# EUROPEAN PATENT SPECIFICATION

(11) **EP 4 313 972 B1**
(45) Date of publication and mention of the grant of the patent: **29.01.2025**
(21) Application number: 22717821.7
(22) Date of filing: 25.03.2022
(51) Int. Cl.: C07D 403/06, C07D 403/14, C07D 471/04, C07D 487/04, A61K 31/519, A61P 11/00

(54) **INDOLINE DERIVATIVES AS DDRS INHIBITORS**
INDOLINDERIVATE ALS DDR HEMMER
DÉRIVÉS D'INDOLINE COMME INHIBITEURS DE DDR

(30) Priority: 26.03.2021 EP 21165281
(43) Date of publication of application: 07.02.2024
(73) Proprietor: Chiesi Farmaceutici S.p.A., 43122 Parma (IT)
(72) Inventor: CARZANIGA, Laura, 43122 Parma (IT); RANCATI, Fabio, 43122 Parma (IT); RIZZI, Andrea, 43122 Parma (IT); IOTTI, Nicolò, 43122 Parma (IT); KNIGHT, Keith Christopher, 43122 Parma (IT); MULLINS, Toby Matthew Grover, 43122 Parma (IT); KARAWAJCZYK, Anna, 43122 Parma (IT); WHITTAKER, Ben Paul, 43122 Parma (IT)
(74) Representative: Chiesi Farmaceutici S.p.A.
(86) International application number: PCT/EP2022/057939
(87) International publication number: WO 2022/200577

(56) References cited:
- ZHU DONGSHENG ET AL: "2-Amino-2,3-dihydro-1 H -indene-5-carboxamide-Based Discoidin Domain Receptor 1 (DDR1) Inhibitors: Design, Synthesis, and in Vivo Antipancreatic Cancer Efficacy", vol. 62, no. 16, 22 August 2019 (2019-08-22), pages 7431 - 7444, XP055828051, ISSN: 0022-2623, Retrieved from the Internet <URL:https://pubs.acs.org/doi/pdf/10.1021/acs.jmedchem.9b00365> [retrieved on 20210727], DOI: 10.1021/acs.jmedchem.9b00365
- GUO JING ET AL: "A patent review of discoidin domain receptor 1 (DDR1) modulators (2014-present)", vol. 30, no. 5, 3 May 2020 (2020-05-03), GB, pages 341 - 350, XP055827937, ISSN: 1354-3776, Retrieved from the Internet <URL:https://www.tandfonline.com/doi/pdf/10.1080/13543776.2020.1732925?needAccess=true> [retrieved on 20210727], DOI: 10.1080/13543776.2020.1732925

## Description

### FIELD OF THE INVENTION

The present invention relates to compounds inhibiting Discoidin Domain Receptors (DDR inhibitors), methods of preparing such compounds, pharmaceutical compositions containing them and therapeutic use thereof.

The compounds of the invention may be useful for instance in the treatment of many disorders associated with DDR mechanisms.

### BACKGROUND OF THE INVENTION

Discoidin Domain Receptors (DDRs) are type I transmembrane receptor tyrosine kinase (RTKs). The DDR family comprises two distinct members, DDR1 and DDR2.

DDRs are unique receptors among the other members of the RTK superfamily, in that DDRs are activated by collagen whereas other members of the RTK superfamily are typically activated by soluble peptide-like growth factors (see Vogel, W. (1997) Mol. Cell 1, 13-23; Shrivastava A. Mol Cell. 1997; 1:25-34.). Moreover, DDRs are unusual RTKs also because they form ligand-independent stable dimers that are non-covalently linked (see Noordeen, N. A. (2006) J. Biol. Chem. 281, 22744-22751; Mihai C. J Mol Biol. 2009; 385:432-445).

The DDR1 subfamily is composed of five membrane-anchored isoforms, and the DDR2 subfamily is represented by a single protein. The five DDR1 isoforms all have in common the extracellular and transmembrane domains but differ in the cytoplasmic region (see Valiathan, R. R. (2012) Cancer Metastasis Rev. 31, 295-321; Alves, F. (2001) FASEB J. 15, 1321-1323).

DDR receptor family has been found involved in a series of fibrotic diseases, such as pulmonary fibrosis, and in particular idiopathic pulmonary fibrosis (IPF). The first evidence for a protective role of DDR1 deletion in lung fibrosis was generated in 2006 by the research group of Dr. Vogel (see Avivi-Green C, Am J Respir Crit Care Med 2006;174:420-427). The authors demonstrated that DDR1-null mice were largely protected against bleomycin (BLM)-induced injury. Furthermore, myofibroblast expansion and apoptosis were much lower in these animals compared with their wild-type counterparts. Absence of inflammation in knockout mice was confirmed by lavage cell count and cytokines ELISA. These results indicated that DDR1 expression is a prerequisite for the development of lung inflammation and fibrosis.

DDR2 deficiency or downregulation reduces bleomycin-induced lung fibrosis (see Zhao H, Bian H, Bu X, Zhang S, Zhang P, Yu J, et al Mol Ther 2016; 24:1734-1744). Zhao et al, demonstrated that DDR2 plays a critical role in the induction of fibrosis and angiogenesis in the lung, in particular that DDR2 synergizes with transforming growth factor (TGF)-β to induce myofibroblast differentiation. Furthermore, they showed that treatment of injured mice with specific siRNA against DDR2 exhibited therapeutic efficacy against lung fibrosis. In a second publication, Jia et al showed that mice lacking DDR2 are protected from bleomycin-induced lung fibrosis (see Jia S, Am J Respir Cell Mol Biol 2018;59:295-305. In addition, DDR2-null fibroblasts are significantly more prone to apoptosis than wild-type fibroblasts, supporting a paradigm in which fibroblast resistance to apoptosis is critical for progression of fibrosis.

Some compounds have been described in the literature as DDR1 or DDR2 antagonists.

WO2016064970 (Guangzhou) discloses isoquinolines derivatives as DDR1 inhibitors useful as therapeutic agents for preventing and treating inflammation, liver fibrosis, kidney fibrosis, lung fibrosis, skin scar, atherosclerosis, and cancer.

Zhu Dongsheng et al, in J. Med. Chem. vol. 62, no. 16, pages 7431-7444, disclose 2-amino-2,3-dihydro-1H-indene-5-carboxamide derivatives as selective DDR1 inhibitors.

Of note, inhibiting the DDR receptors may be useful for the treatment of fibrosis and disease, disorder and conditions that result from fibrosis and even more antagonizing both receptors DDR1 and DDR2 may be particularly efficacious in the treatment of the above-mentioned diseases, disorders and conditions.

Several efforts have been done in the past years to develop novel DDR1 and DDR2 receptor inhibitors useful for the treatment of several diseases and some of those compounds have shown efficacy also in humans.

Despite the above cited prior art, there remains a potential for developing selective inhibitors of both receptors DDR1 and DDR2 useful for the treatment of diseases or conditions associated with a dysregulation of DDR receptors, in the respiratory field, in particular idiopathic pulmonary fibrosis (IPF), to be administered by the inhalation route and characterized by a good inhalatory profile, that corresponds to a good activity in the lung, a good lung retention and to a low metabolic stability in order to minimize the systemic exposure and correlated safety issues.

In this direction, we have surprisingly found a new series of compounds of general formula (I), as herein below reported, that solves the problem of providing inhibitors for receptors DDR1 and DDR2 for administration by inhalation, that are active as selective inhibitors of DDR1 and DDR2 receptors with respect to other human protein kinases. Such compounds show high potency, good inhalatory profile, low metabolic stability, low systemic exposure, improved safety and tolerability.

### SUMMARY OF THE INVENTION

In a first aspect the present invention relates to a compound of formula (I) 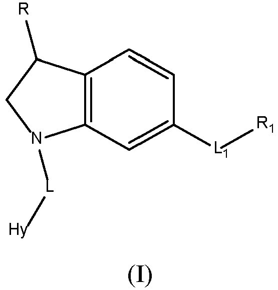 wherein
**Ris** H or -(C₁-C₄)alkyl;
**L** is selected from the group consisting of -CH₂- and -C(O)-;
**Li** is selected from the group consisting of -C(O)NH- and -NHC(O)-;
**Hy** is a bicyclic heteroaryl optionally substituted by one or more groups selected from -(C₁-C₄)alkyl, heterocycloalkyl and -(C₁-C₄)alkylene-NR_{A}R_{B}, or **Hy** is a bicyclic semi saturated heteroaryl;
**R_{A}** is H or -(C₁-C₄)alkyl;
**R_{B}** is H or -(C₁-C₄)alkyl;
**R₁** is selected from the group consisting of:
   - **Het** which is a heteroaryl optionally substituted by one or more groups selected from - (C₁-C₄)alkyl, -(C₁-C₄)haloalkyl, -(C₁-C₄)alkylene-heterocycloalkyl-NR_{A}R_{B}, -(C₁-C₄)alkylene-NR_{A}R_{B} and aryl, wherein said aryl is optionally substituted by one or more -(C₁-C₄)alkyl, and
   - X 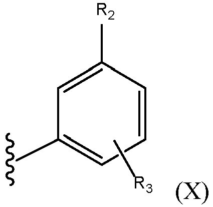 wherein **R₂** is selected from the group consisting of -O(C₁-C₄)haloalkyl, halogen atoms and -(C₁-C₄)haloalkyl;
      **R₃** is H or is selected from the group consisting of -O(C₁-C₄)alkyl, -C(O)NH-(C₁-C₄)alkylene-NR_{A}R_{B}, -C(O)-heterocycloalkyl, wherein said heterocycloalkyl is optionally substituted by one or more -(C₁-C₄)alkyl, -O-heterocycloalkyl, wherein said heterocycloalkyl is optionally substituted by one or more -(C₁-C₄)alkyl, -O(C₁-C₄)alkylene-heterocycloalkyl, -(C₁-C₄)alkylene-NR_{A}R_{B}, heteroaryl optionally substituted by one or more -(C₁-C₄)alkyl, and monocyclic heterocycloalkyl-(C₁-C₄)alkylene-, wherein said heterocycloalkyl is optionally substituted by one or more groups selected from -(C₁-C₄)alkyl and -NR_{A}R_{B};
      and pharmaceutically acceptable salts thereof.

In a second aspect, the invention refers to a pharmaceutical composition comprising a compound of formula (I) and pharmaceutically acceptable salts thereof in a mixture with one or more pharmaceutically acceptable carrier or excipient.

In a third aspect, the invention refers to a compound of formula (I) and pharmaceutically acceptable salts or to a pharmaceutical composition comprising a compound of formula (I) and pharmaceutically acceptable salts thereof for use as a medicament.

In a further aspect, the invention refers to a compound of formula (I) and pharmaceutically acceptable salts thereof or to a pharmaceutical composition comprising a compound of formula (I) and pharmaceutically acceptable salts thereof for use in preventing and/or treating a disease, disorder or condition associated with dysregulation of DDR.

In a further aspect, the invention refers to a compound of formula (I) and pharmaceutically acceptable salts thereof or to a pharmaceutical composition comprising a compound of formula (I) and pharmaceutically acceptable salts thereof for use in the prevention and/or treatment of fibrosis and/or diseases, disorders, or conditions that involve fibrosis.

In a further aspect, the invention refers to a compound of formula (I) and pharmaceutically acceptable salts thereof or to a pharmaceutical composition comprising a compound of formula (I) and pharmaceutically acceptable salts thereof for use in the prevention and/or treatment of idiopathic pulmonary fibrosis (IPF).

### DETAILED DESCRIPTION OF THE INVENTION

### Definitions

Unless otherwise specified, the compound of formula (I) of the present invention is intended to include also its stereoisomers, tautomers or pharmaceutically acceptable salts or solvates thereof.

Unless otherwise specified, the compound of formula (I) of the present invention is intended to include also the compounds of formula (IA), (IA'), (IA"), (IAa), (IAa'), (lAa'S), (IAa"), (IAa"S), (IAb), (IAb'), (IAb"), (IB), (IBa), (IBa'), (IBa'S), (IBa"), (IBa"S), (IBb), (IBb'), (IBb").

The term "pharmaceutically acceptable salts", as used herein, refers to derivatives of compounds of formula (I) wherein the parent compound is suitably modified by converting any of the free acid or basic group, if present, into the corresponding addition salt with any base or acid conventionally intended as being pharmaceutically acceptable.

Suitable examples of said salts may thus include mineral or organic acid addition salts of basic residues such as amino groups, as well as mineral or organic basic addition salts of acid residues such as carboxylic groups.

Cations of inorganic bases which can be suitably used to prepare salts comprise ions of alkali or alkaline earth metals such as potassium, sodium, calcium or magnesium.

Those obtained by reacting the main compound, functioning as a base, with an inorganic or organic acid to form a salt comprise, for example, salts of hydrochloric acid, hydrobromic acid, sulfuric acid, phosphoric acid, methane sulfonic acid, camphor sulfonic acid, acetic acid, oxalic acid, maleic acid, fumaric acid, succinic acid and citric acid.

The term "solvate" means a physical association of a compound of this invention with one or more solvent molecules, whether organic or inorganic. This physical association includes hydrogen bonding. In certain instances, the solvate will be capable of isolation, for example, when one or more solvent molecules are incorporated in the crystal lattice of the crystalline solid. The solvate may comprise either a stoichiometric or nonstoichiometric amount of the solvent molecules.

The term "stereoisomer" refers to isomers of identical constitution that differ in the arrangement of their atoms in space. Enantiomers and diastereomers are examples of stereoisomers.

The term "enantiomer" refers to one of a pair of molecular species that are mirror images of each other and are not superimposable.

The term "diastereomer" refers to stereoisomers that are not mirror images.

The term "racemate" or "racemic mixture" refers to a composition composed of equimolar quantities of two enantiomeric species, wherein the composition is devoid of optical activity.

The term "tautomer" refers to each of two or more isomers of a compound that exist together in equilibrium and are readily interchanged by migration of an atom or group within the molecule.

The term "halogen" or "halogen atoms" or "halo" as used herein includes fluorine, chlorine, bromine, and iodine atom.

The term "(Cₓ-C_{y})alkyl" wherein x and y are integers, refers to a straight or branched chain alkyl group having from x to y carbon atoms. Thus, when x is 1 and y is 4, for example, the term comprises methyl, ethyl, n-propyl, isopropyl, n-butyl, isobutyl, sec-butyl and t-butyl.

The term "O(Cₓ-C_{y})alkyl" wherein x and y are integers, refers to the above defined "(Cₓ-C_{y})alkyl" groups wherein a carbon atom is linked to an oxygen atom, e.g. ethoxy.

The term "(Cₓ-C_{y})haloalkyl" wherein x and y are integers refers to the above defined "(Cₓ-C_{y})alkyl" groups wherein one or more hydrogen atoms are replaced by one or more halogen atoms, which can be the same or different. Examples of said "(Cₓ-C_{y})haloalkyl" groups may include halogenated, poly-halogenated and fully halogenated alkyl groups wherein all hydrogen atoms are replaced by halogen atoms, e.g. trifluoromethyl.

The term "O(Cₓ-C_{y})haloalkyl" wherein x and y are integers, refers to the above defined "(Cₓ-C_{y})haloalkyl" groups wherein the carbon atom is linked to an oxygen atom.

Examples of said "O(Cₓ-C_{y})haloalkyl" groups may thus include halogenated, poly-halogenated and fully halogenated Oalkyl groups wherein all hydrogen atoms are replaced by halogen atoms, e.g. trifluoromethoxy.

The term "aryl" refers to monocyclic carbon ring systems which have 6 ring atoms wherein the ring is aromatic. Examples of suitable aryl monocyclic ring systems include, for instance, phenyl.

The term "heteroaryl" refers to a mono- or bi-cyclic aromatic group containing one or more heteroatoms selected from S, N and O, and includes groups having two such monocyclic rings, or one such monocyclic ring and one monocyclic aryl ring, which are fused through a common bond. Examples of suitable heteroaryl include, for instance, (imidazo[1,2-a]pyridinyl), 1H-pyrrolo[2,3-b]pyridyl, 1H-pyrazolo[3,4-b]pyridinyl, benzo[d]thiazolyl, 1H-indazolyl, 1H-benzo[d]imidazolyl, isoquinolinyl, imidazo[1,2-a]pyrazinyl, 1H-pyrazolo[3,4-b]pyridinyl, 1H-benzo[d]imidazolyl; 1H-pyrrolo[2,3-b]pyridinyl, pyrazolo[1,5-a]pyrimidinyl, pyrazolo[1,5-a]pyrazinyl, pyrazolyl, pyridinyl, isoxazolyl, pyrimidinyl.

The term "semisaturated heteroaryl" refers to a bicyclic group containing one or more heteroatoms selected from S, N and O, and includes monocyclic heteroaryl condensed to one or more monocyclic heterocycloalkyl ring. Examples of suitable semisaturated heteroaryl include, for instance, 2,3-dihydro-1H-pyrrolo[2,3-b]pyridinyl.

The term "heterocycloalkyl" refers to a saturated or partly unsaturated mono- or bi-cyclic ring system of 3 to 12 ring atoms comprising one or more heteroatoms selected from N, S or O, e.g. piperazinyl, morpholinyl, pyrrolidinyl, piperidinyl.

The term "heterocycloalkyl-(Cₓ-C_{y})alkylene" refers to an heterocycloalkyl linked to a straight-chained or branched (Cₓ-C_{y})alkylene group having from x to y carbon atoms, e.g. (4-methylpiperazin-1-yl)methyl, morpholinomethyl, pyrrolidin-1-ylmethyl.

A bond pointing to a wavy or squiggly line, such as 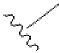 as used in structural formulas herein, depicts the bond that is the point of attachment of the moiety or substituent to the core or backbone structure.

When referring to substituents, a dash ("-") that is not between two letters, words, or symbols is meant to represent the point of attachment for such substituents.

The carbonyl group is herein preferably represented as -C(O)- as an alternative to the other common representations such as -CO-, -(CO)- or -C(=O)-.

When referring to the group -C(O)NH- or -NHC(O)- of **L₁** of Formula (I), the left dash ("-") of the group is the bond between the group and the indoline core, and the right dash ("-") of the group is the bond between the group and the **R₁** group. Accordingly, when referring to -C(O)NH- **of L₁** of Formula (I), the carbon is bonded to the indoline core, and the nitrogen is bonded to the **R₁** group. When referring to -NHC(O)- of **L₁** of Formula (I), the nitrogen is bonded to the indoline core, and the carbon is bonded to the **R₁** group.

Whenever basic amino groups are present in the compounds of formula (I), physiologically acceptable anions may be present, selected among chloride, bromide, iodide, trifluoroacetate, formate, sulfate, phosphate, methanesulfonate, nitrate, maleate, acetate, citrate, fumarate, tartrate, oxalate, succinate, benzoate, p-toluenesulfonate, pamoate and naphthalene disulfonate. Likewise, in the presence of acidic groups, corresponding physiological cation salts may be present as well, for instance including alkaline or alkaline earth metal ions.

The term "half maximal inhibitory concentration" (IC50) indicates the concentration of a particular compound or molecule required for obtaining 50% inhibition of a biological process in vitro.

The term "Ki" indicates the dissociation constant for the enzyme-inhibitor complex, expressed in molar units. It is an indicator of the binding affinity between inhibitor and DDR1 or DDR2 receptors.

The present invention refers to novel compounds differing from the structures disclosed in the art at least for a common new core scaffold. In fact the invention relates to compounds that are indoline derivatives, wherein the nitrogen of the indoline is linked to a bicyclic heteroaryl ring Hy through a linker -CH₂- or -C(O)- represented by the general formula (I) as herein below described in details, which are endowed with an inhibitory activity on receptors DDR1 and DDR2.

As detailed in the experimental part below, the compounds of formula (I) of the present invention are able to act as antagonist of both DDR1 and DDR2 receptors in a substantive and effective way. In particular, Table 7 below shows that for the compounds of the present invention, both the affinity for either DDR1 and DDR2 receptors and the inhibitory activity against either DDR1 and DDR2 receptors are below about 80 nM respectively in the binding (expressed as Ki) and the cell based assays (expressed as IC50). This confirms that the compounds of formula (I) are able to inhibit the two isoforms of DDR receptor mainly involved in fibrosis and diseases resulting from fibrosis. Accordingly, the compounds of formula (I) can be used in the treatment of fibrosis, in particular pulmonary fibrosis, when DDR1 and DDR2 are involved.

Guangzhou discloses tetrahydroisoquinoline-7-carboxamides as selective DDR1 inhibitors.

Of note, in the compounds of formula (I) according to present invention, the presence of at least the indoline moiety and a spacer -CH₂- or -C(O)- between the nitrogen atom of the pyrrolidine and the heteroaryl group Hy unexpectedly and remarkably determines high affinities for both DDR1 and DDR2 receptors and relevant inhibitory activity on the DDR1 and, in addition, also on the DDR2 receptors in the cellular assay.

Moreover, as indicated in the experimental part, comparative examples, in particular in Table 8, it is shown that conversely to the compounds of Examples C1 and C2 characterized by a -CH₂-C(O)- linker between the indoline ring and Hy group, the presence of a -CH₂- or a -C(O)- linker in that position in compounds of the present invention unexpectedly and remarkably determines a relevant increase in the inhibitory activity on both the DDR1 and DDR2 receptors.

In particular, a direct comparison between Example C1 and Examples 14 and 11 of the present invention, differing only for the linker between the indoline group and Hy, highlights that the presence in Examples 14 and 11 of a -C(O)- and a -CH₂- linker respectively, determines an unexpected and noteworthy increase in activity on both the DDR1 and DDR2 receptors. Analogously, a direct comparison between compound of Example C2 and compound of Example 16 of the present invention, differing only for the linker between the indoline group and Hy, highlights that the presence of a -C(O)- linker determines an unexpected and noteworthy increase in activity on both the DDR1 and DDR2 receptors.

Advantageously, the compounds of the present invention are endowed by a very high potency, they could be administered in human at a lower dosage respect to the compounds of the prior art, thus reducing the adverse events that might occur administering higher dosages of drug.

In addition to being notably potent with respect to their inhibitory activity on both receptors DDR1 and DDR2, the compounds of the present invention are also characterized by being selective inhibitors of DDR1 and DDR2 receptors with respect to other human protein kinases, by a good inhalatory profile, that permits to act effectively on the lung compartment and have, at the same time, a low metabolic stability, that allows to minimize the drawbacks associated with the systemic exposure, such as safety and tolerability issues.

Therefore, the compounds of the present invention are particularly appreciated by the skilled person when looking at a suitable and efficacious compounds useful for the treatment of fibrosis, in particular idiopathic pulmonary fibrosis, administered by the inhalation route and characterized by a good inhalatory profile, that corresponds to a good activity on the lung, a good lung retention and to a low metabolic stability, that minimizes the systemic exposure and correlated safety issues.

Thus, in one aspect the present invention relates to a compound of general formula (I) 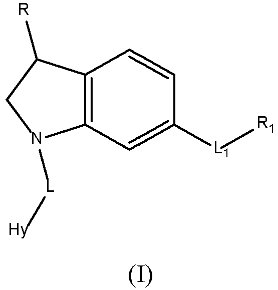 wherein
**R** is H or -(C₁-C₄)alkyl;
**L** is selected from the group consisting of -CH₂- and -C(O)-;
**L₁** is selected from the group consisting of -C(O)NH- and -NHC(O)-;
**Hy** is a bicyclic heteroaryl optionally substituted by one or more groups selected from -
(C₁-C₄)alkyl, heterocycloalkyl and -(C₁-C₄)alkylene-NR_{A}R_{B}, or **Hy** is a bicyclic semi saturated heteroaryl;
**R_{A}** is H or -(C₁-C₄)alkyl;
**R_{B}** is H or -(C₁-C₄)alkyl;
**R₁** is selected from the group consisting of:
   - **Het** which is a heteroaryl optionally substituted by one or more groups selected from - (C₁-C₄)alkyl, -(C₁-C₄)haloalkyl, -(C₁-C₄)alkylene-heterocycloalkyl-NR_{A}R_{B}, -(C₁-C₄)alkylene-NR_{A}R_{B} and aryl, wherein said aryl is optionally substituted by one or more
   - (C₁-C₄)alkyl, and
   - **X** 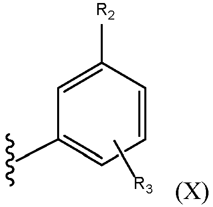 wherein **R₂** is selected from the group consisting of -O(C₁-C₄)haloalkyl, halogen atoms and -(C₁-C₄)haloalkyl;
      **R₃** is H or is selected from the group consisting of -O(C₁-C₄)alkyl, -C(O)NH-(C₁-C₄)alkylene-NR_{A}R_{B}, -C(O)-heterocycloalkyl, wherein said heterocycloalkyl is optionally substituted by one or more -(C₁-C₄)alkyl, -O-heterocycloalkyl, wherein said heterocycloalkyl is optionally substituted by one or more -(C₁-C₄)alkyl, -O(C₁-C₄)alkylene-heterocycloalkyl, -(C₁-C₄)alkylene-NR_{A}R_{B}, heteroaryl optionally substituted by one or more -(C₁-C₄)alkyl, and monocyclic heterocycloalkyl-(C₁-C₄)alkylene-, wherein said heterocycloalkyl is optionally substituted by one or more groups selected from -(C₁-C₄)alkyl and -NR_{A}R_{B};
      and pharmaceutically acceptable salts thereof.

In a preferred embodiment, the present invention relates to a compound of general formula (I) wherein **Hy** is a bicyclic heteroaryl wherein the heteroatom is represented by one or more N atoms.

In another preferred embodiment the present invention refers to a compound of formula (I), wherein **Hy** is a bicyclic heteroaryl optionally substituted by one or more groups selected from methyl, (dimethylamino)methyl and morpholinyl, or **Hy** is a semi saturated heteroaryl.

In another preferred embodiment, the present invention relates to a compound of formula (I), wherein **Hy** is selected from the group consisting of (imidazo[1,2-a]pyridine-3-yl), 1H-pyrrolo[2,3-b]pyridin-5-yl, 1H-pyrazolo[3,4-b]pyridine-5-yl, benzo[d]thiazol-6-yl, 1H-indazol-5-yl, 1H-benzo[d]imidazol-5-yl, isoquinolin-7-yl, imidazo[1,2-a]pyrazin-3-yl, 1H-pyrazolo[3,4-b]pyridin-5-yl, 3-methyl-1H-pyrazolo[3,4-b]pyridin-5-yl, 1H-pyrazolo[3,4-b]pyridin-5-yl, 1H-benzo[d]imidazol-5-yl, 1H-benzo[d]imidazol-5-yl, 3-((dimethylamino)methyl)-1H-pyrrolo[2,3-b]pyridin-5-yl, pyrazolo[1,5-a]pyrimidin-6-yl, 2,3-dihydro-1H-pyrrolo[2,3-b]pyridin-5-yl, 3-morpholino-1H-pyrazolo[3,4-b]pyridin-5-yl, pyrazolo[1,5-a]pyrazin-3-yl and pyrazolo[1,5-a]pyrimidin-6-yl.

In a further preferred embodiment, the present invention relates to a compound of formula (I) wherein **Het** is selected from the group consisting of 3-(tert-butyl)-1-phenyl-1H-pyrazol-5-yl, 2-((3-(dimethylamino)pyrrolidin-1-yl)methyl)-6-(trifluoromethyl)pyridin-4-yl, 5-(trifluoromethyl)pyridin-3-yl, 2-((dimethylamino)methyl)-6-(trifluoromethyl)pyridin-4-yl, ((4-methylpiperazin-1-yl)methyl)-6-(trifluoromethyl)pyridin-4-yl, 3-(tert-butyl)isoxazol-5-yl, 5-(tert-butyl)isoxazol-3-yl, 5-(tert-butyl)-1-methyl-1H-pyrazol-3-yl, 6-(trifluoromethyl)pyrimidin-4-yl and 5-(1,1,1-trifluoro-2-methylpropan-2-yl)isoxazol-3-yl.

In another particularly preferred embodiment, the present invention relates to a compound of formula (I) wherein **X** is selected from the group consisting of 3-((4-methylpiperazin-1-yl)methyl)-5-(trifluoromethyl)phenyl, 3-(4-methyl-1H-imidazol-1-yl)-5-(trifluoromethyl)phenyl, 3-(trifluoromethyl)phenyl, 3-fluoro-5-(trifluoromethyl)phenyl, 4-methoxy-3-(trifluoromethyl)phenyl, 3-(trifluoromethoxy)phenyl, 3-(tert-butyl)-1-phenyl-1H-pyrazol-5-yl, 5-(1,1,1-trifluoro-2-methylpropan-2-yl)isoxazol-3-yl, 3-fluoro-5-(trifluoromethoxy)phenyl, 4-((tetrahydrofuran-3-yl)oxy)-3-(trifluoromethyl)phenyl, 4-((dimethylamino)methyl)-3-(trifluoromethyl)phenyl, 3-(morpholinomethyl)-5-(trifluoromethyl)phenyl, 3-(pyrrolidin-1-ylmethyl)-5-(trifluoromethyl)phenyl, 3-((dimethylamino)methyl)-5-(trifluoromethyl)phenyl, 3-(2-(pyrrolidin-1-yl)ethoxy)-5-(trifluoromethyl)phenyl; 3-(2-morpholinoethoxy)-5-(trifluoromethyl)phenyl, 4-((4-methylpiperazin-1-yl)methyl)-3-(trifluoromethyl)phenyl, 4-((dimethylamino)methyl)-3-(trifluoromethyl)phenyl, 4-(pyrrolidin-1-ylmethyl)-3-(trifluoromethyl)phenyl, 3-((3-(dimethylamino)pyrrolidin-1-yl)methyl)-5-(trifluoromethyl)phenyl, 3-((2-(dimethylamino)ethyl)carbamoyl)-5-(trifluoromethyl)phenyl, 3-(4-methylpiperazine-1-carbonyl)-5-(trifluoromethyl)phenyl and 4-((1-methylpiperidin-4-yl)oxy)-3-(trifluoromethyl)phenyl.

In a particularly preferred embodiment, the present invention relates to a compound of general formula (I) wherein **L₄** is -C(O)NH- represented by formula (IA) 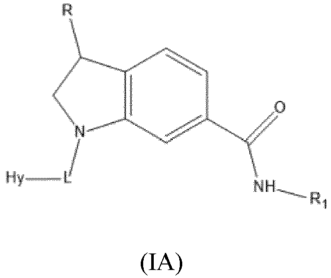 wherein **R, L,** Hy and **R₁** are as defined above.

In another preferred embodiment the present invention refers to a compound of formula (IA) wherein L is -C(O)-, represented by formula (IA') 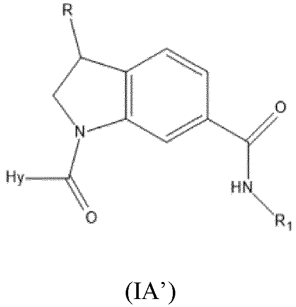 wherein **R, Hy** and **R₁** are as defined above.

In a even more preferred embodiment the present invention refers to a compound of formula (IA'), wherein **R₁** is selected from the group consisting of 3-((4-methylpiperazin-1-yl)methyl)-5-(trifluoromethyl)phenyl and 3-(4-methyl-1H-imidazol-1-yl)-5-(trifluoromethyl)phenyl and **Hy** is selected from the group consisting of imidazo[1,2-a]pyridine-3-yl, imidazo[1,2-a]pyrazine-3-yl and 1H-pyrazolo[3,4-b]pyridine-5-yl.

According to a preferred embodiment, the invention refers to at least one of the compounds of Formula (IA') listed in the Table 1 below and pharmaceutically acceptable salts thereof. These compounds are particularly active on receptors DDR1 and DDR2, as shown in Table 7.

**Table 1: List of preferred compounds of Formula (IA')**

| **Example No.** | **Structure** | **Chemical Name** |
|---|---|---|
| 12 | 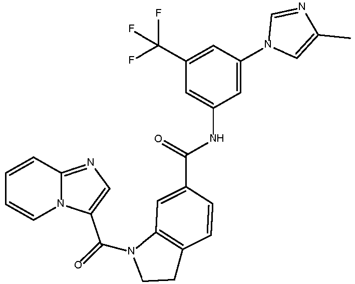 | 1-(imidazo[1,2-a]pyridine-3-carbonyl)-N-(3-(4-methyl-1H-imidazol-1-yl)-5-(trifluoromethyl)phenyl)ind oline-6-carboxamide |
| 13 | 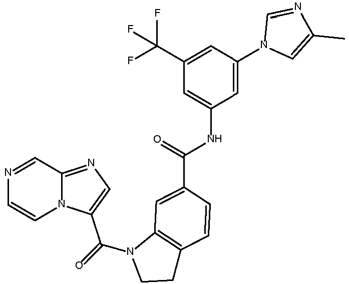 | 1-(imidazo[1,2-a]pyrazine-3-carbonyl)-N-(3-(4-methyl-1H-imidazol-1-yl)-5-(trifluoromethyl)phenyl)ind oline-6-carboxamide |
| 14 | 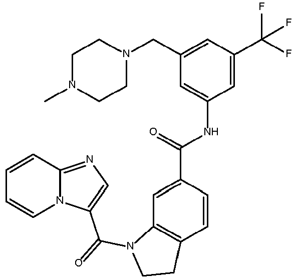 | 1-(imidazo[1,2-a]pyridine-3-carbonyl)-N-(3-((4-methylpiperazin-1-yl)methyl)-5-(trifluoromethyl)phenyl)ind oline-6-carboxamide |
| 15 | 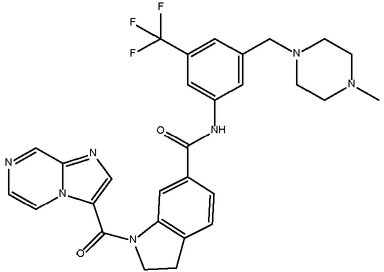 | 1-(imidazo[1,2-a]pyrazine-3-carbonyl)-N-(3-((4-methylpiperazin-1-yl)methyl)-5-(trifluoromethyl)phenyl)ind oline-6-carboxamide |
| 28 | 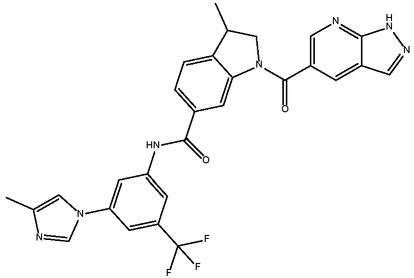 | 3-methyl-N-(3-(4-methyl-1H-imidazol-1-yl)-5-(trifluoromethyl)phenyl)-1 - (1H-pyrazolo[3,4-b]pyridine-5-carbonyl)indoline-6-carboxamide |
| 29 | 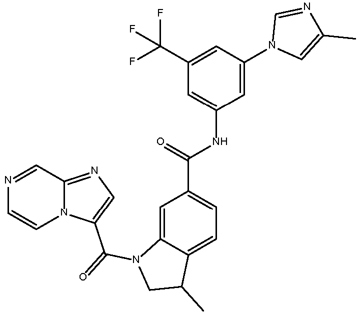 | 1-(imidazo[1,2-a]pyrazine-3-carbonyl)-3-methyl-N-(3-(4-methyl-1H-imidazol-1 - yl)-5-(trifluoromethyl)phenyl)ind oline-6-carboxamide |

In another preferred embodiment the present invention refers to a compound of formula (IA) wherein L is -CH₂-, represented by formula (IA") 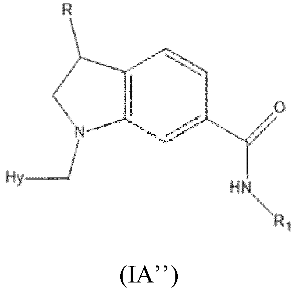 wherein **R**, **Hy** and **R₁** are as defined above.

In a even more preferred embodiment the present invention refers to a compound of formula (IA"), wherein **R₁** is selected from the group consisting of 3-((4-methylpiperazin-1-yl)methyl)-5-(trifluoromethyl)phenyl, 3-(4-methyl-1H-imidazol-1-yl)-5-(trifluoromethyl)phenyl), 3-(trifluoromethyl)phenyl, 3-fluoro-5-(trifluoromethyl)phenyl, 4-methoxy-3-(trifluoromethyl)phenyl, 3-(trifluoromethoxy)phenyl; 3-(tert-butyl)-1-phenyl-1H-pyrazol-5-yl, 5-(1,1,1-trifluoro-2-methylpropan-2-yl)isoxazol-3-yl, 3-fluoro-5-(trifluoromethoxy)phenyl, 4-((tetrahydrofuran-3-yl)oxy)-3-(trifluoromethyl)phenyl, 4-((dimethylamino)methyl)-3-(trifluoromethyl)phenyl, 3-(morpholinomethyl)-5-(trifluoromethyl)phenyl, 3-(pyrrolidin-1-ylmethyl)-5-(trifluoromethyl)phenyl, 3-((dimethylamino)methyl)-5-(trifluoromethyl)phenyl, 3-(2-(pyrrolidin-1-yl)ethoxy)-5-(trifluoromethyl)phenyl; 3-(2-morpholinoethoxy)-5-(trifluoromethyl)phenyl, 4-((4-methylpiperazin-1-yl)methyl)-3-(trifluoromethyl)phenyl, 4-((dimethylamino)methyl)-3-(trifluoromethyl)phenyl, 4-(pyrrolidin-1-ylmethyl)-3-(trifluoromethyl)phenyl, 3-((3-(dimethylamino)pyrrolidin-1-yl)methyl)-5-(trifluoromethyl)phenyl, 3-((2-(dimethylamino)ethyl)carbamoyl)-5-(trifluoromethyl)phenyl, 3-(4-methylpiperazine-1-carbonyl)-5-(trifluoromethyl)phenyl, 2-((3-(dimethylamino)pyrrolidin-1-yl)methyl)-6-(trifluoromethyl)pyridin-4-yl, 5-(trifluoromethyl)pyridin-3-yl, 2-((dimethylamino)methyl)-6-(trifluoromethyl)pyridin-4-yl, 4-methylpiperazin-1-yl)methyl)-6-(trifluoromethyl)pyridin-4-yl, 3-(tert-butyl)isoxazol-5-yl, 5-(tert-butyl)isoxazol-3-yl, 5-(tert-butyl)-1-methyl-1H-pyrazol-3-yl, 6-(trifluoromethyl)pyrimidin-4-yl and 4-((1-methylpiperidin-4-yl)oxy)-3-(trifluoromethyl)phenyl and **Hy** is selected from the group consisting of imidazo[1,2-a]pyridin-3-yl, 1H-pyrrolo[2,3-b]pyridine, 1H-indazol-5-yl, 1H-benzo[d]imidazol-5-yl, isoquinolin-7-yl, imidazo[1,2-a]pyrazin-3-yl, 1H-pyrazolo[3,4-b]pyridin-5-yl, 3-methyl-1H-pyrazolo[3,4-b]pyridin-5-yl, 3-((dimethylamino)methyl)-1H-pyrrolo[2,3-b]pyridin-5-yl, pyrazolo[1,5-a]pyrimidin-6-yl, 2,3-dihydro-1H-pyrrolo[2,3-b]pyridin-5-yl, 3-morpholino-1H-pyrazolo[3,4-b]pyridin-5-yl, pyrazolo[1,5-a]pyrazin-3-yl and 1H-benzo[d]imidazol-5-yl.

According to a preferred embodiment, the invention refers to at least one of the compounds of Formula (IA") listed in the Table 2 below and pharmaceutically acceptable salts thereof. These compounds are particularly active on receptors DDR1 and DDR2, as shown in Table 7.

**Table 2: List of preferred compounds of Formula (IA")**

| **Example No.** | **Structure** | **Chemical Name** |
|---|---|---|
| 1 | 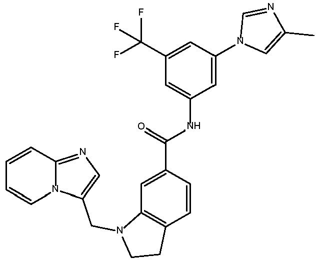 | 1-(imidazo[1,2-a]pyridin-3 -ylmethyl)-N-(3-(4-methyl-1H-imidazol-1-yl)-5-(trifluoromethyl)phenyl )indoline-6-carboxamide |
| 2 | 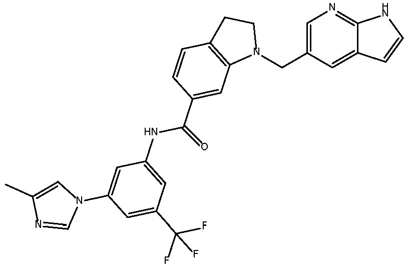 | 1-((1H-pyrrolo[2,3-b]pyridin-5-yl)methyl)-N-(3-(4-methyl-1H-imidazol-1-yl)-5-(trifluoromethyl)phenyl )indoline-6-carboxamide |
| 3 | 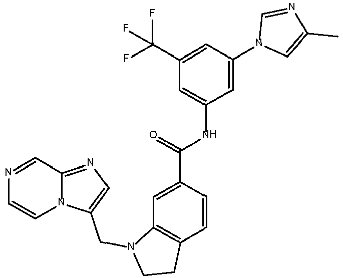 | 1-(imidazo[1,2-a]pyrazin-3 -ylmethyl)-N-(3-(4-methyl-1H-imidazol-1-yl)-5-(trifluoromethyl)phenyl )indoline-6-carboxamide |
| 4 | 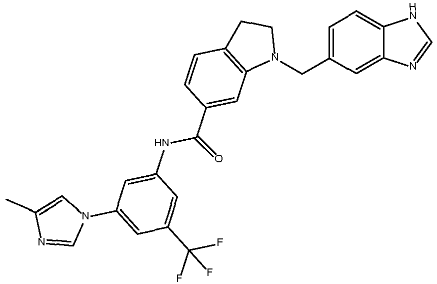 | 1-((1H-benzo[d]imidazol-5-yl)methyl)-N-(3-(4-methyl-1H-imidazol-1-yl)-5-(trifluoromethyl)phenyl )indoline-6-carboxamide |
| 5 | 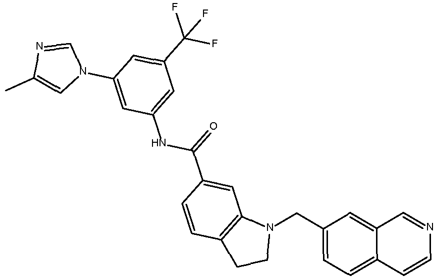 | 1-(isoquinolin-7-ylmethyl)-N-(3-(4-methyl-1H-imidazol-1-yl)-5-(trifluoromethyl)phenyl )indoline-6-carboxamide |
| 6 | 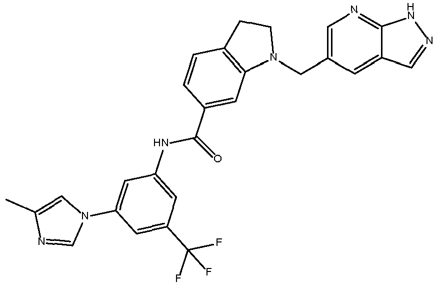 | 1-((1H-pyrazolo[3,4-b]pyridin-5-yl)methyl)-N-(3-(4-methyl-1H-imidazol-1-yl)-5-(trifluoromethyl)phenyl )indoline-6-carboxamide |
| 7 | 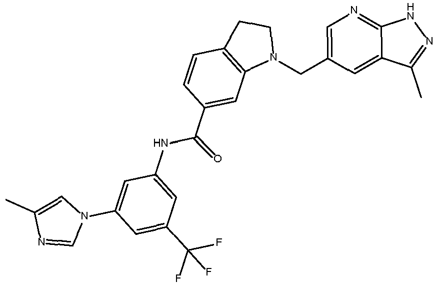 | N-(3-(4-methyl-1H-imidazol-1-yl)-5-(trifluoromethyl)phenyl )-1-((3-methyl-1H-pyrazolo[3,4-b]pyridin-5-yl)methyl)indoline-6-carboxamide |
| 8 | 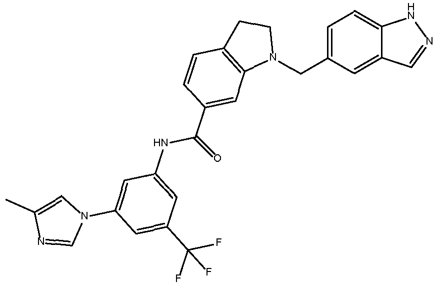 | 1-((1H-indazol-5-yl)methyl)-N-(3-(4-methyl-1H-imidazol-1-yl)-5-(trifluoromethyl)phenyl )indoline-6-carboxamide |
| 9 | 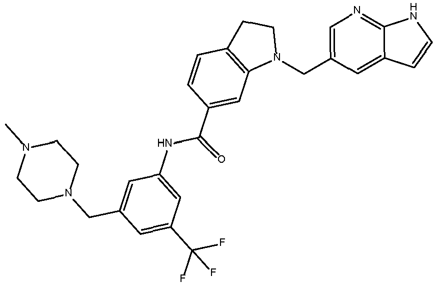 | 1-((1H-pyrrolo[2,3-b]pyridin-5-yl)methyl)-N-(3-((4-methylpiperazin-1-yl)methyl)-5-(trifluoromethyl)phenyl )indoline-6-carboxamide |
| 10 | 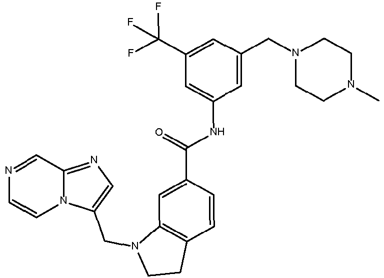 | 1-(imidazo[1,2-a]pyrazin-3 -ylmethyl)-N-(3-((4-methylpiperazin-1- yl)methyl)-5-(trifluoromethyl)phenyl )indoline-6-carboxamide |
| 11 | 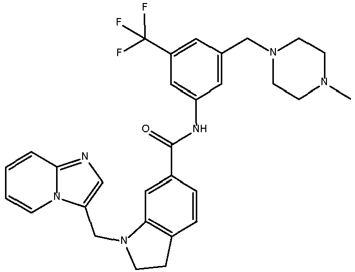 | 1-(imidazo[1,2-a]pyridin-3 -ylmethyl)-N-(3-((4-methylpiperazin-1- yl)methyl)-5-(trifluoromethyl)phenyl )indoline-6-carboxamide |
| 24 | 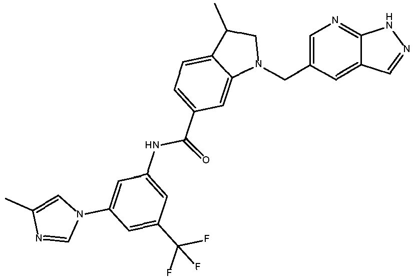 | 1-((1H-pyrazolo[3,4-b]pyridin-5-yl)methyl)-3-methyl-N-(3-(4-methyl-1H-imidazol-1-yl)-5-(trifluoromethyl)phenyl )indoline-6-carboxamide |
| 25 | 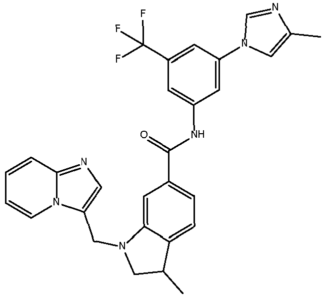 | 1-(imidazo[1,2-a]pyridin-3 -ylmethyl)-3-methyl-N-(3-(4-methyl-1H-imidazol-1-yl)-5-(trifluoromethyl)phenyl )indoline-6-carboxamide |
| 26 racemate | 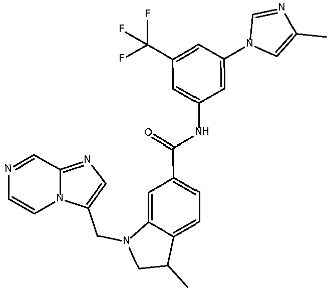 | 1-(imidazo[1,2-a]pyrazin-3 -ylmethyl)-3-methyl-N-(3-(4-methyl-1H-imidazol-1-yl)-5-(trifluoromethyl)phenyl )indoline-6-carboxamide |
| 27 single enantiom er | 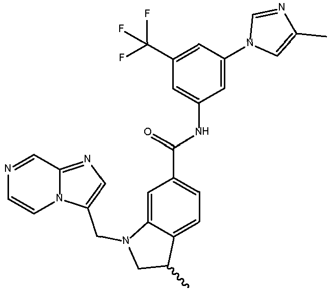 | 1-(imidazo[1,2-a]pyrazin-3 -ylmethyl)-3-methyl-N-(3-(4-methyl-1H-imidazol-1-yl)-5-(trifluoromethyl)phenyl )indoline-6-carboxamide |
| 30 racemate | 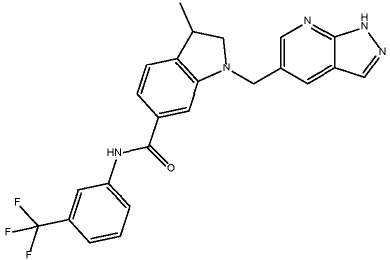 | 1-((1H-pyrazolo[3,4-b]pyridin-5-yl)methyl)-3-methyl-N-(3-(trifluoromethyl)phenyl )indoline-6-carboxamide |
| 31 | 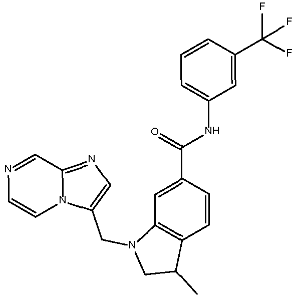 | 1-(imidazo[1,2-a]pyrazin-3 -ylmethyl)-3-methyl-N-(3-(trifluoromethyl)phenyl )indoline-6-carboxamide |
| 32 | 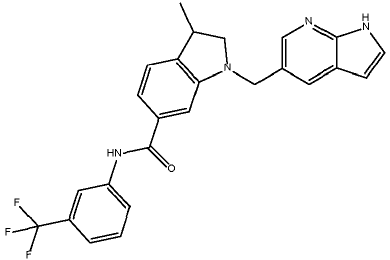 | 1-((1H-pyrrolo[2,3-b]pyridin-5-yl)methyl)-3-methyl-N-(3-(trifluoromethyl)phenyl )indoline-6-carboxamide |
| 33 | 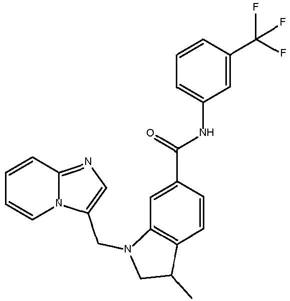 | 1-(imidazo[1,2-a]pyridin-3 -ylmethyl)-3-methyl-N-(3-(trifluoromethyl)phenyl )indoline-6-carboxamide |
| 34 single enantiom er | 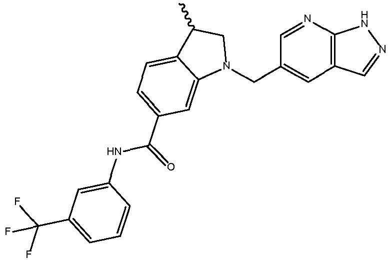 | 1-((1H-pyrazolo[3,4-b]pyridin-5-yl)methyl)-3-methyl-N-(3-(trifluoromethyl)phenyl )indoline-6-carboxamide |
| 36 | 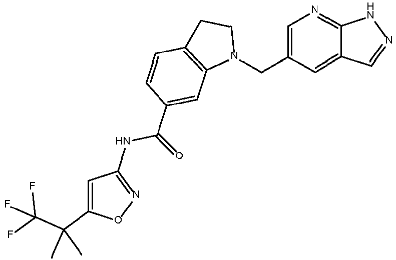 | 1-((1H-pyrazolo[3,4-b]pyridin-5-yl)methyl)-N-(5-(1,1,1-trifluoro-2-methylpropan-2-yl)isoxazol-3-yl)indoline-6-carboxamide |
| 37 | 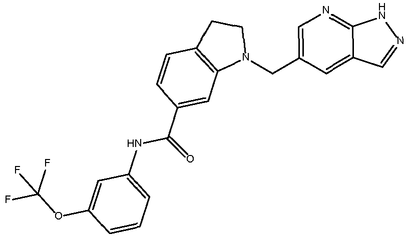 | 1-((1H-pyrazolo[3,4-b]pyridin-5-yl)methyl)-N-(3-(trifluoromethoxy)phen yl)indoline-6-carboxamide |
| 39 | 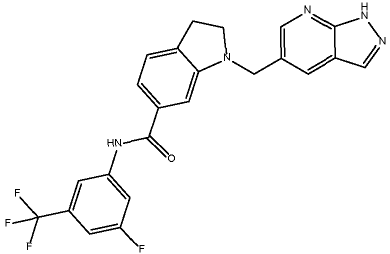 | 1-((1H-pyrazolo[3,4-b]pyridin-5-yl)methyl)-N-(3-fluoro-5-(trifluoromethyl)phenyl )indoline-6-carboxamide |
| 40 | 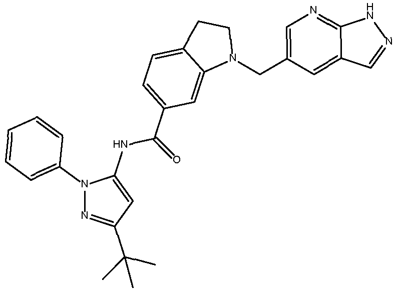 | 1-((1H-pyrazolo[3,4-b]pyridin-5-yl)methyl)-N-(3-(tert-butyl)-1-phenyl-1H-pyrazol-5-yl)indoline-6-carboxamide |
| 41 | 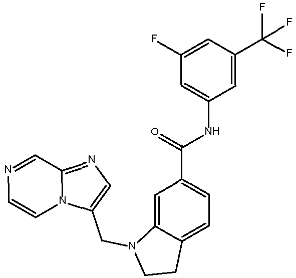 | N-(3-fluoro-5-(trifluoromethyl)phenyl )-1-(imidazo[1,2-a]pyrazin-3-ylmethyl)indoline-6-carboxamide |
| 42 | 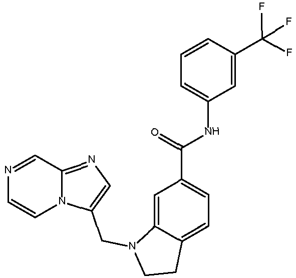 | 1-(imidazo[1,2-a]pyrazin-3 -ylmethyl)-N-(3-(trifluoromethyl)phenyl )indoline-6-carboxamide |
| 43 | 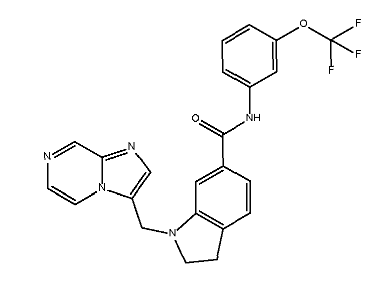 | 1-(imidazo[1,2-a]pyrazin-3 -ylmethyl)-N-(3-(trifluoromethoxy)phen yl)indoline-6-carboxamide |
| 44 | 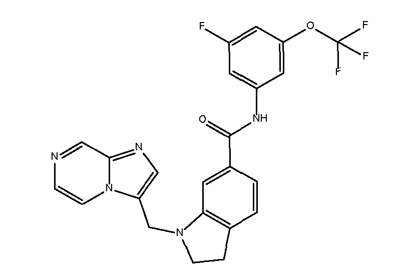 | N-(3-fluoro-5-(trifluoromethoxy)phen yl)-1-(imidazo[1,2-a]pyrazin-3-ylmethyl)indoline-6-carboxamide |
| 45 | 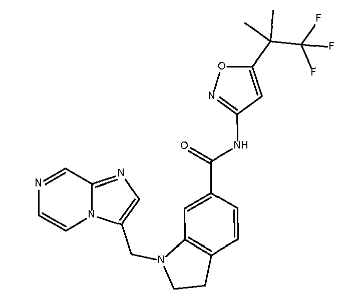 | 1-(imidazo[1,2-a]pyrazin-3 -ylmethyl)-N-(5-(1,1,1-trifluoro-2-methylpropan-2-yl)isoxazol-3-yl)indoline-6-carboxamide |
| 46 | 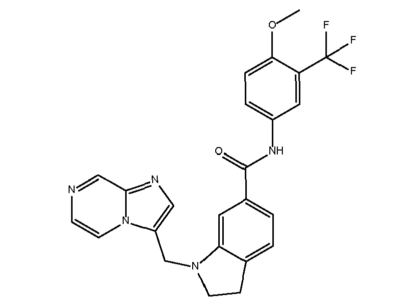 | 1-(imidazo[1,2-a]pyrazin-3 -ylmethyl)-N-(4-methoxy-3-(trifluoromethyl)phenyl )indoline-6-carboxamide |
| 47 | 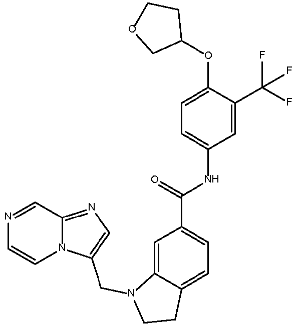 | 1-(imidazo[1,2-a]pyrazin-3 -ylmethyl)-N-(4-((tetrahydrofuran-3-yl)oxy)-3-(trifluoromethyl)phenyl )indoline-6-carboxamide |
| 48 | 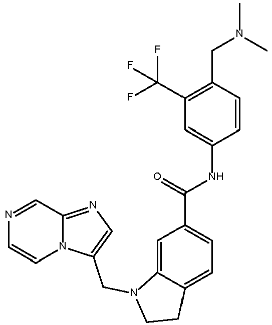 | N-(4-((dimethylamino)methy l)-3-(trifluoromethyl)phenyl )-1-(imidazo[1,2-a]pyrazin-3-ylmethyl)indoline-6-carboxamide |
| 49 | 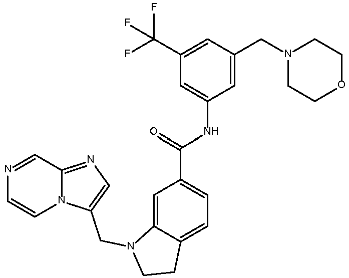 | 1-(imidazo[1,2-a]pyrazin-3 -ylmethyl)-N-(3-(morpholinomethyl)-5-(trifluoromethyl)phenyl )indoline-6-carboxamide |
| 50 | 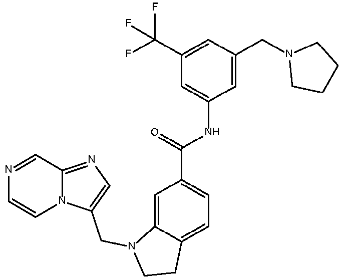 | 1-(imidazo[1,2-a]pyrazin-3 -ylmethyl)-N-(3-(pyrrolidin-1-ylmethyl)-5-(trifluoromethyl)phenyl )indoline-6-carboxamide |
| 51 | 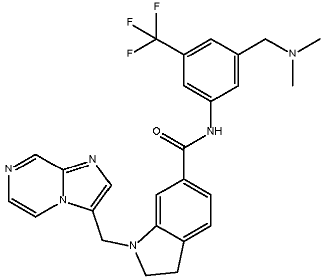 | N-(3-((dimethylamino)methy l)-5-(trifluoromethyl)phenyl )-1-(imidazo[1,2-a]pyrazin-3-ylmethyl)indoline-6-carboxamide |
| 52 | 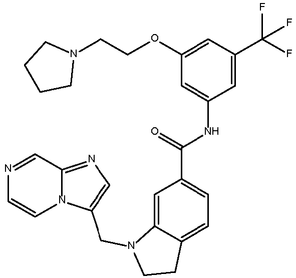 | 1-(imidazo[1,2-a]pyrazin-3 -ylmethyl)-N-(3-(2-(pyrrolidin-1-yl)ethoxy)-5-(trifluoromethyl)phenyl )indoline-6-carboxamide |
| 53 | 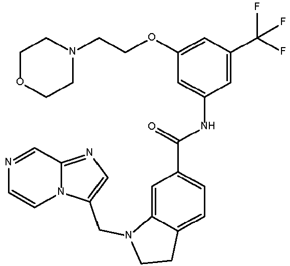 | 1-(imidazo[1,2-a]pyrazin-3 -ylmethyl)-N-(3-(2-morpholinoethoxy)-5-(trifluoromethyl)phenyl )indoline-6-carboxamide |
| 54 | 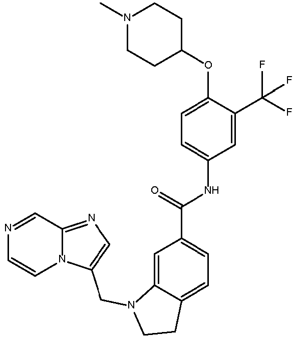 | 1-(imidazo[1,2-a]pyrazin-3 -ylmethyl)-N-(4-((1-methylpiperidin-4-yl)oxy)-3-(trifluoromethyl)phenyl )indoline-6-carboxamide |
| 55 | 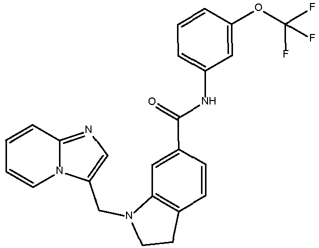 | 1-(imidazo[1,2-a]pyridin-3 -ylmethyl)-N-(3-(trifluoromethoxy)phen yl)indoline-6-carboxamide |
| 56 | 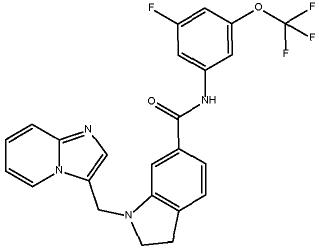 | N-(3-fluoro-5-(trifluoromethoxy)phen yl)-1-(imidazo[1,2-a]pyridin-3-ylmethyl)indoline-6-carboxamide |
| 57 | 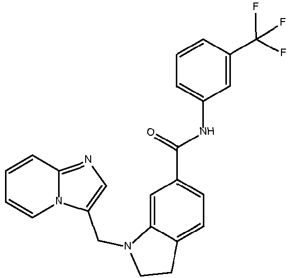 | 1-(imidazo[1,2-a]pyridin-3 -ylmethyl)-N-(3-(trifluoromethyl)phenyl )indoline-6-carboxamide |
| 58 | 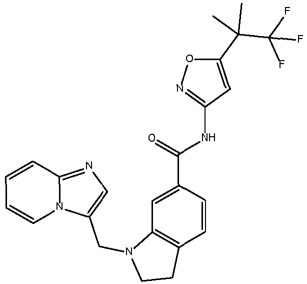 | 1-(imidazo[1,2-a]pyridin-3-ylmethyl)-N-(5-(1,1,1-trifluoro-2-methylpropan-2-yl)isoxazol-3-yl)indoline-6-carboxamide |
| 59 | 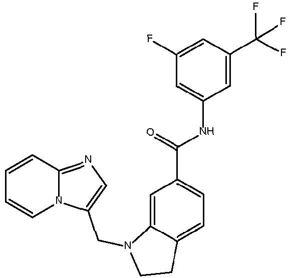 | N-(3-fluoro-5-(trifluoromethyl)phenyl )-1-(imidazo[1,2-a]pyridin-3-ylmethyl)indoline-6-carboxamide |
| 60 | 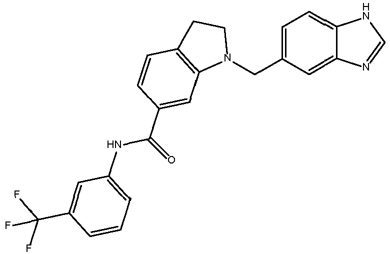 | 1-((1H-benzo[d]imidazol-5-yl)methyl)-N-(3-(trifluoromethyl)phenyl )indoline-6-carboxamide |
| 61 | 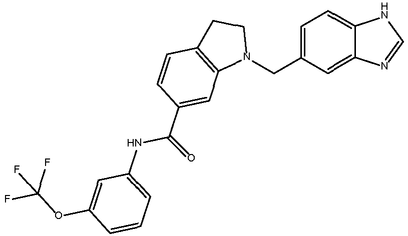 | 1-((1H-benzo[d]imidazol-5-yl)methyl)-N-(3-(trifluoromethoxy)phen yl)indoline-6-carboxamide |
| 62 | 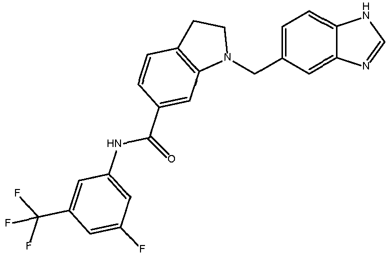 | 1-((1H-benzo[d]imidazol-5-yl)methyl)-N-(3-fluoro-5-(trifluoromethyl)phenyl )indoline-6-carboxamide |
| 63 | 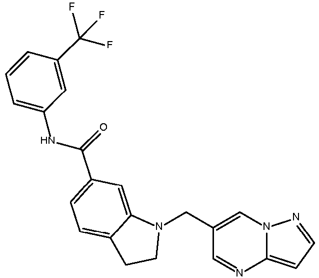 | 1-(pyrazolo[1,5-a]pyrimidin-6-ylmethyl)-N-(3-(trifluoromethyl)phenyl )indoline-6-carboxamide |
| 64 | 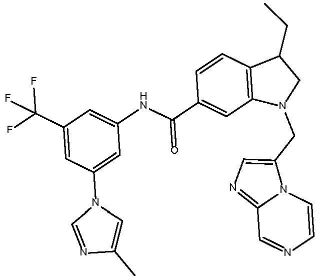 | 3-ethyl-1-(imidazo[1,2-a]pyrazin-3 -ylmethyl)-N-(3-(4-methyl-1H-imidazol-1-yl)-5-(trifluoromethyl)phenyl )indoline-6-carboxamide |
| 65 | 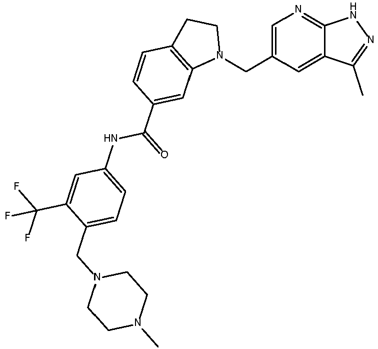 | 1-((3-methyl-1H-pyrazolo[3,4-b]pyridin-5-yl)methyl)-N-(4-((4-methylpiperazin-1-yl)methyl)-3-(trifluoromethyl)phenyl )indoline-6-carboxamide |
| 66 | 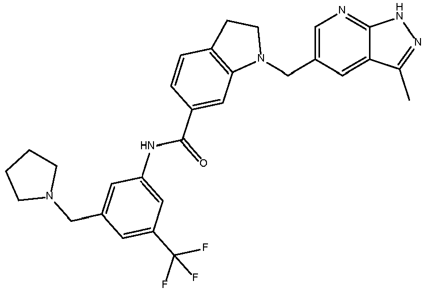 | 1-((3-methyl-1H-pyrazolo[3,4-b]pyridin-5-yl)methyl)-N-(3-(pyrrolidin-1 -ylmethyl)-5-(trifluoromethyl)phenyl )indoline-6-carboxamide |
| 68 | 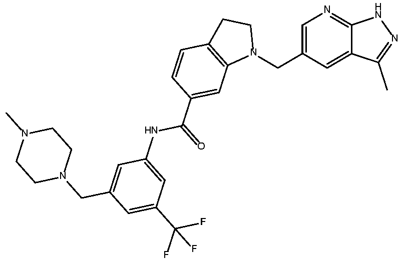 | 1-((3-methyl-1H-pyrazolo[3,4-b]pyridin-5-yl)methyl)-N-(3-((4-methylpiperazin-1-yl)methyl)-5-(trifluoromethyl)phenyl )indoline-6-carboxamide |
| 69 | 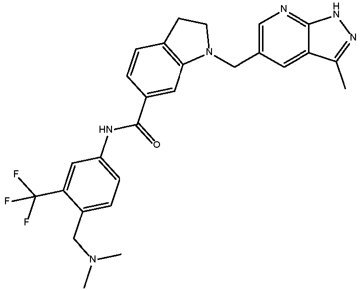 | N-(4-((dimethylamino)methy l)-3-(trifluoromethyl)phenyl )-1-((3-methyl-1H-pyrazolo[3,4-b]pyridin-5-yl)methyl)indoline-6-carboxamide |
| 70 | 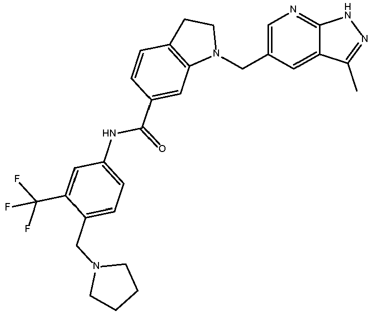 | 1-((3-methyl-1H-pyrazolo[3,4-b]pyridin-5-yl)methyl)-N-(4-(pyrrolidin-1 -ylmethyl)-3-(trifluoromethyl)phenyl )indoline-6-carboxamide |
| 72 | 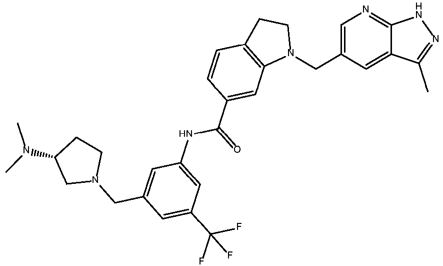 | (R)-N-(3-((3-(dimethylamino)pyrroli din-1-yl)methyl)-5-(trifluoromethyl)phenyl )-1-((3-methyl-1H-pyrazolo[3,4-b]pyridin-5-yl)methyl)indoline-6-carboxamide |
| 73 | 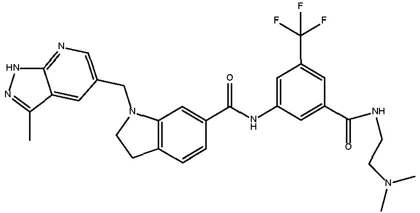 | N-(3-((2-(dimethylamino)ethyl)c arbamoyl)-5-(trifluoromethyl)phenyl )-1-((3-methyl-1H-pyrazolo[3,4-b]pyridin-5-yl)methyl)indoline-6-carboxamide |
| 74 | 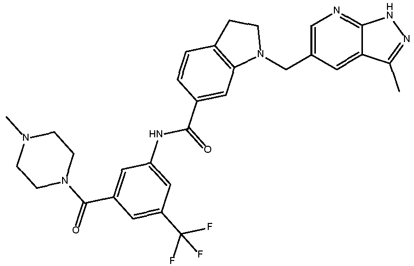 | 1-((3-methyl-1H-pyrazolo[3,4-b]pyridin-5-yl)methyl)-N-(3-(4-methylpiperazine-1-carbonyl)-5-(trifluoromethyl)phenyl )indoline-6-carboxamide |
| 75 | 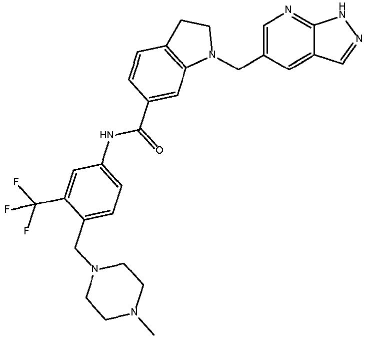 | 1-((1H-pyrazolo[3,4-b]pyridin-5-yl)methyl)-N-(4-((4-methylpiperazin-1- yl)methyl)-3-(trifluoromethyl)phenyl )indoline-6-carboxamide |
| 76 | 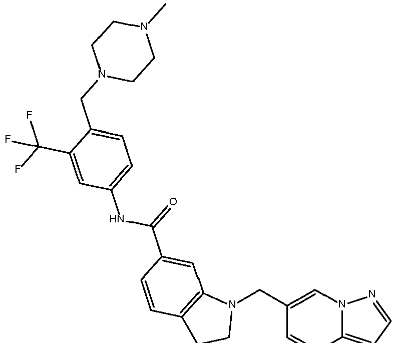 | N-(4-((4-methylpiperazin-1-yl)methyl)-3-(trifluoromethyl)phenyl )-1-(pyrazolo[1,5-a]pyrimidin-6-ylmethyl)indoline-6-carboxamide |
| 77 | 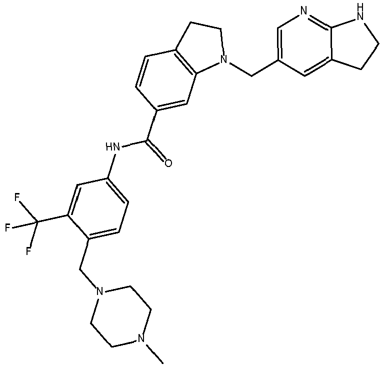 | 1-((2,3-dihydro-1H-pyrrolo[2,3 -b]pyridin-5-yl)methyl)-N-(4-((4-methylpiperazin-1 - yl)methyl)-3-(trifluoromethyl)phenyl )indoline-6-carboxamide |
| 85 | 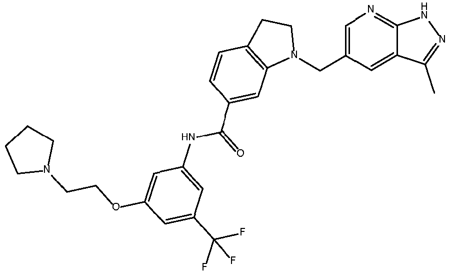 | 1-((3-methyl-1H-pyrazolo[3,4-b]pyridin-5-yl)methyl)-N-(3-(2-(pyrrolidin-1-yl)ethoxy)-5-(trifluoromethyl)phenyl )indoline-6-carboxamide |
| 86 | 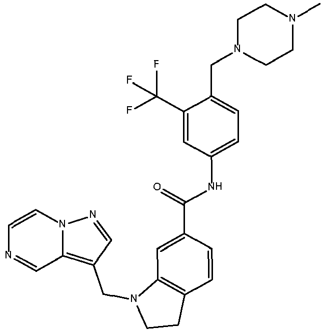 | N-(4-((4-methylpiperazin-1-yl)methyl)-3-(trifluoromethyl)phenyl )-1-(pyrazolo[1,5-a]pyrazin-3-ylmethyl)indoline-6-carboxamide |
| 89 | 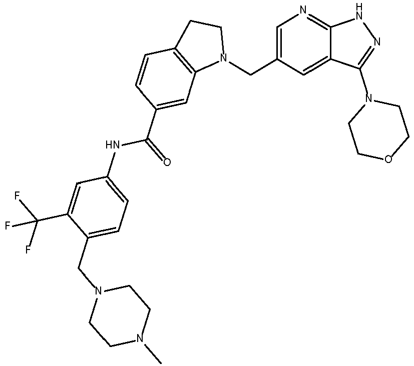 | N-(4-((4-methylpiperazin-1-yl)methyl)-3-(trifluoromethyl)phenyl )-1-((3-morpholino-1H-pyrazolo[3,4-b]pyridin-5-yl)methyl)indoline-6-carboxamide |
| 90 | 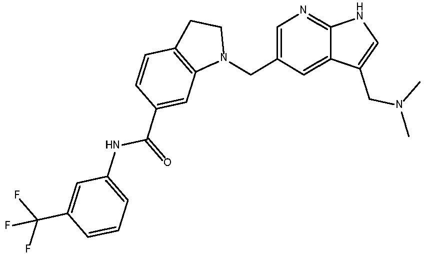 | 1-((3-((dimethylamino)methy l)-1H-pyrrolo[2,3-b]pyridin-5-yl)methyl)-N-(3-(trifluoromethyl)phenyl )indoline-6-carboxamide |

In a particularly preferred embodiment, the present invention relates to a compound of general formula (IA) wherein **R₁** is group X 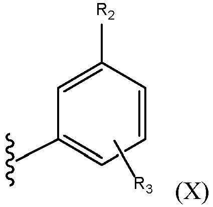 represented by formula (IAa) 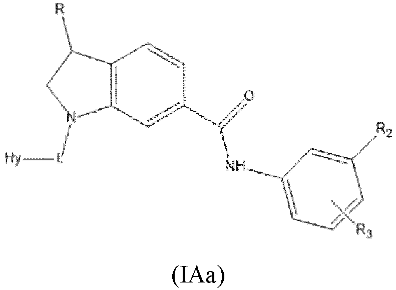 wherein **R, L, Hy, R₂** and **R₃** are as defined above.

In a particularly preferred embodiment the present invention refers to a compound of formula (IAa) wherein L is -C(O)-, represented by formula (IAa') 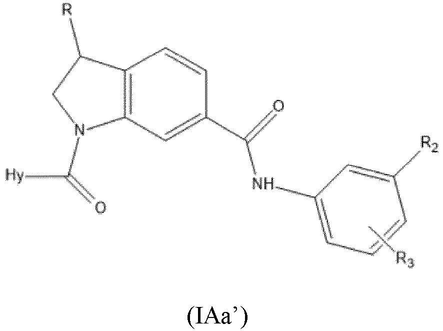 wherein **R, Hy, R₂** and **R₃** are as defined above.

In another particularly preferred embodiment, the present invention relates to a compound of general formula (I) wherein L is -C(O)-, **Li** is-C(O)NH-, **R₁** is X' 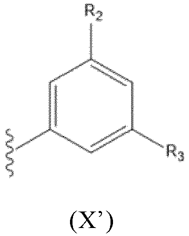 represented by formula (IAa'S) 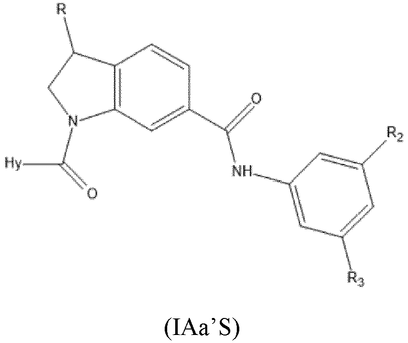 wherein **R, Hy, R₂** and **R₃** are as defined above.

In a further particularly preferred embodiment, the present invention relates to a compound of general formula (IAa) wherein L is -CH₂-, represented by formula (IAa") 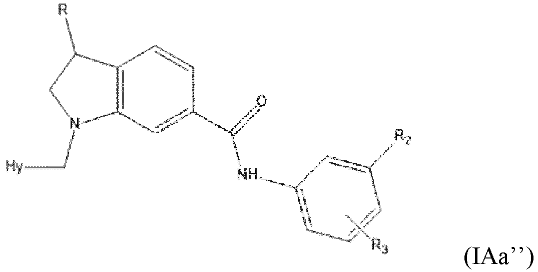 wherein **R, Hy, Ri, R₂** and **R₃** are as defined above.

In a further preferred embodiment, the present invention relates to a compound of general formula (I) wherein **L** is -CH₂-, Li is-C(O)NH-, **R₁** is X' 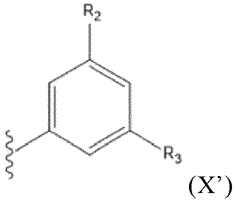 represented by formula (IAa"S) 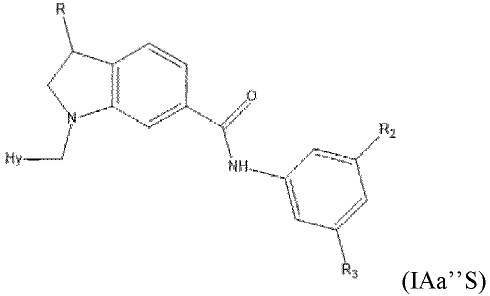 wherein **R, Hy, R₂** and **R₃** are as defined above.

In another particularly preferred embodiment, the present invention relates to a compound of general formula (I) wherein **L₁** is -C(O)NH-, **R₁** is **Het,** represented by formula (IAb) 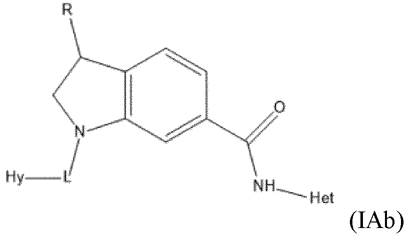 wherein **R, L, Hy** and **Het** are as defined above.

In an even more preferred embodiment, the present invention relates to a compound of general formula (IAb) wherein **L** is -C(O)-, represented by formula (IAb') 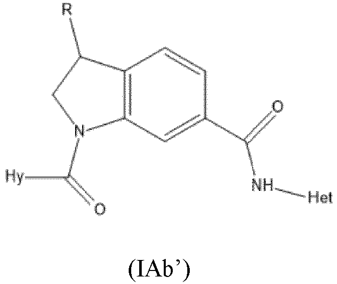 wherein **R, Hy** and **Het** are as defined above.

In another particularly preferred embodiment, the present invention relates to a compound of general formula (IAb) wherein **L** is -CH₂-, represented by formula (IAb") 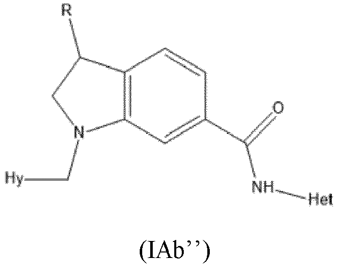 wherein **R, Hy** and **Het** are as defined above.

In a even more preferred embodiment the present invention refers to a compound of formula (IAb"), wherein **Het** is selected from the group consisting of 3-(tert-butyl)-1-phenyl-1H-pyrazol-5-yl, 2-((3-(dimethylamino)pyrrolidin-1-yl)methyl)-6-(trifluoromethyl)pyridin-4-yl, 5-(trifluoromethyl)pyridin-3-yl, 2-((dimethylamino)methyl)-6-(trifluoromethyl)pyridin-4-yl, 4-methylpiperazin-1-yl)methyl)-6-(trifluoromethyl)pyridin-4-yl, 3-(tert-butyl)isoxazol-5-yl, 5-(tert-butyl)isoxazol-3-yl, 5-(tert-butyl)-1-methyl-1H-pyrazol-3-yl, 6-(trifluoromethyl)pyrimidin-4-yl and 5-(1,1,1-trifluoro-2-methylpropan-2-yl)isoxazol-3-yl).

In another preferred embodiment the present invention refers to a compound of formula (IAb"), wherein **Hy** is selected from the group consisting of 1H-pyrazolo[3,4-b]pyridin-5-yl, imidazo[1,2-a]pyridin-3-yl, 3-methyl-1H-pyrazolo[3,4-b]pyridin-5-yl, 3-((dimethylamino)methyl)-1H-pyrrolo[2,3-b]pyridin-5-yl, and imidazo[1,2-a]pyrazin-3-yl.

According to a preferred embodiment, the invention refers to at least one of the compounds of Formula (IAb") listed in the Table 3 below and pharmaceutically acceptable salts thereof. These compounds are particularly active on receptors DDR1 and DDR2, as shown in Table 7.

**Table 3: List of compounds of Formula (IAb")**

| **Example No.** | **Structure** | **Chemical Name** |
|---|---|---|
| 36 | 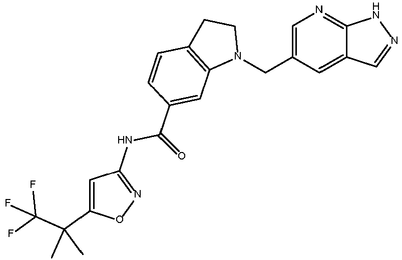 | 1-((1H-pyrazolo[3,4-b]pyridin-5-yl)methyl)-N-(5-(1,1,1-trifluoro-2-methylpropan-2-yl)isoxazol-3-yl)indoline-6-carboxamide |
| 40 | 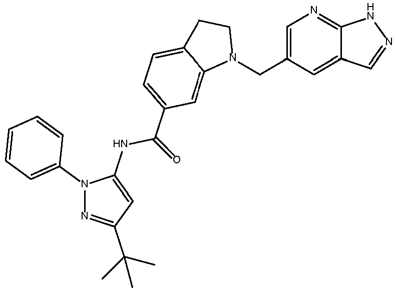 | 1-((1H-pyrazolo[3,4-b]pyridin-5-yl)methyl)-N-(3-(tert-butyl)-1-phenyl-1H-pyrazol-5-yl)indoline-6-carboxamide |
| 45 | 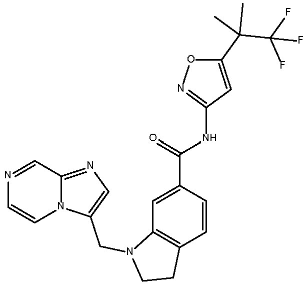 | 1-(imidazo[1,2-a]pyrazin-3-ylmethyl)-N-(5-(1,1,1-trifluoro-2-methylpropan-2-yl)isoxazol-3-yl)indoline-6-carboxamide |
| 58 | 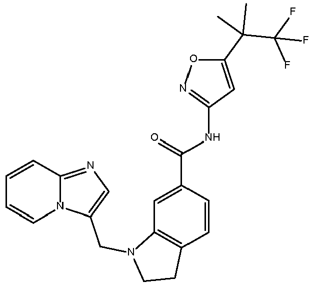 | 1-(imidazo[1,2-a]pyridin-3 -ylmethyl)-N-(5-(1,1,1-trifluoro-2-methylpropan-2-yl)isoxazol-3-yl)indoline-6-carboxamide |
| 67 | 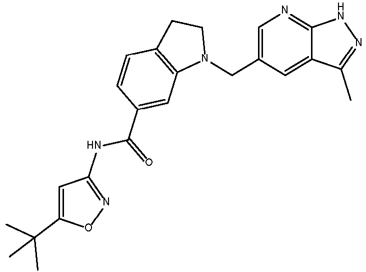 | N-(5-(tert-butyl)isoxazol-3 -yl)-1- ((3-methyl-1H-pyrazolo[3,4-b]pyridin-5-yl)methyl)indoline-6-carboxamide |
| 71 | 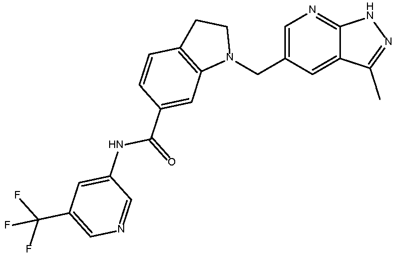 | 1-((3-methyl-1H-pyrazolo[3,4-b]pyridin-5-yl)methyl)-N-(5-(trifluoromethyl)pyridin -3-yl)indoline-6-carboxamide |
| 78 | 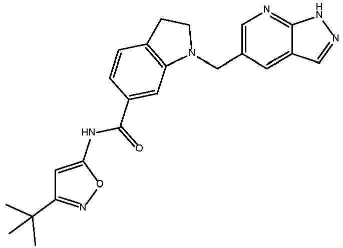 | 1-((1H-pyrazolo[3,4-b]pyridin-5-yl)methyl)-N-(3-(tert-butyl)isoxazol-5-yl)indoline-6-carboxamide |
| 79 | 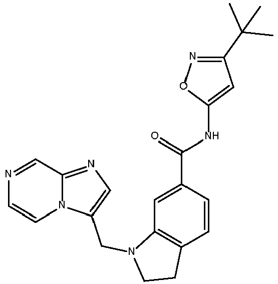 | N-(3-(tert-butyl)isoxazol-5-yl)-1-(imidazo[1,2-a]pyrazin-3 -ylmethyl)indoline-6-carboxamide |
| 80 | 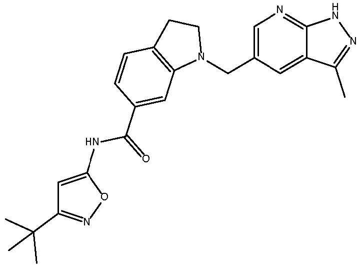 | N-(3-(tert-butyl)isoxazol-5-yl)-1-((3-methyl-1H-pyrazolo[3,4-b]pyridin-5-yl)methyl)indoline-6-carboxamide |
| 81 | 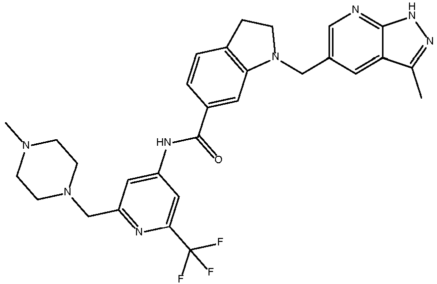 | 1-((3-methyl-1H-pyrazolo[3,4-b]pyridin-5-yl)methyl)-N-(2-((4-methylpiperazin-1 - yl)methyl)-6-(trifluoromethyl)pyridin -4-yl)indoline-6-carboxamide |
| 82 | 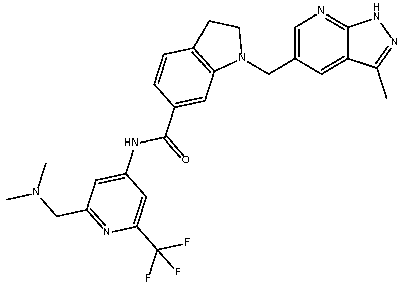 | N-(2-((dimethylamino)methy l)-6-(trifluoromethyl)pyridin -4-yl)-1-((3-methyl-1H-pyrazolo[3,4-b]pyridin-5-yl)methyl)indoline-6-carboxamide |
| 83 | 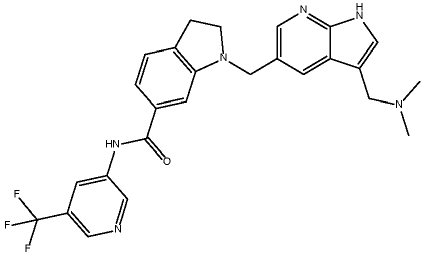 | 1-((3-((dimethylamino)methy l)-1H-pyrrolo[2,3-b]pyridin-5-yl)methyl)-N-(5-(trifluoromethyl)pyridin -3-yl)indoline-6-carboxamide |
| 84 | 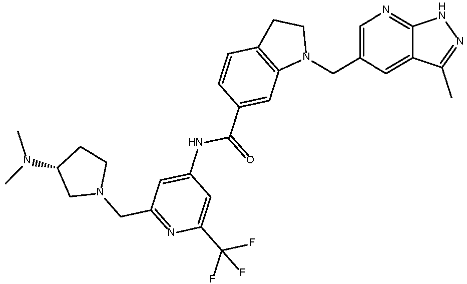 | (R)-N-(2-((3-(dimethylamino)pyrroli din-1-yl)methyl)-6-(trifluoromethyl)pyridin -4-yl)-1-((3-methyl-1H-pyrazolo[3,4-b]pyridin-5-yl)methyl)indoline-6-carboxamide |
| 87 | 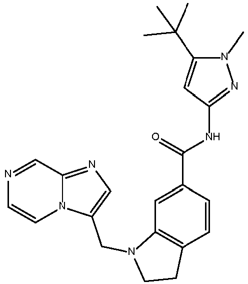 | N-(5-(tert-butyl)-1-methyl-1H-pyrazol-3-yl)-1-(imidazo[1,2-a]pyrazin-3-ylmethyl)indoline-6-carboxamide |
| 88 | 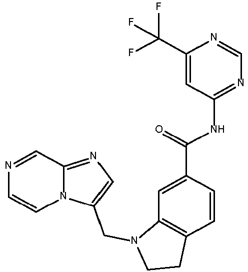 | 1-(imidazo[1,2-a]pyrazin-3-ylmethyl)-N-(6-(trifluoromethyl)pyrimi din-4-yl)indoline-6-carboxamide |
| 91 | 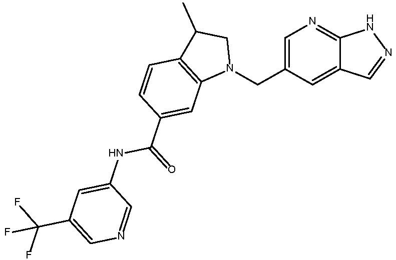 | 1-((1H-pyrazolo[3,4-b]pyridin-5-yl)methyl)-3-methyl-N-(5-(trifluoromethyl)pyridin -3-yl)indoline-6-carboxamide |
| 92 | 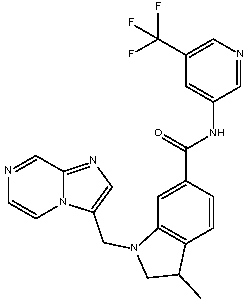 | 1-(imidazo[1,2-a]pyrazin-3-ylmethyl)-3-methyl-N-(5-(trifluoromethyl)pyridin -3-yl)indoline-6-carboxamide |

In another preferred embodiment, the present invention relates to a compound of general formula (I) wherein **L₁** is -NHC(O)-, represented by formula (IB) 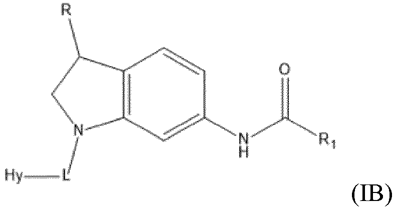 wherein **R, L, Hy** and **R₁** are as defined above.

According to a preferred embodiment, the invention refers to at least one of the compounds of Formula (IB) listed in the Table 4 below and pharmaceutically acceptable salts thereof. These compounds are particularly active on receptors DDR1 and DDR2, as shown in Table 7.

**Table 4: List of compounds of Formula (IB)**

| **Example No.** | **Structure** | **Chemical Name** |
|---|---|---|
| 16 | 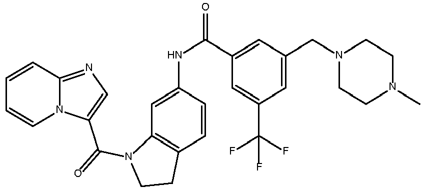 | N-(1-(imidazo[1,2-a]pyridine-3-carbonyl)indolin-6-yl)-3-((4-methylpiperazin-1-yl)methyl)-5-(trifluoromethyl)benzamide |
| 17 | 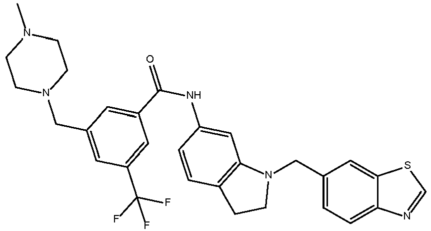 | N-(1-(benzo[d]thiazol-6-ylmethyl)indolin-6-yl)-3-((4-methylpiperazin-1-yl)methyl)-5-(trifluoromethyl)benzamide |
| 18 | 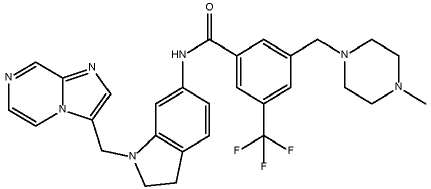 | N-(1-(imidazo[1,2-a]pyrazin-3-ylmethyl)indolin-6-yl)-3 - ((4-methylpiperazin-1-yl)methyl)-5-(trifluoromethyl)benzamide |
| 19 | 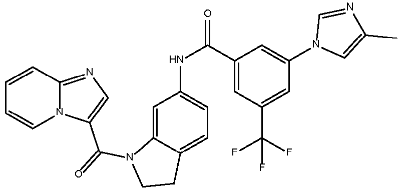 | N-(1-(imidazo[1,2-a]pyridine-3-carbonyl)indolin-6-yl)-3 -(4-methyl-1H-imidazol-1-yl)-5-(trifluoromethyl)benzamide |
| 20 | 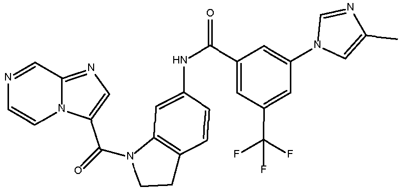 | N-(1-(imidazo[1,2-a]pyrazine-3-carbonyl)indolin-6-yl)-3-(4-methyl-1H-imidazol-1-yl)-5-(trifluoromethyl)benzamide |
| 21 | 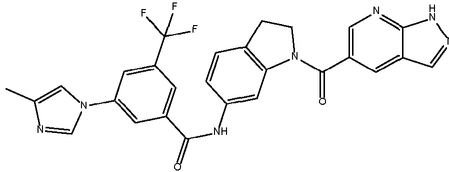 | N-(1-(1H-pyrazolo[3,4-b]pyridine-5-carbonyl)indolin-6-yl)-3 -(4-methyl-1H-imidazol-1-yl)-5-(trifluoromethyl)benzamide |
| 22 | 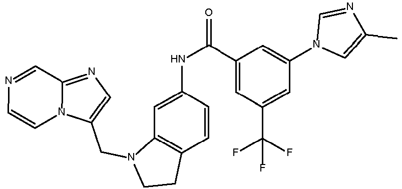 | N-(1-(imidazo[1,2-a]pyrazin-3-ylmethyl)indolin-6-yl)-3-(4-methyl-1H-imidazol-1-yl)-5-(trifluoromethyl)benzamide |
| 23 | 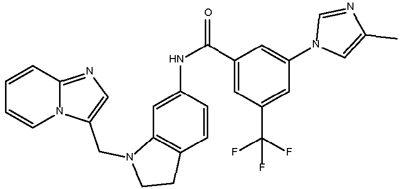 | N-(1-(imidazo[1,2-a]pyridin-3-ylmethyl)indolin-6-yl)-3-(4-methyl-1H-imidazol-1-yl)-5-(trifluoromethyl)benzamide |
| 35 | 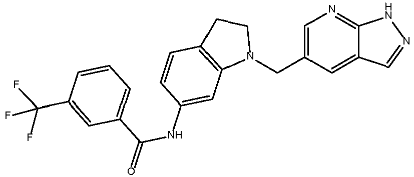 | N-(1-((1H-pyrazolo[3,4-b]pyridin-5-yl)methyl)indolin-6-yl)-3 - (trifluoromethyl)benzamide |
| 38 | 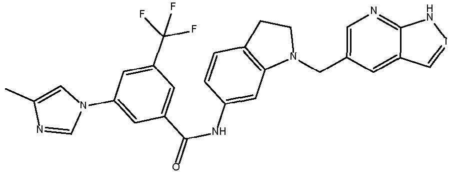 | N-(1-((1H-pyrazolo[3,4-b]pyridin-5-yl)methyl)indolin-6-yl)-3 - (4-methyl-1H-imidazol-1-yl)-5-(trifluoromethyl)benzamide |

In a particularly preferred embodiment, the present invention relates to a compound of general formula (I) wherein **L₁** is -NHC(O)-, **R₁** is group X 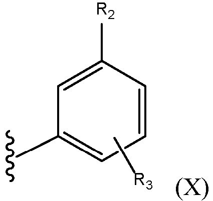 represented by formula (IBa) 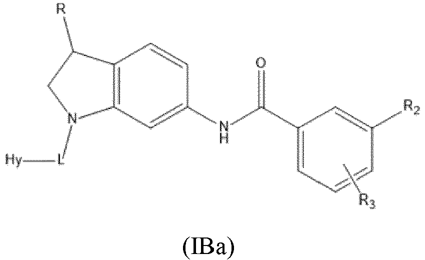 wherein **R, L, Hy, R₂** and **R₃** are as defined above.

In a further preferred embodiment, the present invention relates to a compound of general formula (IBa) wherein **L** is -C(O)-, represented by formula (IBa') 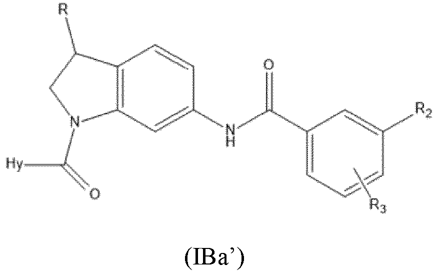 wherein **R, Hy, R₂** and **R₃** are as defined above.

In a another preferred embodiment, the present invention relates to a compound of general formula (I) wherein Li is -NHC(O)-, L is-C(O)-, R₁ is X' 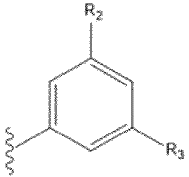 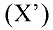 represented by formula (IBa' S) 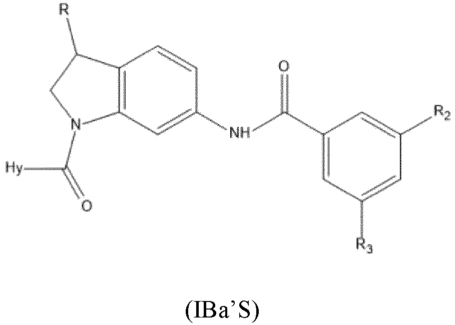 wherein **R, Hy, R₂** and **R₃** are as defined above.

In a even more preferred embodiment the present invention refers to a compound of formula (IBa'S), wherein **R₂** is trifluoromethyl; **R₃** is 4-methyl-1H-imidazol-1-yl and (4-methylpiperazin-1-yl)methyl; and **Hy** is selected from the group consisting of 1H-pyrazolo[3,4-b]pyridine-5-yl, imidazo[1,2-a]pyridine-3-yl and 1-(imidazo[1,2-a]pyrazine-3-yl.

According to a preferred embodiment, the invention refers to at least one of the compounds of Formula (IBa'S) listed in the Table 5 below and pharmaceutically acceptable salts thereof. These compounds are particularly active on receptors DDR1 and DDR2, as shown in Table 7.

**Table 5: List of compounds of Formula (IBa'S)**

| **Example No.** | **Structure** | **Chemical Name** |
|---|---|---|
| 19 | 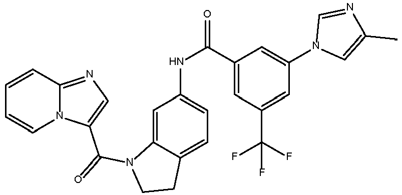 | N-(1-(imidazo[1,2-a]pyridine-3-carbonyl)indolin-6-yl)-3-(4-methyl-1H-imidazol-1-yl)-5-(trifluoromethyl)benza mide |
| 20 | 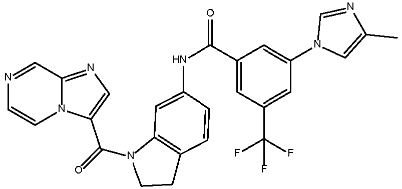 | N-(1-(imidazo[1,2-a]pyrazine-3-carbonyl)indolin-6-yl)-3-(4-methyl-1H-imidazol-1-yl)-5-(trifluoromethyl)benza mide |
| 21 | 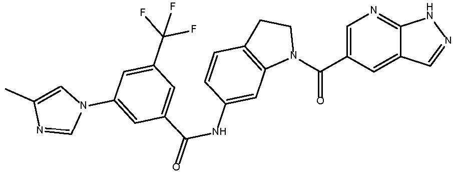 | N-(1-(1H-pyrazolo[3,4-b]pyridine-5-carbonyl)indolin-6-yl)-3-(4-methyl-1H-imidazol-1-yl)-5-(trifluoromethyl)benza mide |
| 16 | 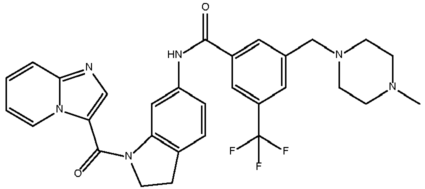 | N-(1-(imidazo[1,2-a]pyridine-3-carbonyl)indolin-6-yl)-3-((4-methylpiperazin-1 - yl)methyl)-5-(trifluoromethyl)benza mide |

In another particularly preferred embodiment the present invention refers to a compound of formula (IBa) wherein **L** is -CH₂-, represented by formula (IBa") 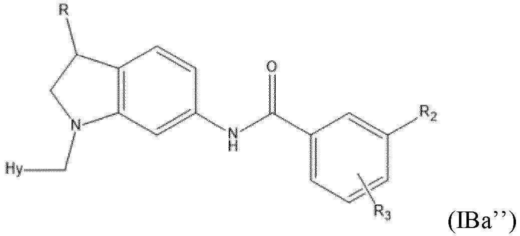 wherein **R, Hy, R₂** and **R₃** are as defined above.

In a particularly preferred embodiment, the present invention relates to a compound of general formula (I) wherein **L₁** is -NHC(O)-, **L** is-CH₂-, **R₁** is X' 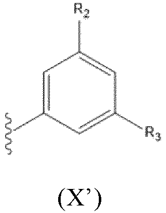 represented by formula (IBa"S) 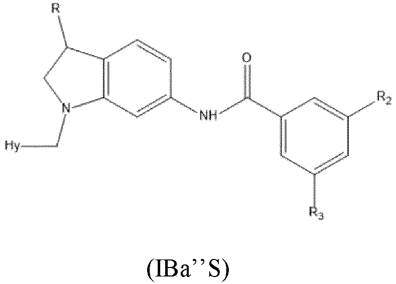 wherein **R, Hy, R₂** and **R₃** are as defined above.

In a even more preferred embodiment the present invention refers to a compound of formula (IBa"S), wherein **R₂** is trifluoromethyl; **R₃** is H or is selected from the group consisting of 4-methyl-1H-imidazol-1-yl and 4-(methylpiperazin-1-yl)methyl; and **Hy** is selected from the group consisting of imidazo[1,2-a]pyridine-3-yl; benzo[d]thiazol-6-yl, imidazo[1,2-a]pyrazin-3-yl and 1H-pyrazolo[3,4-b]pyridin-5-yl.

According to a preferred embodiment, the invention refers to at least one of the compounds of Formula (IBa" S) listed in the Table 6 below and pharmaceutically acceptable salts thereof. These compounds are particularly active on receptors DDR1 and DDR2, as shown in Table 7

**Table 6: List of compounds of Formula (IBa"S)**

| **Ex. No.** | **Structure** | **Chemical Name** |
|---|---|---|
| 17 | 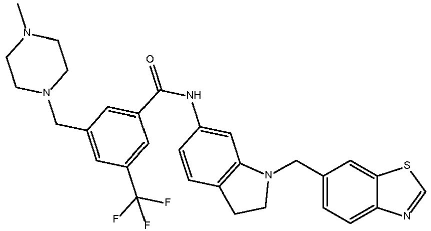 | N-(1-(benzo[d]thiazol-6-ylmethyl)indolin-6-yl)-3-((4-methylpiperazin-1-yl)methyl)-5-(trifluoromethyl)benzamide |
| 18 | 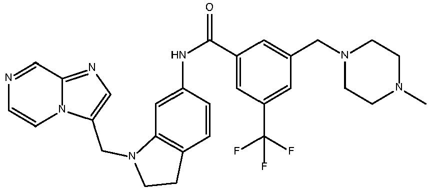 | N-(1-(imidazo[1,2-a]pyrazin-3 -ylmethyl)indolin-6-yl)-3 - ((4-methylpiperazin-1-yl)methyl)-5-(trifluoromethyl)benzamide |
| 22 | 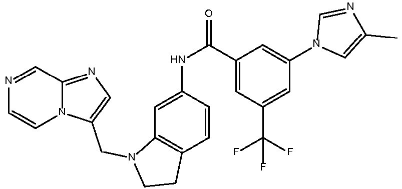 | N-(1-(imidazo[1,2-a]pyrazin-3-ylmethyl)indolin-6-yl)-3-(4-methyl-1H-imidazol-1-yl)-5-(trifluoromethyl)benzamide |
| 23 | 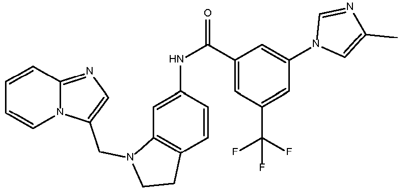 | N-(1-(imidazo[1,2-a]pyridin-3 -ylmethyl)indolin-6-yl)-3 - (4-methyl-1H-imidazol-1-yl)-5-(trifluoromethyl)benzamide |
| 35 | 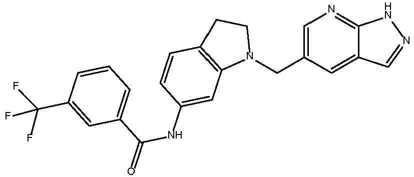 | N-(1-((1H-pyrazolo[3,4-b]pyridin-5-yl)methyl)indolin-6-yl)-3 - (trifluoromethyl)benzamide |
| 38 | 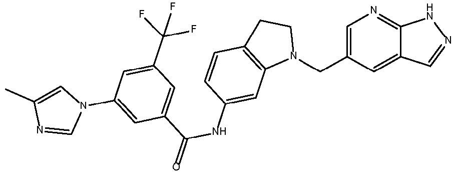 | N-(1-((1H-pyrazolo[3,4-b]pyridin-5-yl)methyl)indolin-6-yl)-3-(4-methyl-1H-imidazol-1-yl)-5-(trifluoromethyl)benzamide |

In a particularly preferred embodiment, the present invention relates to a compound of general formula (I) wherein **L₁** is-NHC(O)-, **R₁** is **Het,** represented by formula (IBb) 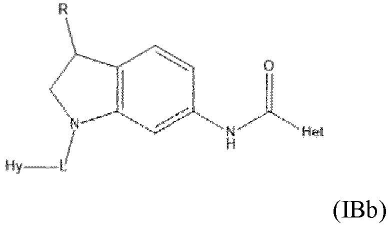 wherein **R, L, Hy** and **Het** are as defined above.

In another preferred embodiment, the present invention relates to a compound of general formula (IBb) wherein **L** is -C(O)-, represented by formula (IBb') 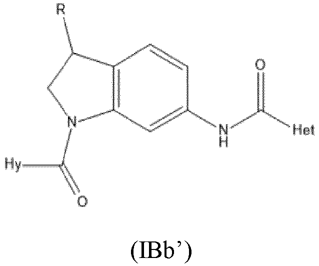 wherein **R, Hy** and **Het** are as defined above.

In another preferred embodiment, the present invention relates to a compound of general formula (IBb) wherein **L** is -CH₂-, represented by formula (IBb") 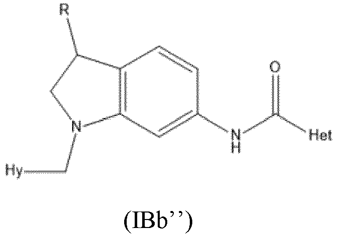 wherein **R, Hy** and **Het** are as defined above.

It is clearly understood that any compound of the present invention may be encompassed by more than one general formula. For instance, and not limited to that, a compound may be encompassed by both formula (IA") and formula (IAb"), as they have both **L** being CH₂ and **L₁** being -C(O)NH-.

The compounds of the invention, including all the compounds here above listed, can be prepared from readily available starting materials using the following general methods and procedures or by using slightly modified processes readily available to those of ordinary skill in the art. Although a particular embodiment of the present invention may be shown or described herein, those skilled in the art will recognize that all embodiments or aspects of the present invention can be obtained using the methods described herein or by using other known methods, reagents and starting materials. When typical or preferred process conditions (i.e. reaction temperatures, times, mole ratios of reactants, solvents, pressures, etc.) are given, other process conditions can also be used unless otherwise stated. While the optimum reaction conditions may vary depending on the particular reactants or solvent used, such conditions can be readily determined by those skilled in the art by routine optimization procedures.

In some cases, generally known protective groups (PG) could be employed when needed to mask or protect sensitive or reactive moieties, in accordance to general principles of chemistry (Protective group in organic syntheses, 3rd ed. T. W. Greene, P. G. M. Wuts).

The compounds of formula (I) of the present invention have surprisingly been found to effectively inhibit both receptor DDR1 and DDR2. Advantageously, the inhibition of receptors DDR1 and DDR2 may result in efficacious treatment of the diseases or conditions wherein the DDR receptors are involved.

In this respect, it has now been found that the compounds of formula (I) of the present invention have an inhibitory drug potency expressed as inhibition constant Ki on DDR1 and DDR2 lower than 80 nM, as shown in the present experimental part. Preferably, the compounds of the present invention have a Ki on DDR1 and DDR2 lower than 50 nM. Even more preferably, the compounds of the present invention have a Ki on DDR1 and DDR2 lower than 25 nM.

In addition, it has been found that the compounds of formula (I) of the present invention have both the affinity for either DDR1 and DDR2 receptors and the inhibitory activity against either DDR1 and DDR2 receptors below about 80 nM respectively in the binding (expressed as Ki) and the cell based assays (expressed as IC50), as shown in the present experimental part. Preferably, the compounds of the present invention have a Ki and/or an IC50 on DDR1 and DDR2 receptors lower than 50 nM. Even more preferably, the compounds of the present invention have a Ki and/or an IC50 on DDR1 and DDR2 receptors lower than 25 nM.

In one aspect, the present invention refers to a compound of formula (I) according to any of the embodiments disclosed above for use as a medicament.

In a preferred embodiment, the invention refers to a compound of formula (I) and pharmaceutically acceptable salts thereof, for use in treating diseases, disorders, or conditions associated with dysregulation of DDR.

In another aspect, the invention refers to the use of a compound of formula (I) as above described in the preparation of a medicament for the treatment of disorders associated with dysregulation of DDR.

In a preferred embodiment, the invention refers to a compound of formula (I) or a pharmaceutically acceptable salt thereof, for use in the prevention and/or treatment of a disease, disorder or condition associated with DDR receptor mechanism. In one embodiment, the present invention refers to a compound of formula (I) useful for the prevention and/or treatment of fibrosis and/or diseases, disorders, or conditions that involve fibrosis.

The terms "fibrosis" or "fibrosing disorder," as used herein, refers to conditions that are associated with the abnormal accumulation of cells and/or fibronectin and/or collagen and/or increased fibroblast recruitment and include but are not limited to fibrosis of individual organs or tissues such as the heart, kidney, liver, joints, lung, pleural tissue, peritoneal tissue, skin, cornea, retina, musculoskeletal and digestive tract.

Preferably, the compounds of formula (I) as above described are useful for the treatment and/or prevention of fibrosis such as pulmonary fibrosis, idiopathic pulmonary fibrosis (IPF), hepatic fibrosis, renal fibrosis, ocular fibrosis, cardiac fibrosis, arterial fibrosis and systemic sclerosis.

More preferably, the compounds of formula (I) as above described are for the treatment of idiopathic pulmonary fibrosis (IPF).

In one aspect, the invention also refers to a method for the prevention and/or treatment of disorders associated with DDR receptors mechanisms, said method comprises administering to a patient in need of such treatment a therapeutically effective amount of a compound of formula (I) as above described.

In a further aspect, the invention refers to the use of a compound of formula (I) as above described for the treatment of disorders associated with DDR receptors mechanism.

In another aspect, the invention refers to the use of a compound of formula (I) as above described in the preparation of a medicament for the treatment of disorders associated with DDR receptors mechanism.

In a further aspect, the invention refers to a method for the prevention and/or treatment of disorder or condition associated with dysregulation of DDR receptors 1 and 2, said method comprising administering a patient in need of such treatment a therapeutically effective amount of a compound of formula (I) as above described.

In a further aspect, the present invention refers to the use of a compound of formula (I) as above described for the treatment of a disease, disorder or condition associated with dysregulation of DDR receptors 1 and 2.

As used herein, "safe and effective amount" in reference to a compound of formula (I) or a pharmaceutically acceptable salt thereof or other pharmaceutically-active agent means an amount of the compound sufficient to treat the patient's condition but low enough to avoid serious side effects and it can nevertheless be routinely determined by the skilled artisan.

The compounds of formula (I) may be administered once or according to a dosing regimen wherein a number of doses are administered at varying intervals of time for a given period of time. Typical daily dosages may vary depending upon the route of administration chosen.

The present invention also refers to a pharmaceutical composition comprising a compound of formula (I) according to any of its embodiment in admixture with at least one or more pharmaceutically acceptable carrier or excipient.

In one embodiment, the invention refers to a pharmaceutical composition of compounds of formula (I) in admixture with one or more pharmaceutically acceptable carrier or excipient, for example those described in Remington's Pharmaceutical Sciences Handbook, XVII Ed., Mack Pub., N.Y., U.S.A.

Administration of the compounds of the invention and their pharmaceutical compositions may be accomplished according to patient needs, for example, orally, nasally, parenterally (subcutaneously, intravenously, intramuscularly, intrasternally and by infusion) and by inhalation.

Preferably, the compounds of the present invention are administered orally or by inhalation.

In one preferred embodiment, the pharmaceutical composition comprising the compound of formula (I) is a solid oral dosage form such as tablets, gelcaps, capsules, caplets, granules, lozenges and bulk powders.

In one embodiment, the pharmaceutical composition comprising the compound of formula (I) is a tablet.

The compounds of the invention can be administered alone or combined with various pharmaceutically acceptable carriers, diluents (such as sucrose, mannitol, lactose, starches) and known excipients, including suspending agents, solubilizers, buffering agents, binders, disintegrants, preservatives, colorants, flavorants, lubricants and the like.

In a further embodiment, the pharmaceutical composition comprising a compound of formula (I) is a liquid oral dosage forms such as aqueous and non-aqueous solutions, emulsions, suspensions, syrups, and elixirs. Such liquid dosage forms can also contain suitable known inert diluents such as water and suitable known excipients such as preservatives, wetting agents, sweeteners, flavorants, as well as agents for emulsifying and/or suspending the compounds of the invention.

In a further embodiment, the pharmaceutical composition comprising the compound of formula (I) is an inhalable preparation such as inhalable powders, propellant-containing metering aerosols or propellant-free inhalable formulations.

For administration as a dry powder, single- or multi-dose inhalers known from the prior art may be utilized. In that case the powder may be filled in gelatine, plastic or other capsules, cartridges or blister packs or in a reservoir.

A diluent or carrier chemically inert to the compounds of the invention, e.g. lactose or any other additive suitable for improving the respirable fraction may be added to the powdered compounds of the invention.

Inhalation aerosols containing propellant gas such as hydrofluoroalkanes may contain the compounds of the invention either in solution or in dispersed form. The propellant-driven formulations may also contain other ingredients such as co-solvents, stabilizers and optionally other excipients.

The propellant-free inhalable formulations comprising the compounds of the invention may be in form of solutions or suspensions in an aqueous, alcoholic or hydroalcoholic medium and they may be delivered by jet or ultrasonic nebulizers known from the prior art or by soft-mist nebulizers.

The compounds of the invention can be administered as the sole active agent or in combination with other pharmaceutical active ingredients.

The dosages of the compounds of the invention depend upon a variety of factors including among others the particular disease to be treated, the severity of the symptoms, the route of administration and the like.

The invention is also directed to a device comprising a pharmaceutical composition comprising a compound of Formula (I) according to the invention, in form of a single- or multi-dose dry powder inhaler or a metered dose inhaler.

All preferred groups or embodiments described above for compounds of formula (I) may be combined among each other and apply as well *mutatis mutandis.*

The compounds of the invention, including all the compounds here above listed, can be prepared from readily available starting materials using the following general methods and procedures or by using slightly modified processes readily available to those of ordinary skill in the art. Although a particular embodiment of the present invention may be shown or described herein, those skilled in the art will recognize that all embodiments or aspects of the present invention can be obtained using the methods described herein or by using other known methods, reagents and starting materials. When typical or preferred process conditions (i.e. reaction temperatures, times, mole ratios of reactants, solvents, pressures, etc.) are given, other process conditions can also be used unless otherwise stated. While the optimum reaction conditions may vary depending on the particular reactants or solvent used, such conditions can be readily determined by those skilled in the art by routine optimization procedures.

Thus, processes described below and reported in the following schemes should not be viewed as limiting the scope of the synthetic methods available for the preparation of the compounds of the invention.

The compounds of formula (I), including all the compounds or at least one of the here above listed, can be generally prepared according to the procedure outlined in detail in the Schemes shown below, using generally known methods.

In a first embodiment of the present invention, compounds of formula (I) wherein R₁, L, L₁, Hy and X are as defined above can be prepared as described in Scheme 1.

Compounds of formula (I) may be prepared according to Scheme 1 as described hereinafter providing at least one non-limiting synthetic route for the preparation of all examples.

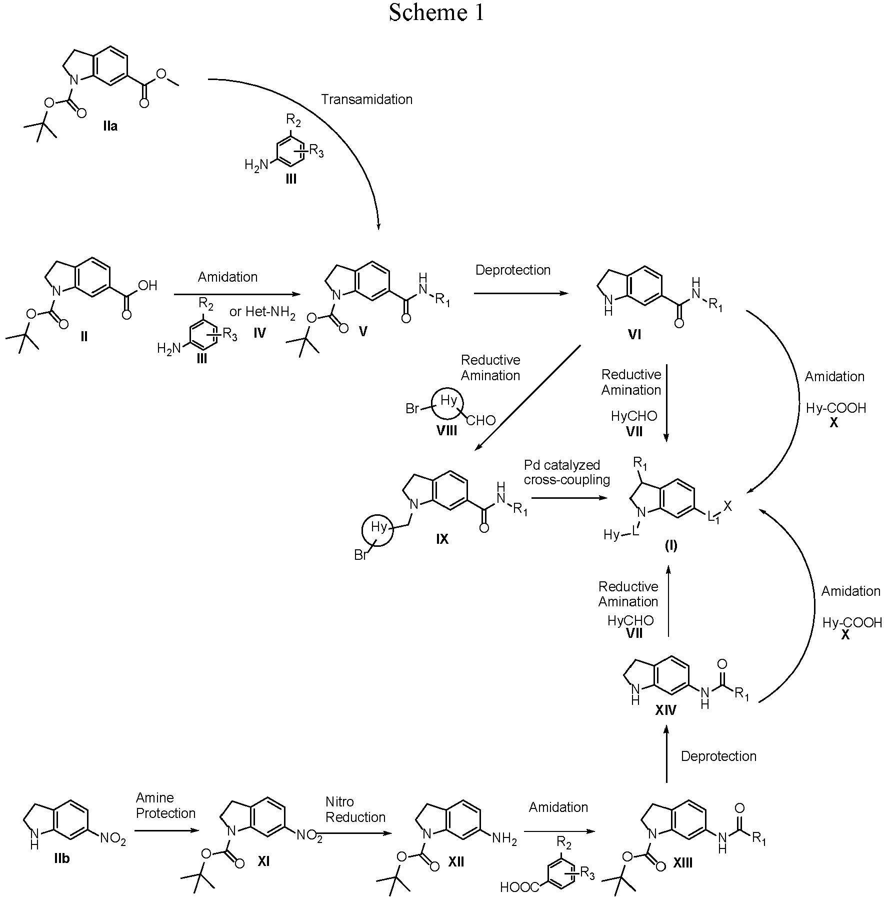

According to Scheme 1, Intermediate V may be prepared following a one-step synthesis starting from intermediate II, under suitable amide coupling reaction conditions. For example, intermediate II may be reacted with the appropriate amine III or IV in the presence of an agent, such as T3P or HATU, that activates the carboxylic acid partner for subsequent reaction with amines; with an organic base, such as DIPEA or TEA, in a suitable organic solvent, such as DCM or DMF, and at a temperature generally around RT for a time ranging from a few hours to overnight. Alternatively, intermediate V may be obtained by reacting intermediate II in the presence of TCFH and 1-methylimidazole to give the transient activated acylimidazolinium intermediate that may be reacted with the appropriate amine III or IV in a suitable solvent, such as DMF, at RT.

In a different approach, direct transamidation may be carried on between intermediate IIa and the appropriate amine III to obtain Intermediate V, using for example butyllithium as a base, in a suitable organic solvent as THF or Dioxane and at a temperature ranging from room temperature to -78 °C for few hours.

Intermediate V can be converted into intermediate VI by deprotection of the BOC-protected amine under acidic conditions such as trifluoroacetic acid in a suitable solvent such as, but not limited to, DCM at room temperature for few hours and subsequently intermediate VI can be transformed into Compounds of formula (I) with the appropriate aldehyde VII by applying reductive amination conditions with a suitable reductant agent, such as Na(OAc)₃BH or NaCNBH₃, in a suitable solvent, such as DCM or EtOH, in presence of an acid, such as acetic acid, and a coordinating agent, such as titanium tetrahydroisopropoxide, if needed, with a proper dehydrating agent, such as Mg₂SO₄, if needed, at room temperature.

Differently, Compounds of formula (I) can be prepared from intermediate VI via amidation conditions with the appropriate carboxylic acid X in the presence of an agent that activates the carboxylic acid partner, such as TBTU or HATU or T3P, in the presence of organic base such as DIPEA or TEA, in a suitable organic solvent such as DCM or DMF, and at a temperature generally around room temperature.

In a different approach, Compounds of formula (I) can be obtained from intermediate VI applying reductive amination conditions as described above with the proper bromo-aldehyde VIII, to give intermediate IX followed by palladium catalyzed cross-coupling condition with the proper amine using a suitable palladium catalyst, such as RuPhos Pd G3, in a suitable solvent, such as dioxane, and a proper base, such as LiHMDS, at a suitable temperature, for example 80 °C.

Intermediate XII can be prepared from intermediate IIb protecting the amine using a suitable protecting agent, such as, but not limited to, Boc anhydride, in a suitable solvent, such as DCM or DMF, and a suitable base, such as DIPEA or TEA, with a suitable promoter, such as DMAP, at room temperature, followed by reduction of the nitro group using a suitable hydrogen source, such as H₂, with a suitable catalyst, such as palladium on carbon, in the suitable solvent, such as ethyl acetate, at room temperature. Intermediate XII may undergo amidation applying the suitable amidation conditions described above, with the appropriate carboxylic acid, followed by Boc deprotection performed as described above to give intermediate XIV, that can be reacted with the appropriate aldehyde VII under reductive amination conditions as described above to give Compounds of formula (I).

Differently, intermediate XIV can undergo amidation with the proper carboxylic acid X using the conditions described above to obtain Compounds of formula (I).

In another embodiment, compounds of formula (I) may be prepared according to Scheme 2.

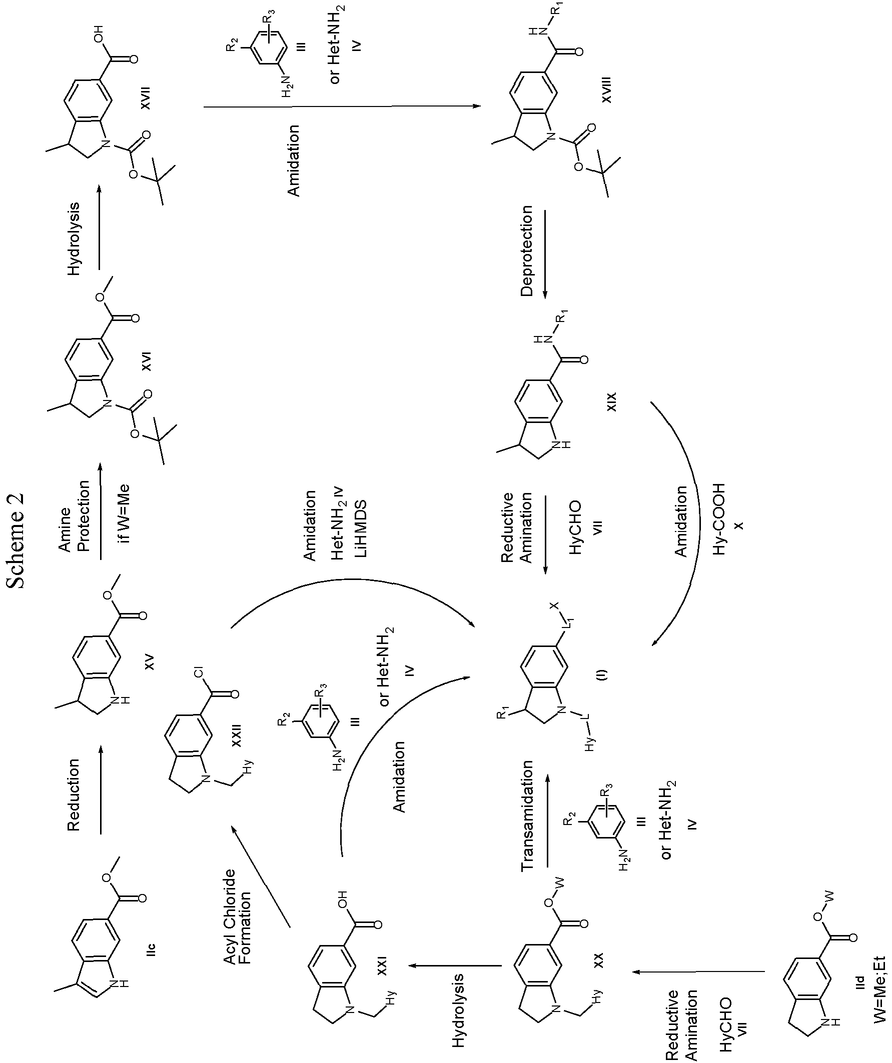

According to Scheme 2, Intermediate XV may be prepared from intermediate IIc applying reduction conditions using a suitable hydrogen source, for example triethylsilane, in acidic conditions, such as trifluoroacetic acid, in a suitable organic solvent, such as THF and at temperature generally around 0°C for a time ranging from a few hours to overnight. A protection of the amine can be performed on intermediate XV to give intermediate XVI in the presence of a proper protecting group, such as BOC anhydride, with a suitable organic base, such as DIPEA or TEA, with a suitable activating agent, such as DMAP, in a suitable organic solvent, such as DCM, and at temperature generally around RT for a time ranging from a few hours to overnight.

Hydrolysis of ester XVI can be performed in basic conditions, such as lithium hydroxide, in a suitable organic solvent, such as MeOH and at temperature generally around room temperature. Intermediate XVIII may be prepared under suitable amide coupling reaction conditions from intermediate XVI. For example, intermediate XVII may be reacted with the appropriate amine III or IV in the presence of an agent that activates the carboxylic acid partner for subsequent reaction with amines such as T3P or HATU, with an organic base such as DIPEA or TEA, in a suitable organic solvent such as DCM or DMF, and at temperature generally around RT for a time ranging from a few hours to overnight.

The intermediate XVIII can be converted into intermediate XIX by deprotection of BOC-protected amine under appropriate acidic conditions, such as trifluoroacetic acid, in a suitable solvent such as, but not limited to, DCM at room temperature for few hours.

Then Compounds of formula (I) may be obtained under suitable amide coupling conditions as described above from intermediate XIX with the proper carboxylic acid X. Alternatively, intermediate XIX can be converted in Compounds of formula (I) with the appropriate aldehyde VII by applying reductive amination conditions with a suitable reductant agent, such as Na(OAc)₃BH or NaCNBH₃, in a suitable solvent, such as DCM or EtOH, in presence of an acid, such as acetic acid, and a coordinating agent, such as titanium tetrahydroisopropoxide, if needed, with a proper dehydrating agent, such as Mg₂SO₄, if needed, at room temperature.

In a different approach, intermediate XXI can be prepared from intermediate Iid performing a reductive amination with the proper aldehyde VII using conditions described above, followed by hydrolysis of the ester, performed using the appropriate aqueous inorganic base, such as LiOH or NaOH, in a suitable solvent such as MeOH or EtOH, at room temperature.

Compounds of formula (I) may be obtained from intermediate XX applying transamidation conditions with the proper amine III or IV, using a suitable base, such as butyllithium or LDA, in a suitable organic solvent, such as THF or Dioxane, at a temperature ranging from room temperature to -78 °C for few hours. Alternatively Compounds of formula (I) may be obtained from intermediate XXI, under suitable amide coupling reaction conditions with the appropriate amine III or IV in the presence of an agent that activates the carboxylic acid partner for subsequent reaction with amines such as T3P or HATU, with an organic base such as DIPEA or TEA, in a suitable organic solvent such as DCM or DMF, and at temperature generally around RT for a time ranging from a few hours to overnight. In a different approach, Compounds of formula (I) can be obtained from intermediate XXI by applying acyl chloride formation conditions using the appropriate chlorinating agent, such as thionyl chloride or oxalyl chloride, in the proper solvent such as DCM, at a temperature for example ranging from 0°C to room temperature, followed by amidation performed for example treating amine IV with LiHMDS in the proper solvent, such as THF, at the appropriate temperature, such as - 78°C.

In a further embodiment, compounds of formula I may be prepared according to Scheme 3.

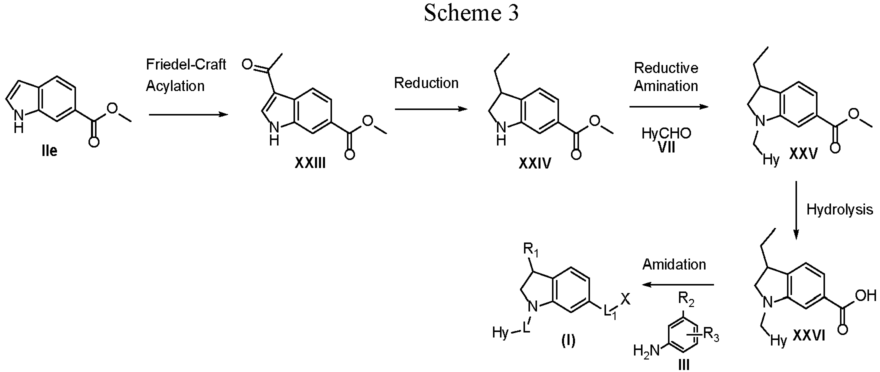

According to Scheme 3, Intermediate XXIII may be prepared from intermediate IIe applying Friedel-Craft acylation conditions with the proper acyl chloride, such as acetyl chloride, using the proper Lewis acid, such AlCl₃, in the proper solvent, such as DCM. Reduction of intermediate XXIII may be performed to obtain intermediate XXIV, using a suitable hydrogen source, for example triethylsilane, in acidic conditions, such as trifluoroacetic acid, in a suitable organic solvent, such as THF and at temperature generally around 0°C for a time ranging from a few hours to overnight. Intermediate XXIV can be converted in intermediate XXV with the appropriate aldehyde VII by applying reductive amination conditions with a suitable reductant agent, such as Na(OAc)₃BH or NaCNBH₃, in a suitable solvent, such as DCM or EtOH, in presence of an acid, such as acetic acid, and a coordinating agent, such as titanium tetrahydroisopropoxide, if needed, with a proper dehydrating agent, such as Mg₂SO₄, if needed, at room temperature. Hydrolysis of ester XXV can be performed in basic conditions, such as lithium hydroxide, in a suitable organic solvent, such as MeOH and at temperature generally around room temperature. Compounds of formula (I) can be obtained starting from intermediate XXVI in the presence of an appropriate chlorinating reagent, such as POCl₃ or thionyl chloride in a solvent such as pyridine or DCM, at 5°C or room temperature, to get the corresponding acyl chloride that is treated directly with the appropriate amine III in a suitable solvent such as DCM at room temperature.

The various aspects of the invention described in this application are illustrated by the following examples which are not meant to limit the invention in any way.

### PREPARATIONS OF INTERMEDIATES AND EXAMPLES

Chemical Names of the compounds were generated with Structure To Name Place IUPAC Name by PerkinElmer ChemDraw Professional 19.1.1.21.

All reagents, for which the synthesis is not described in the experimental part, are either commercially available, or are known compounds or may be formed from known compounds by known methods by a person skilled in the art.

In the procedures that follow, some of the starting materials are identified through an "Intermediate" or "Example" number with indications on step number. This is provided merely for assistance to the skilled chemist.

A "similar" or "analogous" procedure means that such a procedure may involve minor variations, for example reaction temperature, reagent/solvent amount, reaction time, work-up conditions or chromatographic purification conditions.

### ABBREVIATION - MEANING

t*_{R}* = retention time; Boc = tert-butoxycarbonyl; (BOC)₂O= Boc anhydride, Di-*tert-*butyl dicarbonate; TEA = triethylamine; HATU = (Dimethylamino)-N,N-dimethyl(3H-[1,2,3]triazolo[4,5-b]pyridin-3-yloxy)methaniminium hexafluoro phosphate; TBTU = *O-*(Benzotriazol-1-yl)-*N*,*N*,*N'*,*N'*-tetramethyluronium tetrafluoroborate; DMAP = 4-dimethylaminopyridine; DMF = dimethylformamide; EtOAc = Ethyl acetate; ee= Enantiomeric excess; RT or rt = room temperature; THF = tetrahydrofuran; SOCl₂ = Thionyl chloride; DCM = dichloromethane; MeOH = methyl alcohol; LCMS = Liquid Chromatography/Mass Spectrometry; HPLC = high performance liquid chromatography; PrepHPLC = preparative high performance liquid chromatography; Int. = Intermediate; TLC = Thin Layer Chromatography; d-DMSO = deuterated dimethyl sulfoxide. NMR = nuclear magnetic resonance; DIPEA = N,N-Diisopropylethylamine; UPLC = Ultra Performance Liquid Chromatography; tBu XPhos = 2-Di-tert-butylphosphino-2',4',6'-triisopropylbiphenyl; PdCl₂(dppf) = [1,1'-Bis(diphenylphosphino)ferrocene]dichloropalladium(II); STAB = sodium triacetoxyborohydride; Pd(OAc)₂ = palladium(II) acetate; AcOH = Acetic acid; Py = pyridine; T3P = Propanephosphonic acid anhydride; NaBH₃CN =sodium cyanoborohydride; Na₂SO₄ = sodium sulfate; pTLC = preparative thin layer chromatography; FCC= flash column chromatography; nBuLi= nButyllithium; ACN = Acetonitrile; NaHCO₃ = 15 % or saturated solution (sat. aq. sol.) of sodium bicarbonate; RuPhos Pd G3: (2-Dicyclohexylphosphino-2',6'-diisopropoxy-1,1'-biphenyl)[2-(2'-amino-1,1'-biphenyl)]palladium(II) methanesulfonate, generation 3 SCX = strong cation exchange; SFC = supercritical fluid chromatography; SM = starting material; eq.= equivalents.

Xphos Pd G2= (2-Dicyclohexylphosphino-2',4',6'-triisopropyl-1,1'-biphenyl)[2-(2'-amino-1,1'-biphenyl)]palladium(II) methanesulfonate, generation 2; Xphos Pd G3= (2-Dicyclohexylphosphino-2',4',6'-triisopropyl-1,1'-biphenyl)[2-(2'-amino-1,1'-biphenyl)]palladium(II) methanesulfonate, generation 3.

### General Experimental details and methods

### NMR characterization:

¹H NMR spectra were recorded on Varian MR-400 spectrometer operating at 400 MHZ (proton frequency), equipped with: a self-shielded Z-gradient coil 5 mm 1H/nX broadband probe head for reverse detection, deuterium digital lock channel unit, quadrature digital detection unit with trans mitter offset frequency shift, alternatively ¹H NMR spectra were recorded on Bruker Avance III HD 400 MHz or Bruker Fourier 300 MHz. Chemical shifts are reported as δ values in ppm relative to tetramethyl silane (TMS) as an internal standard. Coupling constants (J values) are given in hertz (Hz) and multiplicities are reported using the following abbreviation (s= singlet, d=doublet, t=triplet, q=quartet, m=multiplet, br=broad, nd=not determined).

In some cases, signals NH from amide bond or amine bond (Exchangeable protons) are not visible. In a few cases, signals of CH₂ of indoline ring could be hidden under the signal of water or under the signal of DMSO. In some cases, signals of CH₂ from pyrrolidine or 1-methylpiperazine rings could be hidden under the signal of water or DMSO. In few cases, signals from CH₃ groups could be hidden under the signal of water or DMSO.

### LC/UV/MS Analytical Methods

LC/MS retention times are estimated to be affected by an experimental error of ± 0.5 min.
**Method 1:** Kinetex^{®} XB-C18 column, 4.6x50 mm, 2.6 µm maintained at 25 °C. Mobile phase: water (0.1% formic acid) in MeCN (0.1% formic acid), from 80% to 5% within 3.90 min; Flow rate: 1.0 ml/min; wavelength: 190-340 nm DAD. Dionex UHPLC Ultimate 3000 with DAD detector/Thermo Scientific MSQ Plus.
**Method 2:** Acquity UPLC HSS C18 column, 100 x 2.1mm, 1.8 µm (Plus guard cartridge), maintained at 40°C. Mobile phase: MeCN (0.1% formic acid) in water (0.1% formic acid) from 5% to 95% within 5.6 min. Flow rate: 0.4 ml/min. Wavelength: 210-400 nm DAD. UPLC + Waters DAD + Waters SQD2, single quadrapole UPLC-MS
**Method 3:** Kinetex^{®} XB-C18 column, 4.6x50 mm, 2.6 µm maintained at 25 °C. Mobile phase: water (0.1% formic acid) in MeCN (0.1% formic acid), from 95% to 20% within 4.75 min; Flow rate: 1.0 ml/min; wavelength: 190-340 nm DAD. Dionex UHPLC Ultimate 3000 with DAD detector/Thermo Scientific MSQ Plus.
**Method 4:** Kinetex^{®} XB-C18 column, 4.6x50 mm, 2.6 µm maintained at 25 °C. Mobile phase: water (0.1% formic acid) in MeCN (0.1% formic acid), from 90% to 5% within 3.90 min; Flow rate: 1.0 ml/min; wavelength: 190-340 nm DAD. Dionex UHPLC Ultimate 3000 with DAD detector/Thermo Scientific MSQ Plus.
**Method 5:** Kinetex^{®} XB-C18 column, 4.6x50 mm, 2.6 µm maintained at 25 °C. Mobile phase: water (0.1% formic acid) in MeCN (0.1% formic acid), from 70% to 5% within 3.90 min; Flow rate: 1.0 ml/min; wavelength: 190-340 nm DAD. Dionex UHPLC Ultimate 3000 with DAD detector/Thermo Scientific MSQ Plus.
**Method 6:** Acquity CSH C18 column 50mm x 2.1mm 1.7µm, manteined at 40°C; Mobile Phase: Eluent B (MeCN/water 95:5 +0.05% HCOOH) in Eluent A (water/MeCN 95:5 +0.05% HCOOH) from 1% to 99.9% within 1.5 min. Flow rate: 1 mL/min. Wavelength: 210-400 nm DAD. UPLC + Waters PDA + Waters QDA.
**Method 7:** Kinetex^{®} XB-C18 column, 4.6x50 mm, 2.6 µm maintained at 25 °C. Mobile phase: water (0.1% formic acid) in MeCN (0.1% formic acid), from 80% to 5% within 3.90 min; Flow rate: 1.0 ml/min; wavelength: 190-340 nm DAD. Dionex UHPLC Ultimate 3000 with DAD detector/Thermo Scientific MSQ Plus.
**Method 8:** Kinetex^{®} XB-C18 column, 4.6x50 mm, 2.6 µm maintained at 25 °C. Mobile phase: water (no formic acid) in MeCN (no formic acid), from 60% to 5% within 3.90 min; Flow rate: 1.0 ml/min; wavelength: 190-340 nm DAD. Dionex UHPLC Ultimate 3000 with DAD detector/Thermo Scientific MSQ Plus.
**Method 9:** Kinetex^{®} XB-C18 column, 4.6x50 mm, 2.6 µm maintained at 25 °C. Mobile phase: water (0.1% formic acid) in MeCN (0.1% formic acid), from 60% to 5% within 3.90 min; Flow rate: 1.0 ml/min; wavelength: 190-350 nm DAD. Dionex UHPLC Ultimate 3000 with DAD detector/Thermo Scientific ISQ EC mass spectrometer.
**Method 10:** Kinetex^{®} XB-C18 column, 4.6x50 mm, 2.6 µm maintained at 25 °C. Mobile phase: water (0.1% formic acid) in MeCN (0.1% formic acid), from 70% to 5% within 3.90 min; Flow rate: 1.0 ml/min; wavelength: 190-350 nm DAD. Dionex UHPLC Ultimate 3000 with DAD detector/Thermo Scientific ISQ EC mass spectrometer.
**Method 11:** Kinetex^{®} XB-C18 column, 4.6x50 mm, 2.6 µm maintained at 25 °C. Mobile phase: water (0.1% formic acid) in MeCN (0.1% formic acid), from 80% to 5% within 3.90 min; Flow rate: 1.0 ml/min; wavelength: 190-340 nm DAD. Dionex UHPLC Ultimate 3000 with DAD detector/Thermo Scientific MSQ Plus.
**Method 12:** Kinetex^{®} XB-C18 column, 4.6x50 mm, 2.6 µm maintained at 25 °C. Mobile phase: water (0.1% formic acid) in MeCN (0.1% formic acid), from 75% to 5% within 3.90 min; Flow rate: 1.0 ml/min; wavelength: 190-340 nm DAD. Dionex UHPLC Ultimate 3000 with DAD detector/Thermo Scientific MSQ Plus
**Method 13:** ACQUITY UPLC BEH C8 column, 42.1x150 mm, 1.7 µm maintained at 55 °C. Mobile phase: water (0.1% formic acid) in MeCN (0.1% formic acid), from 80% to 10% within 7.00 min; Flow rate: 0.5 ml/min; wavelength: 190-340 nm DAD. Dionex UHPLC Ultimate 3000 with DAD detector/Thermo Scientific MSQ Plus.
**Method 14:** Kinetex^{®} XB-C18 column, 4.6x50 mm, 2.6 µm maintained at 25 °C. Mobile phase: water (0.1% formic acid) in MeCN (0.1% formic acid), from 50% to 5% within 3.90 min; Flow rate: 1.0 ml/min; wavelength: 190-350 nm DAD. Dionex UHPLC Ultimate 3000 with DAD detector/Thermo Scientific ISQ EC - Mass Spectrometer
**Method 15:** Gemini-NX C18 column, 4.6x150 mm, 3 µm maintained at 35 °C. Mobile phase: water (0.1% formic acid) in MeCN (0.1% formic acid), from 80% to 5% within 8.50 min; Flow rate: 1.0 ml/min; wavelength: 190-340 nm DAD. Dionex UHPLC Ultimate 3000 with DAD detector/Thermo Scientific MSQ Plus.
**Method 16:** Kinetex^{®} XB-C18 column, 4.6x50 mm, 2.6 µm maintained at 25 °C. Mobile phase: water (0.1% formic acid) in MeCN (0.1% formic acid), from 60% to 5% within 3.90 min; Flow rate: 1.0 ml/min; wavelength: 190-340 nm DAD. Dionex UHPLC Ultimate 3000 with DAD detector/Thermo Scientific ISQ EC mass spectrometer.
**Method 20 :** Acquity UPLC BEH C18, 100x2.1mm, 1.7 µm maintained at 40 °C. Mobile phase: MeCN (0.1% formic acid) in water (0.1% formic acid), from 5% to 80% within 1.5 min; Flow rate : 1.5 ml/min; Wavelength: 210-400 nm DAD. Acquity H-Class UPLC with PDA detector and Qda
**Method 21:** Acquity BEH UPLC column, 2.1x50mm, 1.7µm, maintained at 40°C. Mobile phase: MeCN (0.03% ammonia) in water (0.03% ammonia), from 8% to 97% within 1.5 min; Flow rate: 0.8 ml/min; Wavelength: 210-400 nm DAD. Acquity H-Class UPLC with PDA detector and QDa.
**Method 22:** Waters Sunfire C18 column, 4.6x50mm, 3.5µm, maintained at 40°C. Mobile phase MeCN in water + 10mM ammonium bicarbonate, from 5 to 95% within 2.5 mins. Flow rate: 2.0 ml/min. Wavelength: 210-400 nm DAD. Waters 2795 separations module + Waters DAD + Micromass ZQ, single quadrapole LC-MS

### Chiral Supercritical Fluid Chromatography (SFC) separation protocol

The diastereomeric separation of compounds was achieved by Supercritical Fluid Chromatography (SFC) using a Waters Thar Prep100 preparative SFC system (P200 CO₂ pump, 2545 modifier pump, 2998 UV/VIS detector, 2767 liquid handler with Stacked Injection Module). The Waters 2767 liquid handler acted as both auto-sampler and fraction collector. Appropriate isocratic methods were selected based on methanol, ethanol or isopropanol solvent systems under un-modified or basic conditions. The standard SFC method used was modifier, CO₂, 100 mL/min, 120 Bar backpressure, 40 °C column temperature. The modifier used under basic conditions was diethylamine (0.1% V/V). The modifier used under acidic conditions was either formic acid (0.1% V/V) or trifluoroacetic acid (0.1% V/V). The SFC purification was controlled by Waters Fractionlynx software through monitoring at 210-400 nm and triggered at a threshold collection value, typically 260 nm. Collected fractions were analysed by SFC (Waters/Thar SFC systems with Waters SQD). The fractions that contained the desired product were concentrated by vacuum centrifugation.

### Supercritical Fluid Chromatography - Mass Spectrometry analytical conditions:

**Method 17:** SFC-MS was performed on a Waters/Thar SFC systems with Waters SQD using a YMC AMYLOSE-C column with a 45% IPA/CO₂ (with 0.1% diethylamine) isocratic run at 100mL/min, 120 Bar backpressure, 40°C column temperature.

**Method 18:** SFC-MS was performed on a Gilson Preparative LC system (GiIson Pump - 333; Gilson 151;Gilson Valvemate 6 position) using a Lux C1 (21.2mm x 250mm, 5um) column with a isocratic run (50:50 HEXANE:EtOH, 0.2% v/v NH3) at 21mL/min, 30°C column temperature.

**Method 19:** SFC-MS was performed on a Gilson Preparative LC system using a Lux C1 (4.6mm x 250mm, 5um) column with a isocratic run (50:50 HEX:EtOH (0.2% v/v NH3) at 1mL/min, 30°C column temperature.

Where the preparation of starting materials is not described, these are commercially available, known in the literature, or readily obtainable by those skilled in the art using standard procedures. All solvents were purchased from commercial sources and were used without additional purification.

Preparative HPLC was performed using reversed phase (C18) preparative HPLC both in basic conditions (ACN+0.1%NH₃, H2O+0.1%NH₃) and in acidic conditions (ACN+0.1%FA, H₂O+0.1%FA), in the last case, the residue was triturated with NaHCO₃ (15% aq. sol.), then the precipitate was filtered through the Schott funnel, rinsed with water, transferred to the vial and dried on high vacuum overnight at room temperature or alternatively SCX (NH) was utilized to obtained free base of the product, unless differently stated.

Thin layer chromatography was performed on Merck silica gel 60 F254 TLC plates. Preparative thin-layer chromatography (pTLC) was performed with Uniplate 1000 micron or 500 micron silica gel plates.

Flash chromatography (FCC) was performed on Interchim PuriFlash 450 and 520Plus systems or Isolera^{™} flash purification system using pre-packed silica gel cartridges unless otherwise stated in the text.

### PREPARATION OF INTERMEDIATES

### Preparation of lithio 3-[(4-methylpiperazin-1-yl)methyl]-5-(trifluoromethyl)benzoate - Intermediate 1

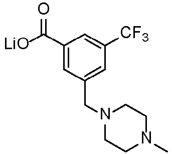

### Step 1; methyl 3-bromo-5-(trifluoromethyl)benzoate - Intermediate 2

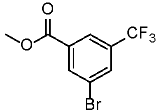

To the solution of 3-bromo-5-(trifluoromethyl)benzoic acid (75.0 g, 279 mmol) in MeOH (282 mL), SOCl₂ (81.0 mL, 1115 mmol) was added dropwise at 0°C. Next, the reaction mixture was stirred under reflux overnight, whereupon volatiles were removed under vacuum. To the residue water (200 mL) was added and aqueous layer was extracted with EtOAc (2x250 mL). Combined organic layers were washed with saturated solution of NaHCO₃, dried over Na₂SO₄ concentrated under vacuum to afford the title compound (74.5 g, 94%).

¹H NMR (300 MHz, DMSO-d6) δ 8.30 (dt, J = 1.8, 0.8 Hz, 2H), 8.13 (td, J = 1.6, 0.8 Hz, 1H), 3.90 (s, 3H).

### Step 2; methyl 3-[(4-methylpiperazin-1-yl)methyl]-5-(trifluoromethyl) benzoate- Intermediate 3

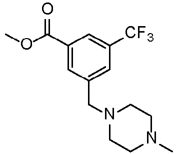

Intermediate 2 (47.5 g, 168 mmol), Cs₂CO₃ (164 g, 503 mmol), potassium 1-methyl-4-trifluoroboratomethylpiperazine (40.6 g, 184.6 mmol) were suspended in a mixture of THF (100 mL) and water (11 mL). The suspension was degassed, then Pd(OAc)₂ (3.76 g, 16.8 mmol) and XPhos (16.0 g, 33.5 mmol) were added and the reaction was carried out at 80 °C for 24 h. The reaction mixture was diluted with water (100 mL) and extracted with EtOAc (2x150 mL). The combined organic phases were concentrated and dried under vacuum to afford the crude, which was purified by column chromatography eluting with DCM/MeOH, 9:1 to give the title compound (25.3 g, 48%).

¹H NMR (300 MHz, DMSO-*d*₆) δ 8.16 (d, *J =* 1.7 Hz, 1H), 8.07 (t, *J =* 1.8 Hz, 1H), 7.97 - 7.86 (m, 1H), 3.90 (s, 3H), 3.62 (s, 2H), 2.38 (s, 8H), 2.15 (s, 3H).

### Step 3; lithio 3-[(4-methylpiperazin-1-yl)methyl]-5-(trifluoromethyl) benzoate - Intermediate 1

Intermediate 3 (25.3 g, 80.0 mmol) was dissolved in MeOH (700 mL). 1M LiOH solution was added (3.8 g, 160 mL) to the reaction mixture and stirred at RT overnight. The solvent was removed under vacuum and the crude material triturated with diethyl ether (2x) and filtered. The solid was collected to give the title compound (26.0 g, 100%).

¹H NMR (300 MHz, DMSO-d6) δ 8.02 (s, 2H), 7.49 (s, 1H), 3.51 (s, 2H), 2.32 (s, 8H), 2.14 (s, 3H).

### Preparation of 2-((1-methyl-1H-pyrazol-4-yl)amino) pyrimidine-5-carbaldehyde - Intermediate 4

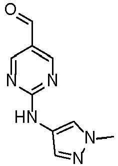

### Step 1; 2-((1-methyl-1H-pyrazol-4-yl)amino) pyrimidine-5-carbaldehyde - Intermediate 4

2-chloropyrimidine-5-carbaldehyde (50 mg, 0.351 mmol) was dissolved in THF (3.5) then 1-methyl-1H-pyrazol-4-amine (41 mg, 0.421 mmol), TEA (0.1 ml, 0.719 mmol) and THF (3.5 ml) were added. Reaction mixture was stirred overnight at room temperature. The mixture was quenched with addition of DCM and brine. The phases were separated and the water layer was washed with DCM (x2). The all combined organic phases were dried over Na₂SO₄, filtered and concentrated. Crude material was purified by FCC eluting with Hexane/ethyl acetate 1:1 to give the title compound (35 mg, 39%).

¹H NMR (300 MHz, DMSO-d6) δ 10.45 (s, 1H), 9.80 (s, 1H), 8.92 - 8.79 (m, 2H), 7.99 (d, J = 0.8 Hz, 1H), 7.56 (d, J = 0.8 Hz, 1H), 3.83 (s, 3H).

### Preparation of 3-(morpholinomethyl)-5-(trifluoromethyl)aniline - Intermediate 5

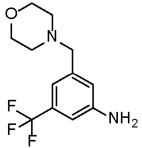

### Step 1; 4-(3-nitro-5-(trifluoromethyl)benzyl)morpholine - Intermediate 6

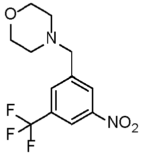

3-nitro-5-(trifluoromethyl)benzaldehyde (150 mg, 0.685 mmol) and morpholine (0.066 mL, 0.753 mmol) were dissolved in DCM (6.8 ml), titanium(IV) isopropoxide (389.4 mg, 1.37 mmol) and acetic acid was added. The reaction mixture was stirred at room temperature for 1 h. STAB (290 mg, 1.37 mmol) was added and the reaction mixture was stirred at room temperature until LCMS indicated consumption of starting material. The reaction mixture was partitioned between DCM and saturated NaHCO_{3 (aq)} and the mixture filtered through a bed of Celite. The aqueous phase was extracted with 2xDCM and the combined organic phases were washed with saturated aqueous NaCl _{(aq)}, separated and concentrated under vacuum to give the title compound (197 mg, 99%).
¹H NMR (400 MHz, CDCl3) δ 8.44 (s, 1H), 8.40 (s, 1H), 7.96 (s, 1H), 3.76 - 3.73 (m, 4H), 3.66 (s, 2H), 2.51 - 2.48 (m, 4H)

The following compounds were prepared as described for **Intermediate 6, step 1** reacting the suitable corresponding amine.

| **Int. N°** | **Chemical name** | **Structure** | **Aldehyde amount** | **Product mount (yield)** | **Purification Method** | **Analytical data** |
|---|---|---|---|---|---|---|
| 7 | N,N-dimethyl-1-(3-nitro-5-(trifluoro methyl)ph enyl)meth anamine | 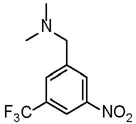 | 0.20 g | 84 mg, (37%) | FCC | ¹H NMR (400 MHz, CDCl₃) δ 8.41 - 8.37 (m, 2H), 7.95 (s, 1H), 3.58 (s, 2H), 2.29 (s, 6H) LCMS (Method 21) t*_{R}* = 1.50 min, m/z 249 [M+H]⁺ |
| 8 | N,N-dimethyl-1-(4-nitro-2-(trifluoro methyl)ph enyl)meth anamine | 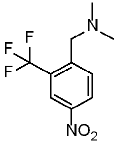 | 0.125 g | 132 mg (Y=93%) | SCX cartridge | ¹H NMR (400 MHz, CDCl₃) δ 8.50 (d, J=2.2 Hz, 1H), 8.38 (dd, J=2.3, 8.6 Hz, 1H), 8.10 (d, J=8.6 Hz, 1H), 3.68 (s, 2H), 2.30 (s, 6H) LCMS (Method 21) t*_{R}* = 1.58 min, m/z 249 [M+H]⁺ |
| 44 | (R)-N,N-dimethyl-1-(3-nitro-5-(trifluoro methyl)be nzyl)pyrro lidin-3-amine | 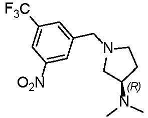 | 0.75 g | 0.78 g (Y=72%) | FCC | ¹H NMR (300 MHz, DMSO) δ 8.43 (s, 1H), 8.38 (s, 1H), 8.13 (s, 1H), {3.86 (d, J=14.2 Hz, 1H), 3.73 (d, J=14.2 Hz, 1H), 2.79 - 2.53 (m, 3H), 2.39 - 2.30 (m, 1H), 2.08 (s, 6H), 1.94 - 1.81 (m, 1H), 1.69 - 1.57 (m, 1H). LCMS (Method 20): t*_{R}* 0.81 m/z 318 [M+H]⁺ |

### Step 2; 3-(morpholinomethyl)-5-(trifluoromethyl)aniline - Intermediate 5

To palladium (10% on carbon) (10 wt%) was added a solution of Intermediate 6 (153 mg, 0.527 mmol) in ethanol (5 ml). The flask was evacuated and purged with argon (x3) then evacuated and purged with hydrogen (x3). The reaction mixture was stirred at room temperature until LCMS indicated consumption of starting material. The reaction mixture was diluted with DCM and filtered through Celite. The filter cake was washed with DCM and the combined filtrates were concentrated under vacuum to give the title compound (134 mg, 98%).

¹H NMR (400 MHz, CDCl3) δ 6.95 (s, 1H), 6.84 (s, 2H), 6.79 (s, 1H), 3.82 (s, 2H), 3.74 - 3.69 (m, 4H), 3.44 - 3.43 (m, 2H), 2.46 - 2.43 (m, 4H).

The following compounds were prepared as described for Intermediate 5, step 2, starting from the suitable corresponding Intermediate 7 and 8, previously described in the step 1.

| **Int. N°** | **Chemical name** | **Structure** | **Amount of nitro derivative** | **Amount of product (yield)** | **Purification Method** | **Analytical data** |
|---|---|---|---|---|---|---|
| 9 | 3-((dimethylami no)methyl)-5-(trifluorometh yl)aniline | 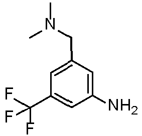 | 0.084 g | 0.067 g (Y=91%) | used without further purification | ¹H NMR (400 MHz, CDCl₃) δ 6.93 (s, 1H), 6.82 (s, 1H), 6.80 (s, 1H), 3.82 (s, 2H), 3.36 (s, 2H), 2.24 - 2.24 (m, 6H) LCMS (Method B) t*_{R}* = 1.26 min, m/z 219 [M+H]⁺ |
| 10 | 4-((dimethylami no)methyl)-3-(trifluorometh yl)aniline | 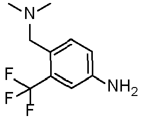 | 0.13 g | 104 mg (Y=90%) | used without further purification | ¹H NMR (400 MHz, CDCl₃) δ 7.45 (d, J=8.5 Hz, 1H), 6.92 (d, J=2.5 Hz, 1H), 6.81 (dd, J=2.4, 8.3 Hz, 1H), 3.78 (s, 2H), 3.45 (s, 2H), 2.24 (s, 6H) |
| | | | | | | LCMS (Method B) t*_{R}* = 1.26 min, m/z 174 [M+H]⁺ |
| 45 | (R)-1-(3-amino-5-(trifluoromethyl )benzyl)-N,N-dimethylpyrrol idin-3-amine | 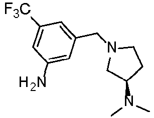 | 0.78 g | 0.71 g (Y=99%) | used without further purification | ¹H NMR (400 MHz, CDCl₃) δ 6.94 (s, 1H), 6.84 (s, 1H), 6.77 (s, 1H), 3.81 (s, 2H), 3.58 (d, J=13.2 Hz, 1H), 3.50 (d, J=13.3 Hz, 1H), 2.83 - 2.69 (m, 3H), 2.53 - 2.45 (m, 1H), 2.31 (dd, J=6.8, 8.3 Hz, 1H), 2.20 (s, 6H), 2.05 - 1.95 (m, 1H), 1.77 - 1.68 (m, 1H) |
| | | | | | | LCMS (Method 21) t*_{R}*: 1.22 min, m/z 288 [M+H]⁺ |

### Preparation of 3-(pyrrolidin-1-ylmethyl)-5-(trifluoromethyl) aniline - Intermediate 11

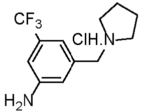

### Step 1; 3-(trifluoromethyl)-5-vinyl-aniline - Intermediate 12

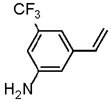

To a mixture of 3-amino-5-bromobenzotrifluoride (2.50 g, 10.4 mmol, 1.00 eq), Vinylboronic acid pinacol ester (2.10 mL, 12.5 mmol, 1.20 eq), XPhos Pd G3 (441 mg, 0.521 mmol, 0.05 eq), XPhos (497 mg, 1.04 mmol, 0.1 eq) and K₃PO₄ (5527 mg, 26.0 mmol, 2.5 eq) was suspended in 1,4-dioxane (45 mL) and water (5 mL). The reaction mixture was sparged with argon for 15 min, and then stirred at 80°C for 1.5 h. The reaction mixture was allowed to cool to room temperature and partitioned between water and EtOAc. The aqueous phase was extracted with 2xEtOAc, and the combined organic phases were washed with saturated NaCl (aq), dried (MgSO₄) and concentrated. The residue was purified by FCC eluting with EtOAc in cyclohexane 0 - 30% to give title compound (1.54 g, 79%).
¹H NMR (400 MHz, CDCl₃) δ 7.04 (s, 1H), 6.85 (s, 1H), 6.79 (s, 1H), 6.64 (dd, *J*=11.0, 17.5 Hz, 1H), 5.75 (d, J=17.5 Hz, 1H), 5.30 (d, J=11.0 Hz, 1H), 3.86 (br s, 2H)

The following Intermediate 46 was prepared as described for Intermediate 12 in step 1, reacting the suitable corresponding bromide. Such procedure may involve minor variations. In some cases, where modification involved chromatographic purification conditions (e.g. prepHPLC or flash chromatography) or catalyst (e.g. XPhos Pd G3 or XPhos Pd G2), such changes were reported in the table.

| **Int. N°** | **Int. chemical name** | **Structure** | **Amount of Bromide** | **Amount of product (yield)** | **Purification Method** | **Analytical data** |
|---|---|---|---|---|---|---|
| 46 | 3-(trifluoromethyl)-4-vinylaniline | 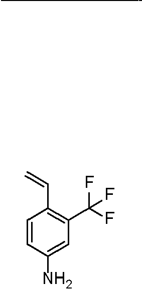 | 2.5 g | 1.12g, (Y=57%) | FCC | ¹H NMR (400 MHz, CDCl₃) δ 7.48 (d, J=8.5 Hz, 1H), 7.04 - 6.93 (m, 1H), 6.91 (d, J=2.5 Hz, 1H), 6.78 (dd, J=2.5, 8.5 Hz, 1H) 5.58 (d, J=17.5 Hz, 1H), 5.22 (dd, J=1.0, 11.0 Hz, 1H), 3.87 (s, 2H) |

### Step 2; tert-butyl N-[3-(trifluoromethyl)-5-vinyl-phenyl]carbamate - Intermediate 13

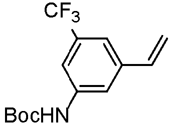

To a mixture of intermediate 12 (500 mg, 2.67 mmol, 1.00 eq) and di-tert-butyl dicarbonate (0.77 mL, 3.34 mmol, 1.25 eq) was added toluene (5 mL). The reaction mixture stirred at 100°C for 19 h then allowed to cool to RT and concentrated under vacuum. The residue was diluted with heptane, and partially concentrated, resulting in formation of a dense precipitate. The suspension was filtered under vacuum to afford the title compound (488 mg, 64%). The filtrate was concentrated under vacuum and the residue was purified by FCC eluting with EtOAc in cyclohexane 0 - 10% to give the title compound (150 mg, 20%). Combined yield = 638 mg, 84%.
¹H NMR (400 MHz, CDCl₃) δ 7.59 - 7.54 (m, 2H), 7.32 (s, 1H), 6.69 (dd, *J=11.0,* 17.5 Hz, 1H), 6.56 (s, 1H), 5.81 (d, J=17.5 Hz, 1H), 5.35 (d, J=11.0 Hz, 1H), 1.53 (s, 9H)

The following Intermediate 47 was prepared as described for Intermediate 13 in step 2.

| **Int. N°** | **Int. chemical name** | **Structure** | **Amount of amine** | **Amount of product (yield)** | **Purification Method** | **Analytical data** |
|---|---|---|---|---|---|---|
| 47 | tert-butyl (3-(trifluoro methyl)-4-vinylphen yl)carbam ate | 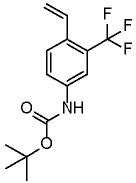 | 1.12 g | 1.91 (>100%) | FCC | ¹H NMR (400 MHz, CDCl₃) δ 7.66 (d, J=2.0 Hz, 1H), 7.59 (d, J=8.5 Hz, 1H), 7.51 (d, J=8.5 Hz, 1H), 7.08 - 6.97 (m, 1H), 6.59 (s, 1H), 5.67 (d, J=17.5 Hz, 1H), 5.35 - 5.29 (m, 1H), 1.53 (s, 9H) |

### Step 3; tert-butyl N-[3-formyl-5-(trifluoromethyl)phenyl]carbamate-Intermediate 14

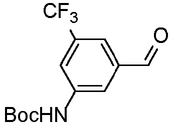

To a solution of Intermediate 13 (488 mg, 1.70 mmol, 1.00 eq) in DCM (20 mL) at -78°C was passed ozone for 30 min at which point a light blue colour was observed. Dimethyl sulfide (0.62 mL, 8.49 mmol, 5.00 eq) was added and the reaction mixture was allowed to warm to room temperature and stirred for 1 h. The reaction mixture was concentrated under vacuum and the residue was purified by column chromatography on silica gel (Ethyl Acetate / Cyclohexane from 0 to 20%) to give the title compound (278 mg, 57%).

¹H NMR (400 MHz, CDCl3) δ 10.02 (s, 1H), 8.06 (s, 1H), 7.98 (s, 1H), 7.80 (s, 1H), 6.76 (s, 1H), 1.54 (s, 9H).

The following Intermediate 48 was prepared as described for Intermediate 14 in step 3.

| **Int. N°** | **Chemical name** | **Structure** | **Amount of reagent** | **Amount of product (yield)** | **Purification Method** | **Analytical data** |
|---|---|---|---|---|---|---|
| 48 | tert-butyl (4-formyl-3-(trifluoromet hyl)phenyl)c arbamate | 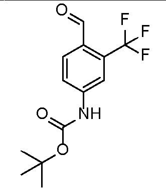 | 1.72 g | 1.19 g, (Y=39%) | FCC | LCMS (Method 20) t*_{R}*= 1.59 min, m/z [M-H]⁻=288 |

### Step 4; tert-butyl N-[3-(pyrrolidin-1-ylmethyl)-5-(trifluoromethyl) phenyl]carbamate - Intermediate 15

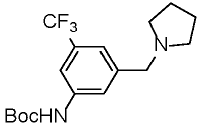

To a solution of Intermediate 14 (140 mg, 0.484 mmol, 1.00 eq) in DCM (3.5 mL) was added Pyrrolidine (440 µL, 0.532 mmol, 1.10 eq), acetic acid (83 µL, 1.45 mmol, 3.00 eq) and titanium(IV) isopropoxide (290 µL, 0.968 mmol, 2.00 eq). The reaction mixture was stirred at room temperature for 1 h. STAB (205 mg, 0.968 mmol, 2.00 eq) was added and the reaction mixture was stirred at room temperature for a further 1.5 h. The reaction mixture was partitioned between saturated NaHCO_{3(aq)} and EtOAc The aqueous phase was re-extracted with 3xEtOAc and the combined organic phases were washed with water, saturated NaCl _{(aq)}, separated and concentrated under vacuum to afford the title compound.

¹H NMR (400 MHz, CDCl3) δ 7.65 (s, 1H), 7.44 (s, 1H), 7.27 (s, 1H), 6.58 (s, 1H), 3.63 (s, 2H), 2.55 - 2.50 (m, 4H), 1.81 - 1.77 (m, 4H), 1.52 (s, 9H).

The following Intermediate 49 was prepared as described for Intermediate 15 in step 4. Intermediate 49 was used in next steps without further purifications.

| **Int. N°** | **Chemical name** | **Structure** | **Amount of reagent** | **Amount of product (yield)** | **Analytical data** |
|---|---|---|---|---|---|
| 49 | tert-butyl (4-(pyrrolidin-1-ylmethyl)-3-(trifluorome thyl)phenyl) carbamate | 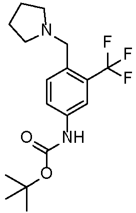 | 0.3 g | 0.3 g (Y=89%) | ¹H NMR (400 MHz, CDCl₃) δ 7.69 (d, J=8.5 Hz, 1H), 7.65 (d, J=2.0 Hz, 1H), 7.49 (m, 1H), 6.54 - 6.52 (m, 1H), 3.74 (s, 2H), 2.58 - 2.51 (m, 4H), 1.82 - 1.77 (m, 4H), 1.52 (s, 9H) |

### Step 5; 3-(pyrrolidin-1-ylmethyl)-5-(trifluoromethyl)aniline - Intermediate 11

To a solution of intermediate 15 (186 mg, 0.540 mmol, 1.0 eq) in MeOH (3.0 ml) was added 4 M HCl in dioxane (0.41 ml, 1.62 mmol, 3.0 eq). The reaction mixture was stirred at room temperature for 2 h. A further portion of 4 M HCl in dioxane (0.41 mL, 1.62 mmol, 3.00 eq) was added and the reaction mixture was stirred at room temperature for a further 4 h. 4 M HCl in dioxane (0.41 mL, 1.62 mmol, 3.00 eq) was added and the reaction mixture was stirred for a further 12 h. The reaction mixture was concentrated under vacuum to give the HCl salt of the title compound (140 mg, 90%).
¹H NMR (400 MHz, DMSO) δ 10.64 (s, 1H), 7.12 (s, 1H), 6.98 (s, 1H), 6.96 (s, 1H), 4.27 (d, J=6.0 Hz, 2H), 3.37 - 3.31 (m, 2H), 3.07 - 2.98 (m, 2H), 2.03 - 1.96 (m, 2H), 1.91 - 1.86 (m, 2H)

The following intermediate 50 was prepared as described for Intermediate 11 in step 5.

| **Int. N°** | **Chemical name** | **Structure** | **Amount of reagent** | **Amount of product (yield)** | **Analytical data** |
|---|---|---|---|---|---|
| 50 | 4-(pyrrolidin-1-ylmethyl)-3-(trifluoromet hyl)aniline | 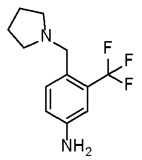 | 0.31 g | 0.28 g. HCl salt (Y>100%) | ¹H NMR (400 MHz, DMSO) δ 10.34 (s, 1H), 7.66 (d, J=9.0 Hz, 1H), 6.98 (d, J=2.5 Hz, 1H), 6.87 (dd, J=2.0, 8.5 Hz, 1H), 4.28 (d, 2H), 3.43 - 3.36 (m, 2H), 3.11 - 3.02 (m, 2H), 2.03 - 1.88 (m, 4H) |

### Preparation of 4-((tetrahydrofuran-3-yl)oxy)-3-(trifluoromethyl)aniline - Intermediate 16

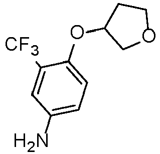

### Step 1; 3-(4-nitro-2-(trifluoromethyl)phenoxy)tetrahydrofuran - Intermediate 17

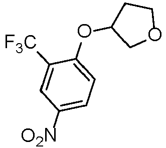

Sodium hydride (60% in mineral oil, 158 mg, 6.60 mmol) was added to a stirred solution of 3-hydroxytetrahydrofuran (0.53 g, 6.00 mmol) in acetonitrile (13 ml) under an inert atmosphere. The reaction mixture was stirred for 1h then the 2-fluoro-5-nitrobenzotrifluoride (0.69 mL, 5.00 mmol) was added in one portion. The reaction mixture was stirred at room temperature until LCMS indicated consumption of starting material. The reaction mixture was partitioned between water and DCM, and the aqueous phase was re-extracted with DCM (x2). The combined organic phases were separated and the concentrated under vacuum to give the title compound (1.52g, 99%).
¹H NMR (400 MHz, CDCl₃) δ 8.52 (d, J=3.0 Hz, 1H), 8.42 (dd, J=3.0, 9.0 Hz, 1H), 7.03 (d, *J*=9.0 Hz, 1H), 5.16 - 5.12 (m, 1H), 4.15 - 4.11 (m, 1H), 4.03 - 3.97 (m, 3H), 2.35 - 2.19 (m, 2H)

The following compound was prepared as described for intermediate Intermediate 17 step 1, starting from the suitable corresponding alcohol.

| **Int. N°** | **Chemical name** | **Structure** | **Amount** | **Product amount (yield)** | **Purification method** | **Analytical data** |
|---|---|---|---|---|---|---|
| 18 | 1-(2-(4-nitro-2- (trifluoro methyl)phen oxy)ethyl)py rrolidine | 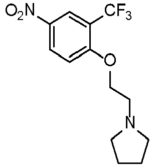 | 0.69 ml | 1.42 g (Y=94%) | No further purification | ¹H NMR (400 MHz, CDCl₃) δ 8.50 (1H, d, *J*=2.5 Hz), 8.41 (1H, dd, J=3.0, 9.0 Hz), 7.10 (1H, d, *J*=9.0 Hz), 4.32 (2H, t, *J*=6.0 Hz), 2.99 (2H, t, *J*=6.0 Hz), 2.68 - 2.62 (4H, m), 1.81 (4H, m). LCMS (Method 20 t*_{R}* = 0.81 min, m/z (M+1)⁺ =305 |

### Step 2; 4-((tetrahydrofuran-3-yl)oxy)-3-(trifluoromethyl)aniline-Intermediate 16

The required Intermediate 17 (1.52 g, 5.48 mmol) and iron powder (1.53 g, 27.4 mmol) were combined in acetic acid (18 ml) and methyl alcohol (18 ml). The reaction mixture was stirred at 50°C until LCMS indicated consumption of starting material. The reaction mixture was allowed to cool to room temperature and concentrated under vacuum. The residue was partitioned between DCM and saturated Na₂CO₃ (aq). The combined organic phases were separated and concentrated in vacuum. The residue was loaded onto an Isolute SCX cartridge, washed with DCM/MeOH, then released with 2M NH₃/MeOH. The eluate was concentrated to give the title compound (1.10 g, 75%).
¹H NMR (400 MHz, CDCl₃) δ 6.91 (d, *J*=2.0 Hz, 1H), 6.79-6.79 (m, 2H), 4.92 - 4.87 (m, 1H), 4.04 - 3.90 (m, 4H), 3.56 (s, 2H), 2.18 - 2.10 (m, 2H)

The following compound was prepared via reduction of nitro residue as described for Intermediate 16, starting from the suitable corresponding nitro derivative.

| **Int. N°** | **Chemical name** | **Structure** | **Amount nitro derivative** | **Amount product (yield)** | **Purification Method** | **Analytical data** |
|---|---|---|---|---|---|---|
| 19 | 4-(2-(pyrrolidin-1-yl) ethoxy)-3-(trifluoromet hyl)aniline | 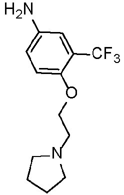 | Intermediate 18: 1.5 g | 1.13 g (Y=83%) | SCX cartridge | ¹H NMR (400 MHz, CDCl₃) δ 6.91 - 6.83 (m, 2H), 6.78 (dd, *J*=3.0, 9.0 Hz, 1H), 4.12 (t, *J*=6.0 Hz, 2H), 3.50 (br.s, 2H), 2.93 (t, J=6.0 Hz, 2H), 2.70 - 2.64 (m, 4H), 1.81 (m, 4H). LCMS (Method 22 t*_{R}* = 1.20 min, m/z (M+1)⁺ = 275 |

### Preparation of 3-(2-morpholinoethoxy)-5-(trifluoromethyl) aniline - Intermediate 20

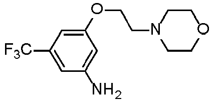

### Step 1; 4-(2-(3-nitro-5-(trifluoromethyl)phenoxy)ethyl)morpholine - Intermediate 21

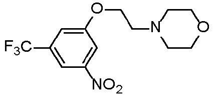

To a solution of 3-nitro-5-(trifluoromethyl)phenol (480.5 mg, 2.32 mmol) in N,N-dimethylformamide (12 ml) was added 4-(2-chloroethyl)morpholine hydrochloride (431 mg, 2.32 mmol) (1.20 eq) followed by cesium carbonate (1.5 g, 4.64 mmol). The mixture was stirred at 80°C until LCMS indicated consumption of starting material. The reaction mixture was diluted with water and extracted with DCM (x2). The organic phases were combined and concentrated.

The residue was purified by FCC eluting with EtOAc in cyclohexane 0 - 100% to give the title compound (0.6 g, 97%).

¹H NMR (400 MHz, DMSO) δ 8.05 (d, J=2.5 Hz, 2H), 7.81 (s, 1H), 4.34 (dd, J=5.6, 5.6 Hz, 2H), 3.58 (dd, J=4.6, 4.6 Hz, 4H), 2.74 (dd, J=5.6, 5.6 Hz, 2H), 2.52 (s, 4H), 2.51 (ddd, J=5.5, 5.5, 4.3 Hz, 4H).

The following Intermediate 51 was prepared as described for Intermediate 21 in step 1.

| **Int. N°** | **Chemical name** | **Structure** | **Amount derivative** | **Amount product (yield)** | **Purification Method** | **Analytical data** |
|---|---|---|---|---|---|---|
| 51 | 1-(2-(3-nitro-5-(trifluoromet hyl)phenoxy) ethyl)pyrroli dine | 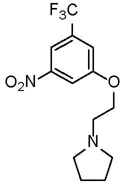 | 0.26 g | 0.36 g (Y=83%) | No further purification | ¹H NMR (400 MHz, CDCl₃) δ 8.08 (s, 1H), 7.94 (t, J=2.1 Hz, 1H), 7.51 - 7.49 (m, 1H), 4.23 (t, J=5.7 Hz, 2H), 2.96 (t, J=5.7 Hz, 2H), 2.67 - 2.62 (m, 4H), 1.86 - 1.81 (m, 4H). |

### Step 2; 3-(2-morpholinoethoxy)-5-(trifluoromethyl)aniline - Intermediate 20

To a solution of Intermediate 21 (100 mg, 0.312 mmol) in ethanol (3 ml) under one atmosphere of nitrogen was sequentially added 1-methyl-1,4-cyclohexadiene (881 mg, 9.36 mmol) and palladium on carbon (10%) (33 mg, 0.312 mmol) and the reaction mixture was stirred at 80°C until LCMS indicated consumption of starting material. The reaction mixture was allowed to cool to room temperature and filtered through a pad of Celite, which was washed with ethanol and ethyl acetate. The filtrate was concentrated under reduced pressure to give title compound (84 mg, 93%).

¹H NMR (400 MHz, CDCl3) δ 6.52 (d, J=9.5 Hz, 2H), 6.36 (t, J=2.0 Hz, 1H), 4.09 (t, J=5.6 Hz, 2H), 3.83 (s, 2H), 3.75 - 3.72 (m, 4H), 2.79 (t, J=5.7 Hz, 2H), 2.59 - 2.55 (m, 4H).

The following Intermediate 52 was prepared as described for Intermediate 20 in step 2.

| **Int. N°** | **Int. chemical name** | **Structure** | **Amount nitro derivative** | **Amount product (yield)** | **Purification Method** | **Analytical data** |
|---|---|---|---|---|---|---|
| 52 | 3-(2-(pyrrolidin-1-yl)ethoxy)-5-(trifluorometh yl)aniline | 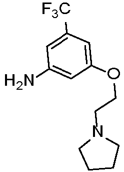 | 0.36 g | 0.31 g (Y=90%) | No further purification | ¹H NMR (400 MHz, CDCl₃) δ 6.55 (s, 1H), 6.50 (s, 1H), 6.37 (d, J=2.0 Hz, 1H), 4.10 - 4.06 (m, 2H), 3.81 (s, 2H), 2.91 - 2.86 (m, 2H), 2.64 - 2.58 (m, 4H), 1.83 - 1.79 (m, 4H). |

### Preparation of 3-((dimethylamino)methyl)-1H-pyrrolo[2,3-b]pyridine-5-carbaldehyde

### Intermediate 53

### Step 1; 3-((dimethylamino)methyl)-1H-pyrrolo[2,3-b]pyridine-5-carbaldehyde

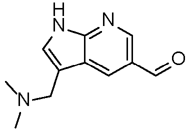

1H-Pyrrolo[2,3-b]pyridine-5-carbaldehyde (100 mg, 0.684 mmol, 1.00 eq) and N,N-Dimethylmethyleneiminium iodide (190 mg, 1.03 mmol, 1.50 eq) were combined in acetonitrile (5.00 mL) and heated with stirring at 80 °C overnight. The reaction mixture was concentrated in vacuo. The residue was dissolved in water (20.00mL), pH adjusted to 12 with 1N NaOH and extracted with DCM. The combined organic phases were filtered through a hydrophobic frit and the solvent was concentrated in vacuo. The resulting material was washed with 1: 1 cyclohexane/diethyl ether and filtered to give the title compound (64 mg, 46%).
¹H NMR (400 MHz, CDCl3) d 10.14 (s, 1H), 10.05 - 10.05 (m, 1H), 8.84 (s, 1H), 8.59 (s, 1H), 7.39 (s, 1H), 3.66 (s, 2H), 2.30 (s, 6H)

### Preparation of 3-bromo-1Hpyrazolo[3,4-b]pyridine-5-carbaldehyde Intermediate 54

### Step 1; 3-bromo-1H-pyrazolo[3,4-b]pyridine-5-carbaldehyde

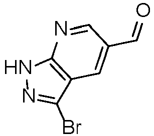

To a solution of 1*H*-pyrazolo[3,4-*b*]pyridine-5-carbaldehyde (650 mg, 4.42 mmol, 1.00 eq) in N,N-dimethylformamide (20 mL) was added N-Bromosuccinimide (983 mg, 5.52 mmol, 1.25 eq) at 20°C. The reaction mixture was stirred at room temperature for 18 h. The mixture was diluted with sat. aq. NH₄Cl (100mL) and extracted with EtOAc. The combined organic phases were dried over magnesium sulfate, filtered and concentrated in vacuo. The material was triturated with water and dried in vacuo to give the title compound (1.02 g, 99.2% yield)
¹H NMR (400 MHz, DMSO) δ 14.61 - 14.58 (m, 1H), 10.23 (s, 1H), 9.13 (d, J=1.8 Hz, 1H), 8.73 (d, J=1.5 Hz, 1H)

### Preparation of 2-((4-methylpiperazin-1-yl)methyl)-6-(trifluoromethyl)pyridin-4-amine

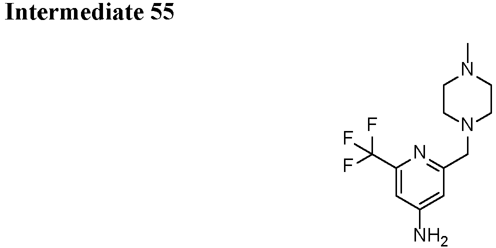

### Step 1; 4-amino-N-methoxy-N-methyl-6-(trifluoromethyl)picolinamide

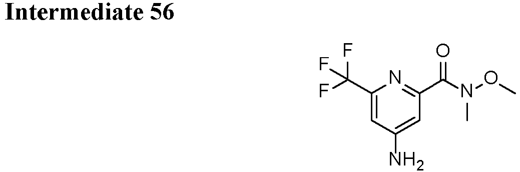

To a mixture of 4-amino-6-(trifluoromethyl)picolinic acid (445 mg, 2.16 mmol) and HATU (1.6 g 4.32 mmol) in DMF (0.1M concentration) was added *N,N-*diisopropylethylamine (2.9 mL, 17.98 mmol). The reaction mixture was stirred at room temperature for 15 minutes then N,O-dimethylhydroxylamine hydrochloride (232 mg, 2.37 mmol) was added. The reaction mixture was stirred at rt until LCMS indicated consumption of starting material and then concentrated. Purification by column chromatography on silica gel (80 g cartridge, 0-50% EtOAc in cyclohexane (+0.1% NEt₃)) gave the title compound (369 mg, 68%).
¹H NMR (400 MHz, DMSO) δ 6.96 (d, J=2.1 Hz, 1H), 6.84 - 6.78 (m, 3H), 3.67 (s, 3H), 3.24 (s, 3H)

### Step 2; 4-amino-6-(trifluoromethyl)picolinaldehyde

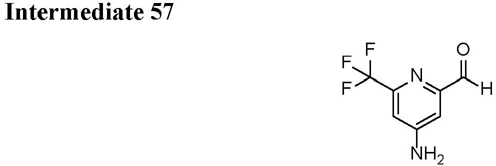

To a stirred solution of Intermediate 56 (308 mg, 1.24 mmol) in THF (4.82 mL) cooled in an ice/water bath was added LiAlH₄ (2M in THF, 0.62 mL, 1.24 mmol) dropwise maintaining the internal temperature below 6 °C. The reaction mixture was stirred for 1 h and diluted with anhydrous diethyl ether (5 mL). Water (47 µL), 15% NaOH_{(aq)} (47 µL) and water (141 µL) were added, the reaction mixture was allowed to warm to room temperature and stirred for 15 min. Anhydrous MgSO₄ was added, the reaction mixture was stirred for 15 min, filtered and the filtrate was concentrated in vacuo to give Intermediate 57 (252 mg, >100%), which was used in the next step without purification.
LCMS (Method 21) t*_{R}*: 0.95 min, m/z (M+1); 191

### Step 3; 2-((4-methylpiperazin-1-yl)methyl)-6-(trifluoromethyl)pyridin-4-amine

### Intermediate 55

To a solution of Intermediate 57 (126 mg, 0.663 mmol) in DCM (0.1 M concentration) was added methylpiperazine (0.074 mL, 0.663 mmol) titanium(IV) isopropoxide (376.87 mg 1.326 mmol, 2.00 eq) and acetic acid (114 uL, 1.988 mmol eq). The reaction mixture was stirred at room temperature for 1 h. Sodium triacetoxyborohydride (281 mg, 1.325 mmol) was added and the reaction mixture was stirred at room temperature until LCMS indicated consumption of starting material. Purification by column chromatography on silica gel (0-10% 2M NH₃/MeOH in DCM) gave the title compound (85 mg, 46%).

Following intermediates were prepared as described for **Intermediate 55** in step 3, starting from **Intermediate 57** using the appropriate commercially available amine.

| **Int. N°** | **Chemical name** | **Structure** | **Amount reagent** | **Amount product (yield)** | **Purification Method** | **Analytical data** |
|---|---|---|---|---|---|---|
| 58 | 2-((dimethylam ino)methyl)-6-(trifluoromet hyl)pyridin-4-amine | 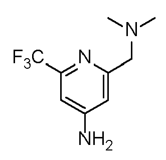 | 0.126 g | 0.065 g (Y=44%) | FCC | ¹H NMR (400 MHz, DMSO) δ 6.78 - 6.77 (m, 2H), 6.49 (s, 2H), 3.34 (s, 2H), 2.18 (s, 6H). |
| 59 | (R)-2-((3-(dimethylami no)pyrrolidin -1-yl)methyl)-6-(trifluoromet hyl)pyridin-4-amine | 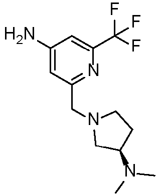 | 0.25 g | 0.37 (Y=98%) | No further purification | LCMS (Method 21) t*_{R}*: 1.00 min, m/z (M+H)⁺=289) |

### Preparation of 3-amino-N-[2-(dimethylamino)ethyl]-5-(trifluoromethyl)benzamide

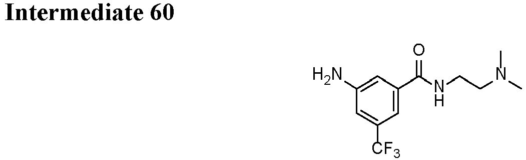

### Step 1: 3-amino-N-[2-(dimethylamino)ethyl]-5-(trifluoromethyl)benzamide Intermediate 60

To a mixture of the required carboxylic acid (0.25 g, 1.21 mmol ) and HATU (0.9, 2.43 mmol) in DMF (0.1M concentration) was added *N*,*N*-diisopropylethylamine (639 µL, 3.66). The reaction mixture was stirred at rt for 15 mins then the N,N-Dimethylethylenediamine (0.13 g mg, 1.46 mmol), was added. The reaction mixture was stirred at room temperature until LCMS indicated consumption of starting material and then concentrated. The residue was purified by column chromatography on silica gel (12g cartridge, 0 - 10% 2M NH₃/MeOH in DCM) to give the title compound (237 mg, 71%).

¹H NMR (300 MHz, DMSO) δ 8.51 (t, J=5.5 Hz, 1H), 7.28 - 7.25 (m, 1H), 7.25 - 7.21 (m, 1H), 6.99 - 6.96 (m, 1H), 5.78 (s, 2H), 3.44 (q, J=6.3 Hz, assumed 2H, overlapping with water peak), 2.75 (t, J=6.2 Hz, 2H), 2.46 (s, 6H)

The following intermediate was prepared as described for Intermediate 60, varying the corresponding commercially available amine.

| **Int. N°** | **chemical name** | **Structure** | **Amount of benzoic acid** | **Amount product (yield)** | **Purification Method** | **Analytical data** |
|---|---|---|---|---|---|---|
| 61 | (3-amino-5-(trifluoromet hyl)phenyl)( 4-methylpipera zin-1-yl)methanon e | 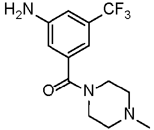 | 0.25 g | 0.18 g (Y=51%) | FCC | ¹H NMR (300 MHz, DMSO) δ 6.90 (s, 1H), 6.76 (s, 1H), 6.70 (s, 1H), 5.80 (s, 2H), 3.57 (br s, 4H), 2.31 (br s, 4H), 2.20 (3H, s) |

### PREPARATIONS OF EXAMPLES

### Example 1; Preparation of compound 1-(imidazo[1,2-a]pyridin-3-ylmethyl)-N-(3-(4-methyl-1H-imidazol-1-yl)-5-(trifluoromethyl)phenyl) indoline-6-carboxamide

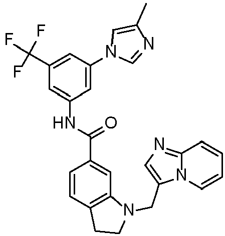

### Example 1

### Step 1; tert-butyl 6-((3-(4-methyl-1H-imidazol-1-yl)-5-(trifluoromethyl) phenyl)carbamoyl)indoline-1-carboxylate - Intermediate 22

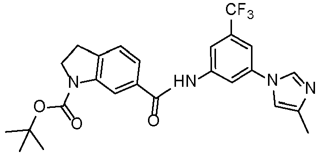

1-(tert-butoxycarbonyl)indoline-6-carboxylic acid (1 g, 3.80 mmol) and 3-(4-methyl-1H-imidazol-1-yl)-5-(trifluoromethyl)aniline (0.916 g, 3.80 mmol) were dissolved in DCM (2 ml). DIPEA (1.990 ml, 11.39 mmol) and 50% T3P in ethyl acetate (4.48 ml, 7.60 mmol) were added. Reaction mixture was stirred at 20 °C over 20 hr. Reaction mixture was diluted with DCM (10 ml), next water (5ml) was added. Phases were separated, organic layer was washed with citric acid (5% aq.sol., 2x10 ml), dried over Na₂SO₄, filtered and concentrated to dryness. Crude material was purified via column chromatography eluting with DCM/MeOH, starting from 100% of DCM to 5% of MeOH in DCM. 200 mg of pure desired product were obtained. 1.4 g of mixture of desired product and starting material (acid) was obtained. This material was dissolved in DCM (100 ml) and washed Na₂CO₃ (3x50 ml) and brine (100 ml) to remove residual of acid. Organic layer was dried over Na₂SO₄, filtered, evaporated to dryness giving the title compound (1.01 g, 55 %).

¹H NMR (300 MHz, DMSO-d6) δ 10.66 (s, 1H), 8.28 (d, J = 2.4 Hz, 1H), 8.20 (d, J = 1.4 Hz, 1H), 8.14 (s, 1H), 7.71 (d, J = 2.1 Hz, 1H), 7.58 (dd, J = 7.7, 1.7 Hz, 1H), 7.49 (t, J = 1.3 Hz, 1H), 7.37 (d, J = 7.8 Hz, 1H), 3.98 (t, J = 8.7 Hz, 2H), 3.15 (t, J = 8.7 Hz, 2H), 2.18 (d, J = 1.0 Hz, 3H), 1.53 (s, 8H).

### Step 2; N-(3-(4-methyl-1H-imidazol-1-yl)-5-(trifluoromethyl)phenyl) indoline-6-carboxamide - Intermediate 23

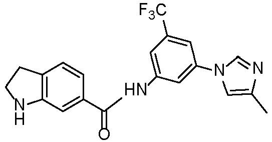

Intermediate 22 (1.01 g, 2.076 mmol) was dissolved in DCM (5 ml) then trifluoroacetic acid (1.440 ml, 18.68 mmol) was added. Reaction mixture was stirred at RT overnight. Reaction mixture was diluted with DCM (10 ml), NaHCO₃ (sat. aq. sol.) was added. Mixture was left for stirring for 30 minutes at room temperature (bubbling was observed). Layers were partitioned. Organic layer was washed with NaHCO₃, dried over Na₂SO₄ and evaporated under vacuum. Material was used further without additional purification. The title compound, as a free base, was obtained (900 mg, 100 %).

¹H NMR (300 MHz, DMSO-d6) δ 10.49 (s, 1H), 8.31 (dd, J = 4.2, 2.1 Hz, 2H), 8.17 (d, J = 1.7 Hz, 1H), 7.71 (d, J = 1.8 Hz, 1H), 7.53 (d, J = 1.4 Hz, 1H), 7.25 - 7.13 (m, 2H), 7.06 (d, J = 1.3 Hz, 1H), 3.47 (d, J = 8.5 Hz, 3H), 2.98 (t, J = 8.5 Hz, 2H), 2.20 (d, J = 1.0 Hz, 3H).

### Step 3; 1-(imidazo[1,2-a]pyridin-3-ylmethyl)-N-(3-(4-methyl-1H-imidazol-1-yl)-5-(trifluoromethyl)phenyl)indoline-6-carboxamide (Example 1)

N-(3-(4-methyl-1H-imidazol-1-yl)-5-(trifluoromethyl)phenyl)indoline-6-carboxamide (0.1 g, 0.259 mmol) was dissolved in DCM (2 ml) then imidazo[1,2-a]pyridine-3-carbaldehyde (0.038 g, 0.259 mmol), NaCNBH₃ (0.024 g, 0.388 mmol) and AcOH (0.030 ml, 0.518 mmol) were added. Reaction mixture was stirred 18 hr at 20 °C. Reaction mixture was evaporated, then diluted with DCM (5 ml), next water (5 ml) was added. Layers were partitioned, organic layer was washed with K₂CO₃ (aq.sat.sol.) 2x10 ml, dried over Na₂SO₄, filtered and concentrated under reduced pressure. Crude material was purified via preparative HPLC eluting with ACN+0.1%NH₃, H2O+0.1%NH₃ to provide the title compound (28 mg, 21 %).

| **Example No.** | **Analytical data** |
|---|---|
| 1 | ¹H NMR (300 MHz, DMSO-d6) δ 10.52 (s, 1H), 8.39 (dt, J = 6.9, 1.2 Hz, 1H), 8.31 (t, J = 1.9 Hz, 1H), 8.21 (d, J = 1.4 Hz, 1H), 8.15 (s, 1H), 7.73 (s, 1H), 7.66 - 7.56 (m, 2H), 7.50 (t, J = 1.3 Hz, 1H), 7.42 (d, J = 1.5 Hz, 1H), 7.35 (dd, J = 7.6, 1.5 Hz, 1H), 7.33 - 7.27 (m, 1H), 7.25 (d, J = 7.8 Hz, 1H), 6.98 (td, J = 6.8, 1.2 Hz, 1H), 4.73 (s, 2H), 3.23 (t, J = 8.2 Hz, 1H), 3.17 (d, J = 5.3 Hz, 1H), 2.94 (t, J = 8.3 Hz, 2H), 2.19 (d, J = 1.0 Hz, 3H). |
| | LC-MS (ESI): (Method 3) t*_{R}*= 3.10 min; m/z (M+1)=517.02 |

The following compounds were prepared via reductive amination as described for Example 1, step 1-3, starting from the suitable corresponding, commercially available carbaldehyde in step 3; if STAB instead of NaCNBH₃, as reductive agent, was used, it is indicated in the table together with equivalents (eq.).

| **Example No** | **Structure** | **Amount SM** | **Product amount (yield)** | **Purification Method** | **Analytical data** |
|---|---|---|---|---|---|
| 2 | 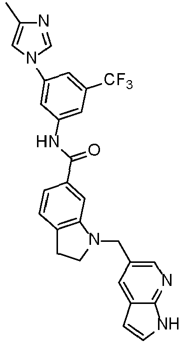 | 0.10 g | 0.027 g (Y=20%) | prep HPLC | ¹H NMR (300 MHz, DMSO-d6) δ 11.64 (s, 1H), 10.50 (s, 1H), 8.31 (d, J = 2.1 Hz, 1H), 8.25 (d, J = 2.0 Hz, 1H), 8.21 (d, J = 1.4 Hz, 1H), 8.15 (s, 1H), 7.94 (d, J = 2.0 Hz, 1H), 7.71 (s, 1H), 7.54 - 7.40 (m, 2H), 7.33 - 7.24 (m, 2H), 7.21 (d, J = 7.4 Hz, 1H), 6.43 (d, J = 3.4 Hz, 1H), 4.48 (s, 2H), 2.98 (t, J = 8.3 Hz, 2H), 2.18 (d, J = 1.0 Hz, 3H). |
| | | | | | LC-MS (ESI): (Method 1) |
| | | | | | t*_{R}* =1.99 min; m/z (M+1)=517.6 |
| 3 | 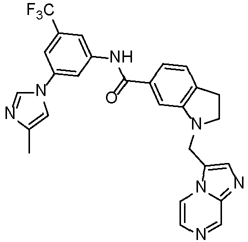 | 0.10 g | 0.050 g (Y=3 7%) | FCC | ¹H NMR (300 MHz, DMSO-d6) δ 10.51 (s, 1H), 9.09 (d, J = 1.5 Hz, 1H), 8.48 (dd, J = 4.7, 1.5 Hz, 1H), 8.30 (s, 1H), 8.25 - 8.09 (m, 2H), 7.96 (d, J = 4.6 Hz, 1H), 7.85 (s, 1H), 7.72 (s, 1H), 7.48 (s, 1H), 7.43 - 7.30 (m, 2H), 7.24 (d, J = 7.6 Hz, 1H), 4.79 (s, 2H), 2.95 (t, J = 8.2 Hz, 2H), 2.18 (s, 3H). |
| | | | | | LC-MS (ESI): (Method 1) |
| | | | | | tR =1.79 min; m/z (M+1)= 518.15 |
| 4 | 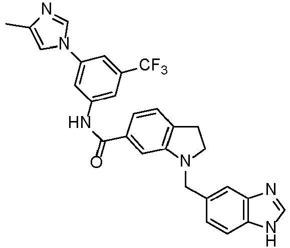 | 0.10 g 1 eq. STAB | 0.040 g (Y=30%) | prep HPLC | ¹H NMR (300 MHz, DMSO-d6) δ 12.39 (s, 1H), 10.46 (s, 1H), 8.28 (d, J = 2.0 Hz, 1H), 8.23 - 8.16 (m, 2H), 8.13 (s, 1H), 7.70 (s, 1H), 7.62 (s, 1H), 7.54 - 7.43 (m, 2H), 7.28 (dd, J = 7.8, 1.4 Hz, 1H), 7.21 (dd, J = 4.6, 3.0 Hz, 3H), 4.49 (s, 2H), 3.38 (d, J = 8.6 Hz, 1H), 2.99 (t, J = 8.4 Hz, 2H), 2.17 (d, J = 1.0 Hz, 3H). |
| | | | | | LC-MS (ESI): (Method 4) |
| | | | | | t*_{R}* =2.037 min; m/z (M+1)= 517.07 |
| 5 | 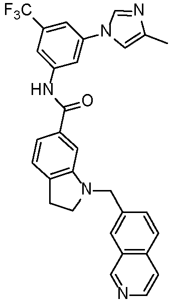 | 0.10 1 eq. STAB | 0.037 g (Y=27%) | FCC | ¹H NMR (300 MHz, DMSO-d6) δ 10.50 (s, 1H), 9.59 (s, 1H), 8.56 (d, J = 5.7 Hz, 1H), 8.28 (s, 1H), 8.19 (d, J = 1.5 Hz, 1H), 8.13 (s, 1H), 7.95 (d, J = 8.2 Hz, 1H), 7.91 - 7.82 (m, 1H), 7.81 - 7.72 (m, 1H), 7.69 (d, J = 8.3 Hz, 2H), 7.51 - 7.44 (m, 1H), 7.34 (d, J = 6.9 Hz, 2H), 7.26 (d, J = 7.7 Hz, 1H), 4.93 (s, 2H), 4.03 (q, J = 7.1 Hz, 1H), 2.99 (t, J = 8.3 Hz, 2H), 2.17 (d, J = 1.0 Hz, 3H). |
| | | | | | LC-MS (ESI): (Method 7) |
| | | | | | t*_{R}* =1.98 min; m/z (M+1)= 528.16 |
| 6 | 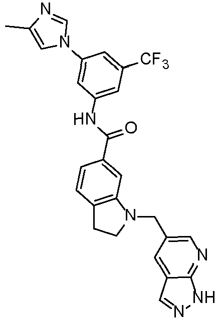 | 0.10 4 eq.STAB | 0.084 g (Y=62%) | FCC | ¹H NMR (300 MHz, DMSO-d6) δ 13.65 (s, 1H), 10.49 (s, 1H), 8.57 (d, J = 2.0 Hz, 1H), 8.29 (s, 1H), 8.23 - 8.18 (m, 2H), 8.14 (s, 2H), 7.71 (s, 1H), 7.49 (t, J = 1.3 Hz, 1H), 7.26 (dt, J = 14.7, 7.4 Hz, 3H), 4.53 (s, 2H), 2.99 (t, J= 8.3 Hz, 2H), 2.18 (d, J = 1.0 Hz, 3H). |
| | | | | | LC-MS (ESI): (Method 1) |
| | | | | | t*_{R}* =1.99 min; m/z (M+1)= 518.16 |
| 7 | 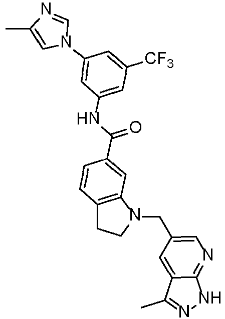 | 0.10 4 eq. STAB | 0.078 g (Y=55.5%) | FCC | ¹H NMR (300 MHz, DMSO-d6) δ 13.21 (s, 1H), 10.49 (s, 1H), 8.53 (d, J = 2.0 Hz, 1H), 8.30 (s, 1H), 8.20 (dd, J = 3.9, 1.7 Hz, 2H), 8.14 (s, 1H), 7.72 (s, 1H), 7.49 (d, J = 1.3 Hz, 1H), 7.33 - 7.20 (m, 3H), 4.51 (s, 2H), 2.99 (t, J = 8.4 Hz, 2H), 2.46 (s, 3H), 2.18 (d, J = 1.0 Hz, 3H). |
| | | | | | LC-MS (ESI): (Method 1) |
| | | | | | t*_{R}* = 2.053 min; m/z (M+1)= 532.14 |
| 8 | 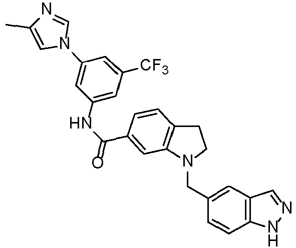 | 0.10 2 eq.STAB | 0.040 g (Y=30%) | prep HPLC | ¹H NMR (300 MHz, DMSO-d6) δ 13.04 (s, 1H), 10.46 (s, 1H), 8.28 (d, J = 2.2 Hz, 1H), 8.19 (d, J = 1.5 Hz, 1H), 8.13 (s, 1H), 8.04 (s, 1H), 7.71 (d, J = 6.9 Hz, 2H), 7.53 (d, J = 8.6 Hz, 1H), 7.48 (t, J = 1.3 Hz, 1H), 7.36 (dd, J = 8.6, 1.5 Hz, 1H), 7.28 (dd, J = 7.6, 1.4 Hz, 1H), 7.25 - 7.18 (m, 2H), 4.46 (s, 2H), 2.99 (t, J = 8.4 Hz, 2H), 2.17 (d, J = 1.0 Hz, 3H). |
| | | | | | LC-MS (ESI): (Method 12) |
| | | | | | t*_{R}* =2.88 min; m/z (M+1)= 576.87 |

### Example 9; Preparation of 1-((1H-pyrrolo[2,3-b]pyridin-5-yl)methyl)-N-(3-((4-methylpiperazin-1-yl)methyl)-5-(trifluoromethyl)phenyl)indoline-6-carboxamide

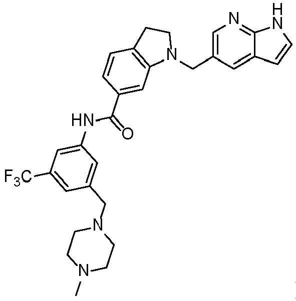

Example 9

### Step 1; tert-butyl 6-((3-((4-methylpiperazin-1-yl)methyl)-5-(trifluoromethyl) phenyl)carbamoyl)indoline-1-carboxylate - Intermediate 24

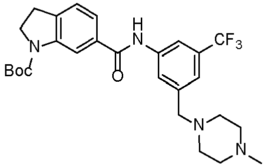

In a dried (heat gun/vacuum/N2 cycles), double-necked 25mL flask, 3-((4-methylpiperazin-1-yl)methyl)-5-(trifluoromethyl)aniline (0.542 g, 1.983 mmol) was dissolved in anhydrous THF (15 ml) and cooled down to -78 °C, then butyllithium (0.793 ml, 1.983 mmol) was added dropwise. The solution was stirred for 15 minutes, then a solution of 1-(tert-butyl) 6-methyl indoline-1,6-dicarboxylate (0.275 g, 0.992 mmol) in anhydrous THF (1 mL) was added dropwise. The mixture was stirred for 16h while temperature raised from -78 °C up to room temperature. Water (50 mL) was added, then extraction with EtOAc (3x10 mL) was done. The organic phase was washed with brine (30mL), dried over Na₂SO₄, filtered and concentrated to dryness. The crude was purified on FCC (Biotage^{®} Sfär KP-Amino D - Duo) eluting system DCM/MeOH, from 100:0 to 90:10 to afford the title compound (518mg, 100%).
¹H NMR (DMSO-d6, 400MHz): δ = 10.48 (s, 1H), 8.15 (s, 1H), 7.99 (s, 1H), 7.57 (d, 1H, J = 7.7 Hz), 7.36-7.32 (m, 2H), 6.76 (s, 1H), 5.51 (s, 2H), 3.97 (t, 2H, J = 8.7 Hz), 3.14 (t, 2H, J = 8.7 Hz), 2.33 (bs, 8H), 2.15 (s, 3H), 1.53 (s, 9H) ppm

The following intermediate 62 was prepared following the same procedure used for the synthesis of Intermediate 24, varying the corresponding commercially available amine.

| **Int. N°** | **Chemical Name** | **Structure** | **Amount reagent** | **Product amount (yield)** | **Purification method** | **Analytical data** |
|---|---|---|---|---|---|---|
| 62 | tert-butyl 6-((3-(trifluorometh yl)phenyl)carb amoyl)indoline -1-carboxylate | 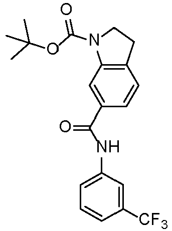 | 1.5g | 1.70 g (Y: 77%) | FCC | 1H NMR (300 MHz, DMSO-d6) δ 10.52 (s, 1H), 8.23 (d, J = 2.0 Hz, 2H), 8.09-8.00 (m, 1H), 7.63 - 7.52 (m, 2H), 7.44 (ddd, J = 7.8, 2.0, 1.0 Hz, 1H), 7.35 (d, J = 7.8 Hz, 1H), 3.97 (t, J = 8.7 Hz, 2H), 3.14 (t, J = 8.6 Hz, 2H), 1.53 (s, 9H). |

### Step 2; N-(3-((4-methylpiperazin-1-yl)methyl)-5-(trifluoromethyl) phenyl) indoline-6-carboxamide - Intermediate 25

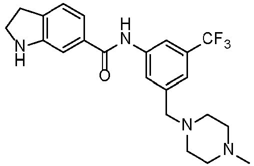

Intermediate 24 (0.514 g, 0.991 mmol) was dissolved in a 3:1 mixture of DCM (12 ml) and 2,2,2-trifluoroacetic acid (3.04 ml, 39.6 mmol). The solution was stirred at room temperature for 1 h. Evaporation of solvents. Crude was diluted with water (20 mL) then pH was adjusted to ca 7-8. Product was extracted with DCM (2x20 mL) via phase separator. The organic phase was then concentrated to dryness to give the title compound (415 mg, 100%).

¹H NMR (DMSO-d₆, 400MHz): δ = 10.25 (s, 1H), 8.13 (s, 1H), 7.96 (s, 1H), 7.28 (s, 1H), 7.15 (d, 1H, J = 7.5 Hz), 7.11 (d, 1H, J = 7.5 Hz), 7.01 (s, 1H), 4.04 (q, 1H, J = 5.1 Hz), 3.49 (s, 2H), 3.44 (t, 2H, J = 8.7 Hz), 2.94 (t, 2H, J = 8.7 Hz), 2.34-2.27 (m, 8H), 2.13 (s, 3H) ppmFollowing Intermediate 63 was prepared following the same procedure reported for Intermediate 25. Modifications of chromatographic purification conditions were reported in the table.

| **Int. N°** | **Chemical name** | **Structure** | **Amount reagent** | **Product amount (yield)** | **Purification method** | **Analytical data** |
|---|---|---|---|---|---|---|
| 63 | N-(3-(trifluoro methyl)ph enyl)indol ine-6-carboxam ide | 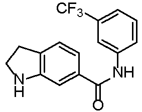 | 1.94 g | 0.37 g (Y=86%) | FCC | ¹H NMR (300 MHz, DMSO-d6) δ 10.33 (s, 1H), 8.25 (d, J = 2.0 Hz, 1H), 8.03 (dt, J = 8.0, 1.3 Hz, 1H), 7.57 (t, J = 8.0 Hz, 1H), 7.41 (ddt, J = 7.8, 1.9, 0.9 Hz, 1H), 7.20 - 7.12 (m, 2H), 7.04 (t, J = 1.0 Hz, 1H), 5.76 (d, J = 2.1 Hz, 1H), 3.48 (td, J = 8.6, 1.9 Hz, 2H), 2.97 (t, J = 8.5 Hz, 2H). |

### Step 3; 1-((1H-pyrrolo[2,3-b]pyridin-5-yl)methyl)-N-(3-((4-methylpiperazin-1-yl)methyl)-5-(trifluoromethyl)phenyl)indoline-6-carboxamide (Example 9)

Intermediate 25 (0.1 g, 0.239 mmol) was dissolved in DCM (2 ml) then 1H-pyrrolo[2,3-b]pyridine-5-carbaldehyde (0.035 g, 0.239 mmol), NaCNBH₃ (0.023 g, 0.358 mmol) and AcOH (0.027 ml, 0.478 mmol) were added. Reaction mixture was stirred overnight at room temperature. Reaction mixture was diluted with DCM (5 ml) and washed with water (5 ml). Organic layer was washed with K₂CO₃ (aq.sat.sol., 2x10 ml), dried over Na₂SO₄, filtered and concentrated under reduced pressure.

Crude material was purified via preparative HPLC eluting with ACN+0.1%FA, H₂O+0.1%FA. Residue was triturated with NaHCO₃ (15% aq. sol.), precipitate was filtered through the Schott funnel, rinsed with water, transferred to the vial and dried on high vacuum overnight at room temperature to provide the title compound (17 mg, 13%).

| **Example No.** | **Analytical data** |
|---|---|
| 9 | ¹H NMR (300 MHz, DMSO-d6) δ 11.63 (s, 1H), 10.32 (s, 1H), 8.25 (d, J = 2.0 Hz, 1H), 8.18 (s, 1H), 8.01 (s, 1H), 7.94 (d, J = 2.0 Hz, 1H), 7.47 (t, J = 2.8 Hz, 1H), 7.33 (s, 1H), 7.28 (d, J = 6.2 Hz, 2H), 7.19 (d, J = 7.7 Hz, 1H), 6.43 (d, J = 3.4 Hz, 1H), 4.47 (s, 2H), 3.53 (s, 2H), 3.31 (s, 2H), 2.96 (t, J = 8.4 Hz, 2H), 2.41 (s, 8H), 2.16 (s, 3H). |
| | LC-MS (ESI): (Method 1) t*_{R}* = 1.91 min; m/z (M+1)= 549.07 |

The following compounds were prepared via reductive amination as described for Example 9, step 1-3, starting from the suitable corresponding, commercially available or synthesized carbaldehyde in step 3 and using either NaCNBH₃ or STAB as reductive agent. In some cases, titanium(IV) isopropoxide were used with reductive agent. These modifications were reported in the table.

| **Example N°** | **Structure** | **Amount SM** | **Product amount (yield)** | **Purification Method** | **Analytical data** |
|---|---|---|---|---|---|
| 10 | 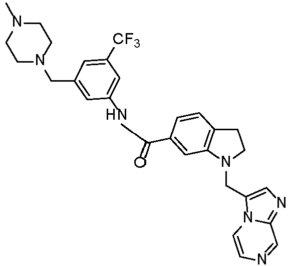 | 0.10g 4 equiv STAB | 0.060 g (Y=46%) | prep HPLC | ¹H NMR (300 MHz, DMSO-d6) δ 10.35 (s, 1H), 9.09 (d, J = 1.5 Hz, 1H), 8.48 (dd, J = 4.7, 1.5 Hz, 1H), 8.18 (s, 1H), 8.00 (s, 1H), 7.96 (d, J = 4.6 Hz, 1H), 7.85 (s, 1H), 7.40 (d, J = 1.5 Hz, 1H), 7.38 - 7.30 (m, 2H), 7.22 (d, J = 7.6 Hz, 1H), 4.78 (s, 2H), 3.53 (s, 2H), 3.25 (t, J = 8.3 Hz, 2H), 2.93 (t, J = 8.2 Hz, 2H), 2.36 (d, J = 19.1 Hz, 7H), 2.15 (s, 3H). |
| | | | | | LC-MS (ESI): (Method 1) t*_{R}* = 1.70 min; m/z (M+1)= 550.19 |
| 11 | 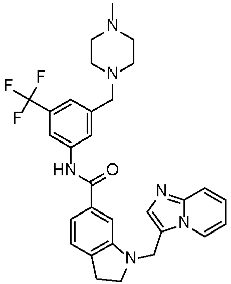 | 0.10 g 1.5 eq. NaCNBH₃ | 0.030 g (Y=23%) | prep HPLC | ¹H NMR (300 MHz, DMSO-d6) δ 10.35 (s, 1H), 8.38 (s, 1H), 8.18 (s, 1H), 8.01 (s, 1H), 7.61 (d, J = 8.8 Hz, 2H), 7.43 (s, 1H), 7.33 (d, J = 6.5 Hz, 2H), 7.27 (d, J = 7.9 Hz, 1H), 7.21 (d, J = 7.6 Hz, 1H), 6.97 (t, J = 6.8 Hz, 1H), 4.71 (s, 2H), 3.53 (s, 2H), 3.20 (t, J = 8.3 Hz, 2H), 2.91 (t, J = 8.1 Hz, 2H), 2.38 (s, 8H), 2.15 (s, 3H). |
| | | | | | LC-MS (ESI): (Method 3) t*_{R}* = 3.06 min; m/z (M+1)= 549.03 |
| 90 | 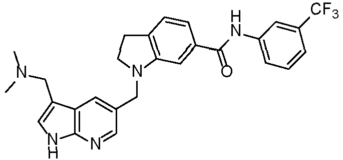 | 0.028 g STAB, Ti(OiPr)₄ Intermediate 53 | 0.022 g (Y= 32%) | prep HPLC | ¹H NMR (400 MHz, CDCl3) d 8.86 (s, 1H), 8.31 (s, 1H), 8.09 - 8.03 (m, 2H), 7.92 (s, 1H), 7.88 (d, J=7.8 Hz, 1H), 7.47 (t, J=7.8, 7.8 Hz, 1H), 7.37 (d, J=7.3 Hz, 1H), 7.24 (m, 1H), 7.17 - 7.12 (m, 2H), 7.09 (s, 1H), 4.43 (s, 2H), 3.61 (s, 2H), 3.39 (t, J=8.3 Hz, 2H), 3.02 (t J=8.3 Hz, 2H), 2.27 (s, 6H) |
| | | | | | LC-MS (ESI): (Method 2) t*_{R}* = 3.75 min; m/z (M+1)= 494.3 |

### Example 12; Preparation of 1-(imidazo[1,2-a]pyridine-3-carbonyl)-N-(3-(4-methyl-1H-imidazol-1-yl)-5-(trifluoromethyl)phenyl)indoline-6-carboxamide

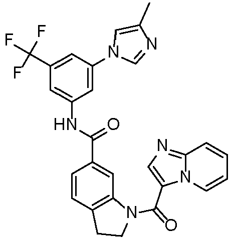

Example 12

### Step 1; 1-(imidazo[1,2-a]pyridine-3-carbonyl)-N-(3-(4-methyl-1H-imidazol-1-yl)-5-(trifluoromethyl)phenyl)indoline-6-carboxamide (Example 12)

Imidazo[1,2-a]pyridine-3-carboxylic acid (0.040 g, 0.246 mmol) was dissolved in DMF (2.5 ml) then TBTU (0.079 g, 0.246 mmol), TEA (0.069 ml, 0.492 mmol) were added. Mixture was stirred ~ 15 min at room temperature, then Intermediate 23 (0.095 g, 0.246 mmol) was added. Reaction mixture was stirred overnight at room temperature.

Reaction mixture was diluted with DCM (5 ml) and water (5 ml). Mixture was stirred 15 minutes at room temperature, then phases were separated, organic layer was washed with water (2x15 ml) and brine (2x5 ml), next dried over Na₂SO₄, filtered and concentrated to dryness under reduced pressure. Crude material has low solubility in organic solvents. Residue was triturated with H₂O overnight, filtered and dried under high vacuum to give the title compound (43 mg, 33%).

| **Example No.** | **Analytical Data** |
|---|---|
| 12 | ¹H NMR (300 MHz, DMSO-d6) δ 10.71 (s, 1H), 9.34 (d, J = 6.9 Hz, 1H), 8.72 (s, 1H), 8.41 (s, 1H), 8.32 (s, 1H), 8.19 (d, J = 15.0 Hz, 2H), 7.89 - 7.64 (m, 3H), 7.53 (d, J = 13.4 Hz, 3H), 7.19 (t, J = 6.8 Hz, 1H), 4.58 (t, J = 8.2 Hz, 2H), 3.27 (s, 2H), 2.18 (s, 3H). |
| | LC-MS (ESI): (Method 1) t*_{R}* = 1.61 min; m/z (M+1)= 530.94 |

The following compound was prepared via amidation as described for Example 12, step 1, starting from the suitable corresponding, commercially available acid in step 1.

| **Example N°** | **Structure** | **Amount SM** | **Product amount (yield)** | **Purification Method** | **Analytical data** |
|---|---|---|---|---|---|
| 13 | 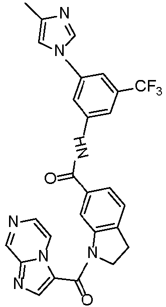 | 0.10 g | 0.062 g (Y=45%) | prep HPLC | ¹H NMR (300 MHz, DMSO-d6) δ 10.74 (s, 1H), 9.30 (d, J = 1.5 Hz, 1H), 9.21 (dd, J = 4.7, 1.5 Hz, 1H), 8.75 (d, J = 1.6 Hz, 1H), 8.58 (s, 1H), 8.41 - 8.23 (m, 2H), 8.20 - 8.14 (m, 2H), 7.80 - 7.72 (m, 2H), 7.52 (d, J = 8.0 Hz, 2H), 4.60 (t, J = 8.2 Hz, 2H), 2.19 (d, J = 1.0 Hz, 3H). |
| | | | | | LC-MS (ESI): (Method 1) t*_{R}* = 1.80 min; m/z (M+1)= 532.1 |

### Example 14; Preparation of 1-(imidazo[1,2-a]pyridine-3-carbonyl)-N-(3-((4-methylpiperazin-1-yl)methyl)-5-(trifluoromethyl)phenyl)indoline-6-carboxamide

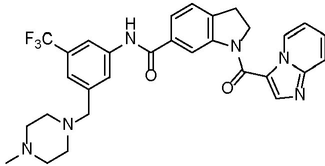

### Example 14

### Step 1; 1-(imidazo[1,2-a]pyridine-3-carbonyl)-N-(3-((4-methylpiperazin-1-yl)methyl)-5-(trifluoromethyl)phenyl)indoline-6-carboxamide

Imidazo[1,2-a]pyridine-3-carboxylic acid (0.012 g, 0.072 mmol) and HATU (0.030 g, 0.079 mmol) were dissolved in a 3:1 solution of anhydrous DCM (3 ml) and DMF (1.000 ml) then N-ethyl-N-isopropylpropan-2-amine (0.027 ml, 0.158 mmol) was added in one portion. The solution was stirred at room temperature for 10 minutes then Intermediate 25 (0.03 g, 0.072 mmol) was added in one portion. Stirring was maintained at room temperature for 16h. After dilution with DCM (10mL) the mixture was washed with sat NH₄Cl (10mL), sat NaHCO₃ (10mL) and brine (10mL). The organic phase was separated, dried over Na₂SO₄, filtered and concentrated to dryness. Crude was purified by reversed phase chromatography eluting with H₂O/MeCNH/HCO₂H, from 95:5:0.1 to 5:95:0.1 to afford the title compound as formate salt (9 mg, 22%).

| **Example No.** | **Analytical Data** |
|---|---|
| 14 | ¹H NMR (DMSO-d6, 400MHz): δ 10.55 (s, 1H), 9.34 (br d, *J*=7.0 Hz, 1H), 8.69 (br d, *J*= 1.1 Hz, 1H), 8.41 (s, 1H), 8.19 (s, 1H), 8.15 (s, 1H), 8.03 (br s, 1H), 7.80 (br d, *J*=9.0 Hz, 1H), 7.72 (br dd, *J*=7.8, 1.4 Hz, 1H), 7.52-7.57 (m, 1H), 7.47 (br d, *J*=7.7 Hz, 1H), 7.35 (s, 1H), 7.16-7.21 (m, 1H), 4.58 (brt, *J*=8.3 Hz, 2H), 3.55 (s, 2H), 2.53-2.55 (m, 2H), 2.35-2.45 (m, 6H), 2.17 ppm (s, 3H) |
| | LC-MS (ESI): (Method 6) t*_{R}* = 0.62 min; m/z (M+1)= 562.77 |

The following compound was prepared via amidation as described for Example 14, step 1, starting from the suitable corresponding, commercially available acid in step 1 and using TBTU as coupling agent.

| **Ex. No** | **Structure** | **Amount SM** | **Amount product (yield)** | **Purification Method** | **Analytical data** |
|---|---|---|---|---|---|
| 15 | 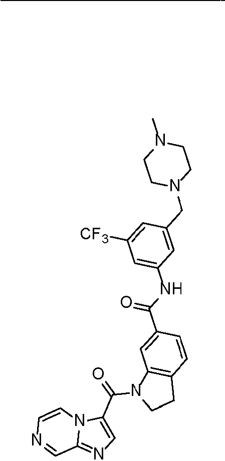 | 0.10 g | 0.050 g (Y=37%) | prep HPLC | ¹H NMR (300 MHz, DMSO-d6) δ 10.57 (s, 1H), 9.30 (d, J = 1.5 Hz, 1H), 9.21 (dd, J = 4.7, 1.5 Hz, 1H), 8.72 (s, 1H), 8.58 (s, 1H), 8.27 - 8.09 (m, 2H), 8.02 (s, 1H), 7.82 - 7.70 (m, 1H), 7.48 (d, J = 7.8 Hz, 1H), 7.34 (s, 1H), 4.60 (t, J = 8.3 Hz, 2H), 3.54 (s, 2H), 3.32 (s, 2H), 2.37 (d, J = 17.2 Hz, 8H), 2.15 (s, 3H). |
| | | | | | LC-MS (ESI): (Method 1) t*_{R}* = 1.71 min; m/z (M+1)= 564.15 |

### Example 16; Preparation of N-(1-(imidazo[1,2-a]pyridine-3-carbonyl)indolin-6-yl)-3-((4-methylpiperazin-1-yl)methyl)-5-(trifluoromethyl)benzamide

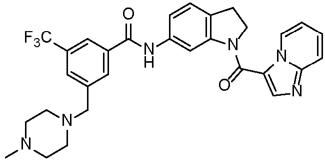

### Example 16

### Step 1; tert-butyl 6-nitroindoline-1-carboxylate - Intermediate 26

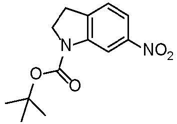

BOC-Anhydride (26.6 g, 122 mmol), 6-nitroindoline (20 g, 122 mmol), DMAP (1.5 g, 12.18 mmol), TEA (51 ml, 365 mmol), DCM (244 ml) were put into the flask. The reaction mixture was stirred at rt overnight. Solvents were removed under vacuum. Extraction with DCM was done. Solvents were removed again. Crude product was purified by FCC eluting with hexane/ethyl acetate 9:1 to give the title compound (29 g, 90%).

¹H NMR (300 MHz, DMSO-d6) δ 8.42 (s, 1H), 7.84 (dd, J = 8.2, 2.3 Hz, 1H), 7.49 - 7.40 (m, 1H), 4.01 (dd, J = 9.2, 8.2 Hz, 2H), 3.19 (t, J = 8.7 Hz, 2H), 1.54 (s, 9H).

### Step 2; tert-butyl 6-aminoindoline-1-carboxylate - Intermediate 27

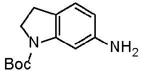

Into a 3-neck round button was charged Intermediate 26 (29.13 g, 110 mmol). Air was evacuated using water pump and purged with Ar - step was repeated 3 times. After that Palladium on Carbon 10 wt. % (2.91 g, 27.3 mmol) and Ethyl acetate (551 ml) was added. The reaction mixture was purged with Argon and removed using water pump - next purged with H₂. The mixture was stirred in hydrogen atmosphere at rt overnight. The reaction mixture was filtered through celite pad and concentrated to give the title compound (27 g, 100%).

¹H NMR (300 MHz, DMSO-d6) δ 7.05 (s, 1H), 6.80 (d, J = 7.9 Hz, 1H), 6.13 (dd, J = 7.9, 2.1 Hz, 1H), 4.92 (d, J = 6.6 Hz, 2H), 3.82 (dd, J = 9.2, 7.9 Hz, 2H), 2.86 (t, J = 8.5 Hz, 2H), 1.50 (s, 9H).

### Step 3; tert-butyl 6-(3-((4-methylpiperazin-1-yl)methyl)-5-(trifluoromethyl)benzamido)indoline-1-carboxylate - Intermediate 28

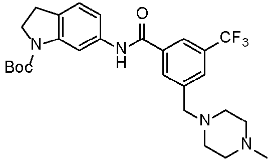

Intermediate 27 (6.45 g, 21.34 mmol) was placed into the flask then HATU (16.23 g, 42.7 mmol), DIPEA (14.91 ml, 85 mmol), DMF (53.4 ml) and Dichloromethane (160 ml) were added. The reaction mixture was stirred at rt for 30 min. After the time tert-butyl 6-aminoindoline-1-carboxylate (5 g, 21.34 mmol) was added. The stirring was continued overnight. Extraction with water and DCM was done. Organic phase was removed under reduce pressure. Crude product was purified by FCC eluting with hexane/ethyl acetate 9:1 to provide the title compound (10.4 g, 92%).

¹H NMR (300 MHz, DMSO-d6) δ 10.40 (s, 1H), 9.32 (s, 1H), 8.24 (d, J = 17.0 Hz, 2H), 8.15 (d, J = 1.9 Hz, 1H), 7.90 (s, 1H), 7.18 (d, J = 8.0 Hz, 1H), 3.94 (t, J = 8.5 Hz, 2H), 3.78 (s, 2H), 3.05 (t, J = 8.6 Hz, 2H), 2.70 (s, 3H), 2.40 (s, 8H), 1.52 (s, 9H).

### Step 4; N-(indolin-6-yl)-3-((4-methylpiperazin-1-yl)methyl)-5-(trifluoromethyl)benzamide - Intermediate 29

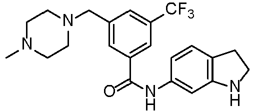

Intermediate 28 (1.938 g, 3.74 mmol) was dissolved in DCM (37.4 ml) and TFA (2.59 ml, 33.6 mmol) was added. The reaction mixture was stirred at rt overnight. The mixture was washed with sat NaHCO₃ and brine (10mL). The organic phase was separated, dried over Na₂SO₄, filtered and concentrated to dryness. Crude material was purified via FCC eluting with DCM/MeOH, 95:5. The pure fractions were washed with water, brine and 5% citric acid and finally the organic phase was dried over Na₂SO₄ and evaporated under vacuum to give the title compound (770 mg, 48%).

¹H NMR (300 MHz, DMSO-d6) δ 10.16 (s, 1H), 8.14 (d, J = 6.8 Hz, 2H), 7.83 (s, 1H), 7.04 (d, J = 1.9 Hz, 1H), 6.98 (d, J = 7.9 Hz, 1H), 6.92 - 6.82 (m, 1H), 5.61 (s, 1H), 3.63 (s, 2H), 3.48 - 3.39 (m, 2H), 2.88 (t, J = 8.4 Hz, 2H), 2.37 (d, J = 22.8 Hz, 8H), 2.16 (s, 3H).

### Step 5; N-(1-(imidazo[1,2-a]pyridine-3-carbonyl)indolin-6-yl)-3-((4-methylpiperazin-1-yl)methyl)-5-(trifluoromethyl)benzamide ( Example 16)

Imidazo[1,2-a]pyridine-3-carboxylic acid (0.012 g, 0.072 mmol) and HATU (0.030 g, 0.079 mmol) were dissolved in a 3:1 solution of anhydrous DCM/DMF (5 ml/ 1 ml), then N-ethyl-N-isopropylpropan-2-amine (0.027 ml, 0.158 mmol) was added in one portion. The solution was stirred at room temperature for 10 minutes then Intermediate 29 (0.03 g, 0.072 mmol) was added in one portion. Stirring was maintained at room temperature for 16 h. The mixture was diluted with DCM (10mL) then washed with sat NH₄Cl (10mL), sat NaHCO₃ (10mL) and brine (10mL). The organic phase was separated, dried over Na₂SO₄, filtered and concentrated to dryness. Crude material was purified via reversed phase column chromatography eluting with H₂O/MeCNH/HCO₂H, from 95:5:0.1 to 5:95:0.1 to provide the title compound as formate (31 mg, 71 %).

| **Example No.** | **Analytical Data** |
|---|---|
| 16 | ¹H NMR (DMSO-d6, 400MHz): d = 10.49 (s, 1H), 9.31 (d, *J*=7.0 Hz, 1H), 8.54 (d, *J*=1.8 Hz, 1H), 8.38 (s, 1H), 8.23 (s, 1H), 8.20 (s, 1H), 8.15 (s, 1H), 7.84 (s, 1H), 7.79 (d, *J*=9.2 Hz, 1H), 7.60 (dd, *J*=8.0, 1.9 Hz, 1H), 7.50-7.56 (m, 1H), 7.29 (br d, *J*=8.3 Hz, 1H), 7.17 (brtd, *J*=6.9, 0.9 Hz, 1H), 4.53 (br t, *J*=8.2 Hz, 2H), 3.65 (s, 2H), 3.19 (br t, *J*=8.1 Hz, 2H), 2.34-2.48 (m, 7H), 2.18 ppm (s, 3H). |
| | LC-MS (ESI): (Method 6) t*_{R}* = 0.65 min; m/z (M+1)= 562.9 |

### Example 17; Preparation of N-(1-(benzo[d]thiazol-6-ylmethyl)indolin-6-yl)-3-((4-methylpiperazin-1-yl)methyl)-5-(trifluoromethyl)benzamide

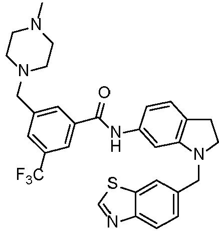

### Example 17

Intermediate 29 (0.1 g, 0.239 mmol) was dissolved in anhydrous DCM (2.4 ml) then benzo[d]thiazole-6-carbaldehyde (0.039 g, 0.239 mmol) was added followed by AcOH (0.027 ml, 0.478 mmol). Reaction mixture was stirred 30 min at rt. Next NaBH3CN (0.023 g, 0.35) was added and reaction mixture stirred at rt overnight. The mixture was quenched with addition of sat. aq. Solution of NaHCO₃. The phases were separated and the water layer was washed with DCM (x2). Collected organic phases were dried over Na₂SO₄ and solvent was removed under vacuum. Crude material was purified by column chromatography eluting with DCM/MeOH 9:1 and by preparative HPLC eluting with ACN, H₂O + 0.05% NH₃ to give the title compound (20 mg, 15 %).

| **Example No.** | **Analytical Data** |
|---|---|
| 17 | ¹H NMR (300 MHz, DMSO-d6) δ 10.23 (s, 1H), 9.37 (s, 1H), 8.16 (d, J = 1.7 Hz, 2H), 8.13 (s, 1H), 8.08 (d, J = 8.4 Hz, 1H), 7.82 (s, 1H), 7.55 (dd, J = 8.4, 1.7 Hz, 1H), 7.04 (s, 3H), 4.44 (s, 2H), 3.62 (s, 2H), 3.39 (d, J = 8.3 Hz, 2H), 2.92 (t, J = 8.3 Hz, 2H), 2.36 (d, J = 22.1 Hz, 8H), 2.15 (s, 3H). LC-MS (ESI): (Method 5) t*_{R}* = 1.82 min; m/z (M+1)= 566.07 |

The following compound was prepared via reductive amination as described for Example 17, step 1, starting from the suitable corresponding, commercially available aldehyde in step 1 using STAB as reductive agent.

| **Ex. No** | **Structure** | **Amount reagents** | **Product amount (yield)** | **Purification Method** | **Analytical data** |
|---|---|---|---|---|---|
| 18 | 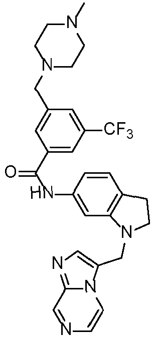 | 0.10 g 2 eq. STAB | 0.080 g (Y=56%) | FCC | ¹H NMR (300 MHz, DMSO-d6) δ 10.32 (s, 1H), 9.09 (d, J = 1.5 Hz, 1H), 8.51 (dd, J = 4.6, 1.5 Hz, 1H), 8.18 (d, J = 7.3 Hz, 2H), 7.96 (d, J = 4.6 Hz, 1H), 7.86 (d, J = 7.1 Hz, 2H), 7.26 (s, 1H), 7.05 (d, J = 1.3 Hz, 2H), 4.69 (s, 2H), 3.64 (s, 2H), 3.25 (t, J = 8.1 Hz, 2H), 2.85 (t, J = 8.1 Hz, 2H), 2.38 (d, J = 21.7 Hz, 8H), 2.17 (s, 3H). |
| | | | | | LC-MS (ESI): (Method 1) |
| | | | | | t*_{R}* = 1.71 min; m/z (M+1)= 548.04 |

### Example 19; Preparation of; N-(1-(imidazo[1,2-a]pyridine-3-carbonyl) indolin-6-yl)-3-(4-methyl-1H-imidazol-1-yl)-5-(trifluoromethyl) benzamide

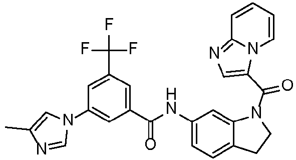

### Example 19

### Step 1; tert-butyl 6-(3-(4-methyl-1H-imidazol-1-yl)-5-(trifluoromethyl) benzamido)indoline-1-carboxylate - Intermediate 30

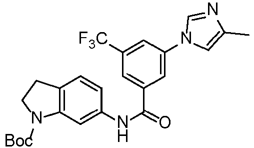

3-(4-methyl-1H-imidazol-1-yl)-5-(trifluoromethyl)benzoic acid (1.15 g, 4.27 mmol), tert-butyl 6-aminoindoline-1-carboxylate(1.0 g, 4.27 mmol), HATU (4.87 g, 12.80 mmol), DIPEA (4.47 ml, 25.6 mmol) were put into the flask then DMF (0.907 ml) and DCM (2.721 ml) were added. The reaction mixture was stirred at rt overnight.

Extraction with water and DCM was done. Solvents were dried over Na₂SO₄ and removed under reduce pressure. Crude material was purified by FCC eluting with Hexane/ethyl acetate 1:1 to give the title compound (2.2 g, 90%).

¹H NMR (300 MHz, DMSO-d6) δ 10.52 (s, 1H), 9.55 (s, 1H), 8.58 (s, 1H), 8.43 (d, J = 8.1 Hz, 2H), 8.15 (s, 2H), 7.21 (d, J = 8.0 Hz, 1H), 4.01 - 3.90 (m, 2H), 3.06 (t, J = 8.5 Hz, 2H), 2.36 (d, J = 1.2 Hz, 3H), 1.52 (s, 9H).

### Step 2; N-(indolin-6-yl)-3-(4-methyl-1H-imidazol-1-yl)-5-(trifluoromethyl) benzamide - Intermediate 31

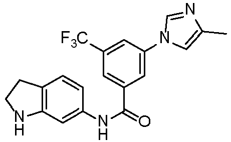

Intermediate 30 (1.87 g, 3.84 mmol) was dissolved in DCM (38.4 ml). Then TFA (2.67 ml, 34.6 mmol) was added. Reaction mixture was stirred at rt overnight. After washing with NaHCO₃, DCM was dried over Na₂SO₄. Solvents were removed under reduce pressure. Crude product was purified by FCC eluting with DCM/MeOH 95:5. After that, extraction with water, brine and 5% citric acid was done to give the title compound (1.09 g, 53%).

¹H NMR (300 MHz, DMSO-d6) δ 10.21 (s, 1H), 8.40 (d, J = 1.5 Hz, 2H), 8.22 (s, 1H), 8.13 (s, 1H), 7.72 (d, J = 1.4 Hz, 1H), 7.05 (d, J = 1.9 Hz, 1H), 7.00 (d, J = 7.8 Hz, 1H), 6.89 (d, J = 8.0 Hz, 1H), 5.64 (s, 1H), 3.44 (d, J = 8.4 Hz, 2H), 2.89 (t, J = 8.4 Hz, 2H), 2.20 (s, 3H).

### Step 3; N-(1-(imidazo[1,2-a]pyridine-3-carbonyl)indolin-6-yl)-3-(4-methyl-1H-imidazol-1-yl)-5-(trifluoromethyl)benzamide (Example 19)

Imidazo[1,2-a]pyridine-3-carboxylic acid (42.0 mg, 0.259 mmol), Intermediate 31 (100 mg, 0.259 mmol), TBTU (0.083 g, 0.259 mmol), TEA (72.1 µl, 0.518 mmol) were put into a vial and DMF (2.5 ml) was added. The reaction mixture was stirred at rt for overnight. The reaction mixture was basified by sat. sol. NaHCO₃. Extraction with DCM was done. Solvents were dried over Na₂SO₄ and removed under reduce pressure. Crude product was purified by FCC eluting with DCM/MeOH 95:5 to provide the title compound (98 mg, 69%).

| **Example No.** | **Analytical data** |
|---|---|
| 19 | ¹H NMR (300 MHz, DMSO-d6) δ 10.56 (s, 1H), 9.31 (dt, J = 7.0,1.2 Hz, 1H), 8.56 (d, J = 1.9 Hz, 1H), 8.49 (s, 1H), 8.45 - 8.38 (m, 2H), 8.25 (s, 1H), 8.21 (s, 1H), 7.80 (dt, J = 9.1, 1.2 Hz, 1H), 7.74 (t, J = 1.3 Hz, 1H), 7.63 (dd, J = 8.1, 2.0 Hz, 1H), 7.58 - 7.50 (m, 1H), 7.32 (d, J = 8.2 Hz, 1H), 7.18 (td, J = 6.9, 1.3 Hz, 1H), 4.55 (t, J = 8.2 Hz, 2H), 3.22 (t, J = 8.3 Hz, 2H), 2.20 (d, J = 1.0 Hz, 3H). |
| | LC-MS (ESI): (Method 13) t_{R} = 3.10 min; m/z (M+1)= 531.02 |

The following compounds were prepared via amidation as described for Example 19, step 1-3, starting from the suitable corresponding, commercially available acid in step 3.

| **Ex. No** | **Structure** | **Amount SM** | **Amount product (yield)** | **Purification Method** | **Analytical data** |
|---|---|---|---|---|---|
| 20 | 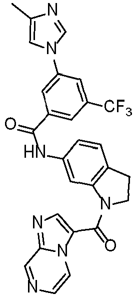 | 0.10 g | 0.045 g (Y=32%) | prep HPLC | ¹H NMR (300 MHz, DMSO-d6) δ 10.59 (s, 1H), 9.30 (d, J = 1.5 Hz, 1H), 9.17 (dd, J = 4.7, 1.5 Hz, 1H), 8.60 (d, J = 1.9 Hz, 1H), 8.57 (s, 1H), 8.49 (s, 1H), 8.42 (d, J = 1.4 Hz, 1H), 8.25 (s, 1H), 8.21 (s, 1H), 8.17 (d, J = 4.7 Hz, 1H), 7.74 (t, J = 1.3 Hz, 1H), 7.65 (dd, J = 8.1, 2.0 Hz, 1H), 7.35 (d, J = 8.2 Hz, 1H), 4.57 (t, J = 8.2 Hz, 2H), 3.22 (t, J = 8.2 Hz, 2H), 2.21 (d, J = 1.0 Hz, 3H). |
| | | | | | LC-MS (ESI): (Method 1) |
| | | | | | t*_{R}* = 1.79 min; m/z (M+1)= 532.14 |
| 21 | 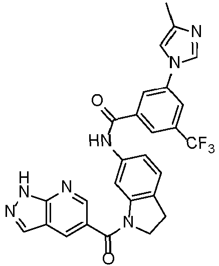 | 0.10 g | 0.020 g (Y=14%) | prep HPLC | ¹H NMR (300 MHz, DMSO-d6) δ 13.94 (s, 1H), 10.55 (s, 1H), 8.78 (d, J = 2.1 Hz, 1H), 8.59 (d, J = 2.1 Hz, 1H), 8.46 (s, 1H), 8.41 (d, J = 1.5 Hz, 1H), 8.28 (s, 1H), 8.24 (s, 1H), 8.18 (s, 1H), 7.73 (s, 1H), 7.63 (d, J = 8.1 Hz, 1H), 7.31 (d, J = 8.2 Hz, 1H), 4.16 (t, J = 8.2 Hz, 2H), 3.10 (t, J = 8.1 Hz, 2H), 2.20 (d, J = 1.0 Hz, 3H). |
| | | | | | LC-MS (ESI): (Method 1) |
| | | | | | t*_{R}* = 1.75 min; m/z (M+1)= 529.83 |

### Example 22; Preparation of N-(1-(imidazo[1,2-a]pyrazin-3-ylmethyl)indolin-6-yl)-3-(4-methyl-1H-imidazol-1-yl)-5-(trifluoromethyl)benzamide

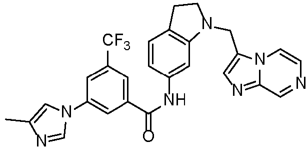

### Example 22

### Step 1; N-(1-(imidazo[1,2-a]pyrazin-3-ylmethyl)indolin-6-yl)-3-(4-methyl-1H-imidazol-1-yl)-5-(trifluoromethyl)benzamide (Example 22)

A flask was charged with Intermediate 31 (100 mg, 0.259 mmol), imidazo[1,2-a]pyrazine-3-carbaldehyde (38.1 mg, 0.259 mmol), magnesium sulfate (62.3 mg, 0.518 mmol) and DCM (2.6 ml). Acetic acid (29.6 µl, 0.518 mmol) was added. The reaction mixture was stirred at rt for 2h, then STAB (110 mg, 0.518 mmol) was added and mixture was stirred overnight. Fresh portion of STAB (110 mg, 0.518 mmol) was added and stirring was continued another day. The mixture was quenched with addition of sat. NaHCO₃. The phases were separated and the water layer was washed with DCM (x2). All combined organic phases were dried over Na₂SO₄, filtered and concentrated under reduce pressure. Crude material was purified by column chromatography eluting with DCM/MeOH 95:05 to afford the title compound (75mg, 53%).

| **Example No.** | **Analytical data** |
|---|---|
| 22 | ¹H NMR (300 MHz, DMSO-d6) δ 10.37 (s, 1H), 9.09 (d, J = 1.5 Hz, 1H), 8.51 (dd, J = 4.7, 1.5 Hz, 1H), 8.44 (s, 1H), 8.41 (d, J = 1.4 Hz, 1H), 8.24 (s, 1H), 8.17 (s, 1H), 7.97 (d, J = 4.6 Hz, 1H), 7.86 (s, 1H), 7.73 (t, J = 1.3 Hz, 1H), 7.26 (s, 1H), 7.12 - 7.01 (m, 2H), 4.70 (s, 2H), 3.26 (t, J = 8.2 Hz, 2H), 2.86 (t, J = 8.1 Hz, 2H), 2.20 (d, J = 1.0 Hz, 3H). |
| | LC-MS (ESI): (Method 11) t*_{R}* = 1.78 min; m/z (M+1)= 518.00 |

The following compound was prepared via amidation as described for Example 22, step 1, starting from the suitable corresponding, commercially available aldehyde in step 1, using NaBH₃CN as reductive agent.

| **Ex. No** | **Structure** | **Amount SM** | **Amount product (yield)** | **Purificatio n Method** | **Analytical data** |
|---|---|---|---|---|---|
| 23 | 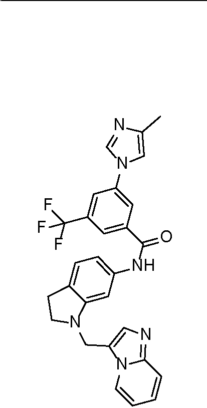 | 0.10 g | 0.035 g (Y=25%) | prep HPLC | ¹H NMR (300 MHz, DMSO-d6) δ 10.37 (s, 1H), 8.48 - 8.36 (m, 3H), 8.24 (s, 1H), 8.17 (s, 1H), 7.73 (t, J = 1.3 Hz, 1H), 7.64 - 7.57 (m, 2H), 7.33 - 7.24 (m, 2H), 7.07 (s, 2H), 6.98 (td, J = 6.8, 1.2 Hz, 1H), 4.62 (s, 2H), 3.23 (t, J = 8.2 Hz, 2H), 2.85 (t, J = 8.1 Hz, 2H), 2.20 (d, J = 1.0 Hz, 3H). |
| | | | | | LC-MS (ESI): (Method 3) |
| | | | | | t*_{R}* = 3.07 min; m/z (M+1)= 517.07 |

### Example 24; Preparation of 1-((1H-pyrazolo[3,4-b]pyridin-5-yl)methyl)-3-methyl-N-(3-(4-methyl-1H-imidazol-1-yl)-5-(trifluoromethyl)phenyl)indoline-6-carboxa mide

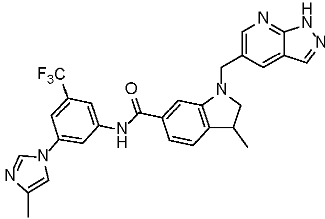

### Example 24

### Step 1; methyl 3-methylindoline-6-carboxylate - Intermediate 32

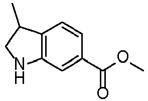

Methyl 3-methyl-1H-indole-6-carboxylate (1.5 g, 7.93 mmol), trifluoroacetic acid (4.86 ml, 63.1 mmol), triethylsilane (5.06 ml, 31.7 mmol) were put into the flask and THF (30 ml) was added. The reaction mixture was stirred at 0 °C overnight. Saturated solution of NaHCO₃ was added, extraction with DCM was done. Solvents were dried over Na₂SO₄ and removed under reduce pressure. Crude material was purified by FCC eluting with Hexane/ethyl acetate 9:1 to afford the title compound (950 mg, 62%).

¹H NMR (300 MHz, DMSO-d6) δ 7.20 (dd, J = 7.6, 1.5 Hz, 1H), 7.11 (d, J = 7.6 Hz, 1H), 7.02 (d, J = 1.5 Hz, 1H), 5.72 (s, 1H), 3.79 (s, 3H), 3.63 (td, J = 8.9, 1.3 Hz, 1H), 3.31 - 3.23 (m, 1H), 3.02 (td, J = 8.6, 2.5 Hz, 1H), 1.24 (d, J = 6.8 Hz, 3H).

### Step 2; 1-(tert-butyl) 6-methyl 3-methylindoline-1,6-dicarboxylate - Intermediate 33

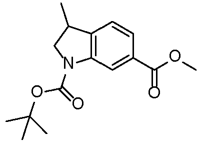

Intermediate 32 (950 mg, 4.97 mmol), BOC-Anhydride (1084 mg, 4.97 mmol), DMAP (61mg, 0.497 mmol), triethylamine (2.077 ml, 14.90 mmol) were put into the flask and DCM (50 ml) was added. The reaction mixture was stirred at rt overnight. Extraction with DCM was done. Solvents were dried over Na₂SO₄ and removed under reduce pressure. Crude material was purified by FCC eluting with Hexane:/ethyl acetate 4:1 to afford the title compound (970 mg, 67%).

¹H NMR (300 MHz, DMSO-d6) δ 8.26 (s, 1H), 7.60 (dd, J = 7.8, 1.6 Hz, 1H), 7.35 (d, J = 7.8 Hz, 1H), 4.13 (d, J = 12.5 Hz, 1H), 3.84 (s, 3H), 3.48 (d, J = 8.1 Hz, 2H), 1.52 (s, 9H), 1.28 (d, J = 6.5 Hz, 3H).

### Step 3; 1-(tert-butoxycarbonyl)-3-methylindoline-6-carboxylic acid - Intermediate 34

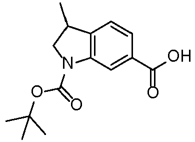

Intermediate 33 (0.95 g, 3.26 mmol), lithium hydroxide 1 M (4.89 ml, 4.89 mmol), MeOH (32.6 ml) were put into the flask and stirred at rt overnight. After the time, further 0.7 eq of 1M LiOH was added and stirring was continued to the next day. Solvents were removed under reduced pressure. Crude material, as lithium salt, was used to the next step without further purification (1.09 g, 100%). Alternatively, the salification was removed by extraction with aqueous solution 5% of citric acid to afford the title compound in quantitative yield.

¹H NMR (300 MHz, DMSO-d6) 6 8.18 (s, 1H), 7.51 (dd, J = 7.6, 1.4 Hz, 1H), 7.06 (d, J = 7.6 Hz, 1H), 4.18 - 4.02 (m, 1H), 3.41 (t, J = 6.1 Hz, 2H), 1.51 (s, 9H), 1.24 (d, J = 6.5 Hz, 3H).

### Step 4; tert-butyl 3-methyl-6-((3-(4-methyl-1H-imidazol-1-yl)-5-(trifluoromethyl)phenyl)carbamoyl)indoline-1-carboxylate - Intermediate 35

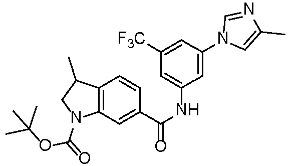

To a solution of Intermediate 34 (1.5 g, 5.41 mmol), 3-(4-methyl-1H-imidazol-1-yl)-5-(trifluoromethyl)aniline (1.305 g, 5.41 mmol) and DIPEA (5.67 ml, 32.5 mmol)in anhydrous DCM (54.1 ml) was added T3P (4.83 ml, 8.11 mmol). The reaction was stirred at rt overnight. Reaction mixture was diluted with DCM (5ml) and water was added. Then phases were separated and organic layer was dried over Na₂SO₄ and concentrated under vacuum. Crude material was purified by FCC eluting with DCM/MeOH, 95:5 to afford the title compound (1.7 g, 61%).

¹H NMR (300 MHz, DMSO-d6) δ 10.67 (s, 1H), 8.28 (s, 1H), 8.20 (d, J = 1.4 Hz, 1H), 8.14 (s, 1H), 7.72 (s, 1H), 7.61 (dd, J = 7.8, 1.6 Hz, 1H), 7.52 - 7.46 (m, 1H), 7.41 (d, J = 7.8 Hz, 1H), 4.16 (d, J = 11.1 Hz, 1H), 3.50 (q, J = 8.7, 7.8 Hz, 2H), 2.18 (d, J = 1.0 Hz, 3H), 1.53 (s, 9H), 1.31 (d, J = 6.5 Hz, 3H).

The following intermediate 64 was prepared via amidation as described for Intermediate 35, step 4, starting from intermediate 34, varying the corresponding amine. Such procedures may involve minor variations. In some cases, where modification involved coupling agents (e.g. TCFH and 1-methylimidazole instead of T3P) or chromatographic purification conditions (e.g. prepHPLC or flash chromatography), such changes were reported in the table.

| **Int N°** | **Chemical Name** | **Structure** | **Amount Reagents** | **Amount Product (yield)** | **Purification Method** | **Analytical Data** |
|---|---|---|---|---|---|---|
| 64 | tert-butyl 3-methyl-6-((5-(trifluorometh yl)pyridin-3-yl)carbamoyl)i ndoline-1-carboxylate | 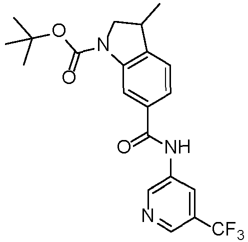 | 0.30 gr 1 eq. amine 1-methylimid azole TCFH ACN | 0.34 g (Y: 75%) | FCC | ¹H NMR (400 MHz, DMSO) δ 10.76 (s, 1H), 9.20 (s, 1H), 8.69 (d, J=1.1 Hz, 1H), 8.62 (m, 1H), 8.24 (brs, 1H), 7.61 (dd, J=1.6, 7.7 Hz, 1H), 7.40 (d, J=7.8 Hz, 1H), 4.20 - 4.15 (m, 1H), 3.54 - 3.43 (m, 2H), 1.53 (s, 9H), 1.30 (m, 3H). |

### Step 5; 3-methyl-N-(3-(4-methyl-1H-imidazol-1-yl)-5-(trifluoromethyl) phenyl)indoline-6-carboxamide - Intermediate 36

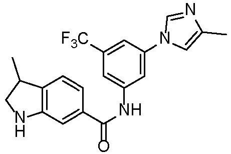

Intermediate 35 (1.71 g, 3.42 mmol) was dissolved in DCM (34.2 ml). Then TFA (2.369 ml, 30.7 mmol) was added. Reaction mixture was stirred at rt for 2 days. Reaction mixture was diluted with DCM (5ml) and aq. Sat. solution NaHCO₃ was added. Then phases were separated and organic layer was dried over Na2SO4 and concentrated under vacuum. Crude product was purified by FCC eluting with DCM:MeOH 95:5 to afford the title compound (1.07 g, 76%).

¹H NMR (300 MHz, DMSO-d6) δ 10.48 (s, 1H), 8.33 - 8.26 (m, 1H), 8.22 - 8.15 (m, 2H), 7.70 (s, 1H), 7.48 (t, J = 1.3 Hz, 1H), 7.26 - 7.14 (m, 2H), 7.05 (d, J = 1.4 Hz, 1H), 3.66 (t, J = 8.9 Hz, 1H), 3.10 - 3.01 (m, 1H), 2.19 (d, J = 1.0 Hz, 3H), 1.27 (d, J = 6.8 Hz, 3H).

The following intermediate 65 was prepared via acidic cleavage as described for Intermediate 36 in step 5. Such procedures may involve minor variations. In some cases, where modification involved chromatographic purification conditions (e.g. FCC replaced by SCX), such changes were reported in the table.

| **Int. No** | **Chemical name** | **Structure** | **Amount of reagent** | **Amount of product (yield)** | **Purification method** | **Analytical data** |
|---|---|---|---|---|---|---|
| 65 | 3-methyl-N-(5-(trifluoromet hyl)pyridin-3-yl)indoline-6-carboxamide | 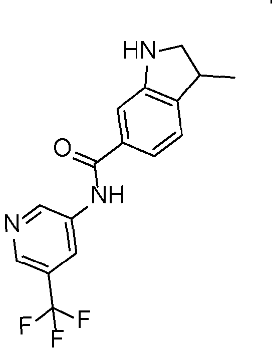 | 0.34 g | 0.21g (Y=80%) | SCX | ¹H NMR (400 MHz, DMSO) δ 10.56 (s, 1H), 9.18 (d, J=2.1 Hz, 1H), 8.68 (s, 1H), 8.64 - 8.62 (m, 1H), 7.22 (dd, J=1.5, 7.6 Hz, 1H), 7.17 (d, J=7.7 Hz, 1H), 7.04 (d, J=1.3 Hz, 1H), 3.68 - 3.62 (dt, J=1.0, 8.9 Hz, 1H), 3.36 - 3.25 (m, 1H), 3.08 - 3.02 (dt, J=2.4, 8.6 Hz, 1H), 1.26 (d, J=6.9 Hz, 3H). |

### Step 6; 1-((1H-pyrazolo[3,4-b]pyridin-5-yl)methyl)-3-methyl-N-(3-(4-methyl-1H-imidazol-1-yl)-5-(trifluoromethyl)phenyl)indoline-6-carboxamide (Example 24)

Into a flask were put 1H-pyrazolo[3,4-b]pyridine-5-carbaldehyde (36.7 mg, 0.250 mmol), Intermediate 36 (100 mg, 0.250 mmol), acetic acid (28.6 µl, 0.499 mmol), magnesium sulfate (60.1 mg, 0.499 mmol) and DCM (2.5 ml). The reaction mixture was stirred at rt for 2h. After the time STAB (212 mg, 0.999 mmol) was added. Stirring was continued to the next day. Reaction mixture was diluted with DCM and aq. Sat. solution NaHCO₃ was added. Then phases were separated and organic layer was dried over Na₂SO₄ and concentrated under vacuum. Crude material was purified by FCC eluting with DCM:MeOH 9:1 to give the title compound (105 mg, 77%).

| **Example No.** | **Analytical data** |
|---|---|
| 24 | ¹H NMR (300 MHz, DMSO-d6) δ 13.65 (s, 1H), 10.49 (s, 1H), 8.57 (d, J = 2.1 Hz, 1H), 8.29 (s, 1H), 8.20 (d, J = 1.6 Hz, 2H), 8.14 (s, 2H), 7.71 (s, 1H), 7.52 - 7.46 (m, 1H), 7.32 (d, J = 7.6 Hz, 1H), 7.27 - 7.20 (m, 2H), 4.63 (d, J = 14.6 Hz, 1H), 4.41 (d, J = 14.7 Hz, 1H), 3.55 (t, J = 8.7 Hz, 1H), 2.90 (t, J = 8.5 Hz, 1H), 2.18 (d, J = 1.0 Hz, 3H), 1.25 (d, J = 6.9 Hz, 3H). |
| | LC-MS (ESI): (Method 1) t*_{R}* =2.14 min; m/z (M+1)= 532.16 |

The following compounds were prepared via reductive amination as described for Example 24, step 1-6, starting from the suitable corresponding, commercially available aldehyde in step 6 and the corresponding amine in step 5. In some cases, where modification involved adding titanium(IV) isopropoxide or chromatographic purification conditions (e.g. prepHPLC or flash chromatography), such changes were reported in the table.

| **Ex. No** | **Structure** | **Amount SM** | **Product amount (yield)** | **Purification Method** | **Analytical data** |
|---|---|---|---|---|---|
| 25 | 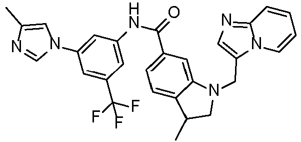 | 0.10 g | 0.075 g (Y=55%) | FCC | ¹H NMR (300 MHz, DMSO-d6) δ 10.52 (s, 1H), 8.38 (d, J = 6.9 Hz, 1H), 8.31 (s, 1H), 8.21 (d, J = 1.4 Hz, 1H), 8.15 (s, 1H), 7.73 (s, 1H), 7.61 (t, J = 4.6 Hz, 2H), 7.51 - 7.48 (m, 1H), 7.41 (d, J = 1.4 Hz, 1H), 7.40 - 7.34 (m, 1H), 7.32 - 7.27 (m, 1H), 7.24 (d, J = 7.8 Hz, 1H), 7.01 - 6.95 (m, 1H), 4.86 (d, J = 14.9 Hz, 1H), 4.57 (d, J = 15.0 Hz, 1H), 3.39 (t, J = 8.6 Hz, 1H), 3.28 - 3.20 (m, 1H), 2.78 (t, J = 8.5 Hz, 1H), 2.18 (d, J = 1.0 Hz, 3H), 1.22 (d, J = 6.8 Hz, 3H). LC-MS (ESI): (Method 1) t*_{R}* =2.38 min; m/z (M+1)= 531.21 |
| 26 | 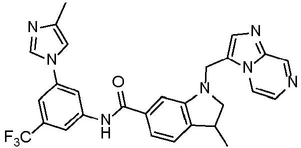 | 0.10 g | 0.090 g (Y=63%) | FCC and prep HPLC | ¹H NMR (300 MHz, DMSO-d6) δ 10.53 (s, 1H), 9.10 (d, J = 1.5 Hz, 1H), 8.48 (dd, J = 4.7, 1.5 Hz, 1H), 8.30 (s, 1H), 8.21 (d, J = 1.4 Hz, 1H), 8.15 (s, 1H), 7.96 (d, J = 4.6 Hz, 1H), 7.85 (s, 1H), 7.72 (s, 1H), 7.49 (t, J = 1.3 Hz, 1H), 7.38 (d, J = 6.8 Hz, 2H), 7.28 - 7.23 (m, 1H), 4.91 (d, J = 15.3 Hz, 1H), 4.65 (d, J = 15.2 Hz, 1H), 3.43 (t, J = 8.6 Hz, 1H), 3.30 - 3.22 (m, 1H), 2.81 (t, J = 8.4 Hz, 1H), 2.18 (d, J = 1.0 Hz, 3H), 1.24 - 1.21 (m, 3H). LC-MS (ESI): (Method 1) t*_{R}* =1.95 min; m/z (M+1)= 532.14 |
| 91 | 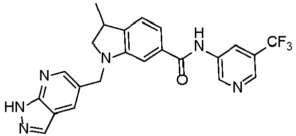 | 0.033 g aldehyde STAB Ti(OiPr)₄, AcOH, DCM | 0.025 g (Y=52%) | prep HPLC | I ¹H NMR (400 MHz, DMSO) δ 13.66 (s, 1H), 10.60 (s, 1H), 9.21 (d, J=2.3 Hz, 1H), 8.70 (d, J=1.1 Hz, 1H), 8.65 - 8.63 (m, 1H), 8.58 (d, J=2.0 Hz, 1H), 8.21 (d, J=2.1 Hz, 1H), 8.15 (s, 1H), 7.34 (dd, J=1.5, 7.5 Hz, 1H), 7.27 (d, J=1.3 Hz, 1H), 7.24 (dd, J=0.7, 7.6 Hz, 1H), 4.64 (d, J=14.7 Hz, 1H), 4.43 (d, J=14.7 Hz, 1H), 3.56 (t, J=8.8 Hz, 1H), 3.33 - 3.27 (m, 1H), 2.91 (t, J=8.5 Hz, 1H), 1.27 (d, J=6.8 Hz, 3H) LC-MS (ESI): (Method J) t*_{R}* =4.61 min; m/z (M+1)= 453.3 |
| 92 | 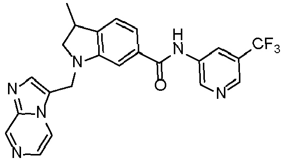 | 0.033 g aldehyde STAB Ti(OiPr)₄, AcOH, DCM | 0.028 g (Y: 59%) | prep HPLC | ¹H NMR (400 MHz, DMSO) δ 10.63 (s, 1H), 9.22 (d, J=2.3 Hz, 1H), 9.11 (d, J=1.5 Hz, 1H), 8.72 - 8.70 (m, 1H), 8.66 - 8.63 (m, 1H), 8.50 (dd, J=1.5, 4.6 Hz, 1H), 7.98 (d, J=4.6 Hz, 1H), 7.87 (s, 1H), 7.41 - 7.38 (m, 2H), 7.26 (dd, J=1.0, 8.1 Hz, 1H), 4.92 (d, J=15.3 Hz, 1H), 4.67 (d, J=15.3 Hz, 1H),), 3.45 (t, J=8.7 Hz, 1H), 3.31 - 3.22 (m, 1H), 2.82 (dd, J=8.5 Hz, 1H), 1.24 (d, J=6.7 Hz, 3H). LC-MS (ESI): (Method 2) t*_{R}* = 4.21 min; m/z (M+1)= 453.3 |

### Example 27; Preparation of 1-(imidazo[1,2-a]pyrazin-3-ylmethyl)-3-methyl-N-(3-(4-methyl-1H-imidazol-1-yl)-5-(trifluoromethyl)phenyl)indoline-6-carboxamide (single enantiomer)

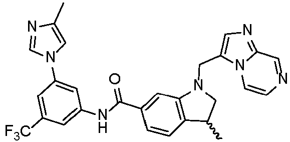

### Example 27

### Step 1; Preparation of 1-(imidazo[1,2-a]pyrazin-3-ylmethyl)-3-methyl-N-(3-(4-methyl-1H-imidazol-1-yl)-5-(trifluoromethyl)phenyl)indoline-6-carboxamide (Example 27)

An amount of isolated racemic Example 26 (65 mg) was dissolved to 5 mg/mL in EtOH and was then purified by chiral HPLC (SFC chiral separation) following Method 18. Combined fractions of second eluting enantiomer were evaporated to near dryness using a rotary evaporator. The resultant solids were transferred into final vessels with DCM which was removed under a stream of compressed air at 35°C before being stored in a vacuum oven at 35°C and 5mbar to afford the title compound (23.10 mg).

| **Example No.** | **Analytical data** |
|---|---|
| 27 | ¹H NMR (300 MHz, DMSO-d6) δ 10.52 (s, 1H), 9.10 (d, J = 1.5 Hz, 1H), 8.48 (dd, J = 4.7, 1.5 Hz, 1H), 8.30 (s, 1H), 8.21 (d, J = 1.4 Hz, 1H), 8.14 (s, 1H), 7.96 (d, J = 4.7 Hz, 1H), 7.85 (s, 1H), 7.72 (s, 1H), 7.49 (t, J = 1.3 Hz, 1H), 7.40 - 7.35 (m, 2H), 7.25 (dd, J = 8.0, 1.0 Hz, 1H), 4.91 (d, J = 15.2 Hz, 1H), 4.65 (d, J = 15.2 Hz, 1H), 3.43 (t, J = 8.6 Hz, 1H), 3.30 - 3.22 (m, 1H), 2.81 (t, J = 8.4 Hz, 1H), 2.18 (d, J = 1.0 Hz, 3H), 1.23 (d, J = 6.7 Hz, 3H). LC-MS (ESI): (Method 1) t*_{R}* =1.96 min; m/z (M+1)= 532.20 Chiral analysis (SFC Method 19): t*_{R}* 10.43 min, ee 100% |

### Example 28; Preparation of 3-methyl-N-(3-(4-methyl-1H-imidazol-1-yl)-5-(trifluoromethyl)phenyl)-1-(1H-pyrazolo[3,4-b]pyridine-5-carbonyl)indoline-6-carboxamide

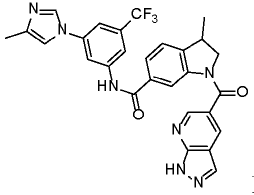

Example 28

Intermediate 36 (0.1 g, 0.250 mmol), 1H-pyrazolo[3,4-b]pyridine-5-carboxylic acid (0.041 g, 0.250 mmol), TEA (69.6 µl, 500 mmol), TBTU (0.08 g, 0.250 mmol) were put into a vial and DMF (2.5 ml was added. The reaction mixture was stirred at rt overnight then the temperature was increased to the 80 °C and stirring was continued to the next day. Extraction with water and DCM was done. Organic phase was separated, dried over Na₂SO₄ and removed under reduce pressure. Crude product was purified by FCC DCM/MeOH; 9:1 and by preparative HPLC eluting with ACN, H2O + 0.05%NH₃ to provide the title compound (15 mg, 11%).

| **Example No.** | **Analytical data** |
|---|---|
| 28 | ¹H NMR (300 MHz, DMSO-d6) δ 13.96 (s, 1H), 10.72 (s, 1H), 8.80 (d, J = 2.1 Hz, 1H), 8.61 (d, J = 2.1 Hz, 1H), 8.56 (s, 1H), 8.30 (d, J = 5.0 Hz, 2H), 8.22 (d, J = 1.4 Hz, 1H), 8.16 (s, 1H), 7.78 (dd, J = 7.8, 1.7 Hz, 1H), 7.73 (s, 1H), 7.55 - 7.47 (m, 2H), 4.34 (t, J = 9.8 Hz, 1H), 3.80 (dd, J = 10.3, 7.3 Hz, 1H), 3.54 (q, J = 7.5 Hz, 1H), 2.18 (d, J = 1.0 Hz, 3H), 1.31 (d, J = 6.8 Hz, 3H). LC-MS (ESI): (Method 1) t*_{R}* =1.91 min; m/z (M+1)= 545.97 |

The following compound was prepared via amidation as described for Example 28, step 1, starting from the suitable corresponding, commercially available acid in step 1.

| **Ex. N°** | **Structure** | **Amount SM** | **Amount product (yield)** | **Purification Method** | **Analytical data** |
|---|---|---|---|---|---|
| 29 | 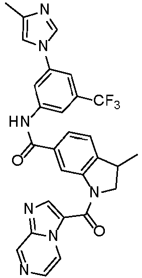 | 0.10 g | 0.120 g (Y=88%) | FCC | ¹H NMR (300 MHz, DMSO-d6) 8 10.74 (s, 1H), 9.31 (d, J = 1.5 Hz, 1H), 9.21 (dd, J = 4.7, 1.5 Hz, 1H), 8.73 (s, 1H), 8.60 (s, 1H), 8.32 (s, 1H), 8.22 (d, J = 1.4 Hz, 1H), 8.18 (d, J = 4.9 Hz, 2H), 7.80 (dd, J = 7.8, 1.7 Hz, 1H), 7.74 (s, 1H), 7.55 (d, J = 7.9 Hz, 1H), 7.51 (s, 1H), 4.77 (t, J = 9.8 Hz, 1H), 4.17 (dd, J = 10.2, 6.9 Hz, 1H), 3.70 - 3.60 (m, 1H), 2.19 (d, J = 1.0 Hz, 3H), 1.39 (d, J = 6.9 Hz, 3H). LC-MS (ESI): (Method 1) t*_{R}* =1.95 min; m/z (M+1)= 546.15 |

### Example 30; Preparation of 1-((1H-pyrazolo[3,4-b]pyridin-5-yl)methyl)-3-methyl-N-(3-(trifluoromethyl)phenyl)indoline-6-carboxamide

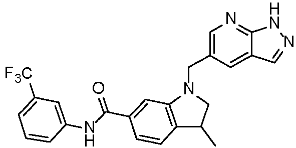

### Example 30

### Step 1; tert-butyl 3-methyl-6-((3-(trifluoromethyl)phenyl)carbamoyl)indoline-1-carboxylate - Intermediate 37

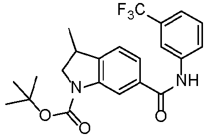

To a solution of 1-(tert-butoxycarbonyl)-3-methylindoline-6-carboxylic acid (935 mg, 3.37 mmol), 3-(trifluoromethyl)aniline (543 mg, 3.37 mmol) and DIPEA (3.53 ml, 20.23 mmol) in anhydrous DCM (33.7 ml) was added T3P (3.01 ml, 5.06 mmol). The reaction was stirred at rt overnight. To the reaction mixture was added water and this mixture was extracted with DCM (2x20 ml). Organic solvents were dried over Na₂SO₄ and removed under reduce pressure. Crude material was purified by FCC eluting with DCM/MeOH, 95:5 to afford the title compound (560 mg, 39%).

¹H NMR (300 MHz, DMSO-d6) δ 10.53 (s, 1H), 8.23 (s, 1H), 8.04 (d, J = 8.2 Hz, 1H), 7.58 (dd, J = 7.7, 1.6 Hz, 2H), 7.45 (d, J = 7.8 Hz, 1H), 7.38 (d, J = 7.8 Hz, 1H), 4.15 (d, J = 11.3 Hz, 1H), 3.54 - 3.44 (m, 2H), 1.53 (s, 9H), 1.30 (d, J = 6.5 Hz, 3H).

### Step 2; 3-methyl-N-(3-(trifluoromethyl)phenyl)indoline-6-carboxamide - Intermediate 38

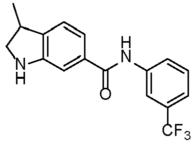

Intermediate 37 (560 mg, 1.332 mmol) was dissolved in DCM (13.32 ml) and TFA (0.924 ml, 11.99 mmol) was added. The reaction mixture was stirred at rt overnight. Reaction mixture was diluted with DCM and aq. sat. solution NaHCO₃ was added. Then phases were separated and organic layer was dried over Na₂SO₄ and concentrated under vacuum. Crude material was purified by FCC eluting with DCM:MeOH 99:1 to give the title compound (375 mg, 86%).

¹H NMR (300 MHz, DMSO-d6) δ 10.34 (s, 1H), 8.25 (s, 1H), 8.03 (d, J = 8.2 Hz, 1H), 7.57 (t, J = 8.0 Hz, 1H), 7.42 (d, J = 7.8 Hz, 1H), 7.19 (dd, J = 7.6, 1.5 Hz, 1H), 7.15 (d, J = 7.6 Hz, 1H), 7.03 (d, J = 1.4 Hz, 1H), 5.73 (s, 1H), 3.64 (td, J = 8.9, 1.3 Hz, 1H), 3.27 (d, J = 7.8 Hz, 1H), 3.04 (td, J = 8.6, 2.5 Hz, 1H), 1.26 (d, J = 6.8 Hz, 3H).

### Step 3; 1-((1H-pyrazolo[3,4-b]pyridin-5-yl)methyl)-3-methyl-N-(3-(trifluoro methyl)phenyl)indoline-6-carboxamide (Example 30)

Into a flask 1H-pyrazolo[3,4-b]pyridine-5-carbaldehyde (32.2 mg, 0.219 mmol) Intermediate 38 (70 mg, 0.219 mmol), acetic acid (25.02 µl, 0.437 mmol) magnesium sulfate (52.6 mg, 0.437 mmol) and DCM (2185 µl) were put. The reaction mixture was stirred at rt for 2h. After the time STAB (185 mg, 0.874 mmol) was added. Stirring was continued to the next day. The reaction was basified by NaHCO₃. Extraction with DCM was done. Then phases were separated and organic layer was dried over Na₂SO₄ and concentrated under vacuum. Crude material was purified by FCC eluting with DCM/MeOH 95:5 to provide the title compound (17 mg, 89%).

| **Example No.** | **Analytical data** |
|---|---|
| 30 | ¹H NMR (300 MHz, DMSO-d6) δ 13.65 (s, 1H), 10.35 (s, 1H), 8.57 (d, J = 2.0 Hz, 1H), 8.24 (s, 1H), 8.20 (d, J = 2.0 Hz, 1H), 8.14 (s, 1H), 8.05 (d, J = 8.3 Hz, 1H), 7.59 (t, J = 8.0 Hz, 1H), 7.44 (d, J = 7.8 Hz, 1H), 7.30 (dd, J = 7.5, 1.5 Hz, 1H), 7.26 (d, J = 1.4 Hz, 1H), 7.20 (d, J = 7.5 Hz, 1H), 4.62 (d, J = 14.7 Hz, 1H), 4.40 (d, J = 14.6 Hz, 1H), 3.53 (t, J = 8.8 Hz, 1H), 2.88 (t, J = 8.5 Hz, 1H), 1.25 (d, J = 6.9 Hz, 3H). LC-MS (ESI): (Method 8) t*_{R}* =2.91 min; m/z (M+1)= 452.15 |

The following compounds were prepared via reductive amination as described for Example 30, step 1-3, starting from the suitable corresponding, commercially available aldehyde in step 3.

| **Ex. No** | **Structure** | **Amount SM** | **Product amount (yield)** | **Purification Method** | **Analytical data** |
|---|---|---|---|---|---|
| 31 | 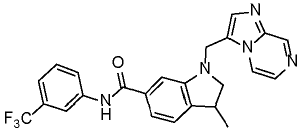 | 0.07 g | 0.055 g (Y=55%) | FCC | ¹H NMR (300 MHz, DMSO-d6) δ 10.38 (s, 1H), 9.10 (d, J = 1.5 Hz, 1H), 8.48 (dd, J = 4.7, 1.5 Hz, 1H), 8.24 (s, 1H), 8.05 (d, J = 8.4 Hz, 1H), 7.96 (d, J = 4.7 Hz, 1H), 7.85 (s, 1H), 7.60 (t, J = 8.0 Hz, 1H), 7.45 (d, J = 7.7 Hz, 1H), 7.38 (d, J = 1.4 Hz, 1H), 7.35 (dd, J = 7.5, 1.5 Hz, 1H), 7.23 (d, J = 7.5 Hz, 1H), 4.91 (d, J = 15.2 Hz, 1H), 4.64 (d, J = 15.2 Hz, 1H), 3.42 (t, J = 8.6 Hz, 1H), 3.24 (dd, J = 14.9, 7.4 Hz, 1H), 2.79 (t, J = 8.4 Hz, 1H), 1.22 (d, J = 6.7 Hz, 3H). LC-MS (ESI): (Method 5) t*_{R}* =3.04 min; m/z (M+1)= 452.11 |
| 32 | 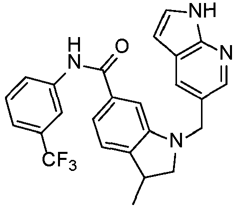 | 0.07 g | 0.080 g (Y=79%) | FCC | ¹H NMR (300 MHz, DMSO-d6) δ 11.63 (s, 1H), 10.35 (s, 1H), 8.24 (d, J = 2.1 Hz, 2H), 8.05 (d, J = 8.4 Hz, 1H), 7.93 (d, J = 2.0 Hz, 1H), 7.59 (t, J = 8.0 Hz, 1H), 7.50 - 7.41 (m, 2H), 7.31 - 7.24 (m, 2H), 7.19 (d, J = 7.4 Hz, 1H), 6.43 (dd, J = 3.4, 1.9 Hz, 1H), 4.56 (d, J = 14.5 Hz, 1H), 4.35 (d, J = 14.4 Hz, 1H), 3.51 (t, J = 8.8 Hz, 1H), 3.29 - 3.23 (m, 1H), 2.86 (t, J = 8.5 Hz, 1H), 1.24 (d, J = 6.8 Hz, 3H). LC-MS (ESI): (Method 8) t*_{R}* =2.90 min; m/z (M+1)= 451.20 |
| 33 | 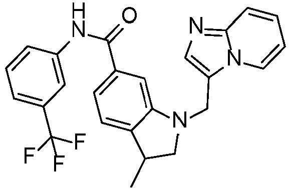 | 0.07 g | 0.080 g (Y=81%) | FCC | ¹H NMR (300 MHz, DMSO-d6) δ 10.38 (s, 1H), 8.38 (dt, J = 7.0, 1.2 Hz, 1H), 8.25 (s, 1H), 8.06 (d, J = 8.5 Hz, 1H), 7.65 - 7.56 (m, 3H), 7.48 - 7.42 (m, 1H), 7.40 (d, J = 1.5 Hz, 1H), 7.34 (dd, J = 7.6, 1.5 Hz, 1H), 7.28 (ddd, J = 9.1, 6.7, 1.3 Hz, 1H), 7.22 (d, J = 7.6 Hz, 1H), 6.97 (td, J = 6.8, 1.2 Hz, 1H), 4.85 (d, J = 15.0 Hz, 1H), 4.56 (d, J = 15.0 Hz, 1H), 3.39 (t, J = 8.5 Hz, 1H), 3.27 - 3.18 (m, 1H), 2.76 (t, J = 8.4 Hz, 1H), 1.22 (d, J = 6.8 Hz, 3H). LC-MS (ESI): (Method 5) t*_{R}* =1.84 min; m/z (M+1)= 451.12 |

### Example 34; Preparation of 1-((1H-pyrazolo[3,4-b]pyridin-5-yl)methyl)-3-methyl-N-(3-(trifluoromethyl)phenyl)indoline-6-carboxamide (single enantiomer)

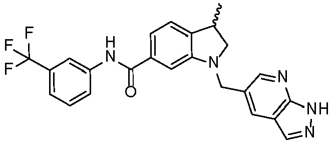

### Example 34

### Step 1; 1-((1H-pyrazolo[3,4-b]pyridin-5-yl)methyl)-3-methyl-N-(3-(trifluoro methyl)phenyl)indoline-6-carboxamide (Example 34)

The title compound was obtained as a single isomer by chiral preparative SFC separation of 70 mg of racemic Example 30. After preparative chiral separation the combined fractions of second eluting enantiomer were evaporated to dryness using a rotary evaporator to afford the title compound (11 mg, 15.6 %).

| **Example No.** | **Analytical data** |
|---|---|
| 34 | ¹H NMR (400 MHz, DMSO) d 13.66 (s, 1H), 10.36 (s, 1H), 8.58 (d, J=2.0 Hz, 1H), 8.25 (s, 1H), 8.21 (d, J=1.5 Hz, 1H), 8.15 (d, J=1.1 Hz, 1H), 8.06 (d, J=8.5 Hz, 1H), 7.60 (dd, J=8.0, 8.0 Hz, 1H), 7.45 (d, J=7.7 Hz, 1H), 7.31 (dd, J=1.4, 7.5 Hz, 1H), 7.27 (s, 1H), 7.22 (d, J=7.5 Hz, 1H), 4.64 (d, J=14.7 Hz, 1H), 4.42 (d, J=14.7 Hz, 1H), 3.55 (t, 1H), 3.32 - 3.28 (m, 1H - partially obscured by D₂O), 2.90 (t, 1H), 1.26 (d, J=6.8 Hz, 3H). LCMS (Method 2): t*_{R}* = 5.10 min, m/z 452.4 [M+H]⁺ Chiral analysis (SFC Method 17): t*_{R}* = 1.8 min, ee 91.2%. |

### Example 35; Preparation of 1-((1H-pyrazolo[3,4-b]pyridin-5-yl)methyl)-N-(3-(trifluoromethyl)phenyl)indoline-6-carboxamide

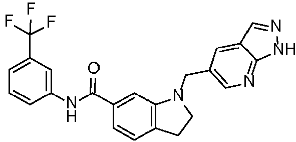

### Example 35

### Step 1; methyl 1-((1H-pyrazolo[3,4-b]pyridin-5-yl)methyl)indoline-6-carboxylate - Intermediate 39

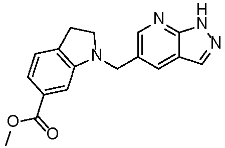

Methyl indoline-6-carboxylate (1 g, 5.64 mmol), 1H-pyrazolo[3,4-b]pyridine-5-carbaldehyde (0.830 g, 5.64 mmol) , acetic acid (0.646 ml, 11.29 mmol), magnesium sulfate (1.358 g, 11.29 mmol) were dissolved in DCM (56.4 ml). The reaction mixture was stirred at rt for 2h. After the time STAB (2.392 g, 11.29 mmol) was added. The stirring was continued overnight. Sat. solution of NaHCO₃ was added and extraction with DCM was done. Organic phase was dried over Na₂SO₄ and removed under reduced pressure. Crude material was purified by FCC eluting with hexane/ethyl acetate 0-100% to afford the title compound (571 mg, 32.7 %).

¹H NMR (300 MHz, DMSO-d6) δ 13.64 (s, 1H), 8.54 (d, J = 2.1 Hz, 1H), 8.17 (d, J = 2.0 Hz, 1H), 8.13 (s, 1H), 7.27 (dd, J = 7.5, 1.4 Hz, 1H), 7.19 - 7.14 (m, 2H), 4.49 (s, 2H), 3.79 (s, 3H), 2.97 (t, J = 8.4 Hz, 2H).

### Step 2; 1-((1H-pyrazolo[3,4-b]pyridin-5-yl)methyl)-N-(3-(trifluoromethyl) phenyl)indoline-6-carboxamide (Example 35)

A solution of 3-(trifluoromethyl)aniline (85 µl, 0.681 mmol) in anhydrous THF (1135 µl), under argon flow, was cooled down to -78°C. Next, nBuLi (2.5M in hexane) (272 µl, 0.681 mmol) was added. The mixture was stirred for 25 minutes, then a solution of Intermediate 39 (70 mg, 0.227 mmol) in anhydrous THF (1135 µl) was added dropwise. The mixture was stirred at -78°C for 0.5 h and slowly warmed to room temperature. The reaction mixture was stirred at room temperature overnight. Water was added and extraction with DCM was done. Organic phase was separated, dried over Na₂SO₄ and removed under reduce pressure. Crude material was purified by FCC eluting with DCM/MeOH 95:5 to provide the title compound (77.4 mg, 77 %).

| **Example No.** | **Analytical data** |
|---|---|
| 35 | ¹H NMR (400 MHz, DMSO-d6) δ 13.64 (s, 1H), 10.34 (s, 1H), 8.57 (d, J = 2.0 Hz, 1H), 8.25 - 8.18 (m, 2H), 8.13 (d, J = 1.4 Hz, 1H), 8.04 (dt, J = 8.3, 1.4 Hz, 1H), 7.58 (t, J = 8.0 Hz, 1H), 7.43 (dd, J = 7.2, 1.5 Hz, 1H), 7.30 - 7.23 (m, 2H), 7.20 (d, J = 7.5 Hz, 1H), 4.52 (s, 2H), 3.36 (t, J = 8.5 Hz, 2H), 2.97 (t, J = 8.4 Hz, 2H). LC-MS (ESI): (Method 9) t*_{R}* =2.56 min; m/z (M+1)= 438.04 |

The following compounds were prepared via transamidation as described for Example 35, step 1-2, starting from the suitable corresponding, commercially available amines in step 2.

| **Example No** | **Structure** | **Amount SM** | **Product amount (yield)** | **Purification Method** | **Analytical data** |
|---|---|---|---|---|---|
| 36 | 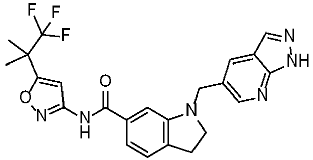 | 0.07 g | 0.040 g (Y=36.7 %) | FCC followed by prep HPLC | ¹H NMR (300 MHz, DMSO-d6) δ 13.61 (s, 1H), 11.27 (s, 1H), 8.57 (d, J = 2.1 Hz, 1H), 8.20 (d, J = 2.0 Hz, 1H), 8.14 (s, 1H), 7.36 (d, J = 1.5 Hz, 1H), 7.32 (dd, J = 7.6, 1.5 Hz, 1H), 7.17 (d, J = 7.6 Hz, 1H), 7.10 (s, 1H), 4.51 (s, 2H), 2.97 (t, J = 8.4 Hz, 2H), 1.58 (s, 6H). |
| | | | | | LC-MS (ESI): (Method 5) t*_{R}* =3.03 min; m/z (M+1)=471.74 |
| 37 | 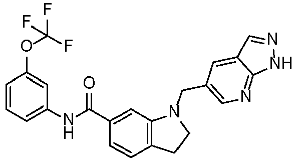 | 0.07 g | 0.073 g (Y=70.5 %) | FCC | ¹H NMR (300 MHz, DMSO-d6) δ 13.64 (s, 1H), 10.30 (s, 1H), 8.57 (d, J = 2.0 Hz, 1H), 8.20 (d, J = 2.0 Hz, 1H), 8.13 (s, 1H), 7.94 (s, 1H), 7.75 (d, J = 8.2 Hz, 1H), 7.47 (t, J = 8.3 Hz, 1H), 7.29 - 7.16 (m, 3H), 7.07 (d, J = 8.2 Hz, 1H), 4.52 (s, 2H), 2.97 (t, J = 8.4 Hz, 2H). |
| | | | | | LC-MS (ESI): (Method 8) t*_{R}* =2.74 min; m/z (M+1)=454.12 |
| 38 | 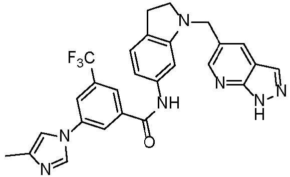 | 0.10 g | 0.093 g (Y=66.8 %) | FCC | ¹H NMR (300 MHz, DMSO-d6) δ 13.64 (s, 1H), 10.31 (s, 1H), 8.56 (d, J = 2.1 Hz, 1H), 8.45 - 8.38 (m, 2H), 8.22 (s, 1H), 8.19 (d, J = 2.0 Hz, 1H), 8.13 (d, J = 2.2 Hz, 2H), 7.71 (s, 1H), 7.12 (s, 1H), 7.05 (t, J = 5.9 Hz, 2H), 4.43 (s, 2H), 2.90 (t, J = 8.2 Hz, 2H), 2.19 (d, J = 1.0 Hz, 3H). |
| | | | | | LC-MS (ESI): (Method 15) t*_{R}* =5.65 min; m/z (M+1)=518.13 |
| 39 | 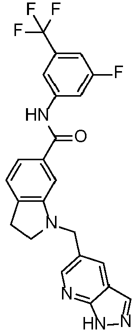 | 0.07 g | 0.077 g (Y=74.1 %) | Trituration with DCM, MeOH followed by filtration | ¹H NMR (300 MHz, DMSO-d6) δ 13.64 (s, 1H), 10.51 (s, 1H), 8.57 (d, J = 2.0 Hz, 1H), 8.20 (d, J = 2.0 Hz, 1H), 8.13 (s, 1H), 8.02 (d, J = 7.2 Hz, 2H), 7.38 (d, J = 8.5 Hz, 1H), 7.30 - 7.16 (m, 3H), 4.53 (s, 2H), 2.98 (t, J = 8.4 Hz, 2H). |
| | | | | | LC-MS (ESI): (Method 8) t*_{R}* =2.94 min; m/z (M+1)=456.13 |
| 40 | 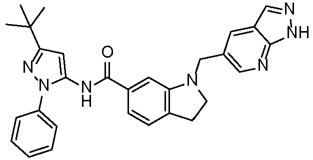 | 0.07 g | 0.020 g (Y=17.5 %) | prep HPLC | ¹H NMR (300 MHz, DMSO-d6) δ 13.56 (s, 1H), 10.08 (s, 1H), 8.55 (d, J = 2.0 Hz, 1H), 8.17 (d, J = 2.0 Hz, 1H), 8.12 (s, 1H), 7.64 (s, 2H), 7.37 (t, J = 7.7 Hz, 2H), 7.23 (d, J = 7.4 Hz, 1H), 7.18 (d, J = 8.3 Hz, 2H), 7.11 (d, J = 7.5 Hz, 1H), 6.40 (s, 1H), 4.46 (s, 2H), 2.94 (t, J = 8.3 Hz, 2H), 1.30 (s, 9H). |
| | | | | | LC-MS (ESI): (Method 8) t*_{R}* =2.62 min; m/z (M+1)=492.23 |

### Example 41; Preparation of N-(3-fluoro-5-(trifluoromethyl)phenyl)-1-(imidazo[1,2-a]pyrazin-3-ylmethyl)indoline-6-carboxamide

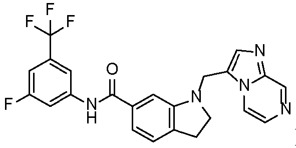

Example 41

### Step 1; methyl 1-(imidazo[1,2-a]pyrazin-3-ylmethyl)indoline-6-carboxylate - Intermediate 40

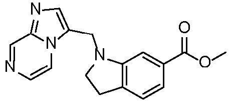

Ethyl indoline-6-carboxylate hydrochloride (0.5 g, 2.340 mmol), imidazo[1,2-a]pyrazine-3-carbaldehyde (0.344 g, 2.340 mmol) and magnesium sulfate (0.563 g, 4.68 mmol) were placed in the flask under argon. Anhydrous DCM (23.40 ml) was added followed by AcOH (0.268 ml, 4.68 mmol). Reaction mixture was stirred 2h at RT. Next STAB (1.736 g, 8.19 mmol) was added and the reaction mixture was stirred at RT overnight. The reaction mixture was diluted with DCM and 10% aqueous solution of NaHSO₃ was added. This mixture was stirred for 20 min and the phases were separated. The organic phase was washed with 10% aqua solution of NaHSO₃ three times to remove the residue of the aldehyde. The organic layer was dried over Na₂SO₄, filtered and concentrated under vacuum. The crude product was purified via FCC eluting with DCM/MeOH, 1:0 - 1:1 to give the title compound (0.319 g, 44.2 %)

¹H NMR (300 MHz, DMSO-d6) δ 9.08 (d, J = 1.5 Hz, 1H), 8.46 (dd, J = 4.7, 1.5 Hz, 1H), 7.94 (d, J = 4.7 Hz, 1H), 7.81 (s, 1H), 7.33 (d, J = 7.1 Hz, 2H), 7.22 - 7.16 (m, 1H), 4.76 (s, 2H), 3.82 (s, 3H), 3.22 (t, J = 8.3 Hz, 2H), 2.92 (t, J = 8.3 Hz, 2H).

### Step 2; N-(3-fluoro-5-(trifluoromethyl)phenyl)-1-(imidazo[1,2-a]pyrazin-3-ylmethyl)indoline-6-carboxamide (Example 41)

The solution of 3-fluoro-5-(trifluoromethyl)aniline (0.095 ml, 0.730 mmol) in anhydrous THF (1.216 ml) was cooled down to -78°C. Next, nBuLi (2.5M in hexane) (0.243 ml, 0.608 mmol) was added. The mixture was stirred for 25 minutes, then a solution of Intermediate 40 (0.075 g, 0.243 mmol) in anhydrous THF (1.2 ml) was added dropwise. The mixture was stirred at -78°C and slowly warmed to room temperature. The reaction mixture was stirred at room temperature overnight. Water and ethyl acetate were added to reaction mixture. The two layers were separated and the water layer was extracted with ethyl acetate. Organic layers were combined, dried over Na₂SO₄, filtered and concentrated to dryness. The crude product was purified via preparative HPLC eluting with ACN, H₂O + 0.05% NH₃ to give the title compound (0.061 g, 55 % yield).

| **Example No.** | **Analytical data** |
|---|---|
| 41 | ¹H NMR (300 MHz, DMSO-d6) δ 10.55 (s, 1H), 9.09 (d, J = 1.5 Hz, 1H), 8.48 (dd, J = 4.7, 1.5 Hz, 1H), 8.07 - 8.00 (m, 2H), 7.96 (d, J = 4.6 Hz, 1H), 7.85 (s, 1H), 7.41 - 7.35 (m, 2H), 7.32 (dd, J = 7.6, 1.5 Hz, 1H), 7.23 (d, J = 7.6 Hz, 1H), 4.78 (s, 2H), 3.25 (t, J = 8.3 Hz, 2H), 2.94 (t, J = 8.2 Hz, 2H). |
| | LC-MS (ESI): (Method 5) t*_{R}* =3.05 min; m/z (M+1)=456.11 |

The following compounds were prepared via transamidation as described for Example 41, step 1-2, starting from the suitable corresponding, commercially available amines in step 2.

| **Example No** | **Structure** | **Amount SM** | **Product amount (yield)** | **Purification Method** | **Analytical data** |
|---|---|---|---|---|---|
| 42 | 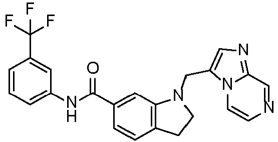 | 0.075 g | 0.061 g (Y=57.3 %) | prep HPLC | ¹H NMR (300 MHz, DMSO-d6) δ 10.38 (s, 1H), 9.09 (d, J = 1.5 Hz, 1H), 8.48 (dd, J = 4.7, 1.6 Hz, 1H), 8.24 (d, J = 2.0 Hz, 1H), 8.05 (dd, J = 8.3, 2.0 Hz, 1H), 7.96 (d, J = 4.7 Hz, 1H), 7.85 (s, 1H), 7.60 (t, J = 8.0 Hz, 1H), 7.44 (d, J = 7.8 Hz, 1H), 7.39 (d, J = 1.4 Hz, 1H), 7.33 (dd, J = 7.5, 1.5 Hz, 1H), 7.22 (d, J = 7.6 Hz, 1H), 4.78 (s, 2H), 3.25 (t, J = 8.3 Hz, 2H), 2.93 (t, J = 8.2 Hz, 2H). |
| | | | | | LC-MS (ESI): (Method 5) t*_{R}* =2.76 min; m/z (M+1)=438.11 |
| 43 | 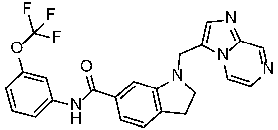 | 0.07 g | 0.035 g (Y=34 %) | prep HPLC | ¹H NMR (300 MHz, DMSO-d6) δ 10.34 (s, 1H), 9.09 (d, J = 1.5 Hz, 1H), 8.48 (dd, J = 4.7, 1.5 Hz, 1H), 7.99 - 7.93 (m, 2H), 7.85 (s, 1H), 7.79 - 7.73 (m, 1H), 7.48 (t, J = 8.3 Hz, 1H), 7.38 (d, J = 1.4 Hz, 1H), 7.31 (dd, J = 7.6, 1.5 Hz, 1H), 7.22 (d, J = 7.6 Hz, 1H), 7.08 (d, J = 8.2 Hz, 1H), 4. 78 (s, 2H), 3.23 (t, J = 8.1 Hz, 2H), 2.94 (t, J = 8.3 Hz, 2H). |
| | | | | | LC-MS (ESI): (Method 16) t*_{R}* =2.89 min; m/z (M+1)=454.03 |
| 44 | 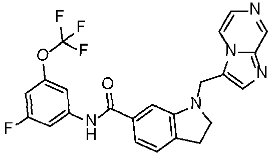 | 0.05 g | 0.046 g (Y=60 %) | prep HPLC | ¹H NMR (300 MHz, DMSO-d6) δ 10.49 (s, 1H), 9.09 (d, J = 1.5 Hz, 1H), 8.47 (dd, J = 4.7, 1.5 Hz, 1H), 7.95 (d, J = 4.7 Hz, 1H), 7.84 (s, 1H), 7.81 - 7.70 (m, 2H), 7.35 (d, J = 1.4 Hz, 1H), 7.30 (dd, J = 7.6, 1.5 Hz, 1H), 7.23 (d, J = 7.6 Hz, 1H), 7.09 (d, J = 9.0 Hz, 1H), 4.78 (s, 2H), 3.25 (t, J = 8.3 Hz, 2H), 2.94 (t, J = 8.2 Hz, 2H). |
| | | | | | LC-MS (ESI): (Method 14) t*_{R}* =2.18 min; m/z (M+1)=472.03 |
| 45 | 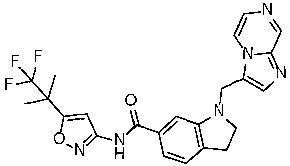 | 0.05 g | 0.058 g (Y=76 %) | prep HPLC | ¹H NMR (300 MHz, DMSO-d6) δ 11.31 (s, 1H), 9.09 (d, J = 1.5 Hz, 1H), 8.47 (dd, J = 4.7, 1.5 Hz, 1H), 7.96 (d, J = 4.6 Hz, 1H), 7.86 (s, 1H), 7.48 (d, J = 1.5 Hz, 1H), 7.36 (dd, J = 7.6, 1.5 Hz, 1H), 7.19 (d, J = 7.7 Hz, 1H), 7.11 (s, 1H), 4.76 (s, 2H), 3.24 (t, J = 8.3 Hz, 2H), 2.92 (t, J = 8.2 Hz, 2H), 1.58 (s, 6H). |
| | | | | | LC-MS (ESI): (Method 10) t*_{R}* =2.68 min; m/z (M+1)=471.26 |
| 46 | 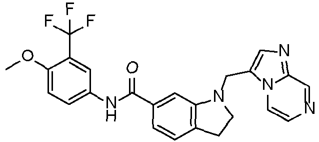 | 0.07 g | 0.060 g, (Y=56.5) | prep HPLC | ¹H NMR (300 MHz, DMSO-d6) δ 10.18 (s, 1H), 9.09 (d, J = 1.5 Hz, 1H), 8.48 (dd, J = 4.7, 1.5 Hz, 1H), 8.10 (d, J = 2.6 Hz, 1H), 8.01 (dd, J = 9.1, 2.6 Hz, 1H), 7.95 (d, J = 4.6 Hz, 1H), 7.85 (s, 1H), 7.38 (d, J = 1.5 Hz, 1H), 7.35 - 7.24 (m, 2H), 7.21 (d, J = 7.6 Hz, 1H), 4.77 (s, 2H), 3.88 (s, 3H), 3.24 (t, J = 8.3 Hz, 2H), 2.93 (t, J = 8.2 Hz, 2H). |
| | | | | | LC-MS (ESI): (Method 10) t*_{R}* =2.59 min; m/z (M+1)=468.02 |

### Example 47; 1-(imidazo[1,2-a]pyrazin-3-ylmethyl)-N-[4-(oxetan-3-ylmethoxy)-3-(trifluoromethyl)phenyl]indoline-6-carboxamide

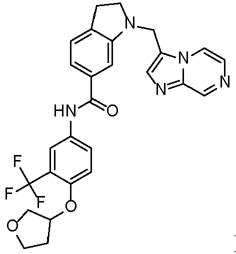

Example 47

### Step 1; 1-(imidazo[1,2-a]pyrazin-3-ylmethyl)indoline-6-carboxylic acid - Intermediate 41

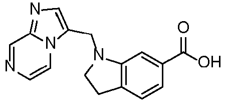

Intermediate 40 (68 mg, 0.21 mmol) and NaOH 1 N (0.6 ml) were stirred for 2 h at rt. The mixture was concentrated under reduced pressure. Residue was washed with water, filtered and dried under vacuum to yield the title compound (65 mg, 100%).

### Step 2; 1-(imidazo[1,2-a]pyrazin-3-ylmethyl)-N-[4-(oxetan-3-ylmethoxy)-3-(trifluoromethyl)phenyl]indoline-6-carboxamide (Example 47)

To a mixture of the Intermediate 41 (65 mg, 0.221 mmol) and HATU (100.8 mg, 0.26) in DMF (0.6 ml), *N*,*N-*diisopropylethylamine (85.68 mg, 129.2 mmol) was added. The reaction mixture was stirred at room temperature for 15 mins then Intermediate 16 (55 mg, 0.221 mmol) was added. The reaction mixture was stirred at room temperature until LCMS indicated consumption of starting material and then concentrated. Water and ethyl acetate were added to reaction mixture. The two layers were separated and the water layer was extracted with ethyl acetate. Organic layers were combined, dried over Na₂SO₄, filtered and concentrated to dryness. The crude product was purified via reverse phase preparative HPLC eluting with ACN / H₂O (10mM NH₄CO₃) to give the title compound (15.28mg, 13.1%).

| **Example No.** | **Analytical data** |
|---|---|
| 47 | ¹H NMR (400 MHz, DMSO) δ 10.20 (s, 1H), 9.11 (d, J=1.5 Hz, 1H), 8.50 (dd, J=1.5, 4.6 Hz, 1H), 8.12 (d, J=2.6 Hz, 1H), 8.02 (dd, J=2.5, 9.0 Hz, 1H), 7.97 (d, J=4.6 Hz, 1H), 7.86 (s, 1H), 7.39 (s, 1H), 7.36 - 7.31 (m, 2H), 7.23 (d, J=7.5 Hz, 1H), 4.79 (s, 2H), 4.71 (dd, J=6.0, 8.0 Hz, 2H), 4.47 (t, J=6.1 Hz, 2H), 4.32 (d, J=6.4 Hz, 2H), 3.47 - 3.38 (m, 1H), 3.26 (t, J=8.3 Hz, 2H), 2.95 (t, J=8.2 Hz, 2H) |
| | LCMS (Method 2) t*_{R}* = 4.12min, m/z 524.3 [M+H]+ |

The following compounds were prepared via amidation as described for Example 47, step 1-2, starting from the suitable corresponding amines in step 2.

| **Example No** | **Structure** | **Amount SM (g)** | **Product amount (yield)** | **Purification Method** | **Analytical data** |
|---|---|---|---|---|---|
| 48 | 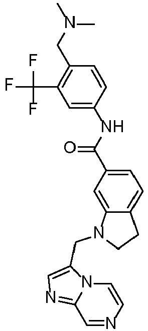 | 0.060 | 0.013 g (Y=19%) | prep HPLC | ¹H NMR (400 MHz, DMSO) δ 10.34 (1H, s), 9.11 (1H, d, J=1.5 Hz), 8.50 (1H, dd, J=1.5, 4.6 Hz), 8.22 (1H, d, J=2.1 Hz), 8.06 (1H, dd, J=1.9, 8.5 Hz), 7.97 (1H, d, J=4.6 Hz), 7.87 (1H, s), 7.73 - 7.70 (1H, m), 7.41 - 7.33 (2H, m), 7.23 (1H, d, J=7.5 Hz), 4.80 (2H, s), 3.51 (2H, s), 3.27 (2H, t, J=7.6 Hz), 2.95 (2H, t, J=8.3 Hz), 2.20 (6H, s) LCMS (Method 2) t*_{R}*= 2.93 min, m/z 495 [M+H]⁺ |
| 49 | 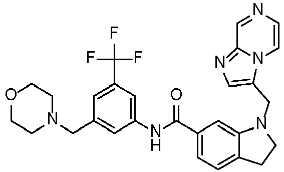 | 0.100 | 0.010 g (Y=13%) | prep HPLC | ¹H NMR (400 MHz, DMSO) δ 10.37 (s, 1H), 9.11 (d, J=1.5 Hz, 1H), 8.50 (dd, J=1.5, 4.6 Hz, 1H), 8.20 (s, 1H), 8.04 (s, 1H), 7.97 (d, J=4.6 Hz, 1H), 7.87 (s, 1H), 7.42 - 7.34 (m, 3H), 7.24 (d, J=7.7 Hz, 1H), 4.80 (s, 2H), 3.63 - 3.59 (m, 4H), 3.57 (s, 2H), 3.27 (t, J=8.3 Hz, 2H), 2.95 (t, J=8.3 Hz, 2H), 2.43 - 2.38 (m, 4H) LCMS (Method 2) t*_{R}* = 3.13 min, m/z 537.3 [M+H]⁺ |
| 50 | 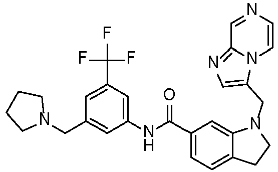 | 0.10 | 0.012 g (Y=16%) | prep HPLC | ¹H NMR (400 MHz, DMSO) δ 10.34 (s, 1H), 9.11 (d, J=1.5 Hz, 1H), 8.50 (dd, J=1.5, 4.6 Hz, 1H), 8.19 (s, 1H), 8.03 (s, 1H), 7.97 (d, J=4.7 Hz, 1H), 7.87 (s, 1H), 7.42 (d, J=1.2 Hz, 1H), 7.38 - 7.34 (m, 2H), 7.25 - 7.22 (m, 1H), 4.80 (s, 2H), 3.67 (s, 2H), 3.26 (t, J=8.3 Hz, 2H), 2.95 (t, J=8.3 Hz, 2H), 2.50 - 2.47 (m, 4H), 1.76 - 1.71 (m, 4H) LCMS (Method 2) t*_{R}* = 3.21 min, m/z 521.4 [M+H]+ |
| 51 | 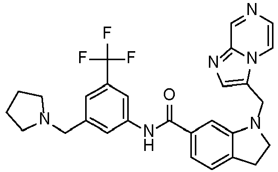 | 0.10 | 0.005 g (Y=7%) | prep HPLC | ¹H NMR (400 MHz, DMSO) δ 10.35 (s, 1H), 9.11 (d, J=1.5 Hz, 1H), 8.50 (dd, J=1.5, 4.6 Hz, 1H), 8.20 (s, 1H), 8.03 (s, 1H), 7.97 (d, J=4.6 Hz, 1H), 7.87 (s, 1H), 7.42 (d, J=1.3 Hz, 1H), 7.37 - 7.34 (m, 2H), 7.23 (d, J=7.7 Hz, 1H), 4.80 (s, 2H), 3.49 (s, 2H), 3.26 (t, J=8.3 Hz, 2H), 2.95 (t, J=8.3 Hz, 2H), 2.20 (s, 6H) LCMS (Method 2) t*_{R}* = 3.09 min, m/z 495.3 [M+H]+ |
| 52 | 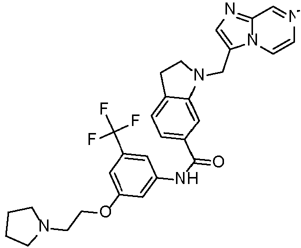 | 0.04 | 0.007 g (Y=31%) | prep HPLC (Luna PhenylHexyl 212x150mm, 10um 20-80% MeOH / H2O (0.1% FA), 20ml/min, RT) then lyophilization | ¹H NMR (400 MHz, DMSO) δ 10.31 (s, 1H), 9.11 (d, J=1.5 Hz, 1H), 8.49 (dd, J=1.5, 4.6 Hz, 1H), 7.97 (d, J=4.6 Hz, 1H), 7.87 - 7.81 (m, 2H), 7.76 (d, J=1.9 Hz, 1H), 7.39 - 7.31 (m, 2H), 7.24 (d, J=7.5 Hz, 1H), 7.00 (s, 1H), 4.80 (s, 2H), 4.16 (t, J=5.8 Hz, 2H), 3.35 - 3.29 (m, 4H), 2.95 (t, J=8.3 Hz, 2H), 2.83 (t, J=5.8 Hz, 2H), 1.73 - 1.68 (m, 4H) LCMS (Method 2) t*_{R}* = 3.36 min, m/z 551 [M+H]+ |
| 53 | 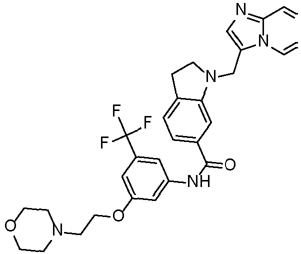 | 0.04 | 0.009 g (Y=12%) | prep HPLC (Sunfire C18 19x15 0mm, 10um 5-60% ACN / H2O (0.1% FA), 20ml/min, RT | ¹H NMR (400 MHz, DMSO) δ 10.31 (s, 1H), 9.11 (d, J=1.4 Hz, 1H), 8.50 (dd, J=1.6, 4.7 Hz, 1H), 7.97 (d, J=4.6 Hz, 1H), 7.87 - 7.91 (m, 2H), 7.76 (s, 1H), 7.39 - 7.31 (m, 2H), 7.24 (d, J=7.5 Hz, 1H), 7.01 (s, 1H), 4.80 (s, 2H), 4.18 (t, J=5.7 Hz, 2H), 3.59 (t, J=4.6 Hz, 4H), 3.26 (t, J=10.3 Hz, 2H), 2.95 (t, J=8.2 Hz, 2H), 2.74 (t, J=5.6 Hz, 2H), 2.53- 2.51 (m, 4H) LCMS (Method 2) t_{R} = 3.24min, m/z 567 [M+H]+ |
| 54 | 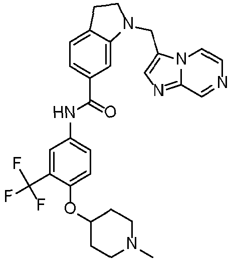 | 0.06 | 0.007 g (Y=5%) | prep HPLC formate salt | ¹H NMR (400 MHz, DMSO) δ 10.18 (s, 1H), 9.11 (d, J=1.6 Hz, 1H), 8.49 (dd, J=1.5, 4.7 Hz, 1H), 8.10 (d, J=2.7 Hz, 1H), 7.99 - 7.95 (m, 2H), 7.86 (s, 1H), 7.39 (d, J=1.1 Hz, 1H), 7.35 - 7.31 (m, 2H), 7.22 (d, J=7.3 Hz, 1H), 4.79 (s, 2H), 4.62 - 4.56 (m, 1H), 3.26 (t, J=7.9 Hz, 2H), 2.95 (t, J=7.9 Hz, 2H), 2.29 - 2.22 (m, 2H), 2.19 (s, 3H), 1.97 - 1.89 (m, 2H), 1.75 - 1.66 (m, 2H). One. LCMS (Method 2) t*_{R}* = 3.17min, m/z 551.4 [M⁺H]⁺ |

### Example 55; Preparation of 1-(imidazo[1,2-a]pyridin-3-ylmethyl)-N-(3-(trifluoromethoxy)phenyl)indoline-6-carboxamide

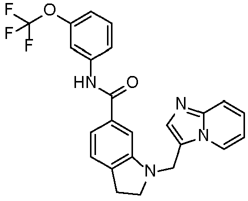

Example 55

### Step 1; methyl 1-(imidazo[1,2-a]pyridin-3-ylmethyl)indoline-6-carboxylate - Intermediate 42

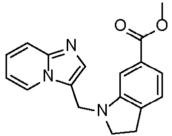

Methyl indoline-6-carboxylate (1 g, 5.64 mmol), imidazo[1,2-a]pyridine-3-carbaldehyde (0.825 g, 5.64 mmol) acetic acid (0.646 ml, 11.29 mmol), magnesium sulfate (1.358 g, 11.29 mmol) and DCM (56.4 ml) were put in a flask, reaction mixture was stirred at rt for 2h, then STAB (2.392 g, 11.29 mmol) was added. The stirring was continued to the next day. NaHCO₃ saturated solution was added. Extraction with DCM was done. Solvents were removed under reduce pressure. Crude material was purified by FCC eluting with DCM/MeOH 95:5 to provide the title compound (760 mg, 43%).

¹H NMR (300 MHz, DMSO-d6) δ 8.36 (dt, J = 6.9, 1.2 Hz, 1H), 7.60 (dt, J = 9.1, 1.1 Hz, 1H), 7.56 (s, 1H), 7.35 (d, J = 1.4 Hz, 1H), 7.34 - 7.24 (m, 2H), 7.18 (d, J = 7.5 Hz, 1H), 6.96 (td, J = 6.8, 1.2 Hz, 1H), 4.69 (s, 2H), 3.82 (s, 3H), 3.20 (t, J = 8.3 Hz, 2H), 2.91 (t, J = 8.3 Hz, 2H).

### Step 2; 1-(imidazo[1,2-a]pyridin-3-ylmethyl)-N-(3-(trifluoromethoxy)phenyl) indoline-6-carboxamide (Example 55)

A solution of 3-(trifluoromethoxy)aniline (121 mg, 0.683 mmol) in anhydrous THF (1139 µl) was cooled down to -78°C. Next, nBuLi (2.5M in hexane) (228 µl, 0.569 mmol) was added. The mixture was stirred for 25 minutes, then a solution of Intermediate 42 (70 mg, 0.228 mmol) in anhydrous THF (1139 µl) was added dropwise. The mixture was stirred at -78°C and slowly warmed to room temperature. The reaction mixture was stirred at room temperature overnight. Water and DCM were added to the reaction mixture. Two layers were separated and water layer was extracted again with DCM. Organic layers were combined, dried over Na₂SO₄, filtered and concentrated to dryness. Crude material was purified by FCC eluting with DCM/MeOH 95:5 to provide the title compound (87 mg, 84%).

| **Example No** | **Analytical data** |
|---|---|
| 55 | ¹H NMR (300 MHz, DMSO-d6) δ 10.32 (s, 1H), 8.38 (d, J = 7.0 Hz, 1H), 7.96 (s, 1H), 7.80 - 7.74 (m, 1H), 7.63 - 7.57 (m, 2H), 7.48 (t, J = 8.2 Hz, 1H), 7.40 (s, 1H), 7.33 - 7.25 (m, 2H), 7.21 (d, J = 7.6 Hz, 1H), 7.11-7.04 (m, 1H), 7.00 - 6.93 (m, 1H), 4.71 (s, 2H), 3.21 (t, J = 8.3 Hz, 2H), 2.92 (t, J = 8.2 Hz, 2H). LC-MS (ESI): (Method 1) t*_{R}* =2.23 min; m/z (M+1)=453.12 |

The following compounds were prepared via transamidation as described for Example 55, step 1-2, starting from the suitable corresponding, commercially available amines in step 2.

| **Example N°** | **Structure** | **Amou nt SM** | **Product amount (yield)** | **Purification Method** | **Analytical data** |
|---|---|---|---|---|---|
| 56 | 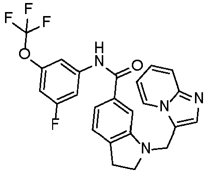 | 0.07 g | 0.045 g (Y=41 %) | FCC | ¹H NMR (300 MHz, DMSO-d6) δ 10.49 (s, 1H), 8.37 (d, J = 6.9 Hz, 1H), 7.77 (d, J = 7.3 Hz, 2H), 7.60 (t, J = 4.6 Hz, 2H), 7.38 (d, J = 1.3 Hz, 1H), 7.32 - 7.19 (m, 3H), 7.09 (d, J = 9.2 Hz, 1H), 7.01 - 6.94 (m, 1H), 4.71 (s, 2H), 3.21 (t, J = 8.2 Hz, 2H), 2.92 (t, J = 8.3 Hz, 2H). |
| | | | | | LC-MS (ESI): (Method 5) t*_{R}* =1.94 min; m/z (M+1)=471.06 |
| 57 | 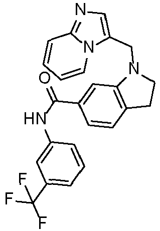 | 0.07 g | 0.068 g (Y=68 %) | FCC | ¹H NMR (300 MHz, DMSO-d6) δ 10.36 (s, 1H), 8.38 (d, J = 6.9 Hz, 1H), 8.25 (s, 1H), 8.06 (d, J = 8.3 Hz, 1H), 7.64 - 7.56 (m, 3H), 7.44 (d, J = 7.8 Hz, 1H), 7.41 (s, 1H), 7.35 - 7.24 (m, 2H), 7.22 (d, J = 7.6 Hz, 1H), 7.01 - 6.94 (m, 1H), 4.71 (s, 2H), 3.21 (t, J = 8.4 Hz, 2H), 2.92 (t, J = 8.3 Hz, 2H). |
| | | | | | LC-MS (ESI): (Method 1) t*_{R}* =2.16 min; m/z (M+1)=437.13 |
| 58 | 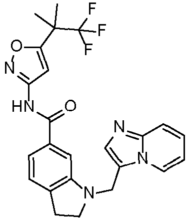 | 0.07 g | 0.025 g (Y=23 %) | prep HPLC | ¹H NMR (300 MHz, DMSO-d6) δ 11.29 (s, 1H), 8.40 - 8.34 (m, 1H), 7.65 - 7.57 (m, 2H), 7.52 (d, J = 1.5 Hz, 1H), 7.36 (dd, J = 7.6, 1.5 Hz, 1H), 7.32 - 7.24 (m, 1H), 7.19 (d, J = 7.6 Hz, 1H), 7.12 (s, 1H), 6.98 (dd, J = 7.3, 6.1 Hz, 1H), 4.69 (s, 2H), 3.21 (t, 2H), 2.91 (t, J = 8.2 Hz, 2H), 1.59 (s, 6H). |
| | | | | | LC-MS (ESI): (Method 1) t*_{R}* =2.14 min; m/z (M+1)=470.12 |
| 59 | 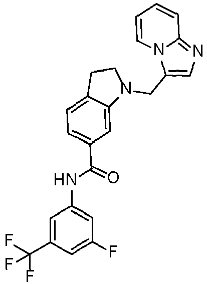 | 0.07 g | 0.025 g (Y=23 %) | FCC | ¹H NMR (300 MHz, DMSO-d6) δ 10.54 (s, 1H), 8.38 (d, J = 6.8 Hz, 1H), 8.08 - 7.98 (m, 2H), 7.61 (t, J = 4.6 Hz, 2H), 7.38 (d, J = 7.0 Hz, 2H), 7.34 - 7.18 (m, 3H), 7.00 - 6.93 (m, 1H), 4.72 (s, 2H), 3.22 (t, J = 8.3 Hz, 2H), 2.92 (t, J = 8.2 Hz, 2H). |
| | | | | | LC-MS (ESI): (Method 5) t*_{R}* =1.85 min; m/z (M+1)=455.06 |

### Example 60; Preparation of 1-((1H-benzo[d]imidazol-5-yl)methyl)-N-(3-(trifluoromethyl)phenyl)indoline-6-carboxamide

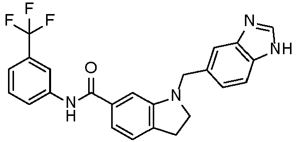

### Example 60

### Step 1; methyl 1-((1H-benzo[d]imidazol-5-yl)methyl)indoline-6-carboxylate - Intermediate 43

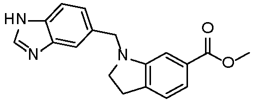

Methyl indoline-6-carboxylate hydrochloride (1.5 g, 7.02 mmol), 1H-benzo[d]imidazole-5-carbaldehyde (1.026 g, 7.02 mmol) and magnesium sulfate (1.690 g, 14.04 mmol) were placed in the flask under argon. Anhydrous DCM (46.8 ml) was added followed by AcOH (0.804 ml, 14.04 mmol). Reaction mixture was stirred 2h at RT. Next STAB (5.21 g, 24.57 mmol) was added and the reaction mixture was stirred at RT overnight. The reaction mixture was diluted with DCM and 10% aqua solution of NaHSO₃ was added. This mixture was stirred for 20 min and the phases were separated. The organic phase was washed with 10% aqueous solution of NaHSO₃ three times. The organic layer was dried over Na₂SO₄, filtered and concentrated under vacuum. The crude product was purified via flash column chromatography eluting with DCM/MeOH, 1:0 - 1:1. The obtained solid was triturated with water to give the title compound (1.068 g, 49.5 % yield).

¹H NMR (300 MHz, DMSO-d6) δ 8.32 (s, 1H), 7.58 (d, J = 8.3 Hz, 1H), 7.54 (d, J = 1.5 Hz, 1H), 7.23 (td, J = 7.9, 1.5 Hz, 2H), 7.15 (d, J = 7.6 Hz, 1H), 7.07 (d, J = 1.4 Hz, 1H), 4.45 (s, 2H), 3.78 (s, 3H), 3.37 (t, 2H), 2.97 (t, J = 8.5 Hz, 2H).

### Step 2; 1-((1H-benzo[d]imidazol-5-yl)methyl)-N-(3-(trifluoromethyl)phenyl) indoline-6-carboxamide (Example 60)

A solution of 3-(trifluoromethyl)aniline (0.091 ml, 0.732 mmol) in anhydrous THF (1.220 ml) was cooled down to -78°C. Next, nBuLi (2.5M in hexane) (0.244 ml, 0.610 mmol) was added. The mixture was stirred for 25 minutes, then a solution of Intermediate 43 (0.075 g, 0.244 mmol) in anhydrous THF (1.2 ml) was added dropwise. The mixture was stirred at -78°C and slowly warmed to room temperature. The reaction mixture was stirred at room temperature overnight. Water and ethyl acetate were added to the reaction mixture. Two layers were separated and water layer was extracted with ethyl acetate. Organic layers were combined, dried over Na₂SO₄, filtered and concentrated to dryness. The crude product was purified via preparative HPLC eluting with ACN, H₂O + 0.05% NH₃ to provide the title compound (0.044 g, 41.3 % yield).

| **Example No** | **Analytical data** |
|---|---|
| 60 | ¹H NMR (300 MHz, DMSO-d6) δ 10.32 (s, 1H), 8.20 (d, J = 13.6 Hz, 2H), 8.09 - 7.98 (m, 1H), 7.56 (d, J = 7.8 Hz, 3H), 7.46 - 7.39 (m, 1H), 7.28 - 7.15 (m, 4H), 4.48 (s, 2H), 3.38 (t, 2H), 2.98 (t, J = 8.4 Hz, 2H). |
| | LC-MS (ESI): (Method 10) t*_{R}* =1.81 min; m/z (M+1)=437.05 |

The following compounds were prepared via transamidation as described for Example 60, step 1-2, starting from the suitable corresponding, commercially available amines in step 2.

| **Example No** | **Structure** | **Amount SM** | **Product amount (yield)** | **Purification Method** | **Analytical data** |
|---|---|---|---|---|---|
| 61 | 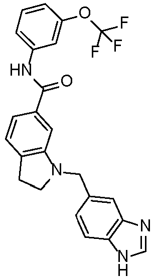 | 0.07 g | 0.032 g (Y=31 %) | prep HPLC | ¹H NMR (300 MHz, DMSO-d6) δ 10.27 (s, 1H), 8.18 (s, 1H), 7.93 (d, J = 2.7 Hz, 1H), 7.75 (dd, J = 8.1, 2.0 Hz, 1H), 7.56 (d, J = 7.9 Hz, 2H), 7.46 (t, J = 8.2 Hz, 1H), 7.25 - 7.15 (m, 4H), 7.09 - 7.01 (m, 1H), 4.47 (s, 2H), 3.38 (t, 2H), 2.97 (t, J = 8.4 Hz, 2H). |
| | | | | | LC-MS (ESI): (Method 10) t*_{R}* =1.88 min; m/z (M+1)=453.07 |
| 62 | 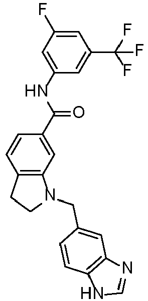 | 0.075 g | 0.061 g (Y=55 %) | Prep HPLC | ¹H NMR (300 MHz, DMSO-d6) δ 8.18 (s, 1H), 8.06 - 7.98 (m, 2H), 7.56 (d, J = 8.0 Hz, 2H), 7.40 - 7.33 (m, 1H), 7.27 - 7.16 (m, 4H), 4.48 (s, 2H), 3.39 (t, 3H), 2.98 (t, J = 8.4 Hz, 2H). |
| | | | | | LC-MS (ESI): (Method 10) t*_{R}* =1.99 min; m/z (M+1)=454.79 |

### Example 63; Preparation of 1-(pyrazolo[1,5-a]pyrimidin-6-ylmethyl)-N-(3-(trifluoromethyl)phenyl)indoline-6-carboxamide

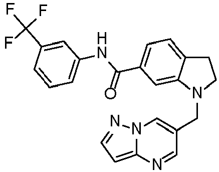

### Example 63

### Step 1; 1-(pyrazolo[1,5-a]pyrimidin-6-ylmethyl)-N-(3-(trifluoromethyl)phenyl) indoline-6-carboxamide (Example 63)

To a solution of pyrazolo[1,5-a]pyrimidine-6-carbaldehyde (24 mg, 0.163 mmol), N-(3-(trifluoromethyl)phenyl)indoline-6-carboxamide (50 mg, 0.163 mmol), obtained as described in Example 32, step 1-2, starting from 1-(tert-butoxycarbonyl)-indoline-6-carboxylic acid, in MeOH (5 ml) titanium(IV) isopropoxide (139 mg, 0.489) was added and the mixture refluxed for 2 h. The reaction was cooled to room temperature and NaBH₃CN (26 mg ,0.407 mmol) was added and stirring continued at room temperature overnight. The reaction was quenched with water, filtered through celite and concentrated under vacuum. Residue was purified by reverse phase preparative HPLC eluting with ACN / H₂O (0.1% FA) to give the title compound (15 mg, 39%).

| **Example No** | **Analytical data** |
|---|---|
| 63 | ¹H NMR (400 MHz, DMSO) d 10.37 (s, 1H), 9.17 - 9.16 (m, 1H), 8.64 (d, J=2.0 Hz, 1H), 8.25 (s, 1H), 8.23 (d, J=2.4 Hz, 1H), 8.06 (d, J=7.9 Hz, 1H), 7.61 (t, J=8.0 Hz, 1H), 7.45 (d, J=7.8 Hz, 1H), 7.32 - 7.29 (m, 2H), 7.23 (d, J=8.0 Hz, 1H), 6.76 - 6.75 (m, 1H), 4.49 (s, 2H), 3.42 (t, J=8.4 Hz, 2H), 3.01 (t, J=8.3 Hz, 2H). |
| | LCMS (Method 2): t*_{R}* = 5.10 min, m/z 438.3 [M+H]+. |

### Example 64; Preparation of 3-ethyl-1-(imidazo[1,2-a]pyrazin-3-ylmethyl)-N-(3-(4-methyl-1H-imidazol-1-yl)-5-(trifluoromethyl)phenyl)indoline-6-carboxamide

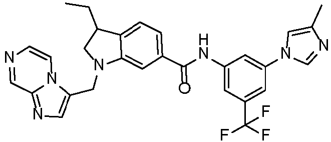

### Example 64

### Step 1; Methyl 3-acetyl-1H indole-6-carboxylate - Intermediate 66

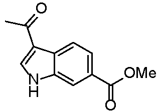

To a suspension of aluminum chloride (9.13 g, 68.5 mmol, 2.40 eq) and acetyl chloride (2.4 mL, 34.3 mmol, 1.20 eq) in DCM (100.00 mL) methyl indole-6-carboxylate (5.00 g, 28.5 mmol, 1.00 eq) was added and the mixture was stirred at room temperature for 1h. The mixture was poured onto ice and extracted with ethyl acetate (x3) (filtration through Celite was required). The combined organic extracts were washed with brine and dried (Na₂SO₄). The solvent was evaporated, and the residue was triturated under ether and filtered to give the title compound (1.19 g, 18%)
LCMS (Method 21 Report) t*_{R}*=1.04 min, m/z (M+1)= 218.1

### Step 2; Methyl 3-ethylindoline-6-carboxylate - Intermediate 67

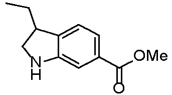

Triethylsilane (14 mL, 90.3 mmol, 18.0 eq) was added to a suspension of Intermediate 66 (1.09 g, 5.02 mmol, 1.00 eq) in TFA (16 mL, 0.214 mol, 42.6 eq) and the mixture was stirred for 18h. The mixture was concentrated in vacuo and the residue was partitioned between NaHCO₃ solution and DCM. The combined organic extracts were dried (Na₂SO₄) and evaporated. The residue was purified by FCC eluting with 50% EtOAC in cyclohexane to give the title compound (0. 31 g, 29%)

¹H NMR (400 MHz, CDCl3) d 7.43 (d, J=7.6 Hz, 1H), 7.25 (s, 1H), 7.13 - 7.10 (m, 1H), 3.87 (s, 3H), 3.81 (s, 1H), 3.74 - 3.69 (m, 1H), 3.30 - 3.19 (m, 2H), 1.91 - 1.80 (m, 1H), 1.65 - 1.52 (m, 1H), 1.01 - 0.96 (m, 3H).

### Step 3; Methyl 3-ethyl-1-(imidazo[1,2-a]pyrazin-3-ylmethyl)indoline-6-carboxylate Intermediate-68

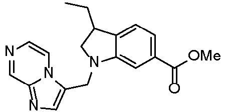

STAB (613 mg, 2.89 mmol, 2.00 eq) was added to a mixture of imidazo[1,2-a]pyrazine-3-carbaldehyde (266 mg, 1.81 mmol, 1.25 eq), Intermediate 67 (297 mg, 1.45 mmol, 1.00 eq), acetic acid (15 uL) and magnesium sulfate (697 mg, 5.79 mmol, 4.00 eq) in DCM (5.00 mL) and the mixture stirred for 5h. The mixture was partitioned between NaHCO₃ solution and DCM (x3). The combined organic extracts were dried (Na₂SO₄) and evaporated. The residue was purified by FCC eluting with 1% of MeOH in DCM to give the title compound (382 mg, 83%).

¹H NMR (400 MHz, CDCl3) d 9.14 (s, 1H), 8.08 (d, J=4.6 Hz, 1H), 7.90 (d, J=4.6 Hz, 1H), 7.79 - 7.78 (m, 1H), 7.53 (d, J=7.6 Hz, 1H), 7.36 - 7.35 (m, 1H), 7.17 - 7.14 (m, 1H), 4.70 - 4.65 (m, 1H), 4.56 - 4.51 (m, 1H), 3.92 - 3.91 (s, 3H), 3.32 - 3.26 (m, 1H), 3.18 - 3.09 (m, 1H), 2.84 - 2.78 (m, 1H), 1.87 - 1.76 (m, 1H), 1.56 - 1.44 (m, 1H), 0.89 (t, J=7.4 Hz, 3H).

### Step 4; Ethyl-1-(imidazo[1,2-a]pyrazin-3-ylmethyl)indoline-6-carboxylic acid -Intermediate 69

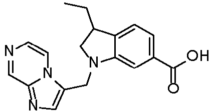

LiOH (54 mg, 2.27 mmol, 2.00 eq) was added to a solution of Intermediate 68 (382 mg, 1.14 mmol, 1.00 eq) in MeOH (2.00 mL) and water (0.50 mL) and the mixture stirred at 50°C for 64h. The mixture was cooled to room temp and HCl (1M; 2.27 ml, 2.27 mmol) added. The reaction mixture was freeze dried. The resulting material was purified by FCC on silica gel (40g cartridge, 0-100% DCM/ MeOH 1:5 in DCM) to give the title compound (249 mg, 61%).

¹H NMR (400 MHz, DMSO) d 9.38 (d, J=1.0 Hz, 1H), 8.69 - 8.67 (m, 1H), 8.21 (d, J=4.8 Hz, 1H), 8.15 (s, 1H), 7.37 - 7.35 (m, 2H), 7.20 - 7.17 (m, 1H), 4.93 - 4.86 (m, 1H), 4.75 - 4.69 (m, 1H), 3.70 (br s, 1H), 3.42 (s, 1H), 3.45 - 3.39 (m, 1H), 3.16 - 3.07 (m, 1H), 2.93 (t, J=8.1 Hz, 1H), 1.86 - 1.72 (m, 1H), 0.89 - 0.83 (m, 3H).

### Step 5; 3-Ethyl-1-(imidazo[1,2-a]pyrazin-3-ylmethyl)-N-(3-(4-methyl-1H-imidazol-1-yl)-5-(trifluoromethyl)phenyl)indoline-6-carboxamide-Intermediate 70

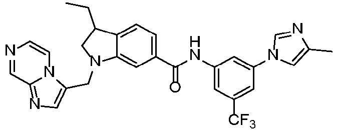

Thionyl chloride (0.79 mL, 10.9 mmol, 50.0 eq) was added to Intermediate 69 (70 mg, 0.217 mmol, 1.00 eq), and the mixture stirred for 10 mins. The mixture was taken to dryness in vacuo to give the intermediate acid chloride. The material was suspended in DCM (2 mL) and 3-(4-methyl-1H-imidazol-1-yl)-5-(trifluoromethyl)aniline (105 mg, 0.434 mmol, 2.00 eq) was added and the mixture stirred for 20 mins and left overnight, The mixture was taken to dryness in vacuo and the residue passed down a SCX-2 (10g) cartridge and eluted with MeOH (30 mL), followed by 2 M methanolic ammonia solution (50 mL). The ammonia solution eluent was combined and concentrated in vacuo. Purification by reverse phase preparative HPLC gave the title compound (7.96 mg, 6.7%).

¹H NMR (400 MHz, DMSO) d 10.54 (s, 1H), 9.11 (d, J=1.5 Hz, 1H), 8.49 - 8.47 (m, 1H), 8.31 (d, J=1.8 Hz, 1H), 8.21 (d, J=1.4 Hz, 1H), 8.16 (s, 1H), 7.98 (d, J=4.6 Hz, 1H), 7.86 (s, 1H), 7.73 (s, 1H), 7.50 - 7.50 (m, 1H), 7.39 - 7.35 (m, 2H), 7.26 (d, J=7.4 Hz, 1H), 4.87 (d, J=15.3 Hz, 1H), 4.73 (d, J=15.3 Hz, 1H), 3.45 - 3.39 (m, 1H), 3.20 - 3.12 (m, 1H), 2.98 - 2.93 (m, 1H), 2.19 (s, 3H), 1.83 - 1.76 (m, 1H), 1.53 - 1.45 (m, 1H), 0.91 - 0.85 (m, 3H).

### Step 6: 3-ethyl-1-(imidazo[1,2-a]pyrazin-3-ylmethyl)-N-(3-(4-methyl-1H-imidazol-1-yl)-5-(trifluoromethyl)phenyl)indoline-6-carboxamide (Example 64 )

Example 64 was obtained as single enantiomer (2^{nd} eluted) by running a chiral preparative SFC purification (Waters/Thar SFC systems with Waters SQD using a YMC Cellulose-C column with a 40/60 IPA (0.1% diethylamine) / heptane isocratic run at 1.0 ml/min ) on the racemic mixture of Intermediate 70 .

| **Example No** | **Analytical data** |
|---|---|
| **64** | ¹H NMR (400 MHz, DMSO) δ 10.54 (s, 1H), 9.11 (d, J=1.5 Hz, 1H), 8.48 (dd, J=1.5, 4.6 Hz, 1H), 8.31 (t, J=1.8 Hz, 1H), 8.21 (d, J=1.4 Hz, 1H), 8.16 (s, 1H), 7.97 (d, J=4.6 Hz, 1H), 7.86 (s, 1H), 7.73 (s, 1H), 7.51 - 7.49 (m, 1H), 7.39 - 7.35 (m, 2H), 7.26 (d, J=7.4 Hz, 1H), 4.87 (d, J=15.3 Hz, 1H), 4.73 (d, J=15.3 Hz, 1H), 3.45 - 3.39 (m, 1H), 3.20 - 3.13 (m, 1H), 2.96 (dd, J=7.5, 8.7 Hz, 1H), 2.20 (d, J=0.9 Hz, 3H), 1.83 - 1.74 (m, 1H), 1.55 - 1.45 (m, 1H), 0.88 (t, J=7.4 Hz, 3H). |
| | LC-MS (ESI): (Method 2) t*_{R}* =3.64 min; m/z (M+1)= 546.5 Chiral analysis (DFC, Method described above): t_{R} 9.8 min, ee >99.9% |

### Example 65; Preparation of 1-((3-methyl-1H-pyrazolo[3,4-b]pyridin-5-yl)methyl)-N-(4-((4-methylpiperazin-1-yl)methyl)-3-(trifluoromethyl)phenyl)indoline-6-carboxamide

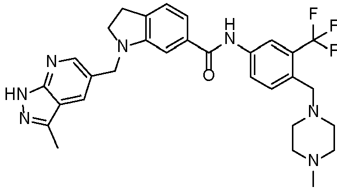

### Example 65

### Step 1; Ethyl 1-((3-methyl-1H-pyrazolo[3,4-b]pyridin-6-yl)methyl)indoline-6-carboxylate - Intermediate 71

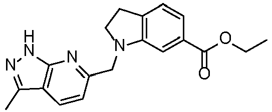

STAB (1108 mg, 5.23 mmol, 2.00 eq) was added to a mixture of 3-methyl-1H-pyrazolo[3,4-b]pyridine-5-carbaldehyde (527 mg, 3.27 mmol, 1.25 eq), ethyl indoline-6-carboxylate (500 mg, 2.61 mmol, 1.00 eq), acetic acid (15 uL) and magnesium sulfate (1259 mg, 10.5 mmol, 4.00 eq) in DCM (15.00 mL) and the mixture stirred at room temperature for 5h. The mixture was partitioned between NaHCO₃ solution and DCM (x3). The combined organic extracts were dried (Na₂SO₄) and evaporated. The residue was triturated under ether and filtered to give the title compound (785mg, 89%).
LCMS (Method 21) t*_{R}* =1.41 min, m/z (M+1)= 337.2

### Step 2; 1-((3-methyl-1H-pyrazolo[3,4-b]pyridin-6-yl)methyl)indoline-6-carboxylic acid - Intermediate 72

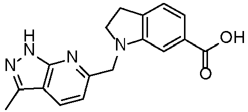

NaOH (192 mg, 4.80 mmol, 2.13 eq) was added to a solution of Intermediate 71 (758 mg, 2.25 mmol, 1.00 eq) in MeOH (10.00 mL) and water (0.50 mL) and the mixture stirred at 50°C for 64h. The mixture was cooled to rt and 1 M HCl (4.8 mL, 4.80 mmol, 2.13 eq) added dropwise resulting in the precipitation of a solid which was filtered off and washed with acetone (4 mL) to give the title compound (652 mg, 94%).
LCMS (Method 21) t*_{R}* 0.77 min, m/z (M+1) =309.2

### Step 3; 1-((3-methyl-1H-pyrazolo[3,4-b]pyridin-5-yl)methyl)-N-(4-((4-methylpiperazin-1-yl)methyl)-3-(trifluoromethyl)phenyl)indoline-6-carboxamide (Example 65)

To a mixture of Intermediate **72** (50 mg, 0.162 mmol) and HATU (74 mg, 0.194 mmol) in DMF (0.1M concentration) was added *N*,*N-*diisopropylethylamine (84 µL, 0.480 mmol ). The reaction mixture was stirred at room temperature for 15 mins then 4-[(4-methylpiperazin-1-yl)methyl]-3-(trifluoromethyl)aniline (53 mg, 0.195 mmol eq) was added. The reaction mixture was applied to a MeOH pre-conditioned 5 g Isolute SCX-II cartridge, washed with MeOH, then released with 2 M NH₃/MeOH. The 2 M NH₃/MeOH eluent was concentrated in vacuo. Purification by reverse phase preparative HPLC (Xbridge Phenyl 19x150mm, 10 µm 40-100% MeOHH₂O (10mM NH₄HCO₃), 20 mL/min, RT) gave the title compound (33.59 mg, 36%).

| **Example No.** | **Analytical data** |
|---|---|
| **65** | ¹H NMR (400 MHz, DMSO) δ 13.21 (s, 1H), 10.31 (s, 1H), 8.54 (d, J=2.0 Hz, 1H), 8.22 - 8.19 (m, 2H), 8.04 (dd, J=2.0, 8.5 Hz, 1H), 7.70 (d, J=8.6 Hz, 1H), 7.31 - 7.27 (m, 2H), 7.21 (d, J=7.5 Hz, 1H), 4.51 (s, 2H), 3.57 (s, 2H), 3.38 - 3.31 (m, 2H), 2.98 (t, J=8.3 Hz, 2H), 2.47 - 2.31 (m, 7H), 2.17 (s, 3H). |
| | LCMS (Method 2) t*_{R}*= 3.52 min, m/z (M+1)= 564.5 |

The following compounds were prepared via amidation as described for example 65, step 1-3, using the previously synthesized or commercially available amine in step 3. Such procedures may involve minor variations. In some cases, where modification involved coupling agents (e.g., TCFH and methylimidazole), solvents or chromatographic purification conditions, such changes were reported in the table.

| **Ex. No** | **Structure** | **Amount SM** | **Product amount (yield)** | **Purification Method** | **Analytical data** |
|---|---|---|---|---|---|
| 66 | 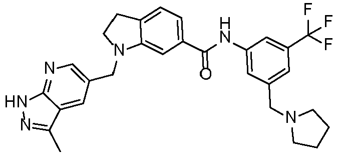 | 0.050 g of Intermediate 72 1.2 eq. amine | 0.076 g, (Y=8%). | Prep HPLC | ¹H NMR (400 MHz, DMSO) δ 13.21 (s, 1H), 10.32 (s, 1H), 8.55 (d, J=2.0 Hz, 1H), 8.21 - 8.17 (m, 2H), 8.03 (s, 1H), 7.35 (s, 1H), 7.33 - 7.30 (m, 2H), 7.23 - 7.20 (m, 1H), 4.51 (s, 2H), 3.67 (s, 2H), 2.98 (t, J=8.3 Hz, 2H), 2.50 - 2.45 (m, 4H), 1.76 - 1.71 (m, 4H). |
| | | | | | LC-MS (ESI): (Method 2) |
| | | | | | t*_{R}* =3.54 min min; m/z (M+1)= 535.3 |
| 67 | 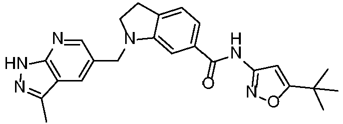 | 0.050 g of Intermediate 72 1.2 eq amine 1-methylimida zole TCFH DMF | 0.019 g, (Y=28 %) | prep HPLC | ¹H NMR (400 MHz, DMSO) δ 13.23 - 13.19 (m, 1H), 11.13 - 11.11 (m, 1H), 8.55 (d, J=2.0 Hz, 1H), 8.20 (d, J=2.0 Hz, 1H), 7.39 (d, J=1.3 Hz, 1H), 7.32 (dd, J=1.5, 7.7 Hz, 1H), 7.18 (d, J=7.5 Hz, 1H), 6.74 (s, 1H), 4.50 - 4.49 (m, 2H), 3.35 (t, J=8.5 Hz, 2H), 2.98 (t, J=8.5 Hz, 2H), 2.52 (s, 3H),1.34 - 1.33 (s, 9H). |
| | | | | | LC-MS (ESI): (Method 2) |
| | | | | | t*_{R}* =4.92 min; m/z (M+1)= 448.3 |
| 68 | 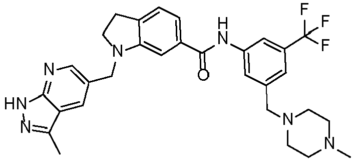 | 0.050 g of Intermediate 72 1.2 eq of amine | 0.011 mg, (Y=12%) | prep HPLC | ¹H NMR (400 MHz, DMSO) δ 13.21 (s, 1H), 10.34 (s, 1H), 8.55 (d, J=2.0 Hz, 1H), 8.21 - 8.17 (m, 2H), 8.01 (s, 1H), 7.35 (s, 1H), 7.32 - 7.29 (m, 2H), 7.21 (d, J=7.9 Hz, 1H), 4.51 (s, 2H), 3.54 (s, 2H), 2.99 (t, J=8.3 Hz, 2H), 2.45 - 2.27 (m, 8H), 2.17 (s, 3H). |
| | | | | | LC-MS (ESI): (Method 2) |
| | | | | | t*_{R}* = 3.55 min; m/z (M+1)= 564.5 |
| 69 | 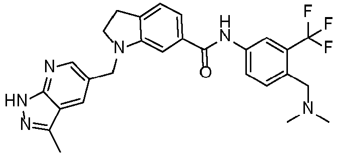 | 0.050 g of Intermediate 72 1.2 eq amine | 0.015 g, (Y=18%) | prep HPLC | ¹H NMR (400 MHz, DMSO) δ 13.22 (s, 1H), 10.31 (s, 1H), 8.54 (d, J=2.0 Hz, 1H), 8.22 - 8.20 (m, 2H), 8.05 (dd, J=2.0, 8.5 Hz, 1H), 7.70 (d, J=8.6 Hz, 1H), 7.30 - 7.28 (m, 2H), 7.22 (d, J=7.4 Hz, 1H), 4.51 (s, 2H), 3.50 (s, 2H), 2.98 (t, J=8.3 Hz, 2H), 2.19 (s, 6H). |
| | | | | | LC-MS (ESI): (Method 2) |
| | | | | | t*_{R}* = 3.33 min; m/z (M+1)= 509.3 |
| 70 | 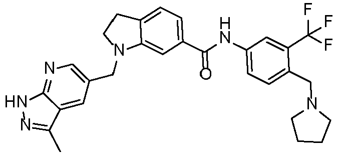 | 0.050 g of Intermediate 72 1 eq. amine | 0.019 g, (Y=22%) | prep HPLC | ¹H NMR (400 MHz, DMSO) δ 13.21 (s, 1H), 10.30 (s, 1H), 8.54 (d, J=2.0 Hz, 1H), 8.20 (d, J=2.0 Hz, 2H), 8.04 (dd, J=2.0, 8.5 Hz, 1H), 7.74 - 7.70 (m, 1H), 7.30 - 7.28 (m, 2H), 7.22 (d, J=7.3 Hz, 1H), 4.51 (s, 2H), 3.71 (s, 2H), 2.98 (t, J=8.3 Hz, 2H), 2.50 - 2.46 (m, 4H), 1.76 - 1.71 (m, 4H). |
| | | | | | LC-MS (ESI): (Method 2) |
| | | | | | t*_{R}* = 3.49 min; m/z (M+1)= 535.3 |
| 71 | 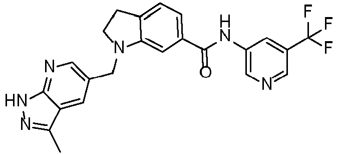 | 0.036 g of Intermediate 72 1.2 eq amine | 0.003 g (Y=5%) | prep HPLC | ¹H NMR (400 MHz, DMSO) δ 13.21 (s, 1H), 10.60 (s, 1H), 9.22 (d, J=2.1 Hz, 1H), 8.70 (s, 1H), 8.64 (s, 1H), 8.54 (d, J=1.9 Hz, 1H), 8.20 (d, J=1.9 Hz, 1H), 7.34 - 7.22 (m, 3H), 4.52 (s, 2H), 3.00 (t, J=8.4 Hz, 2H). |
| | | | | | LC-MS (ESI): (Method 2) |
| | | | | | t*_{R}* = 4.33 min; m/z (M+1)= 453.4 |
| 72 | 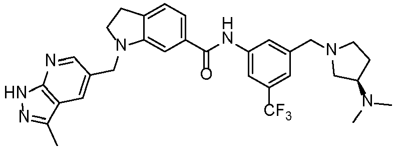 | 0.086 g of Intermediate 72 1 eq of amine TCFH, 1-methylimida zole DMF | 0.076 (Y=47%) | prep HPLC | ¹H NMR (400 MHz, DMSO) δ 13.21 (s, 1H), 10.32 (s, 1H), 8.55 (d, J=2.0 Hz, 1H), 8.20 (d, J=1.9 Hz, 1H), 8.18 (s, 1H), 8.01 (s, 1H), 7.35 (s, 1H), 7.32 - 7.28 (m, 2H), 7.21 (d, J=8.0 Hz, 1H), 4.51 (s, 2H), 3.71 (d, J=13.6 Hz, 1H), 3.58 (d, J=13.6 Hz, 1H), 3.38 - 3.34 (m, 2H), 2.98 (t, J=8.3 Hz, 2H), 2.73 - 2.67 (m, 2H), 2.65 - 2.58 (m, 1H), 2.49 - 2.45 (m, 1H), 2.35 - 2.27 (m, 1H), 2.09 - 2.08 (m, 6H), 1.93 - 1.83 (m, 1H), 1.68 - 1.59 (m, 1H). |
| | | | | | LC-MS (ESI): (Method 2) |
| | | | | | t*_{R}* = 3.13 min; m/z (M+1)= 578.4 |
| 73 | 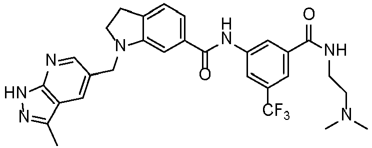 | 0.086 g of Intermediate 72 1.1 eq amine TCFH, 1-methylimida zole DMF | 0.006 g (Y= 7%) | Prep HPLC, SCX | ¹H NMR (400 MHz, DMSO) δ 13.21 (s, 1H), 10.49 (s, 1H), 8.68 (t, J=5.8 Hz, 1H), 8.55 (d, J=2.1 Hz, 1H), 8.52 (s, 1H), 8.43 (s, 1H), 8.21 (d, J=1.7 Hz, 1H), 7.91 (s, 1H), 7.35 - 7.31 (m, 2H), 7.23 (d, J=8.1 Hz, 1H), 4.52 (s, 2H), 3.43 - 3.36 (m, 4H), 3.00 (t, J=7.6 Hz, 2H), 2.46 (br s, 2H), 2.23 (s, 6H). |
| | | | | | LC-MS (ESI): (Method 2) |
| | | | | | t*_{R}* =3.35 min; m/z (M+1)= 566.2 |
| 74 | 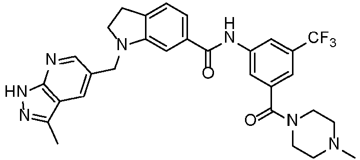 | 0.05 g of Intermediate 72 1.1 eq of amine TCFH, 1-methylimida zole DMF | 0.02 g (Y: 26%) | Prep HPLC, SCX | ¹H NMR (400 MHz, DMSO) δ 13.21 (s, 1H), 10.47 (s, 1H), 8.54 (d, J=2.0 Hz, 1H), 8.31 (s, 1H), 8.20 (d, J=2.0 Hz, 1H), 8.11 (s, 1H), 7.44 (s, 1H), 7.32 - 7.28 (m, 2H), 7.23 (d, J=7.6 Hz, 1H), 4.51 (s, 2H), 3.66 (s, 2H), 2.99 (t, J=8.4 Hz, 2H), 2.42 - 2.31 (m, 4H), 2.22 (s, 3H). |
| | | | | | LCMS (Method 2): t*_{R}* 3.28 min, m/z 578.2 [M+H]+, |

### Example 75; Preparation of 1-((1H-pyrazolo[3,4-b]pyridin-5-yl)methyl-N-(4-((4-methylpiperazin-1-yl)methyl)-3-(trifluoromethyl)phenyl)indoline-6-carboxamide

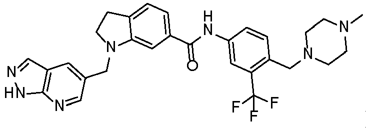

Example 75

### Step 1; tert-butyl 6-((4-((4-methylpiperazin-1-yl)methyl)-3-trifluoromethyl)phenyl)carbamoyl)indoline-1-carboxamide - Intermediate 73

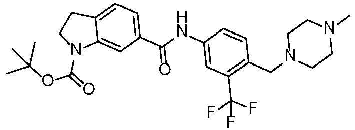

To a suspension of 1-tert-butoxycarbonylindoline-6-carboxylic acid (1.00 g, 3.80 mmol) and 4-[(4-methylpiperazin-1-yl)methyl]-3-(trifluoromethyl)aniline (1.25 g, 4.56 mmol) in DMF (10 mL) was added 1-methylimidazole (1.1 mL, 13.3 mmol) and TCFH (1.60 g, 5.70 mmol). The reaction mixture was stirred at rt for 24h. The mixture was partitioned between brine (3x10 mL) and EtOAc (10 mL). The organic phase was dried (Na₂SO₄) and concentrated. The residue was purified by FCC eluting with 10% of 2M NH₃/MeOH in DCM to give the title compound (2.22 g, 100%)
LCMS (Method 21) t*_{R}* =1.72 min, m/z (M+1)= 519

Following intermediates were prepared via amidation as described for intermediate 73, step 1 using appropriate, commercially available or previously sinthesized amines. Such procedures may involve minor variations. In some cases, where modification involved coupling agents (e.g. TCFH and 1-methylimidazole replaced by HATU and DIPEA), solvents (DMF replaced by ACN) or chromatographic purification conditions (e.g. prepHPLC or flash chromatography), such changes were reported in the table.

| **Intermediate N°** | **Intermediate name** | **Structure** | **Amount Reagent** | **Amount product (yield)** | **Purification Method** | **Analytical data** |
|---|---|---|---|---|---|---|
| 74 | tert-butyl 6-((3-(tert-butyl)isoxazol-5-yl)carbamoyl)ind oline-1-carboxylate | 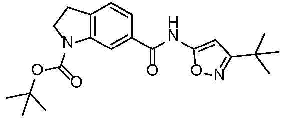 | 0.025 g 1.4 amine | 0.02 g (Y: 54%) | FCC | LCMS (Method 21) -t*_{R}* =1.68 min; m/z (M+1)= 386.4 |
| 75 | tert-butyl 6-((2-((4-methylpiperazin-1-yl)methyl)-6-(trifluoromethyl) pyridin-4-yl)carbamoyl)ind oline-1-carboxylate | 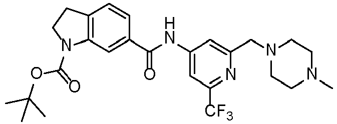 | 0.08 g 1 eq. Intermediate N ACN | 0.054 g (Y: 34%) | FCC | ¹H NMR (400 MHz, DMSO) δ 10.88 (s, 1H), 8.84 (brs, 1H), 8.23 (d, J=1.4 Hz, 1H), 8.15 (s, 1H), 7.60 (dd, J=1.6, 7.8 Hz, 1H), 7.38 (d, J=7.7 Hz, 1H), 4.00 - 3.95 (m, 2H), 3.65 (s, 2H), 3.18 - 3.02 (m, 10H), 2.27 (brs, 3H), 1.53 (brs, 9H) |
| 76 | tert-butyl 6-((2-((dimethylamino) methyl)-6-(trifluoromethyl) pyridin-4-yl)carbamoyl)ind oline-1-carboxylate | 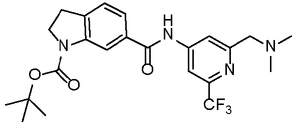 | 0.08 1 eq. intermediate N ACN | 0.07 g (Y=53%) | FCC | ¹H NMR (400 MHz, DMSO) δ 10.99 (s, 1H), 8.27 (s, 1H), 8.23 (s, 1H), 7.59 (dd, J=1.5, 7.7 Hz, 1H), 7.39 (d, J=7.6 Hz, 1H), 6.93 - 6.87 (m, 1H), 4.22 (s, 2H), 3.98 (t, J=8.6 Hz, 2H), 3.15 (t, J=8.7 Hz, 2H), 2.63 (s, 6H), 1.54 (s, 9H). |
| 77 | tert-butyl 6-((5-(trifluoromethyl) pyridin-3-yl)carbamoyl)ind oline-1-carboxylate | 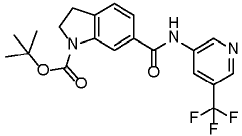 | 0.78 g 1.05 eq. amine ACN | 1.08 g (Y=81%) | FCC | ¹H NMR (300 MHz, DMSO) d 10.74 (s, 1H), 9.19 (d, J=1.5 Hz, 1H), 8.70 - 8.68 (m, 1H), 8.64 - 8.60 (m, 1H), 8.22 (brs, 1H),7.59 (dd, J=1.6, 7.7 Hz, 1H), 7.37 (d, J=7.8 Hz, 1H), 3.97 (t, J=8.8 Hz, 2H), 3.15 (t, J=8.7 Hz, 2H), 1.53 |
| 78 | tert-butyl (R)-6-((2-((3-(dimethylamino) pyrrolidin-1-yl)methyl)-6-(trifluoromethyl) pyridin-4-yl)carbamoyl)ind oline-1-carboxylate | 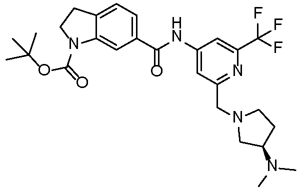 | 0.3 g 1.05 eq. Intermediate R ACN | 0.1 g (Y= 18%) | FCC | ¹H NMR (400 MHz, DMSO) δ 10.90 (s, 1H), 8.32 - 8.22 (m, 2H), 8.12 (s, 1H), 7.62 (dd, J=1.5, 7.7 Hz, 1H), 7.40 (d, J=7.6 Hz, 1H), 4.00 (t, J=9.1 Hz, 2H), 3.82 (d, J=14.6 Hz, 1H), 3.70 (d, J=14.7 Hz, 1H), 3.17 (t, J=8.6 Hz, 2H), 2.80 - 2.64 (m, 3H), 2.60 - 2.55 (m, 1H), 2.42 (dd, J=5.6, 7.7 Hz, 1H), 2.12 (s, 6H), 1.96 - 1.87 (m, 1H), 1.72 - 1.63 (m, 1H), 1.55 (s, 9H). |
| 79 | tert-butyl 6-((3-(2-(pyrrolidin-1-yl)ethoxy)-5-(trifluoromethyl) phenyl)carbamoy 1)indoline-1-carboxylate | 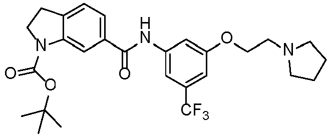 | 0.18 g 1 eq. Intermediate I HATU, eq DIPEA DMF | 0.1 g (Y=27%) | FCC | No data |

### Step 2; N-((4-((4-methylpiperazin-1-yl)methyl)-3-(trifluoromethyl)phenyl)carbamoyl)indoline-6-carboxamide (Intermediate 80)

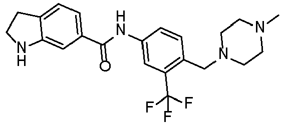

Trifluoroacetic acid (3.3 mL, 42.8 mmol) was added to a solution of Intermediate 73 (2.22 g, 4.28 mmol) in DCM (15 mL) and the mixture was stirred for 4h. The mixture was concentrated in vacuo and the residue passed down a SCX-2 (25g) cartridge and eluted with MeOH (30 mL), followed by 2 M methanolic ammonia solution (50 mL). The ammonia solution eluent was concentrated in vacuo to give a residue which was triturated under ether and filtered to give the title compound (1.47 g, 82%).
¹H NMR (400 MHz, DMSO) δ 10.28 - 10.26 (m, 1H), 8.21 - 8.19 (m, 1H), 8.01 (dd, J=1.9, 8.5 Hz, 1H), 7.68 - 7.65 (m, 1H), 7.16 - 7.15 (m, 2H), 7.03 (s, 1H), 5.75 (s, 1H), 3.55 (s, 2H), 3.48 (dt, J=1.4, 8.6 Hz, 2H), 3.00 - 2.94 (m, 2H), 2.38 - 2.33 (m, 8H), 2.16 - 2.15 (m, 3H)

Following intermediates were prepared via acidic cleavage as described for intermediate 80, step 2. Such procedure may involve minor variations. In some cases, where modification involved chromatographic purification conditions (e.g. prepHPLC or flash chromatography), such changes were reported in the table.

| **Intermediate N°** | **Intermediate name** | **Structure** | **Amount Reagent** | **Amount product (yield)** | **Purification Method** | **Analytical data** |
|---|---|---|---|---|---|---|
| 81 | N-(3-(tert-butyl)isoxazol-5-yl)indoline-6-carboxamide | 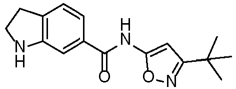 | 0.985 g | 0.4 g (Y: 55%) | No purification | ¹H NMR (400 MHz, DMSO) δ 10.85 (s, 1H), 7.22 - 7.12 (m, 2H), 7.06 (d, J=1.5 Hz, 1H), 6.37 (s, 1H), 5.78 (s, 1H), 4.09 (br s, 1H), 3.46 (t, J=8.1 Hz, 2H), 2.96 (t, J=8.5 Hz, 2H), 1.28 - 1.27 (s, 9H). |
| 82 | N-(2-((4-methylpiperazin-1-yl)methyl)-6-(trifluoromethyl)p yridin-4-yl)indoline-6-carboxamide | 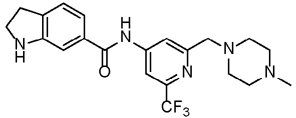 | 0.05 g | 0.02 g (Y=49%) | SCX | ¹H NMR (400 MHz, DMSO) δ 10.68 (s, 1H), 8.25 (d, J=1.8 Hz, 1H), 8.12 (d, J=1.5 Hz, 1H), 7.05 (d, J=1.2 Hz, 1H), 5.81 (s, 1H), 5.76 (s, 1H), 3.64 (s, 1H), 3.62 (s, 2H), 3.49 (dt, J=1.5, 8.6 Hz, 2H), 3.02 - 2.95 (m, 2H), 2.46 - 2.31 (m, 8H), 2.17 (s, 3H) |
| 83 | N-(2-((dimethylamino) methyl)-6-(trifluoromethyl)p yridin-4-yl)indoline-6-carboxamide | 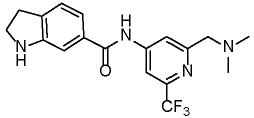 | 0.073 g | 0.02 g (Y=33%) | SCX | ¹H NMR (400 MHz, DMSO) δ 10.66 (s, 1H), 8.23 (d, J=1.8 Hz, 1H), 8.16 (d, J=1.7 Hz, 1H), 7.10 - 7.08 (m, 1H), 7.05 (d, J=1.4 Hz, 1H), 6.87 - 6.86 (m, 1H), 5.80 (s, 1H), 3.56 (s, 2H), 3.48 (dt, J=1.7, 8.8 Hz, 2H), 2.98 (t, J=8.5 Hz, 2H), 2.23 (s, 6H). |
| 84 | (R)-N-(2-((3-(dimethylamino)p yrrolidin-1-yl)methyl)-6-(trifluoromethyl)p yridin-4-yl)indoline-6-carboxamide | 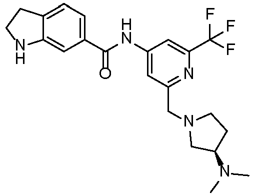 | 0.11 g | 009 g (Y=99%) | SCX | ¹H NMR (400 MHz, DMSO) δ 10.67 (s, 1H), 8.24 (d, J=1.8 Hz, 1H), 8.10 (d, J=1.5 Hz, 1H), 7.21 - 7.15 (m, 2H), 7.05 (s, 1H), 3.78 (d, J=14.7 Hz, 1H), 3.67 (d, J=14.7 Hz, 1H), 3.48 (t, J=9.1 Hz, 2H), 2.98 (t, J=9.1 Hz, 2H), 2.78 - 2.62 (m, 4H), 2.39 (dd, J=5.9, 7.8 Hz, 1H), 2.10 (s, 6H), 1.93 - 1.85 (m, 1H), 1.69 - 1.59 (m, 1H). |
| 85 | N-(5-(trifluoromethyl)p yridin-3-yl)indoline-6-carboxamide | 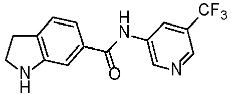 | 1 g | 0.67 g (Y= 88%) | SCX | ¹H NMR (300 MHz, DMSO) d 10.55 (s, 1H), 9.19 (d, J=2.3 Hz, 1H), 8.67 (dd, J=0.8, 1.9 Hz, 1H), 8.65 - 8.61 (m, 1H), 7.22 - 7.15 (m, 2H), 7.05 (s, 1H), 5.79 (s, 1H), 3.49 (dt, J=1.8, 8.6 Hz, 2H), 2.98 (t, J=8.6 Hz, 2H) |
| 86 | N-(3-(2-(pyrrolidin-1-yl)ethoxy)-5-(trifluoromethyl)p henyl)indoline-6-carboxamide | 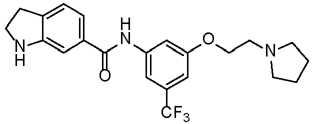 | 0.1 g | 0.04 g (Y= 50%) | FCC | LCMS (Method 22) t*_{R}*: 1.69 min, m/z (M+1)= 420.3 |

### Step 3; 1-((1H pyrazolo[3,4-b]pyridin-5-yl)methyl-N (4-((4-methylpiperazin-1-yl)methyl)-3-(trifluoromethyl)phenyl)indoline-6-carboxamide, Example 75

Sodium triacetoxyborohydride (50.4 mg, 0.238 mmol) was added to a mixture of 1*H*-pyrazolo[3,4-b]pyridine-5-carbaldehyde (22 mg, 0.149 mmol) , Intermediate 80 (50 mg, 0.119 mmol), acetic acid (0.0001 mmol, 7 µL eq) and MgSO₄ (117.32 mg, 0.476 mmol) in DCM (15.00 mL) and the mixture stirred for at room temperature until LCMS indicated consumption of starting material. The mixture was partitioned between saturated NaHCO₃(aq) and DCM, then re-extracted with DCM. The combined organic extracts were dried (Na₂SO₄) and concentrated in vacuo. The crude product was purified by reverse phase HPLC (Xbridge Phenyl 19x150mm, 10um 40-100% MeOH / H₂O (10mM NH₄CO₃), 20ml/min, RT) to give the title compound (50 mg, 76%).

| **Example No.** | **Analytical data** |
|---|---|
| 75 | ¹H NMR (400 MHz, DMSO) δ 13.65 (s, 1H), 10.30 (s, 1H), 8.58 (d, J=2.1 Hz, 1H), 8.22 - 8.20 (m, 2H), 8.15 (s, 1H), 8.04 (dd, J=1.9, 8.4 Hz, 1H), 7.70 (d, J=8.4 Hz, 1H), 7.30 - 7.19 (m, 3H), 4.54 (s, 2H), 3.57 (s, 2H), 3.39 - 3.35 (m, 2H), 2.99 (t, J=8.3 Hz, 2H), 2.40 - 2.32 (m, 8H), 2.17 (s, 3H) |
| | LC-MS (ESI): (Method 2) |
| | t*_{R}* =3.46 min; m/z (M+1)= 550.2 |

The following compounds were prepared via reductive amination as described for Example 75, step 1-3, starting from corresponding intermediate described in step 2 and the suitable corresponding, commercially available or synthesized carbaldehyde in step 3. Such procedures may involve minor variations. In some cases, where modification involved reductive agents (e.g. STAB replaced NaBH₃CN), solvents (DCM replaced by MeOH) use of titanium(IV) isopropoxide or chromatographic purification conditions (e.g. prepHPLC or flash chromatography), such changes were reported in the table.

| **Ex. N°** | **Structure** | **Amount Reagent** | **Amount product (yield)** | **Purification Method** | **Analytical data** |
|---|---|---|---|---|---|
| 76 | 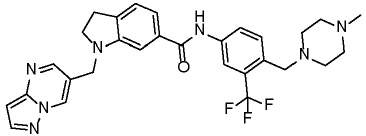 | 0.04 g | 0.033 g (Y: 62%) | prep HPLC | ¹H NMR (400 MHz, DMSO) δ 10.32 (s, 1H), 9.16 (d, J=1.3 Hz, 1H), 8.63 (d, J=2.1 Hz, 1H), 8.23 (d, J=2.4 Hz, 1H), 8.21 (d, J=2.3 Hz, 1H), 8.04 (dd, J=1.9, 8.5 Hz, 1H), 7.70 (d, J=8.5 Hz, 1H), 7.32 - 7.29 (m, 2H), 7.22 (d, J=8.0 Hz, 1H), 6.75 (dd, J=0.9, 2.4 Hz, 1H), 4.49 (s, 2H), 3.58 - 3.56 (m, 2H), 3.42 (t, J=8.6 Hz, 2H), 3.01 (t, J=8.3 Hz, 2H), 2.43 - 2.33 (m, 8H), 2.18 - 2.16 (m, 3H) |
| | | | | | LC-MS (ESI): (Method 2)t*_{R}* =3.54 min; m/z (M+1)= 550.4 |
| 77 | 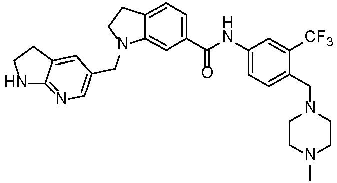 | 0.06 g | 0.04 g (Y=54%) | prep HPLC | ¹H NMR (400 MHz, DMSO) δ 10.28 (s, 1H), 8.21 (d, J=2.1 Hz, 1H), 8.04 (dd, J=2.0, 8.5 Hz, 1H), 7.70 (d, J=8.4 Hz, 2H), 7.25 (dd, J=1.6, 5.5 Hz, 2H), 7.21 - 7.16 (m, 2H), 6.31 (s, 1H), 4.16 (s, 2H), 3.57 (s, 2H), 3.46 (t, J=8.5 Hz, 2H), 3.29 (t, J=8.4 Hz, 2H), 2.99 - 2.91 (m, 4H), 2.39 - 2.34 (m, 6H), 2.17 (s, 3H). |
| | | | | | LC-MS (ESI): (Method 2) t*_{R}* = 2.67 min; m/z (M+1)= 551.2 |
| 78 | 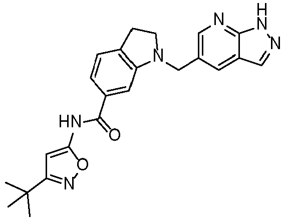 | 0.030 g | 0.024 g (Y:54%) | prep HPLC | ¹H NMR (400 MHz, DMSO) δ 13.68 - 13.64 (m, 1H), 11.69 (s, 1H), 8.59 (d, J=2.0 Hz, 1H), 8.22 (d, J=2.0 Hz, 1H), 8.15 (s, 1H), 7.34 (s, 1H), 7.32 (dd, J=1.2, 7.5 Hz, 1H), 7.19 (d, J=7.5 Hz, 1H), 6.40 (s, 1H), 4.52 (s, 2H), 2.98 (t, J=8.4 Hz, 2H), 1.29 (s, 9H). [2H (3.35) |
| | | | | | LC-MS (ESI): (Method 2) t*_{R}* = 4.60 min; m/z (M+1)= 417.6 |
| 79 | 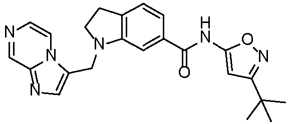 | 0.050 g | 0.050 g (Y:68%) | prep HPLC | ¹H NMR (400 MHz, DMSO) δ 11.71 (s, 1H), 9.11 (d, J=1.5 Hz, 1H), 8.49 (dd, J=1.5, 4.6 Hz, 1H), 7.98 (d, J=4.6 Hz, 1H), 7.88 - 7.87 (m, 1H), 7.46 (d, J=1.3 Hz, 1H), 7.37 (dd, J=1.5, 7.5 Hz, 1H), 7.22 (d, J=7.7 Hz, 1H), 6.42 (s, 1H), 4.78 (s, 2H), 3.26 (t, J=8.4 Hz, 2H), 2.94 (t, J=8.3 Hz, 2H), 1.30 (s, 9H). |
| | | | | | LC-MS (ESI): (Method 2) t*_{R}* = 4.23 min; m/z (M+1)= 417.2 |
| 80 | 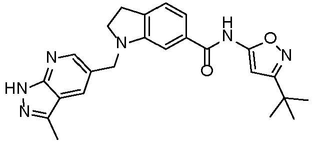 | 0.05 g | 0.009 g (Y=31%) | prep HPLC | ¹H NMR (400 MHz, DMSO) δ 13.23 - 13.20 (m, 1H), 11.72 - 11.68 (m, 1H), 8.55 (d, J=2.0 Hz, 1H), 8.20 (d, J=1.9 Hz, 1H), 7.35 (d, J=1.5 Hz, 1H), 7.32 (dd, J=1.6, 7.6 Hz, 1H), 7.20 (d, J=7.7 Hz, 1H), 6.41 (s, 1H), 4.50 (s, 2H), 2.98 (t, J=8.3 Hz, 2H), 1.30 - 1.29 (m, 9H). |
| | | | | | LC-MS (ESI): (Method 2) t_{R} = 4.72 min; m/z (M+1)= 431.2 |
| 81 | 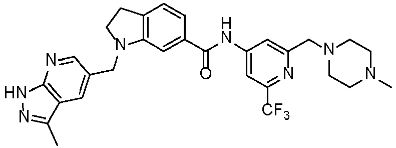 | 0.02 g NaBH₃CN, Ti(OiPr)₄, AcOH aldehyde DCM/MeOH | 0.009 g (Y=31%) | prep HPLC | ¹H NMR (400 MHz, DMSO) δ 13.21 (s, 1H), 10.72 (s, 1H), 8.54 (d, J=2.0 Hz, 1H), 8.26 (d, J=1.8 Hz, 1H), 8.20 (d, J=1.8 Hz, 1H), 8.16 (d, J=1.6 Hz, 1H), 7.34 - 7.28 (m, 2H), 7.23 (d, J=7.5 Hz, 1H), 4.52 (s, 2H), 3.64 (s, 2H), 3.40 - 3.35 (m, 2H), 3.00 (t, J=8.4 Hz, 2H), 2.49 - 2.36 (m, 8H), 2.18 (s, 3H). |
| | | | | | LC-MS (ESI): (Method 2) t_{R}= 3.46 min; m/z (M+1)= 565.4 |
| 82 | 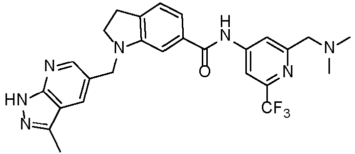 | 0.02 g NaBH₃CN, Ti(OiPr)₄, AcOH aldehyde DCM/MeOH | 0.006 g (Y= 21%) | prep HPLC | ¹H NMR (400 MHz, DMSO) δ 13.23 (s, 1H), 10.69 (s, 1H), 8.55 (d, J=2.0 Hz, 1H), 8.27 (d, J=1.9 Hz, 1H), 8.20 (d, J=1.9 Hz, 1H), 8.17 (d, J=1.6 Hz, 1H), 7.35 - 7.30 (m, 2H), 7.24 (d, J=7.5 Hz, 1H), 4.52 (s, 2H), 3.58 (s, 2H), 3.40 - 3.34 (m, 2H), 3.00 (t, J=8.3 Hz, 2H), 2.51 (s, 3H), 2.25 (s, 6H) |
| | | | | | LC-MS (ESI): (Method 2) t_{R} = 3.35 min; m/z (M+1)= 510.5 |
| 3 | 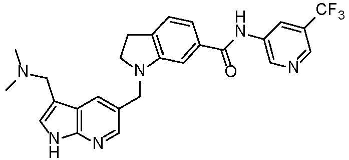 | 0.06 g STAB Ti(OiPr)4, AcOH, aldehyde DCM | 0.04 g (Y= 43%) | prep HPLC | ¹H NMR (400 MHz, DMSO) δ 11.45 (d, J=1.4 Hz, 1H), 10.59 (s, 1H), 9.23 (d, J=2.3 Hz, 1H), 8.70 (m, 1H), 8.64 (m, 1H), 8.24 (d, J=2.0 Hz, 1H), 8.01 (d, J=1.9 Hz, 1H), 7.35 (d, J=2.4 Hz, 1H), 7.32 - 7.28 (m, 2H), 7.22 (d, J=7.5 Hz, 1H), 4.48 (s, 2H), 3.51 (s, 2H), 3.35 (t, J=8.4 Hz, 2H),2.98 (t, J=8.4 Hz, 2H), 2.12 (s, 6H) |
| | | | | | LC-MS (ESI): (Method 2) t_{R} =3.59 min; m/z (M+1)= 495.4 |
| 84 | 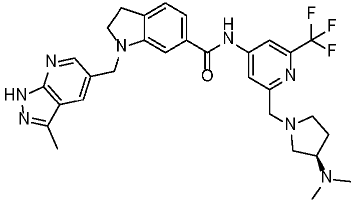 | 0.09 g STAB Ti(OiPr)4, AcOH, aldehyde DCM | 0.036 g (Y= 29%) | prep HPLC | ¹H NMR (400 MHz, DMSO) δ 13.21 (s, 1H), 1070 (s, 1H), 8.54 (d, J=2.0 Hz, 1H), 8.26 (d, J=1.8 Hz, 1H), 8.20 (d, J=1.5 Hz, 1H), 8.12 (d, J=1.6 Hz, 1H), 7.34 - 7.28 (m, 2H), 7.24 (d, J=7.6 Hz, 1H), 4.52 (s, 2H), 3.81 (d, J=14.8 Hz, 1H), 3.71 - 3.67 (m, 1H), 3.37 (t, J=8.1 Hz, 2H), 3.00 (t, J=8.3 Hz, 2H), 2.81 - 2.67 (m, 3H), 2.59 - 2.54 (m, 1H), 2.40 (dd, J=6.4, 8.2 Hz, 1H), 2.11 (s, 6H), 1.94 - 1.85 (m, 1H), 1.70 - 1.62 (m, 1H). 3H of LC-MS (ESI): (Method 2) t*_{R}* =3.03 min; m/z (M+1)= 579.4 |
| 85 | 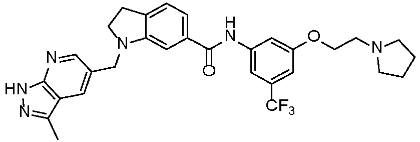 | 0.016 g NaBH₃CN, Ti(OiPr)₄, aldehyde MeOH | 0.008 g (Y=12%) | prep HPLC | ¹H NMR (400 MHz, DMSO) δ 10.28 (s, 1H), 8.54 (d, J=2.0 Hz, 1H), 8.20 (d, J=1.6 Hz, 1H), 7.82 (s, 1H), 7.75 (s, 1H), 7.28 (d, J=8.7 Hz, 3H), 7.22 (d, J=7.4 Hz, 1H), 6.99 (s, 1H), 4.51 (s, 2H), 4.16 (t, J=5.8 Hz, 2H), 3.39 - 3.35 (m, 2H), 2.99 (t, J=8.2 Hz, 2H), 2.82 (t, J=5.8 Hz, 2H), 2.58 - 2.54 (m, 4H), 1.72 - 1.68 (m, 4H). |
| | | | | | LC-MS (ESI): (Method 2) t*_{R}* = 3.86 min; m/z (M+1)= 565.2 |
| 86 | 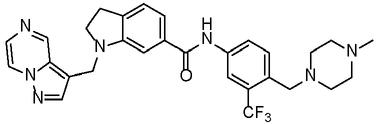 | 0.06 g NaBH₃CN, Ti(OiPr)₄, aldehyde MeOH | 0.01 g (Y= 27%) | Prep HPLC | ¹H NMR (400 MHz, DMSO) δ 10.29 (s, 1H), 9.33 (d, J=1.5 Hz, 1H), 8.75 (dd, J=1.5, 4.8 Hz, 1H), 8.21 - 8.16 (m, 3H), 7.90 (d, J=4.8 Hz, 1H), 7.85 (s, 1H), 7.27 (s, 1H), 7.04 - 6.97 (m, 2H), 4.59 (s, 2H), 3.65 (s, 2H), 3.30 (d, J=8.2 Hz, 2H), 2.85 (t, J=8.2 Hz, 2H), 2.43 - 2.41 (m, 4H), 2.35 - 2.34 (m, 2H), 2.17 (s, 3H). |
| | | | | | LC-MS (ESI): (Method 2) t*_{R}* =3.44 min; m/z (M+1)= 550.5 |

### Example 87; Preparation of N (5-(tert-butyl)-1-methyl -1H-pyrazol-3-yl) -1-(imidazo[1,2-a]pyrazin-3-ylmethyl)indoline-6-carboxamide:

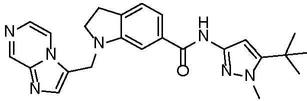

### Example 87

Intermediate 41 (40%, 100 mg, 0.136 mmol) was suspended in thionyl chloride (6.42 mmol, 0.46 mL ) and the reaction mixture was stirred at 50°C for 2h. The reaction mixture was allowed to cool to room temperature and concentrated in vacuo. The residue was suspended in toluene and re-concentrated to give the intermediate acyl chloride.

To a solution of 5-tert-butyl-1-methyl-pyrazol-3-amine (31 mg, 0.204 mmol), in pyridine (0.1 M concentration), was added the acyl chloride. The reaction mixture was stirred at 40°C until LCMS indicated consumption of starting material. The reaction mixture was evaporated, dissolved in MeOH (1mL) and loaded onto an SCX-2 (5g) cartridge, which was washed with MeOH. The compound was released using methanolic ammonia (2M) and the basic solution evaporated. Purification by reverse phase preparative HPLC Sunfire C18 19x150mm, 10um 20-80% acetonitrile / water (10mM NH₄HCO₃), 20ml/min, RT followed by lyophilization gave the title compound (3.3 mg, 6%) .

| **Example No.** | **Analytical data** |
|---|---|
| 87 | ¹H NMR (400 MHz, DMSO) d 10.47 (s, 1H), 9.11 (d, J=1.5 Hz, 1H), 8.49 (dd, J=1.5, 4.8 Hz, 1H), 7.98 (d, J=4.6 Hz, 1H), 7.87 (s, 1H), 7.51 (d, J=1.3 Hz, 1H), 7.34 (dd, J=1.4, 7.6 Hz, 1H), 7.18 - 7.15 (m, 1H), 6.49 (s, 1H), 4.76 (s, 2H), 3.85 (s, 3H), 3.23 (t, J=7.8 Hz, 2H), 2.91 (t, J=8.2 Hz, 2H), 1.36 (s, 9H) |
| | LCMS (Method 2) t*_{R}* =3.50 min, m/z (M+1)= 430.3 |

### Example 88; Preparation of 1-(imidazo[1,2-a]pyrazin-3-ylmethyl)-N-(6-trifluoromethyl)pyrimidin-4-yl)indoline-6-carboxamide

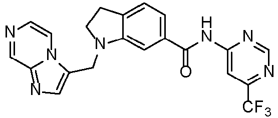

### Example 88

Thionyl chloride (0.40 mL, 5.44 mmol, 40.0 eq) was added to Intermediate 41 and the mixture stirred at room temp for 15 mins. The mixture was taken to dryness in vacuo and the residue suspended in dry DCM (2.00 mL). In a separate flask.1M Lithium bis(trimethylsilyl)amide in THF (0.30 mL, 0.299 mmol, 2.20 eq) was added dropwise to a solution of 6-(trifluoromethyl)pyrimidin-4-amine (27 mg, 0.163 mmol, 1.20 eq)in dry tetrahydrofuran (2.00 mL) at-78°C and the mixture stirred for 15 mins then added dropwise to the suspension of the acid chloride at-78°C. The reaction mixture was allowed to warm to room temperature and stirred for 1h. The solvent was evaporated and the residue dissolved in MeOH (1 mL) and passed down a SCX-2 (10g) cartridge and eluted with MeOH (30 mL), followed by 2 M methanolic ammonia solution (20 mL). The ammonia solution eluent was combined and concentrated in vacuo. The residue (113mg) was dissolved in 9:1 DMSO:water (1.0 mL) and submitted for purification by reverse phase preparative HPLC (Xbridge Phenyl 19x150mm, 10um 40-100% MeOH / H₂O (10mM NH4CO3), 20ml/min) to give the title compound (13.04 mg, 21.04%).

| **Example No.** | **Analytical data** |
|---|---|
| 88 | ¹H NMR (400 MHz, DMSO) d 11.61 - 11.58 (m, 1H), 9.19 (s, 1H), 9.11 (d, J=1.4 Hz, 1H), 8.64 (d, J=1.1 Hz, 1H), 8.50 (dd, J=1.5, 4.8 Hz, 1H), 7.98 (d, J=4.6 Hz, 1H), 7.89 - 7.88 (m, 1H), 7.57 (d, J=1.3 Hz, 1H), 7.43 (dd, J=1.5, 7.7 Hz, 1H), 7.26 - 7.23 (m, 1H), 4.81 (s, 2H), 3.30 - 3.24 (m, 2H), 2.99 - 2.93 (m, 2H). |
| | LC-MS (ESI): (Method 2) t*_{R}* = 4.19 min; m/z (M+1)= 440 |

### Example 89; Preparation of N-[4-[(4-methylpiperazin-1-yl)methyl]-3-(trifluoromethyl)phenyl]-1-[(3-morpholino-1H-pyrazolo[3,4-b]pyridin-5-yl)methyl]indoline-6-carboxamide

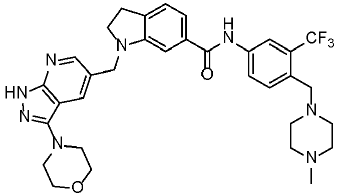

### Example 89

### Step 1; 1-[(3-bromo-1H-pyrazolo[3,4-b]pyridin-5-yl)methyl]-N-[4-[(4-methylpiperazin-1-yl)methyl]-3-(trifluoromethyl)phenyl]indoline-6-carboxamide-Intermediate 87

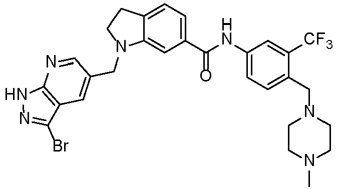

To a solution of Intermediate 54 (74 mg, 0.33 mmol, 1.1 eq) in DCM (0.1 M concentration) was added Intermediate 80(125 mg, 0.30 mmol, 1 eq), titanium(IV) isopropoxide (170.53 mg, 0.060 mmol) and acetic acid (63 µL, 0.90 mmol). The reaction mixture was stirred at room temperature for 1 h. STAB (127.16 mg 0.6 mmol), was added and the reaction mixture was stirred at room temperature until LCMS indicated consumption of starting material to give Intermediate 87 (180 mg, 0.229 mmol, 77%). Taken onto the next step without further purification.
LCMS (Method 22): t*_{R}* =1.69 min, m/z (M+1)= 628/630

### Step 2: N-[4-[(4-methylpiperazin-1-yl)methyl]-3-(trifluoromethyl)phenyl]-1-[(3-morpholino-1H-pyrazolo[3,4-b]pyridin-5-yl)methyl]indoline-6-carboxamide (Example 75)

A solution of Intermediate 87 (80%, 70 mg, 0.0891 mmol) and morpholine (0.0086 mL, 0.0980 mmol) in 1,4-dioxane (3.00 mL) was sparged with N₂. 1M Lithium bis(trimethylsilyl)amide (0.45 mL, 0.446 mmol) was added dropwise and the resulting solution degassed prior to addition of RuPhos Pd G3 (7.5 mg, 8.91 µmol, 0.1000 eq). The mixture was capped and heated to 80°C for 1 h. This sample was cooled to room temperature and quenched with H₂O (1 mL). The mixture was extracted into EtOAc (3 x5 mL), the combined organics dried (MgSO₄) and evaporated. The residue was purified by preparative HPLC (Sunfire C18 19x150mm, 10um 5-60% ACN / H₂O (0.1% FA), 20ml/min, RT). The resulting material was dissolved in 10% MeOH in DCM and applied to a 2 g SCX cartridge. The cartridge was washed with 50:50 MeOH:DCM and the product subsequently eluted with 1N NH₃ in MeOH (20 mL). The solvent was evaporated under high vacuum to afford the title compound (12 mg, 21%).

| **Example No.** | **Analytical data** |
|---|---|
| 89 | ¹H NMR (400 MHz, DMSO) δ 12.61 (s, 1H), 10.31 (s, 1H), 8.48 (d, J=1.9 Hz, 1H), 8.33 (d, J=1.5 Hz, 1H), 8.23 - 8.20 (m, 1H), 8.06 - 8.03 (m, 1H), 7.70 (d, J=8.7 Hz, 1H), 7.31 - 7.26 (m, 2H), 7.20 (d, J=7.5 Hz, 1H), 4.47 (s, 2H), 3.78 (dd, J=4.7, 4.7 Hz, 3H), 3.58 (s, 2H), 2.96 (dd, J=8.3, 8.3 Hz, 2H), 2.66 - 2.12 (m, 11H LCMS (Method 2) t*_{R}* 3.43 min, m/z (M+1) 635.42 |

Comparative newly synthesised compounds having a -C(O)CH₂- linker between the indoline ring and Hy group instead of a -C(O)- or CH₂- linker, either when L₁ is - C(O)NH- or -NHC(O)-.

### Example C1: 1-(2-(imidazo[1,2-a]pyridin-3-yl)acetyl)-N-(3-((4-methylpiperazin-1-yl)methyl)-5-(trifluoromethyl)phenyl)indoline-6-carboxamide

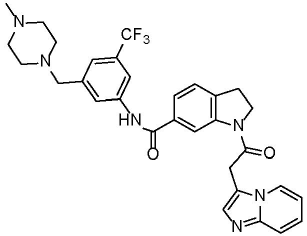

### Example C1

2-(imidazo[1,2-a]pyridin-3-yl)acetic acid (0.042 g, 0.239 mmol) was dissolved in DMF (2.4 ml) then TBTU (0.077 g, 0.239 mmol), TEA (0.067 ml, 0.478 mmol) were added. Mixture was stirred for 15 min at room temperature, then Intermediate 25 (0.1 g, 0.239 mmol) was added. Reaction mixture was stirred overnight at room temperature. Reaction mixture was diluted with DCM (5 ml) and water (5 ml). Mixture was stirred 15 minutes at room temperature, then phases were separated, organic layer was washed with brine (2x15 ml), dried over Na₂SO₄, filtered and concentrated to dryness under reduced pressure. Crude material was triturated with mixture of pentane and DCM 97/3. Residue was purified via preparative HPLC eluting with ACN+0.1%NH₃, H₂O+0.1%NH₃ to afford the title compound (26 mg, 19%).

| **Example No** | **Analytical Data** |
|---|---|
| C1 | ¹H NMR (300 MHz, DMSO-d6) δ 10.48 (s, 1H), 8.55 (s, 1H), 8.37 (d, J = 6.9 Hz, 1H), 8.12 (s, 1H), 7.96 (s, 1H), 7.72 - 7.60 (m, 1H), 7.61 - 7.53 (m, 1H), 7.51 (s, 1H), 7.42 (d, J = 7.8 Hz, 1H), 7.31 (s, 1H), 7.29 - 7.18 (m, 1H), 6.92 (t, J = 6.7 Hz, 1H), 4.39 (t, J = 8.4 Hz, 2H), 4.33 (s, 2H), 3.51 (s, 2H), 2.32 (d, J = 27.0 Hz, 8H), 2.14 (s, 3H). 2 protons are hidden under water. |
| | LC-MS (ESI): (Method 3) t*_{R}* = 2.95 min; m/z (M+1)= 576.9 |

### Example C2: N-(1-(2-(imidazo[1,2-a]pyridin-3-yl)acetyl)indolin-6-yl)-3-((4-methylpiperazin-1-yl)methyl)-5-(trifluoromethyl)benzamide

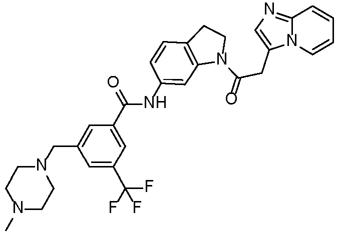

Example C2

2-(imidazo[1,2-a]pyridin-3-yl)acetic acid (37.9 mg, 0.215 mmol), Intermediate 29 (90 mg, 0.215 mmol) and TEA (0.060 ml, 0.430 mmol) were dissolved in DMF (0.2 ml) and DCM (0.6 ml), then TBTU (69.1 mg, 0.215 mmol) was added. Reaction was carried out at RT for 16h. The reaction mixture was diluted with DCM, then extracted with water (1×5mL) and brine (1×5mL). Combined water phases were extracted with DCM. Combined organic phases were dried over Na₂SO₄, filtered and concentrated to dryness under vacuum. The crude material was purified by FCC eluting with DCM/MeOH, from 100:0to 80:20 to afford title compound (83mg, 67%)

| **Example No.** | **Analytical Data** |
|---|---|
| C2 | ¹H NMR (400 MHz, DMSO-d6) δ 10.39 (s, 1H), 8.41 - 8.36 (m, 2H), 8.18 (s, 1H), 8.15 (s, 1H), 7.82 (s, 1H), 7.59 - 7.54 (m, 2H), 7.51 (s, 1H), 7.27 - 7.21 (m, 2H), 6.91 (td, J = 6.8, 1.2 Hz, 1H), 4.35 (t, J = 8.4 Hz, 2H), 4.31 (s, 2H), 3.62 (s, 2H), 3.25-3.23 (m, 2H), 2.37 (s, 8H), 2.18 (s, 3H). |
| | LC-MS (ESI): (Method 3) t*_{R}* = 2.96 min; m/z (M-1)= 575.1 |

### PHARMACOLOGICAL ACTIVITY OF THE COMPOUNDS OF THE INVENTION

### In vitro Assays

### Binding Assays

DDR1 and DDR2 binding assays were performed using Life Technologies LanthaScreen^{™} Europium Kinase Binding assay. The compounds were incubated with 5 nM DDR1 (Carna Biosciences) or 5 nM DDR2 (Life Technologies) for 1 hour at room temperature in white 384-well OptiPlate (PerkinElmer), containing 20 nM or 10 nM Kinase Tracer 178 respectively and 2 nM Europium labelled anti-GST antibody (Life Technologies) in assay buffer (50 mM HEPES pH 7.5, 10 mM MgCI2, 1 mM EGTA and 0.01% BRIJ35). The ratio of fluorescence emission 665 nm/ 615 nm after excitation at 340 nm was obtained using the Tecan Spark 20M plate reader. IC50 values were determined in GraphPad Prism 7.0 software, using 4 parameter model: log(inhibitor) vs. response. IC50 values were converted in Ki using the Cheng-Prusoff equation (Ki=IC50/(1+[Tracer]/Kd).

### DDR1 cell based assay

The inhibition of DDR1 receptor activation by compounds was evaluated by PathHunter^{®} U2OS DDR1 assay (Eurofins DiscoverX), according to the manufacturer's instructions. Briefly, U2OS-DDR1 cells were seeded in white 384-well plates at a density of 5000 cells/well and incubated for 2 hours at 37°C and 5% CO₂. Cells were then treated with compounds at different concentrations and incubated for 30 minutes, before stimulation with bovine Type II Collagen 20 µg/ml and incubation overnight at 37°C and 5% CO₂. PathHunter Detection Reagents were prepared according to the protocol provided by DiscoverX and 20 µl/well of this mix were added to each well. After incubating the plates for 1 hour at room temperature in the dark, luminescence signal was acquired with a plate reader. Raw data were normalized to vehicle control (0% for normalization) and positive control (100% for normalization; cells treated with 20 µg/ml collagen II) and IC50 parameters were calculated in GraphPad Prism 8.0 software, using sigmoidal dose-response curve fitting with variable slope.

### DDR2 cell based assay

The inhibition of DDR2 phosphorylation by compounds was evaluated in HEK293T-DDR2 recombinant cells by phospho-ELISA assay. Briefly, HEK293T-DDR2 cells were seeded in poly-D-lysine-coated 24-well plates at a density of 250.000 cells/well and incubated for 1.5 hours at 37°C and 5% CO₂ in DMEM + 10% FBS. After that, the medium was changed to serum-free DMEM and cells were incubated for 3 hours. Then. test compounds were added at different concentrations 30 minutes before stimulation with bovine Type II Collagen at 50 µg/ml for further 3 hours. For DDR2 phospho-ELISA assay (DuoSet IC Human Phospho-DDR2; R&D Systems), protein extracts were obtained by adding 60 µl/well of lysis buffer prepared according to the manufacturer's instructions. Protein concentration in the samples was determined by BCA assay and the levels of phospho-DDR2 were determined following R&D Systems indications. Raw data were normalized to maximal inhibition control (0% for normalization) and positive control (100% for normalization; cells treated with 20 µg/ml collagen II) and IC50 parameters were calculated in GraphPad Prism 8.0 software, using sigmoidal dose-response curve fitting with variable slope.

The results for individual compounds are provided below in Table 7 wherein the compounds are classified in term of binding affinity for DDR1 and DDR2 receptors (Ki) and in term of potency (IC50) with respect to their inhibitory activity on DDR1 and DDR2 receptors.

| **Example No** | **Ki DDR1** | **Ki DDR2** | **IC50 DDR1** | **IC50 DDR2** |
|---|---|---|---|---|
| **1** | +++ | +++ | +++ | + |
| **2** | +++ | +++ | +++ | +++ |
| **3** | +++ | +++ | +++ | + |
| **4** | +++ | +++ | ++ | - |
| **5** | ++ | + | ++ | - |
| **6** | +++ | +++ | +++ | + |
| **7** | +++ | +++ | +++ | ++ |
| **8** | ++ | ++ | + | - |
| **9** | +++ | +++ | +++ | +++ |
| **10** | +++ | +++ | ++ | - |
| **11** | +++ | +++ | + | - |
| **12** | +++ | +++ | + | - |
| **13** | +++ | +++ | + | - |
| **14** | +++ | +++ | ++ | - |
| **15** | +++ | +++ | + | - |
| **16** | +++ | +++ | ++ | - |
| **17** | +++ | +++ | ++ | - |
| **18** | +++ | +++ | ++ | - |
| **19** | +++ | +++ | ++ | - |
| **20** | +++ | +++ | ++ | - |
| **21** | +++ | +++ | ++ | +++ |
| **22** | +++ | +++ | +++ | ++ |
| **23** | +++ | +++ | + | - |
| **24** | ++ | ++ | +++ | - |
| **25** | +++ | ++ | ++ | - |
| **26** | +++ | +++ | ++ | - |
| **27** | +++ | +++ | +++ | +++ |
| **28** | +++ | ++ | +++ | ++ |
| **29** | +++ | +++ | + | - |
| **30** | +++ | +++ | +++ | - |
| **31** | +++ | +++ | +++ | + |
| **32** | ++ | ++ | +++ | - |
| **33** | +++ | ++ | + | - |
| **34** | +++ | +++ | +++ | ++ |
| **35** | +++ | +++ | +++ | +++ |
| **36** | +++ | +++ | +++ | + |
| **37** | +++ | +++ | +++ | +++ |
| **38** | +++ | +++ | +++ | + |
| **39** | ++ | +++ | +++ | ++ |
| **40** | ++ | +++ | ++ | - |
| **41** | +++ | +++ | ++ | - |
| **42** | +++ | +++ | +++ | - |
| **43** | +++ | +++ | ++ | - |
| **44** | +++ | +++ | +++ | - |
| **45** | +++ | +++ | ++ | - |
| **46** | +++ | ++ | + | - |
| **47** | +++ | +++ | ++ | - |
| **48** | +++ | +++ | ++ | - |
| **49** | +++ | +++ | + | - |
| **50** | +++ | +++ | ++ | - |
| **51** | +++ | +++ | ++ | - |
| **52** | +++ | +++ | + | - |
| **53** | +++ | +++ | + | - |
| **54** | +++ | +++ | + | - |
| **55** | +++ | ++ | +++ | - |
| **56** | +++ | +++ | ++ | - |
| **57** | ++ | + | ++ | - |
| **58** | ++ | +++ | +++ | - |
| **59** | +++ | +++ | +++ | + |
| **60** | +++ | + | + | - |
| **61** | ++ | ++ | + | - |
| **62** | ++ | + | + | - |
| **63** | +++ | +++ | + | - |
| **64** | +++ | +++ | ++ | - |
| **65** | +++ | +++ | +++ | + |
| **66** | +++ | +++ | +++ | - |
| **67** | +++ | +++ | +++ | - |
| **68** | +++ | +++ | +++ | - |
| **69** | +++ | +++ | +++ | +++ |
| **70** | +++ | +++ | ++ | - |
| **71** | +++ | +++ | +++ | - |
| **72** | +++ | +++ | +++ | - |
| **73** | +++ | +++ | +++ | - |
| **74** | +++ | +++ | +++ | - |
| **75** | +++ | +++ | +++ | +++ |
| **76** | +++ | +++ | +++ | - |
| **77** | +++ | ++ | +++ | - |
| **78** | +++ | +++ | +++ | - |
| **79** | +++ | +++ | + | - |
| **80** | +++ | +++ | +++ | +++ |
| **81** | +++ | +++ | +++ | - |
| **82** | +++ | +++ | +++ | - |
| **83** | +++ | +++ | +++ | - |
| **84** | +++ | +++ | +++ | - |
| **85** | +++ | +++ | +++ | - |
| **86** | +++ | +++ | ++ | - |
| **87** | +++ | +++ | ++ | - |
| **88** | +++ | +++ | + | - |
| **89** | +++ | +++ | +++ | + |
| **90** | +++ | +++ | +++ | - |
| **91** | +++ | +++ | +++ | - |
| **92** | +++ | +++ | ++ | - |

| | | | | |
|---|---|---|---|---|
| +: Ki between 50 and 80 nM ++: Ki between 25 and 50 nM +++: Ki lower than 25 nM +: IC50 between 50 and 80 nM ++: IC50 between 25 and 50 nM +++: IC50 lower than 25 nM - : not available | | | | |

As it can be appreciated, the compounds of Table 7, i.e. the compounds of the invention, show a good activity as antagonists of DDR1 and DDR2 receptors. Accordingly, the compounds of the invention can be effectively used for treating disease, disorder or condition associated with DDR receptors, such as fibrosis, e.g. pulmonary fibrosis, idiopathic pulmonary fibrosis (IPF), hepatic fibrosis, renal fibrosis, ocular fibrosis, cardiac fibrosis, arterial fibrosis and systemic sclerosis.

### Comparative Examples

Compounds of the Examples C1 and C2 were tested in the same binding assay described above.

**Table 8**

| **Example No** | **DDR1 Ki (nM)** | **DDR2 Ki (nM)** |
|---|---|---|
| C1 | 58000 | 58000 |
| C2 | 600 | 58000 |

The compounds of the present invention, as shown in Table 7, have both a binding affinity for DDR1 and DDR2 receptors expressed as Ki and an inhibitory potency expressed as IC50 against DDR1 and DDR2 receptors lower than 80 nM, and for most of the compounds lower than 50 nM or even lower than 25 nM. Conversely, comparative Examples C1 and C2, as shown in Table 8, have a binding affinity higher than 600 nM on DDR1 receptor, even of 58000 for C1, and of 58000 on DDR2 receptor for both C1 and C2. These data demonstrate that, conversely to the compounds of Examples C1 and C2 characterized by a -CH₂-C(O)- linker between the indoline ring and Hy group, the presence of a -CH₂- or -C(O)- linker in that position in the present invention compounds unexpectedly and remarkably determines a relevant increase in the inhibitory activity on the DDR1 and DDR2 receptors. In particular, a direct comparison between the compound of Example C1 and the compounds of Examples 14 and 11 of the present invention, differing only for the linker between the indoline group and Hy, highlights that the presence in Examples 14 and 11 of a -C(O)- and a -CH₂- linker respectively determines an unexpected and noteworthy increase in activity on both the DDR1 and DDR2 receptors.

Analogously, a direct comparison between the compound of Example C2 and the compound of Example 16 of the present invention, differing only for the linker between the indoline group and Hy, highlights that the presence of a -C(O)- linker determines an unexpected and valuable increase in activity on both the DDR1 and DDR2 receptors.

## Claims

1. A compound of formula (I) 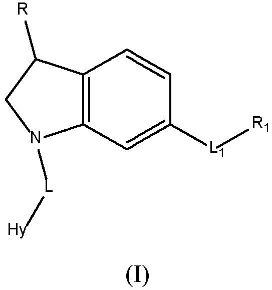 wherein
Ris H or -(C₁-C₄)alkyl;
L is selected from the group consisting of -CH₂- and -C(O)-;
Li is selected from the group consisting of -C(O)NH- and -NHC(O)-;
Hy is a bicyclic heteroaryl optionally substituted by one or more groups selected from
-(C₁-C₄)alkyl, heterocycloalkyl and -(C₁-C₄)alkylene-NR_{A}R_{B}, or Hy is a bicyclic semi saturated heteroaryl;
**R_{A}** is H or -(C₁-C₄)alkyl;
**R_{B}** is H or -(C₁-C₄)alkyl;
**R₁** is selected from the group consisting of:
- **Het** which is a heteroaryl optionally substituted by one or more groups selected from -(C₁-C₄)alkyl, -(C₁-C₄)haloalkyl, -(C₁-C₄)alkylene-heterocycloalkyl-NR_{A}R_{B},
- (C₁-C₄)alkylene-NR_{A}R_{B} and aryl, wherein said aryl is optionally substituted by one or more -(C₁-C₄)alkyl, and
- **X** 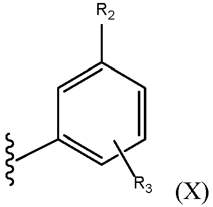
wherein **R₂** is selected from the group consisting of -O(C₁-C₄)haloalkyl, halogen atoms and -(C₁-C₄)haloalkyl;
**R₃** is H or is selected from the group consisting of -O(C₁-C₄)alkyl, -C(O)NH-(C₁-C₄)alkylene-NR_{A}R_{B}, -C(O)-heterocycloalkyl, wherein said heterocycloalkyl is optionally substituted by one or more -(C₁-C₄)alkyl, -O-heterocycloalkyl, wherein said heterocycloalkyl is optionally substituted by one or more -(C₁-C₄)alkyl, -O(Ci-C₄)alkylene-heterocycloalkyl, -(C₁-C₄)alkylene-NR_{A}R_{B}, heteroaryl optionally substituted by one or more -(C₁-C₄)alkyl, and monocyclic heterocycloalkyl-(C₁-C₄)alkylene-, wherein said heterocycloalkyl is optionally substituted by one or more groups selected from -(C₁-C₄)alkyl and -NR_{A}R_{B};
and pharmaceutically acceptable salts thereof.

2. The compound of formula (I) according to claim 1, wherein Li is -C(O)NH-represented by formula (IA)

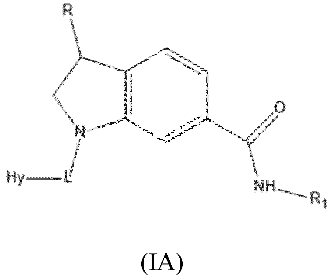

3. The compound of formula (IA) according to claim 2, wherein L is -C(O)-, represented by formula (IA')

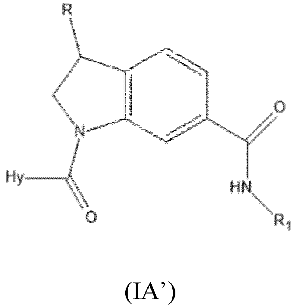

4. The compound of formula (IA') according to claim 3 selected from at least one of:
1-(imidazo[1,2-a]pyridine-3-carbonyl)-N-(3-(4-methyl-1H-imidazol-1-yl)-5-(trifluoromethyl)phenyl)indoline-6-carboxamide;
1-(imidazo[1,2-a]pyrazine-3-carbonyl)-N-(3-(4-methyl-1H-imidazol-1-yl)-5-(trifluoromethyl)phenyl)indoline-6-carboxamide;
1-(imidazo[1,2-a]pyridine-3-carbonyl)-N-(3-((4-methylpiperazin-1-yl)methyl)-5-(trifluoromethyl)phenyl)indoline-6-carboxamide;
1-(imidazo[1,2-a]pyrazine-3-carbonyl)-N-(3-((4-methylpiperazin-1-yl)methyl)-5-(trifluoromethyl)phenyl)indoline-6-carboxamide;
3-methyl-N-(3-(4-methyl-1H-imidazol-1-yl)-5-(trifluoromethyl)phenyl)-1-(1H-pyrazolo[3,4-b]pyridine-5-carbonyl)indoline-6-carboxamide; and
1-(imidazo[1,2-a]pyrazine-3-carbonyl)-3-methyl-N-(3-(4-methyl-1H-imidazol-1-yl)-5-(trifluoromethyl)phenyl)indoline-6-carboxamide.

5. The compound of formula (IA) according to claim 2, wherein L is -CH₂-, represented by formula (IA")

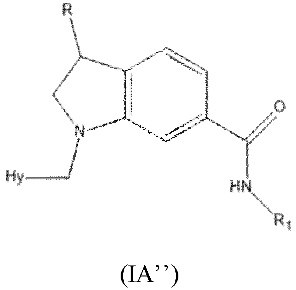

6. The compound of formula (IA") according to claim 5, selected from at least one of:
1-(imidazo[1,2-a]pyridin-3-ylmethyl)-N-(3-(4-methyl-1H-imidazol-1-yl)-5-(trifluoromethyl)phenyl)indoline-6-carboxamide;
1-((1H-pyrrolo[2,3-b]pyridin-5-yl)methyl)-N-(3-(4-methyl-1H-imidazol-1-yl)-5-(trifluoromethyl)phenyl)indoline-6-carboxamide;
1-(imidazo[1,2-a]pyrazin-3-ylmethyl)-N-(3-(4-methyl-1H-imidazol-1-yl)-5 -(trifluoromethyl)phenyl)indoline-6-carboxamide-1-1-((1H-benzo[d]imidazol-5-yl)methyl)-N-(3-(4-methyl-1H-imidazol-1-yl)-5-(trifluoromethyl)phenyl)indoline-6-carboxamide;
1-(isoquinolin-7-ylmethyl)-N-(3-(4-methyl-1H-imidazol-1-yl)-5-(trifluoromethyl)phenyl)indoline-6-carboxamide;
1-((1H-pyrazolo[3,4-b]pyridin-5-yl)methyl)-N-(3-(4-methyl-1H-imidazol-1-yl)-5-(trifluoromethyl)phenyl)indoline-6-carboxamide;
N-(3-(4-methyl-1H-imidazol-1-yl)-5-(trifluoromethyl)phenyl)-1-((3-methyl-1H-pyrazolo[3,4-b]pyridin-5-yl)methyl)indoline-6-carboxamide;
1-((1H-indazol-5-yl)methyl)-N-(3-(4-methyl-1H-imidazol-1-yl)-5-(trifluoromethyl)phenyl)indoline-6-carboxamide;
1-((1H-pyrrolo[2,3-b]pyridin-5-yl)methyl)-N-(3-((4-methylpiperazin-1-yl)methyl)-5-(trifluoromethyl)phenyl)indoline-6-carboxamide;
1-(imidazo[1,2-a]pyrazin-3-ylmethyl)-N-(3-((4-methylpiperazin-1-yl)methyl)-5-(trifluoromethyl)phenyl)indoline-6-carboxamide;
1-(imidazo[1,2-a]pyridin-3-ylmethyl)-N-(3-((4-methylpiperazin-1-yl)methyl)-5-(trifluoromethyl)phenyl)indoline-6-carboxamide;
1-((1H-pyrazolo[3,4-b]pyridin-5-yl)methyl)-3-methyl-N-(3-(4-methyl-1H-imidazol-1-yl)-5-(trifluoromethyl)phenyl)indoline-6-carboxamide;
1-(imidazo[1,2-a]pyridin-3-ylmethyl)-3-methyl-N-(3-(4-methyl-1H-imidazol-1-yl)-5-(trifluoromethyl)phenyl)indoline-6-carboxamide;
1-(imidazo[1,2-a]pyrazin-3-ylmethyl)-3-methyl-N-(3-(4-methyl-1H-imidazol-1-yl)-5-(trifluoromethyl)phenyl)indoline-6-carboxamide;
1-(imidazo[1,2-a]pyrazin-3-ylmethyl)-3-methyl-N-(3-(4-methyl-1H-imidazol-1-yl)-5-(trifluoromethyl)phenyl)indoline-6-carboxamide;
1-((1H-pyrazolo[3,4-b]pyridin-5-yl)methyl)-3-methyl-N-(3-(trifluoromethyl)phenyl)indoline-6-carboxamide;
1-(imidazo[1,2-a]pyrazin-3-ylmethyl)-3-methyl-N-(3-(trifluoromethyl)phenyl)indoline-6-carboxamide;
1-((1H-pyrrolo[2,3-b]pyridin-5-yl)methyl)-3-methyl-N-(3-(trifluoromethyl)phenyl)indoline-6-carboxamide;
1-(imidazo[1,2-a]pyridin-3-ylmethyl)-3-methyl-N-(3-(trifluoromethyl)phenyl)indoline-6-carboxamide;
1-((1H-pyrazolo[3,4-b]pyridin-5-yl)methyl)-3-methyl-N-(3-(trifluoromethyl)phenyl)indoline-6-carboxamide;
1-((1H-pyrazolo[3,4-b]pyridin-5-yl)methyl)-N-(5-(1,1,1-trifluoro-2-methylpropan-2-yl)isoxazol-3-yl)indoline-6-carboxamide;
1-((1H-pyrazolo[3,4-b]pyridin-5-yl)methyl)-N-(3-(trifluoromethoxy)phenyl)indoline-6-carboxamide;
1-((1H-pyrazolo[3,4-b]pyridin-5-yl)methyl)-N-(3-fluoro-5-(trifluoromethyl)phenyl)indoline-6-carboxamide;
1-((1H-pyrazolo[3,4-b]pyridin-5-yl)methyl)-N-(3-(tert-butyl)-1-phenyl-1H-pyrazol-5-yl)indoline-6-carboxamide;
N-(3-fluoro-5-(trifluoromethyl)phenyl)-1-(imidazo[1,2-a]pyrazin-3-ylmethyl)indoline-6-carboxamide;
1-(imidazo[1,2-a]pyrazin-3-ylmethyl)-N-(3-(trifluoromethyl)phenyl)indoline-6-carboxamide;
1-(imidazo[1,2-a]pyrazin-3-ylmethyl)-N-(3-(trifluoromethoxy)phenyl)indoline-6-carboxamide;
N-(3-fluoro-5-(trifluoromethoxy)phenyl)-1-(imidazo[1,2-a]pyrazin-3-ylmethyl)indoline-6-carboxamide;
1-(imidazo[1,2-a]pyrazin-3-ylmethyl)-N-(5-(1,1,1-trifluoro-2-methylpropan-2-yl)isoxazol-3-yl)indoline-6-carboxamide;
1-(imidazo[1,2-a]pyrazin-3-ylmethyl)-N-(4-methoxy-3-(trifluoromethyl)phenyl)indoline-6-carboxamide;
1-(imidazo[1,2-a]pyrazin-3-ylmethyl)-N-(4-((tetrahydrofuran-3-yl)oxy)-3-(trifluoromethyl)phenyl)indoline-6-carboxamide;
N-(4-((dimethylamino)methyl)-3-(trifluoromethyl)phenyl)-1-(imidazo[1,2-a]pyrazin-3-ylmethyl)indoline-6-carboxamide;
1-(imidazo[1,2-a]pyrazin-3-ylmethyl)-N-(3-(morpholinomethyl)-5-(trifluoromethyl)phenyl)indoline-6-carboxamide;
1-(imidazof 1,2-a]pyrazin-3-ylmethyl)-N-(3-(pyrrolidin-1-ylmethyl)-5-(trifluoromethyl)phenyl)indoline-6-carboxamide;
N-(3-((dimethylamino)methyl)-5-(trifluoromethyl)phenyl)-1-(imidazo[1,2-a]pyrazin-3-ylmethyl)indoline-6-carboxamide;
1-(imidazo[1,2-a]pyrazin-3-ylmethyl)-N-(3-(2-(pyrrolidin-1-yl)ethoxy)-5-(trifluoromethyl)phenyl)indoline-6-carboxamide;
1-(imidazo[1,2-a]pyrazin-3-ylmethyl)-N-(3-(2-morpholinoethoxy)-5-(trifluoromethyl)phenyl)indoline-6-carboxamide;
1-(imidazo[1,2-a]pyrazin-3-ylmethyl)-N-(4-((1-methylpiperidin-4-yl)oxy)-3-(trifluoromethyl)phenyl)indoline-6-carboxamide;
1-(imidazo[1,2-a]pyridin-3-ylmethyl)-N-(3-(trifluoromethoxy)phenyl)indoline-6-carboxamide;
N-(3-fluoro-5-(trifluoromethoxy)phenyl)-1-(imidazo[1,2-a]pyridin-3-ylmethyl)indoline-6-carboxamide;
1-(imidazo[1,2-a]pyridin-3-ylmethyl)-N-(3-(trifluoromethyl)phenyl)indoline-6-carboxamide;
1-(imidazo[1,2-a]pyridin-3-ylmethyl)-N-(5-(1,1,1-trifluoro-2-methylpropan-2-yl)isoxazol-3-yl)indoline-6-carboxamide;
N-(3-fluoro-5-(trifluoromethyl)phenyl)-1-(imidazo[1,2-a]pyridin-3-ylmethyl)indoline-6-carboxamide;
1-((1H-benzo[d]imidazol-5-yl)methyl)-N-(3-(trifluoromethyl)phenyl)indoline-6-carboxamide;
1-((1H-benzo[d]imidazol-5-yl)methyl)-N-(3-(trifluoromethoxy)phenyl)indoline-6-carboxamide;
1-((1H-benzo[d]imidazol-5-yl)methyl)-N-(3-fluoro-5-(trifluoromethyl)phenyl)indoline-6-carboxamide;
1-(pyrazolo[1,5-a]pyrimidin-6-ylmethyl)-N-(3-(trifluoromethyl)phenyl)indoline-6-carboxamide;
3-ethyl-1-(imidazo[1,2-a]pyrazin-3-ylmethyl)-N-(3-(4-methyl-1H-imidazol-1-yl)-5-(trifluoromethyl)phenyl)indoline-6-carboxamide;
1-((3-methyl-1H-pyrazolo[3,4-b]pyridin-5-yl)methyl)-N-(4-((4-methylpiperazin-1-yl)methyl)-3-(trifluoromethyl)phenyl)indoline-6-carboxamide;
1-((3-methyl-1H-pyrazolo[3,4-b]pyridin-5-yl)methyl)-N-(3-(pyrrolidin-1-ylmethyl)-5-(trifluoromethyl)phenyl)indoline-6-carboxamide;
N-(5-(tert-butyl)isoxazol-3-yl)-1-((3-methyl-1H-pyrazolo[3,4-b]pyridin-5-yl)methyl)indoline-6-carboxamide;
1-((3-methyl-1H-pyrazolo[3,4-b]pyridin-5-yl)methyl)-N-(3-((4-methylpiperazin-1-yl)methyl)-5-(trifluoromethyl)phenyl)indoline-6-carboxamide;
N-(4-((dimethylamino)methyl)-3-(trifluoromethyl)phenyl)-1-((3-methyl-1H-pyrazolo[3,4-b]pyridin-5-yl)methyl)indoline-6-carboxamide;
1-((3-methyl-1H-pyrazolo[3,4-b]pyridin-5-yl)methyl)-N-(4-(pyrrolidin-1-ylmethyl)-3-(trifluoromethyl)phenyl)indoline-6-carboxamide;
1-((3-methyl-1H-pyrazolo[3,4-b]pyridin-5-yl)methyl)-N-(5-(trifluoromethyl)pyridin-3-yl)indoline-6-carboxamide;
(R)-N-(3-((3-(dimethylamino)pyrrolidin-1-yl)methyl)-5-(trifluoromethyl)phenyl)-1-((3-methyl-1H-pyrazolo[3,4-b]pyridin-5-yl)methyl)indoline-6-carboxamide;
N-(3-((2-(dimethylamino)ethyl)carbamoyl)-5-(trifluoromethyl)phenyl)-1-((3-methyl-1H-pyrazolo[3,4-b]pyridin-5-yl)methyl)indoline-6-carboxamide;
1-((3-methyl-1H-pyrazolo[3,4-b]pyridin-5-yl)methyl)-N-(3-(4-methylpiperazine-1-carbonyl)-5-(trifluoromethyl)phenyl)indoline-6-carboxamide;
1-((1H-pyrazolo[3,4-b]pyridin-5-yl)methyl)-N-(4-((4-methylpiperazin-1-yl)methyl)-3-(trifluoromethyl)phenyl)indoline-6-carboxamide;
N-(4-((4-methylpiperazin-1-yl)methyl)-3-(trifluoromethyl)phenyl)-1-(pyrazolo[1,5-a]pyrimidin-6-ylmethyl)indoline-6-carboxamide;
1-((2,3-dihydro-1H-pyrrolo[2,3-b]pyridin-5-yl)methyl)-N-(4-((4-methylpiperazin-1-yl)methyl)-3-(trifluoromethyl)phenyl)indoline-6-carboxamide;
1-((1H-pyrazolo[3,4-b]pyridin-5-yl)methyl)-N-(3-(tert-butyl)isoxazol-5-yl)indoline-6-carboxamide;
N-(3 -(tert-butyl)isoxazol-5-yl)-1-(imidazo[1,2-a]pyrazin-3-ylmethyl)indoline-6-carboxamide;
N-(3-(tert-butyl)isoxazol-5-yl)-1-((3-methyl-1H-pyrazolo[3,4-b]pyridin-5-yl)methyl)indoline-6-carboxamide;
1-((3-methyl-1H-pyrazolo[3,4-b]pyridin-5-yl)methyl)-N-(2-((4-methylpiperazin-1-yl)methyl)-6-(trifluoromethyl)pyridin-4-yl)indoline-6-carboxamide;
N-(2-((dimethylamino)methyl)-6-(trifluoromethyl)pyridin-4-yl)-1-((3-methyl-1H-pyrazolo[3,4-b]pyridin-5-yl)methyl)indoline-6-carboxamide;
1-((3-((dimethylamino)methyl)-1H-pyrrolo[2,3-b]pyridin-5-yl)methyl)-N-(5-(trifluoromethyl)pyridin-3-yl)indoline-6-carboxamide;
(R)-N-(2-((3-(dimethylamino)pyrrolidin-1-yl)methyl)-6-(trifluoromethyl)pyridin-4-yl)-1-((3-methyl-1H-pyrazolo[3,4-b]pyridin-5-yl)methyl)indoline-6-carboxamide;
1-((3-methyl-1H-pyrazolo[3,4-b]pyridin-5-yl)methyl)-N-(3-(2-(pyrrolidin-1-yl)ethoxy)-5-(trifluoromethyl)phenyl)indoline-6-carboxamide;
N-(4-((4-methylpiperazin-1-yl)methyl)-3-(trifluoromethyl)phenyl)-1-(pyrazolo[1,5-a]pyrazin-3-ylmethyl)indoline-6-carboxamide;
N-(5-(tert-butyl)-1-methyl-1H-pyrazol-3-yl)-1-(imidazo[1,2-a]pyrazin-3-ylmethyl)indoline-6-carboxamide;
1-(imidazo[1,2-a]pyrazin-3-ylmethyl)-N-(6-(trifluoromethyl)pyrimidin-4-yl)indoline-6-carboxamide;
N-(4-((4-methylpiperazin-1-yl)methyl)-3-(trifluoromethyl)phenyl)-1-((3-morpholino-1H-pyrazolo[3,4-b]pyridin-5-yl)methyl)indoline-6-carboxamide;
1-((3-((dimethylamino)methyl)-1H-pyrrolo[2,3-b]pyridin-5-yl)methyl)-N-(3-(trifluoromethyl)phenyl)indoline-6-carboxamide;
1-((1H-pyrazolo[3,4-b]pyridin-5-yl)methyl)-3-methyl-N-(5-(trifluoromethyl)pyridin-3-yl)indoline-6-carboxamide; and
1-(imidazo[1,2-a]pyrazin-3-ylmethyl)-3-methyl-N-(5-(trifluoromethyl)pyridin-3-yl)indoline-6-carboxamide.

7. The compound of formula (I) according to claim 1, wherein Li is -NHC(O)-, represented by formula (IB)

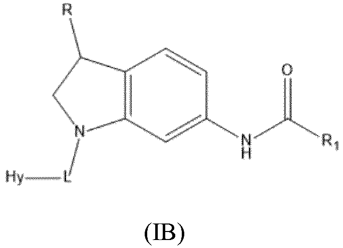

8. The compound of formula (IB) according to claim 7 selected from at least one of:
N-(1-(imidazo[1,2-a]pyridine-3-carbonyl)indolin-6-yl)-3-((4-methylpiperazin-1-yl)methyl)-5-(trifluoromethyl)benzamide;
N-(1-(benzo[d]thiazol-6-ylmethyl)indolin-6-yl)-3-((4-methylpiperazin-1-yl)methyl)-5-(trifluoromethyl)benzamide;
N-(1-(imidazo[1,2-a]pyrazin-3-ylmethyl)indolin-6-yl)-3-((4-methylpiperazin-1-yl)methyl)-5-(trifluoromethyl)benzamide;
N-(1-(imidazo[1,2-a]pyridine-3-carbonyl)indolin-6-yl)-3-(4-methyl-1H-imidazol-1-yl)-5-(trifluoromethyl)benzamide;
N-(1-(imidazo[1,2-a]pyrazine-3-carbonyl)indolin-6-yl)-3-(4-methyl-1H-imidazol-1-yl)-5-(trifluoromethyl)benzamide;
N-(1-(1H-pyrazolo[3,4-b]pyridine-5-carbonyl)indolin-6-yl)-3-(4-methyl-1H-imidazol-1-yl)-5-(trifluoromethyl)benzamide;
N-(1-(imidazo[1,2-a]pyrazin-3-ylmethyl)indolin-6-yl)-3-(4-methyl-1H-imidazol-1-yl)-5-(trifluoromethyl)benzamide;
N-(1-(imidazo[1,2-a]pyridin-3-ylmethyl)indolin-6-yl)-3-(4-methyl-1H-imidazol-1-yl)-5-(trifluoromethyl)benzamide;
N-(1-((1H-pyrazolo[3,4-b]pyridin-5-yl)methyl)indolin-6-yl)-3-(trifluoromethyl)benzamide; and
N-(1-((1H-pyrazolo[3,4-b]pyridin-5-yl)methyl)indolin-6-yl)-3-(4-methyl-1H-imidazol-1-yl)-5-(trifluoromethyl)benzamide.

9. A pharmaceutical composition comprising a compound of formula (I) according to any one of claims 1 to 8, in admixture with one or more pharmaceutically acceptable carrier or excipient.

10. The pharmaceutical composition according to claim 9 for administration by inhalation.

11. The compound of formula (I) according to any one of claims 1 to 8 or the pharmaceutical composition according to claim 9 or 10 for use as a medicament.

12. The compound of formula (I) or the pharmaceutical composition for use according to claim 11 in the prevention and/or treatment of a disease, disorder or condition associated with dysregulation of DDR.

13. The compound of formula (I) or the pharmaceutical composition for use according to claim 11 or 12 in the prevention and/or treatment of fibrosis and/or diseases, disorders or conditions that involve fibrosis.

14. The compound of formula (I) or the pharmaceutical composition for use according to claim 13 in the prevention and/or treatment of fibrosis including pulmonary fibrosis, idiopathic pulmonary fibrosis (IPF), hepatic fibrosis, renal fibrosis, ocular fibrosis, cardiac fibrosis, arterial fibrosis and systemic sclerosis.

15. The compound of formula (I) or the pharmaceutical composition for use according to claim 14 in the prevention and/or treatment of idiopathic pulmonary fibrosis (IPF).

## Patentansprüche

1. Eine Verbindung der Formel (I), 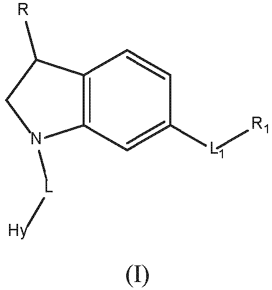 wobei
R H oder -(C₁-C₄)Alkyl ist,
L aus der Gruppe ausgewählt wird, die aus -CH₂- und -C(O)- besteht,
Li aus der Gruppe ausgewählt ist, die aus -C(O)NH- und -NHC(O)- besteht, Hy ein bizyklisches Heteroaryl ist, das optional durch eine oder mehrere Gruppen ersetzt wird, die ausgewählt wurde(n) aus
-(C₁-C₄)Alkyl, Heterocycloalkyl und -(C₁-C₄)Alkylen-NR_{A}R_{B} oder wobei Hy ein bizyklisches, teilgesättigtes Heteroaryl ist,
**R_{A}** H oder -(C₁-C₄)Alkyl ist,
**R_{B}** H oder (C₁-C₄)Alkyl ist,
**R₁** aus der Gruppe ausgewählt ist, die aus Folgendem besteht:
- **Het,** welches ein Heteroaryl ist, das optional durch eine oder mehrere Gruppen substituiert wird, ausgewählt aus -(C₁-C₄)Alkyl, -(C₁-C₄)Halogenalkyl, -(C₁-C₄)Alkylen-Heterocycloalkyl-NR_{A}R_{B}, -(C₁-C₄)Alkylen-NR_{A}R_{B} und Aryl, wobei das besagte Aryl optional substituiert wird durch ein oder mehrere -(C₁-C₄)Alkyle, und
- **X,** 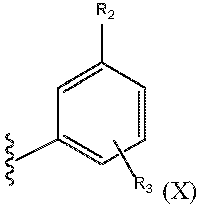
wobei **R₂** ausgewählt wird aus der Gruppe bestehend aus -O(C₁-C₄)-Halogenalkyl, Halogenatomen und -(C₁-C₄)-Halogenalkyl,
**R₃** H ist oder ausgewählt wird aus der Gruppe bestehend aus -O(C₁-C₄)Alkyl, - C(O)NH-(C₁-C₄)Alkylen-NR_{A}R_{B}, -C(O)-Heterocycloalkyl, wobei das besagte Heterocycloalkyl optional substituiert wird durch ein oder mehrere der Folgenden: -(C₁-C₄)Alkyl, -O-Heterocycloalkyl, wobei das besagte Heterocycloalkyl optional substituiuert wird durch ein oder mehrere der Folgenden: -(C₁-C₄)Alkyl, -O(C₁-C₄)Alkylen-Heterocycloalkyl, -(C₁-C₄)Alkylen-NR_{A}R_{B}, wobei das Heteroaryl optional substituiert wird durch ein oder mehrere der Folgenden: -(C₁-C₄)Alkyl und monozyklisches Heterocycloalkyl-(C₁-C₄)Alkylen-, wobei das besagte Heterocycloalkyl optional substituiert wird durch eine oder mehrere Gruppen, ausgewählt aus -(C₁-C₄)Alkyl und -NR_{A}R_{B}
und pharmazeutisch akzeptablen Salzen davon.

2. Die Verbindung der Formel (I) nach Anspruch 1, wobei Li gleich -C(O)NH-, dargestellt durch die Formel (IA)

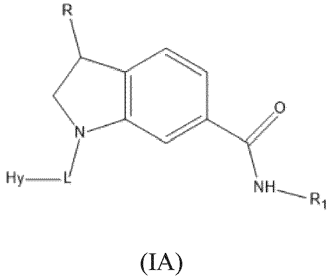

3. Die Verbindung der Formel (IA) nach Anspruch 2, wobei L gleich -C(O)-, dargestellt durch die Formel (IA')

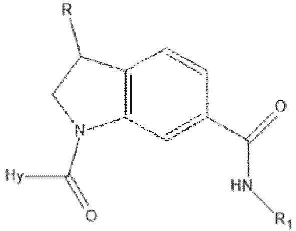

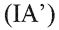

4. Die Verbindung der Formel (IA') nach Anspruch 3, ausgewählt aus mindestens einer der folgenden Verbindungen:
1-(Imidazo[1,2-a]pyridin-3-carbonyl)-N-(3-(4-methyl-1H-imidazol-1-yl)-5-(trifluormethyl)phenyl)indolin-6-carboxamid,
1-(Imidazo[1,2-a]pyrazin-3-carbony1)-N-(3-(4-methyl-1H-imidazol-1-yl)-5-(trifluormethyl)phenyl)indolin-6-carboxamid,
1-(Imidazo[1,2-a]pyridin-3-carbonyl)-N-(3-((4-methylpiperazin-1-yl)methyl)-5-(trifluormethyl)phenyl)indolin-6-carboxamid,
1-(Imidazo[1,2-a]pyrazin-3-carbonyl)-N-(3-((4-methylpiperazin-1-yl)methyl)-5-(trifluormethyl)phenyl)indolin-6-carboxamid,
3-Methyl-N-(3-(4-Methyl-1H-imidazol-1-yl)-5-(trifluormethyl)phenyl)-1-(1H-pyrazol[3,4-b]pyridin-5-carbonyl)indolin-6-carboxamid und
1-(Imidazo[1,2-a]pyrazin-3-carbonyl)-3-methyl-N-(3-(4-methyl-1H-imidazol-1-yl)-5-(trifluormethyl)phenyl)indolin-6-carboxamid.

5. Die Verbindung der Formel (IA) nach Anspruch 2, wobei L gleich -CH₂-, dargestellt durch die Formel (IA")

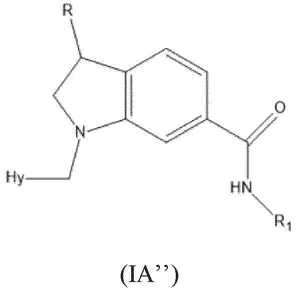

6. Die Verbindung der Formel (IA") nach Anspruch 5, ausgewählt aus mindestens einer der folgenden Verbindungen:
1-(Imidazo[1,2-a]pyridin-3-ylmethyl)-N-(3-(4-methyl-1H-imidazol-1-yl)-5-(trifluormethyl)phenyl)indolin-6-carboxamid;
1-((1H-pyrrol[2,3-b]pyridin-5-yl)methyl)-N-(3-(4-methyl-1H-imidazol-1-yl)-5-(trifluormethyl)phenyl)indolin-6-carboxamid,
1-(Imidazo[1,2-a]pyrazin-3-ylmethyl)-N-(3-(4-methyl-1H-imidazol-1-yl)-5-(trifluormethyl)phenyl)indolin-6-carboxamid;
1-((1H-benzo[d]imidazol-5-yl)methyl)-N-(3-(4-methyl-1H-imidazol-1-yl)-5-(trifluormethyl)phenyl)indolin-6-carboxamid,
1-(Isoquinolin-7-ylmethyl)-N-(3-(4-methyl-1H-imidazol-1-yl)-5-(trifluormethyl)phenyl)indolin-6-carboxamid,
1-((1H-Pyrazol[3,4-b]pyridin-5-yl)methyl)-N-(3-(4-methyl-1H-imidazol-1-yl)-5-(trifluormethyl)phenyl)indolin-6-carboxamid,
N-(3-(4-Methyl-1H-imidazol-1-yl)-5-(trifluormethyl)phenyl)-1-((3-methyl-1H-pyrazol[3,4-b]pyridin-5-yl)methyl)indolin-6-carboxamid,
1-((1H-Indazol-5-yl)methyl)-N-(3-(4-methyl-1H-imidazol-1-yl)-5-(trifluormethyl)phenyl)indolin-6-Carboxamid,
1-((1H-Pyrrol[2,3-b]pyridin-5-yl)methyl)-N-(3-((4-methylpiperazin-1-yl)methyl)-5-(trifluormethyl)phenyl)indolin-6-carboxamid,
1-(Imidazo[1,2-a]pyrazin-3 -ylmethyl)-N-(3 -((4-methylpiperazin-1-yl)methyl)-5-(trifluormethyl)phenyl)indolin-6-carboxamid,
1-(Imidazo[1,2-a]pyridin-3 -ylmethyl)-N-(3-((4-methylpiperazin-1 - yl)methyl)-5-(trifluormethyl)phenyl)indolin-6-carboxamid,
1-((1H-Pyrazol[3,4-b]pyridin-5-yl)methyl)-3-methyl-N-(3-(4-methyl-1H-imidazol-1-yl)-5-(trifluormethyl)phenyl)indolin-6-carboxamid,
1-(Imidazo[1,2-a]pyridin-3-ylmethyl)-3-methyl-N-(3-(4-methyl-1H-imidazol-1-yl)-5-(trifluormethyl)phenyl)indolin-6-carboxamid,
1-(Imidazo[1,2-a]pyrazin-3 -ylmethyl)-3 -methyl-N-(3 -(4-methyl- 1H-imidazol-1-yl)-5-(trifluormethyl)phenyl)indolin-6-carboxamid,
1-(Imidazo[1,2-a]pyrazin-3 -ylmethyl)-3 -methyl-N-(3 -(4-methyl- 1H-imidazol-1-yl)-5-(trifluormethyl)phenyl)indolin-6-carboxamid,
1-((1H-Pyrazol[3,4-b]pyridin-5-yl)methyl)-3-methyl-N-(3-(trifluormethyl)phenyl)indolin-6-carboxamid,
1-(Imidazo[1,2-a]pyrazin-3-ylmethyl)-3-methyl-N-(3-(trifluormethyl)phenyl)indolin-6-carboxamid,
1-((1H-Pyrrol[2,3-b]pyridin-5-yl)methyl)-3-methyl-N-(3-(trifluormethyl)phenyl)Indolin-6-carboxamid,
1-(Imidazo[1,2-a]pyridin-3-ylmethyl)-3-methyl-N-(3-(trifluormethyl)phenyl)indolin-6-carboxamid,
1-((1H-Pyrazol[3,4-b]pyridin-5-yl)methyl)-3-methyl-N-(3-(trifluormethyl)phenyl)indolin-6-carboxamid,
1-(1H-Pyrazol[3,4-b]pyridin-5-yl)methyl)-N-(5-(1,1,1-trifluor-2-methylpropan-2-yl)isoxazol-3-yl)indolin-6-carboxamid,
1-((1H-Pyrazol[3,4-b]pyridin-5-yl)methyl)-N-(3-(trifluormethoxy)phenyl)indolin-6-Carboxamid,
1-((1H-Pyrazol[3,4-b]pyridin-5-yl)methyl)-N-(3-fluoro-5-(trifluormethyl)phenyl)indolin-6-carboxamid,
1-((1H-Pyrazol[3,4-b]pyridin-5-yl)methyl)-N-(3-(tert-butyl)-1-phenyl-1H-pyrazol-5-yl)indolin-6-carboxamid,
N-(3-Fluor-5-(trifluormethyl)phenyl)-1-(imidazo[1,2-a]pyrazin-3-ylmethyl)indolin-6-carboxamid,
1-(Imidazo[1,2-a]pyrazin-3-ylmethyl)-N-(3-(trifluormethyl)phenyl)indolin-6-carboxamid,
1-(Imidazo[1,2-a]pyrazin-3-ylmethyl)-N-(3-(trifluormethoxy)phenyl)indolin-6-carboxamid,
N-(3-Fluor-5-(trifluormethoxy)phenyl)-1-(imidazo[1,2-a]pyrazin-3-ylmethyl)indolin-6-carboxamid,
1-(Imidazo[1,2-a]pyrazin-3-ylmethyl)-N-(5-(1,1,1-trifluor-2-methylpropan-2-yl)isoxazol-3-yl)indolin-6-carboxamid,
1-(Imidazo[1,2-a]pyrazin-3-ylmethyl)-N-(4-methoxy-3-(trifluormethyl)phenyl)indolin-6-carboxamid,
1-(Imidazo[1,2-a]pyrazin-3-ylmethyl)-N-(4-((tetrahydrofuran-3-yl)oxy)-3-(trifluormethyl)phenyl)indolin-6-carboxamid,
N-(4-((Dimethylamino)methyl)-3-(trifluormethyl)phenyl)-1-(imidazo[1,2-a]pyrazin-3-ylmethyl)indolin-6-carboxamid,
1-(Imidazo[1,2-a]pyrazin-3-ylmethyl)-N-(3-(morpholinomethyl)-5-(trifluormethyl)phenyl)indolin-6-carboxamid,
1-(Imidazo[1,2-a]pyrazin-3-ylmethyl)-N-(3-(pyrrolidin-1-ylmethyl)-5-(trifluormethyl)phenyl)indolin-6-carboxamid,
N-(3-((Dimethylamino)methyl)-5-(trifluormethyl)phenyl)-1-(imidazo[1,2-a]pyrazin-3-ylmethyl)indolin-6-carboxamid,
1-(Imidazo[1,2-a]pyrazin-3-ylmethyl)-N-(3-(2-(pyrrolidin-1-yl)ethoxy)-5-(trifluormethyl)phenyl)indolin-6-carboxamid,
1-(Imidazo[1,2-a]pyrazin-3-ylmethyl)-N-(3-(2-morpholinoethoxy)-5-(trifluormethyl)phenyl)indolin-6-carboxamid,
1-(Imidazo[1,2-a]pyrazin-3-ylmethyl)-N-(4-((1-methylpiperidin-4-yl)oxy)-3-(trifluormethyl)phenyl)indolin-6-carboxamid,
1-(Imidazo[1,2-a]pyridin-3-ylmethyl)-N-(3-(trifluormethoxy)phenyl)indolin-6-carboxamid,
N-(3-Fluor-5-(trifluormethoxy)phenyl)-1-(imidazo[1,2-a]pyridin-3-ylmethyl)indolin-6-carboxamid,
1-(Imidazo[1,2-a]pyridin-3-ylmethyl)-N-(3-(trifluormethyl)phenyl)indolin-6-carboxamid,
1-(Imidazo[1,2-a]pyridin-3-ylmethyl)-N-(5-(1, 1,1 -trifluor-2-methylpropan-2-yl)isoxazol-3-yl)indolin-6-carboxamid,
N-(3-Fluor-5-(trifluormethyl)phenyl)-1-(imidazo[1,2-a]pyridin-3-ylmethyl)indolin-6-carboxamid,
1-((1H-benzo[d]imidazol-5-yl)methyl)-N-(3-(trifluormethyl)phenyl)indolin-6-carboxamid,
1-((1H-benzo[d]imidazol-5-yl)methyl)-N-(3-(trifluormethoxy)phenyl)indolin-6-carboxamid,
1-((1H-benzo[d]imidazol-5-yl)methyl)-N-(3-fluor-5-(trifluormethyl)phenyl)indolin-6-carboxamid,
1-(Pyrazol[1,5-a]pyrimidin-6-ylmethyl)-N-(3-(trifluormethyl)phenyl)indolin-6-carboxamid,
3-Ethyl-1-(imidazo[1,2-a]pyrazin-3-ylmethyl)-N-(3-(4-methyl-1H-imidazol-1-yl)-5-(trifluormethyl)phenyl)indolin-6-carboxamid,
1-((3-Methyl-1H-pyrazol[3,4-b]pyridin-5-yl)methyl)-N-(4-((4-methylpiperazin-1-yl)methyl)-3-(trifluormethyl)phenyl)indolin-6-carboxamid,
1-((3-Methyl-1H-pyrazol[3,4-b]pyridin-5-yl)methyl)-N-(3-(pyrrolidin-1-ylmethyl)-5-(trifluormethyl)phenyl)indolin-6-carboxamid,
N-(5-(Tert-butyl)isoxazol-3-yl)-1-((3-methyl-1H-pyrazol[3,4-b]pyridin-5-yl)methyl)indolin-6-carboxamid,
1-((3-Methyl-1H-pyrazol[3,4-b]pyridin-5-yl)methyl)-N-(3-((4-methylpiperazin-1-yl)methyl)-5-(trifluormethyl)phenyl)indolin-6-carboxamid,
N-(4-((Dimethylamino)methyl)-3-(trifluormethyl)phenyl)-1-((3-methyl-1H-pyrazol[3,4-b]pyridin-5-yl)methyl)indolin-6-carboxamid,
1-((3-Methyl-1H-pyrazol[3,4-b]pyridin-5-yl)methyl)-N-(4-(pyrrolidin-1-ylmethyl)-3-(trifluormethyl)phenyl)indolin-6-carboxamid,
1-((3-Methyl-1H-pyrazol[3,4-b]pyridin-5-yl)methyl)-N-(5-(trifluormethyl)pyridin-3-yl)indolin-6-carboxamid,
(R)-N-(3-((3-(Dimethylamino)pyrrolidin-1-yl)methyl)-5-(trifluormethyl)phenyl)-1-((3-methyl-1H-pyrazol[3,4-b]pyridin-5-yl)methyl)indolin-6-carboxamid,
N-(3-((2-(Dimethylamino)ethyl)carbamoyl)-5-(trifluormethyl)phenyl)-1-((3-methyl-1H-pyrazol[3,4-b]pyridin-5-yl)methyl)indolin-6-carboxamid,
1-((3-Methyl-1H-pyrazol[3,4-b]pyridin-5-yl)methyl)-N-(3-(4-methylpiperazin-1-carbonyl)-5-(trifluormethyl)phenyl)indolin-6-carboxamid,
1-((1H-Pyrazol[3,4-b]pyridin-5-yl)methyl)-N-(4-((4-methylpiperazin-1-yl)methyl)-3-(trifluormethyl)phenyl)indolin-6-carboxamid,
N-(4-((4-Methylpiperazin-1-yl)methyl)-3-(trifluormethyl)phenyl)-1-(pyrazol[1,5-a]pyrimidin-6-ylmethyl)indolin-6-carboxamid,
1-((2,3-dihydro-1H-pyrrol[2,3-b]pyridin-5-yl)methyl)-N-(4-((4-methylpiperazin-1-yl)methyl)-3-(trifluormethyl)phenyl)indolin-6-carboxamid,
1-((1H-Pyrazol[3,4-b]pyridin-5-yl)methyl)-N-(3-(tert-butyl)isoxazol-5-yl)indolin-6-carboxamid,
N-(3-(Tert-butyl)isoxazol-5-yl)-1-(imidazo[1,2-a]pyrazin-3-ylmethyl)indolin-6-carboxamid,
N-(3-(Tert-butyl)isoxazol-5-yl)-1-((3-methyl-1H-pyrazol[3,4-b]pyridin-5-yl)methyl)indolin-6-carboxamid,
1-((3-Methyl-1H-pyrazol[3,4-b]pyridin-5-yl)methyl)-N-(2-((4-methylpiperazin-1-yl)methyl)-6-(trifluormethyl)pyridin-4-yl)indolin-6-carboxamid,
N-(2-((Dimethylamino)methyl)-6-(trifluormethyl)pyridin-4-yl)-1-((3-methyl-1H-pyrazol[3,4-b]pyridin-5-yl)methyl)indolin-6-carboxamid,
1-((3-((Dimethylamino)methyl)-1H-pyrrol[2,3-b]pyridin-5-yl)methyl)-N-(5-(trifluormethyl)pyridin-3-yl)indolin-6-carboxamid,
(R)-N-(2-((3-(Dimethylamino)pyrrolidin-1-yl)methyl)-6-(trifluormethyl)pyridin-4-yl)-1-((3-methyl-1H-pyrazol[3,4-b]pyridin-5-yl)methyl)indolin-6-carboxamid,
1-((3-Methyl-1H-pyrazol[3,4-b]pyridin-5-yl)methyl)-N-(3-(2-(pyrrolidin-1-yl)ethoxy)-5-(trifluormethyl)phenyl)indolin-6-carboxamid,
N-(4-((4-Methylpiperazin-1-yl)methyl)-3-(trifluormethyl)phenyl)-1-(pyrazol[1,5-a]pyrazin-3-ylmethyl)indolin-6-carboxamid,
N-(5-(Tert-butyl)-1-methyl-1H-pyrazol-3-yl)-1-(imidazo[1,2-a]pyrazin-3-ylmethyl)indolin-6-carboxamid,
1-(Imidazof1,2-a]pyrazin-3-ylmethyl)-N-(6-(trifluormethyl)pyrimidin-4-yl)indolin-6-carboxamid,
N-(4-((4-Methylpiperazin-1-yl)methyl)-3-(trifluormethyl)phenyl)-1-((3-morpholino-1H-pyrazol[3,4-b]pyridin-5-yl)methyl)indolin-6-carboxamid,
1-((3-((Dimethylamino)methyl)-1H-pyrrol[2,3-b]pyridin-5-yl)methyl)-N-(3-(trifluormethyl)phenyl)indolin-6-carboxamid,
1-((1H-Pyrazol[3,4-b]pyridin-5-yl)methyl)-3-methyl-N-(5-(trifluormethyl)pyridin-3-yl)indolin-6-carboxamid, und
1-(Imidazo[1,2-a]pyrazin-3-ylmethyl)-3-methyl-N-(5-(trifluormethyl)pyridin-3-yl)indolin-6-carboxamid.

7. Die Verbindung der Formel (I) nach Anspruch 1, wobei Li gleich -NHC(O)-, dargestellt durch die Formel (IB)

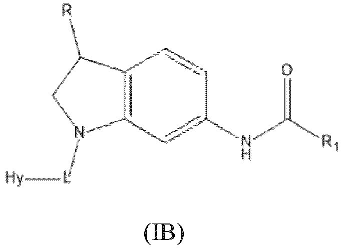

8. Die Verbindung der Formel (IB) nach Anspruch 7, ausgewählt aus mindestens einer der folgenden Verbindungen:
N-(1-(Imidazo[1,2-a]pyridin-3-carbonyl)indolin-6-yl)-3-((4-methylpiperazin-1-yl)methyl)-5-(trifluormethyl)benzamid,
N-(1-(Benzo[d]thiazol-6-ylmethyl)indolin-6-yl)-3-((4-methylpiperazin-1-yl)methyl)-5-(trifluormethyl)benzamid,
N-(1-(Imidazo[1,2-a]pyrazin-3-ylmethyl)indolin-6-yl)-3-((4-methylpiperazin-1-yl)methyl)-5-(trifluormethyl)benzamid,
N-(1-(Imidazo[1,2-a]pyridin-3-carbonyl)indolin-6-yl)-3-(4-methyl-1H-imidazol-1-yl)-5-(trifluormethyl)benzamid,
N-(1-(Imidazo[1,2-a]pyrazin-3-carbonyl)indolin-6-yl)-3-(4-methyl-1H-imidazol-1-yl)-5-(trifluormethyl)benzamid,
N-(1-(1H-Pyrazol[3,4-b]pyridin-5-carbonyl)indolin-6-yl)-3-(4-methyl-1H-imidazol-1-yl)-5-(trifluormethyl)benzamid,
N-(1-(Imidazo[1,2-a]pyrazin-3-ylmethyl)indolin-6-yl)-3-(4-methyl-1H-imidazol-1-yl)-5-(trifluormethyl)benzamid,
N-(1-(Imidazo[1,2-a]pyridin-3-ylmethyl)indolin-6-yl)-3-(4-methyl-1H-imidazol-1-yl)-5-(trifluormethyl)benzamid,
N-(1-((1H-Pyrazol[3,4-b]pyridin-5-yl)methyl)indolin-6-yl)-3-(trifluormethyl)benzamid, und
N-(1-((1H-Pyrazol[3,4-b]pyridin-5-yl)methyl)indolin-6-yl)-3-(4-methyl-1H-imidazol-1-yl)-5-(trifluormethyl)benzamid.

9. Pharmazeutische Zusammensetzung, die eine Verbindung der Formel (I) umfasst, nach einem der Ansprüche 1 bis 8, unter Beigabe eines oder mehrerer pharmazeutisch annehmbarer Träger oder Hilfsstoffe.

10. Pharmazeutische Zusammensetzung nach Anspruch 9 zur Verabreichung durch Inhalation.

11. Verbindung der Formel (I) gemäß einem der Ansprüche 1 bis 8 oder pharmazeutische Zusammensetzung gemäß den Ansprüchen 9 und 10 zur Verwendung als Arzneimittel.

12. Verbindung der Formel (I) oder der pharmazeutischen Zusammensetzung zur Verwendung nach Anspruch 11 bei der Prävention und/oder Behandlung einer Erkrankung, Störung oder eines Zustands, die mit der Dysregulation der DDR verbunden ist.

13. Verbindung der Formel (I) oder pharmazeutische Zusammensetzung zur Verwendung gemäß den Ansprüchen 11 und 12 bei der Prävention und/oder Behandlung von Fibrose und/oder Krankheiten, Störungen oder Zuständen, die Fibrose umfassen.

14. Verbindung der Formel (I) oder pharmazeutische Zusammensetzung zur Verwendung gemäß Anspruch 13 bei der Prävention und/oder Behandlung von Fibrose, einschließlich pulmonaler Fibrose, idiopathischer pulmonaler Fibrose (IPF), hepatischer Fibrose, renaler Fibrose, okularer Fibrose, kardialer Fibrose, arterieller Fibrose und systemischer Sklerose.

15. Verbindung der Formel (I) oder pharmazeutische Zusammensetzung zur Verwendung gemäß Anspruch 14 bei der Prävention und/oder Behandlung von idiopathischer pulmonaler Fibrose (IPF).

## Revendications

1. Composé de formule (I) 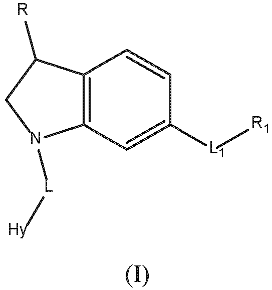 dans lequel
**R** est H ou -(C₁-C₄)alkyle ;
**L** est choisi dans le groupe constitué de -CH₂- et -C(O)- ;
**Li** est choisi dans le groupe constitué de -C(O)NH- et -NHC(O)- ;
**Hy** est un hétéroaryle bicyclique éventuellement substitué par un ou plusieurs groupes choisis parmi -(C₁-C₄)alkyle, hétérocycloalkyle et -(C₁-C₄)alkylène-NR_{A}R_{B}, ou **Hy** est un hétéroaryle bicyclique semi-saturé ;
**R_{A}** est H ou -(C₁-C₄)alkyle ;
**R_{B}** est H ou -(C₁-C₄)alkyle ;
**R₁** est choisi dans le groupe constitué de :
- **Het,** qui est un hétéroaryle éventuellement substitué par un ou plusieurs groupes choisis parmi -(C₁-C₄)alkyle, -(C₁-C₄)haloalkyle, -(C₁-C₄)alkylène-hétérocycloalkyle-NR_{A}R_{B}, -(C₁-C₄)alkylène-NR_{A}R_{B} et aryle, dans lequel ledit aryle est éventuellement substitué par un ou plusieurs -(C₁-C₄)alkyle, et
- **X** 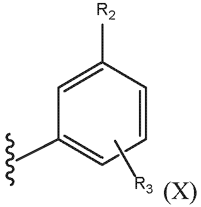
dans lequel **R₂** est choisi dans le groupe constitué de -O(C₁-C₄)haloalkyle, d'atomes d'halogène et de -(C₁-C₄)haloalkyle ;
**R₃** est H ou est choisi dans le groupe constitué de -O(C₁-C₄)alkyle, -C(O)NH-(C₁-C₄)alkylène-NR_{A}R_{B}, -C(O)-hétérocycloalkyle, dans lequel ledit hétérocycloalkyle est éventuellement substitué par un ou plusieurs -(C₁-C₄)alkyle, -O-hétérocycloalkyle, dans lequel ledit hétérocycloalkyle est éventuellement substitué par un ou plusieurs -(C₁-C₄)alkyle, -O(C₁-C₄)alkylène-hétérocycloalkyle, -(C₁-C₄)alkylène-NR_{A}R_{B}, hétéroaryle éventuellement substitué par un ou plusieurs -(C₁-C₄)alkyles, et hétérocycloalkyle-(C₁-C₄)alkylène- monocyclique, dans lequel ledit hétérocycloalkyle est éventuellement substitué par un ou plusieurs groupes choisis parmi -(C₁-C₄)alkyle et -NR_{A}R_{B} ;
et ses sels acceptables du point de vue pharmaceutique.

2. Composé de formule (I) selon la revendication 1, dans lequel Li est -C(O)NH-, représenté par la formule (IA)

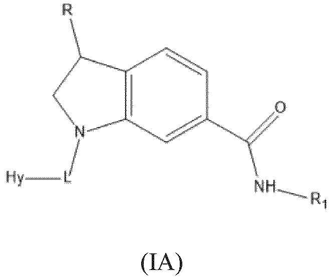

3. Composé de formule (IA) selon la revendication 2, dans lequel L est -C(O)-, représenté par la formule (IA')

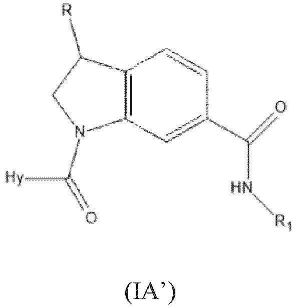

4. Composé de formule (IA') selon la revendication 3, choisi parmi au moins l'un des composés suivants :
1-(imidazo[1,2-a]pyridine-3-carbonyle)-N-(3-(4-méthyle-1H-imidazole-1-yl)-5-(trifluorométhyle)phényl)indoline-6-carboxamide ;
1-(imidazo[1,2-a]pyrazine-3-carbonyle)-N-(3-(4-méthyle-1H-imidazole-1-yl)-5-(trifluorométhyle)phényl)indoline-6-carboxamide ;
1-(imidazo[1,2-a]pyridine-3 -carbonyle)-N-(3-((4-méthylpipérazine-1-yl)méthyle)-5-(trifluorométhyle)phényl)indoline-6-carboxamide ;
1-(imidazo[1,2-a]pyrazine-3-carbonyle)-N-(3-((4-méthylpipérazine-1-yl)méthyle)-5-(trifluorométhyle)phényl)indoline-6-carboxamide ;
3-méthyle-N-(3-(4-méthyle-1H-imidazole-1-yl)-5-(trifluorométhyle)phényl)-1-(1H-pyrazolo[3,4-b]pyridine-5-carbonyle)indoline-6-carboxamide ; et
1-(imidazo[1,2-a]pyrazine-3-carbonyle)-3-méthyle-N-(3-(4-méthyle-1H-imidazole-1-yl)-5-(trifluorométhyle)phényl)indoline-6-carboxamide.

5. Composé de formule (IA) selon la revendication 2, dans lequel L est -CH₂-, représenté par la formule (IA")

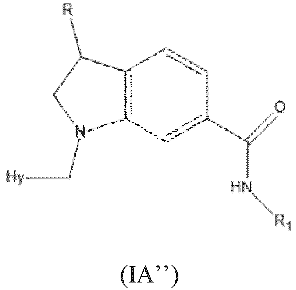

6. Composé de formule (IA") selon la revendication 5, choisi parmi au moins l'un des composés suivants :
1-(imidazo[1,2-a]pyridine-3-ylméthyle)-N-(3-(4-méthyle-1H-imidazole-1-yl)-5-(trifluorométhyle)phényl)indoline-6-carboxamide ;
1-((1H-pyrrolo[2,3-b]pyridine-5-yl)méthyle)-N-(3-(4-méthyle-1H-imidazole-1-yl)-5-(trifluorométhyle)phényl)indoline-6-carboxamide ;
1-(imidazo[1,2-a]pyrazine-3-ylméthyle)-N-(3-(4-méthyle-1H-imidazole-1-yl)-5-(trifluorométhyle)phényl)indoline-6-carboxamide ;
1-((1H-benzo[d]imidazole-5-yl)méthyle)-N-(3-(4-méthyle-1H-imidazole-1-yl)-5-(trifluorométhyle)phényl)indoline-6-carboxamide ;
1-(isoquinoléine-7-ylméthyle)-N-(3-(4-méthyle-1H-imidazole-1-yl)-5-(trifluorométhyle)phényl)indoline-6-carboxamide ;
1-((1H-pyrazolo[3,4-b]pyridine-5-yl)méthyle)-N-(3-(4-méthyle-1H-imidazole-1-yl)-5-(trifluorométhyle)phényl)indoline-6-carboxamide ;
N-(3-(4-méthyle-1H-imidazole-1-yl)-5-(trifluorométhyle)phényl)-1-((3-méthyle-1H-pyrazolo[3,4-b]pyridine-5-yl)méthyle)indoline-6-carboxamide ;
1-((1H-indazole-5-yl)méthyle)-N-(3-(4-méthyle-1H-imidazole-1-yl)-5-(trifluorométhyle)phényl)indoline-6-carboxamide ;
1-((1H-pyrrolo[2,3-b]pyridine-5-yl)méthyle)-N-(3-((4-méthylpipérazine-1-yl)méthyle)-5-(trifluorométhyle)phényl)indoline-6-carboxamide ;
1-(imidazo[1,2-a]pyrazine-3 -ylméthyle)-N-(3 -((4-méthylpipérazine-1-yl)méthyle)-5-(trifluorométhyle)phényl)indoline-6-carboxamide ;
1-(imidazo[1,2-a]pyridine-3 -ylméthyle)-N-(3-((4-méthylpipérazine-1-yl)méthyle)-5-(trifluorométhyle)phényl)indoline-6-carboxamide ;
1-((1H-pyrazolo[3,4-b]pyridine-5-yl)méthyle)-3-méthyle-N-(3-(4-méthyle-1H-imidazole-1-yl)-5-(trifluorométhyle)phényl)indoline-6-carboxamide ;
1-(imidazo[1,2-a]pyridine-3-ylméthyle)-3-méthyle-N-(3-(4-méthyle-1H-imidazole-1-yl)-5-(trifluorométhyle)phényl)indoline-6-carboxamide ;
1-(imidazo[1,2-a]pyrazine-3-ylméthyle)-3-méthyle-N-(3-(4-méthyle-1H-imidazole-1-yl)-5-(trifluorométhyle)phényl)indoline-6-carboxamide ;
1-(imidazo[1,2-a]pyrazine-3-ylméthyle)-3-méthyle-N-(3-(4-méthyle-1H-imidazole-1-yl)-5-(trifluorométhyle)phényl)indoline-6-carboxamide ;
1-((1H-pyrazolo[3,4-b]pyridine-5-yl)méthyle)-3-méthyle-N-(3-(trifluorométhyle)phényl)indoline-6-carboxamide ;
1-(imidazo[1,2-a]pyrazine-3-ylméthyle)-3-méthyle-N-(3-(trifluorométhyle)phényl)indoline-6-carboxamide ;
1-((1H-pyrrolo[2,3-b]pyridine-5-yl)méthyle)-3-méthyle-N-(3-(trifluorométhyle)phényl)indoline-6-carboxamide ;
1-(imidazo[1,2-a]pyridine-3-ylméthyle)-3-méthyle-N-(3-(trifluorométhyle)phényl)indoline-6-carboxamide ;
1-((1H-pyrazolo[3,4-b]pyridine-5-yl)méthyle)-3-méthyle-N-(3-(trifluorométhyle)phényl)indoline-6-carboxamide ;
1-((1H-pyrazolo[3,4-b]pyridine-5-yl)méthyle)-N-(5-(1,1,1-trifluoro-2-méthylpropane-2-yl)isoxazole-3 -yl)indoline-6-carboxamide ;
1-((1H-pyrazolo[3,4-b]pyridine-5-yl)méthyle)-N-(3-(trifluorométhoxy)phényl)indoline-6-carboxamide ;
1-((1H-pyrazolo[3,4-b]pyridine-5-yl)méthyle)-N-(3-fluoro-5-(trifluorométhyle)phényl)indoline-6-carboxamide ;
1-((1H-pyrazolo[3,4-b]pyridine-5-yl)méthyle)-N-(3-(tert-butyle)-1-phényl-1H-pyrazole-5-yl)indoline-6-carboxamide ;
N-(3-fluoro-5-(trifluorométhyle)phényl)-1-(imidazo[1,2-a]pyrazine-3-ylméthyle)indoline-6-carboxamide ;
1-(imidazo[1,2-a]pyrazine-3-ylméthyle)-N-(3 - (trifluorométhyle)phényl)indoline-6-carboxamide ;
1-(imidazo[1,2-a]pyrazine-3-ylméthyle)-N-(3 - (trifluorométhoxy)phényl)indoline-6-carboxamide ;
N-(3-fluoro-5-(trifluorométhoxy)phényl)-1-(imidazo[1,2-a]pyrazine-3-ylméthyle)indoline-6-carboxamide ;
1-(imidazo[1,2-a]pyrazine-3-ylméthyle)-N-(5-(1,1,1-trifluoro-2-méthylpropane-2-yl)isoxazole-3 -yl)indoline-6-carboxamide ;
1-(imidazo[1,2-a]pyrazine-3-ylméthyle)-N-(4-méthoxy-3-(trifluorométhyle)phényl)indoline-6-carboxamide ;
1-(imidazo[1,2-a]pyrazine-3-ylméthyle)-N-(4-((tétrahydrofurane-3-yl)oxy)-3-(trifluorométhyle)phényl)indoline-6-carboxamide ;
N-(4-((diméthylamino)méthyle)-3-(trifluorométhyle)phényl)-1-(imidazo[1,2-a]pyrazine-3-ylméthyle)indoline-6-carboxamide ;
1-(imidazo[1,2-a]pyrazine-3-ylméthyle)-N-(3-(morpholinométhyle)-5-(trifluorométhyle)phényl)indoline-6-carboxamide ;
1-(imidazo[1,2-a]pyrazine-3 -ylméthyle)-N-(3-(pyrrolidine-1-ylméthyle)-5-(trifluorométhyle)phényl)indoline-6-carboxamide ;
N-(3-((diméthylamino)méthyle)-5-(trifluorométhyle)phényl)-1-(imidazo[1,2-a]pyrazine-3-ylméthyle)indoline-6-carboxamide ;
1-(imidazo[1,2-a]pyrazine-3-ylméthyle)-N-(3-(2-(pyrrolidine-1-yl)éthoxy)-5-(trifluorométhyle)phényl)indoline-6-carboxamide ;
1-(imidazo[1,2-a]pyrazine-3-ylméthyle)-N-(3-(2-morpholinoéthoxy)-5-(trifluorométhyle)phényl)indoline-6-carboxamide ;
1-(imidazo[1,2-a]pyrazine-3-ylméthyle)-N-(4-((1-méthylpipéridine-4-yl)oxy)-3-(trifluorométhyle)phényl)indoline-6-carboxamide ;
1-(imidazo[1,2-a]pyridine-3-ylméthyle)-N-(3-(trifluorométhoxy)phényl)indoline-6-carboxamide ;
N-(3-fluoro-5-(trifluorométhoxy)phényl)-1-(imidazo[1,2-a]pyridine-3-ylméthyle)indoline-6-carboxamide ;
1-(imidazo[1,2-a]pyridine-3-ylméthyle)-N-(3-(trifluorométhyle)phényl)indoline-6-carboxamide ;
1-(imidazo[1,2-a]pyridine-3-ylméthyle)-N-(5-(1,1,1-trifluoro-2-méthylpropane-2-yl)isoxazole-3 -yl)indoline-6-carboxamide ;
N-(3-fluoro-5-(trifluorométhyle)phényl)-1-(imidazo[1,2-a]pyridine-3-ylméthyle)indoline-6-carboxamide ;
1-((1H-benzo[d]imidazol-5-yl)méthyle)-N-(3-(trifluorométhyle)phényl)indoline-6-carboxamide ;
1-((1H-benzo[d]imidazole-5-yl)méthyle)-N-(3-(trifluorométhoxy)phényl)indoline-6-carboxamide ;
1-((1H-benzo[d]imidazole-5-yl)méthyle)-N-(3-fluoro-5-(trifluorométhyle)phényl)indoline-6-carboxamide ;
1-(pyrazolo[1,5-a]pyrimidine-6-ylméthyle)-N-(3-(trifluorométhyle)phényl)indoline-6-carboxamide ;
3-éthyle-1-(imidazo[1,2-a]pyrazine-3-ylméthyle)-N-(3-(4-méthyle-1H-imidazole-1-yl)-5-(trifluorométhyle)phényl)indoline-6-carboxamide ;
1-((3-méthyle-1H-pyrazolo[3,4-b]pyridine-5-yl)méthyle)-N-(4-((4-méthylpipérazine-1-yl)méthyle)-3-(trifluorométhyle)phényl)indoline-6-carboxamide ;
1-((3-méthyle-1H-pyrazolo[3,4-b]pyridine-5-yl)méthyle)-N-(3-(pyrrolidine-1-ylméthyle)-5-(trifluorométhyle)phényl)indoline-6-carboxamide ;
N-(5-(tert-butyle)isoxazole-3-yl)-1-((3-méthyle-1H-pyrazolo[3,4-b]pyridine-5-yl)méthyle)indoline-6-carboxamide ;
1-((3-méthyle-1H-pyrazolo[3,4-b]pyridine-5-yl)méthyle)-N-(3-(((4-méthylpipérazine-1-yl)méthyle)-5-(trifluorométhyle)phényl)indoline-6-carboxamide ;
N-(4-((diméthylamino)méthyle)-3-(trifluorométhyle)phényl)-1-((3-méthyle-1H-pyrazolo[3,4-b]pyridine-5-yl)méthyle)indoline-6-carboxamide ;
1-((3-méthyle-1H-pyrazolo[3,4-b]pyridine-5-yl)méthyle)-N-(4-(pyrrolidine-1-ylméthyle)-3-(trifluorométhyle)phényl)indoline-6-carboxamide ;
1-((3-méthyle-1H-pyrazolo[3,4-b]pyridine-5-yl)méthyle)-N-(5-(trifluorométhyle)pyridine-3-yl)indoline-6-carboxamide ;
(R)-N-(3-((3-(diméthylamino)pyrrolidine-1-yl)méthyle)-5-(trifluorométhyle)phényl)-1-((3-méthyle-1H-pyrazolo[3,4-b]pyridine-5-yl)méthyle)indoline-6-carboxamide ;
N-(3-((2-(diméthylamino)éthyle)carbamoyle)-5-(trifluorométhyle)phényl)-1-((3-méthyle-1H-pyrazolo[3,4-b]pyridine-5-yl)méthyle)indoline-6-carboxamide ;
1-((3-méthyle-1H-pyrazolo[3,4-b]pyridine-5-yl)méthyle)-N-(3-(4-méthylpipérazine-1-carbonyle)-5-(trifluorométhyle)phényl)indoline-6-carboxamide ;
1-((1H-pyrazolo[3,4-b]pyridine-5-yl)méthyle)-N-(4-((4-méthylpipérazine-1-yl)méthyle)-3-(trifluorométhyle)phényl)indoline-6-carboxamide;
N-(4-((4-méthylpipérazine-1-yl)méthyle)-3-(trifluorométhyle)phényl)-1-(pyrazolo[1,5-a]pyrimidine-6-ylméthyle)indoline-6-carboxamide ;
1-((2,3-dihydro-1H-pyrrolo[2,3-b]pyridine-5-yl)méthyle)-N-(4-((4-méthylpipérazine-1-yl)méthyle)-3-(trifluorométhyle)phényl)indoline-6-carboxamide ;
1-((1H-pyrazolo[3,4-b]pyridine-5-yl)méthyle)-N-(3-(tert-butyle)isoxazole-5-yl)indoline-6-carboxamide ;
N-(3-(tert-butyle)isoxazole-5-yl)-1-(imidazo[1,2-a]pyrazine-3-ylméthyle)indoline-6-carboxamide ;
N-(3-(tert-butyle)isoxazole-5-yl)-1-((3-méthyle-1H-pyrazolo[3,4-b]pyridine-5-yl)méthyle)indoline-6-carboxamide ;
1-((3-méthyle-1H-pyrazolo[3,4-b]pyridine-5-yl)méthyle)-N-(2-(((4-méthylpipérazine-1-yl)méthyle)-6-(trifluorométhyle)pyridine-4-yl)indoline-6-carboxamide ;
N-(2-((diméthylamino)méthyle)-6-(trifluorométhyle)pyridine-4-yl)-1-((3-méthyle-1H-pyrazolo[3,4-b]pyridine-5-yl)méthyle)indoline-6-carboxamide ;
1-((3-((diméthylamino)méthyle)-1H-pyrrolo[2,3-b]pyridine-5-yl)méthyle)-N-(5-(trifluorométhyle)pyridin-3-yl)indoline-6-carboxamide ;
(R)-N-(2-((3-(diméthylamino)pyrrolidine-1-yl)méthyle)-6-(trifluorométhyle)pyridine-4-yl)-1-((3-méthyle-1H-pyrazolo[3,4-b]pyridine-5-yl)méthyle)indoline-6-carboxamide ;
1-((3-méthyle-1H-pyrazolo[3,4-b]pyridine-5-yl)méthyle)-N-(3-(2-(pyrrolidine-1-yl)éthoxy)-5-(trifluorométhyle)phényl)indoline-6-carboxamide ;
N-(4-((4-méthylpipérazine-1-yl)méthyle)-3-(trifluorométhyle)phényl)-1-(pyrazolo[1,5-a]pyrazine-3-ylméthyle)indoline-6-carboxamide ;
N-(5-(tert-butyle)-1-méthyle-1H-pyrazole-3-yl)-1-(imidazo[1,2-a]pyrazine-3-ylméthyle)indoline-6-carboxamide ;
1-(imidazo[1,2-a]pyrazine-3-ylméthyle)-N-(6-(trifluorométhyle)pyrimidine-4-yl)indoline-6-carboxamide ;
N-(4-((4-méthylpipérazine-1-yl)méthyle)-3-(trifluorométhyle)phényl)-1-((3-morpholino-1H-pyrazolo[3,4-b]pyridine-5-yl)méthyle)indoline-6-carboxamide ;
1-((3-((diméthylamino)méthyle)-1H-pyrrolo[2,3-b]pyridine-5-yl)méthyle)-N-(3-(trifluorométhyle)phényl)indoline-6-carboxamide ;
1-((1H-pyrazolo[3,4-b]pyridine-5-yl)méthyle)-3-méthyle-N-(5-(trifluorométhyle)pyridine-3-yl)indoline-6-carboxamide ; et
1-(imidazo[1,2-a]pyrazine-3-ylméthyle)-3-méthyle-N-(5-(trifluorométhyle)pyridine-3-yl)indoline-6-carboxamide.

7. Composé de formule (I) selon la revendication 1, dans lequel Li est -NHC(O)-, représenté par la formule (IB)

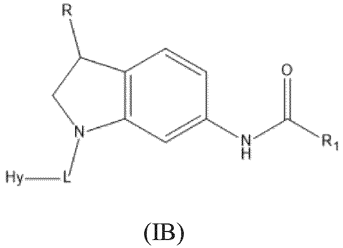

8. Composé de formule (IB) selon la revendication 7, choisi parmi au moins l'un des composés suivants :
N-(1-(imidazo[1,2-a]pyridine-3-carbonyle)indoline-6-yl)-3-((4-méthylpipérazine-1-yl)méthyle)-5-(trifluorométhyle)benzamide ;
N-(1-(benzo[d]thiazole-6-ylméthyle)indoline-6-yl)-3-((4-méthylpipérazine-1-yl)méthyle)-5-(trifluorométhyle)benzamide ;
N-(1-(imidazo[1,2-a]pyrazine-3-ylméthyle)indoline-6-yl)-3-((4-méthylpipérazine-1-yl)méthyle)-5-(trifluorométhyle)benzamide ;
N-(1-(imidazo[1,2-a]pyridine-3-carbonyle)indoline-6-yl)-3-(4-méthyle-1H-imidazole-1-yl)-5-(trifluorométhyle)benzamide ;
N-(1-(imidazo[1,2-a]pyrazine-3-carbonyle)indoline-6-yl)-3-(4-méthyle-1H-imidazole-1-yl)-5-(trifluorométhyle)benzamide ;
N-(1-(1H-pyrazolo[3,4-b]pyridine-5-carbonyle)indoline-6-yl)-3-(4-méthyle-1H-imidazole-1-yl)-5-(trifluorométhyle)benzamide ;
N-(1-(imidazo[1,2-a]pyrazine-3-ylméthyle)indoline-6-yl)-3-(4-méthyle-1H-imidazole-1-yl)-5-(trifluorométhyle)benzamide ;
N-(1-(imidazo[1,2-a]pyridine-3-ylméthyle)indoline-6-yl)-3-(4-méthyle-1H-imidazole-1-yl)-5-(trifluorométhyle)benzamide ;
N-(1-((1H-pyrazolo[3,4-b]pyridine-5-yl)méthyle)indoline-6-yl)-3-(trifluorométhyle)benzamide ; et
N-(1-((1H-pyrazolo[3,4-b]pyridine-5-yl)méthyle)indoline-6-yl)-3-(4-méthyle-1H-imidazole-1-yl)-5-(trifluorométhyle)benzamide.

9. Composition pharmaceutique comprenant un composé de formule (I) selon l'une des revendications 1 à 8, en mélange avec un ou plusieurs transporteurs ou excipients acceptables du point de vue pharmaceutique.

10. Composition pharmaceutique selon la revendication 9 pour administration par inhalation.

11. Composé de formule (I) selon l'une des revendications 1 à 8, ou composition pharmaceutique selon la revendication 9 ou 10 pour une utilisation comme médicament.

12. Composé de formule (I) ou composition pharmaceutique à utiliser selon la revendication 11 dans la prévention et/ou le traitement d'une maladie, d'un trouble ou d'une affection associés à un dérèglement dans la réponse aux dommages à l'ADN.

13. Composé de formule (I) ou composition pharmaceutique pour une utilisation selon la revendication 11 ou 12 dans la prévention et/ou le traitement de la fibrose et/ou de maladies, troubles ou affections qui impliquent la fibrose.

14. Composé de formule (I) ou composition pharmaceutique pour une utilisation selon la revendication 13 dans la prévention et/ou le traitement de la fibrose, y compris la fibrose pulmonaire, la fibrose pulmonaire idiopathique (FPI), la fibrose hépatique, la fibrose rénale, la fibrose oculaire, la fibrose cardiaque, la fibrose artérielle et la sclérose systémique.

15. Composé de formule (I) ou composition pharmaceutique pour une utilisation selon la revendication 14 dans la prévention et/ou le traitement de la fibrose pulmonaire idiopathique (FPI).
